# EUROPEAN PATENT APPLICATION

(11) **EP 3 001 990 A1**
(43) Date of publication of application: **06.04.2016**
(21) Application number: 15169877.6
(22) Date of filing: 25.05.2005
(51) Int. Cl.: A61F 13/00, A61K 9/70, A61K 9/00, A61K 47/36, A61K 47/44, A61K 8/00, A61K 31/00, A61L 26/00, A61K 31/616

(54) **A MIXTURE FOR TRANSDERMAL DELIVERY OF LOW& AND HIGH MOLECULAR WEIGHT COMPOUNDS**

(30) Priority: 28.05.2004 US 856567; 28.05.2004 WO PCT/US2004/017169
(62) Divisional of application: 05826138.9
(71) Applicant: ORYXE, Irvine, California 92618 (US)
(72) Inventor: JORDAN, Frederick, Santa Ana, CA California 92705 (US); DOLBEAR, Geoff E., Diamond Bar, CA California 91765 (US)
(74) Representative: Plougmann Vingtoft a/s

(57) **Abstract**

Aspects of the present invention concern the discovery of a transdermal delivery composition that can deliver low, medium and high molecular weight pharmaceuticals and cosmetic agents. Embodiments include transdermal delivery compositions with therapeutic and cosmetic application, transdermal delivery devices for providing said transdermal delivery compositions to subjects in need thereof, and methods of making and using of the foregoing.

SEQUENCE LISTING

<110> Jordan, Fred
      Dolbear, Geoff E.

<120> A MIXTURE FOR TRANSDERMAL DELIVERY OF
  LOW AND HIGH MOLECULAR WEIGHT COMPOUNDS

<130> ORYXE.066VPC

<150> PCT/US2004/017169
<151> 2004-05-28

<150> 10/856,567
<151> 2004-05-28

<150> 60/510,615
<151> 2003-10-10

<160> 162

<170> FastSEQ for Windows Version 4.0

<210> 1
<211> 5
<212> PRT
<213> Homo sapiens

<400> 1
Leu Lys Glu Lys Lys
 1               5

<210> 2
<211> 9
<212> PRT
<213> Homo sapiens

<400> 2
Cys Tyr Ile Gln Asn Cys Pro Leu Gly
 1               5

<210> 3
<211> 9
<212> PRT
<213> Homo sapiens

<400> 3
Cys Tyr Phe Gln Asn Cys Pro Arg Gly
 1               5

<210> 4
<211> 13
<212> PRT
<213> Homo sapiens

<400> 4
Ser Tyr Ser Met Glu His Phe Arg Trp Gly Lys Pro Val
 1               5                  10

<210> 5
<211> 22
<212> PRT
<213> Homo sapiens

<400> 5
Ala Glu Lys Lys Asp Glu Gly Pro Tyr Arg Met Glu His Phe Arg Trp
1               5                   10                  15
Gly Ser Pro Pro Lys Asp
            20

<210> 6
<211> 12
<212> PRT
<213> Homo sapiens

<400> 6
Tyr Val Met Gly His Phe Arg Trp Asp Arg Phe Gly
1               5                   10

<210> 7
<211> 38
<212> PRT
<213> Homo sapiens

<400> 7
Ser Tyr Ser Met Glu His Phe Arg Trp Gly Lys Pro Val Gly Lys Lys
1               5                   10                  15
Arg Arg Pro Val Lys Val Tyr Pro Asn Gly Ala Glu Asp Glu Ser Ala
            20                  25                  30
Gln Ala Phe Pro Leu Glu
        35

<210> 8
<211> 58
<212> PRT
<213> Homo sapiens

<400> 8
Glu Leu Thr Gly Glu Arg Leu Glu Gln Ala Arg Gly Pro Glu Ala Gln
1               5                   10                  15
Ala Glu Ser Ala Ala Ala Arg Ala Glu Leu Glu Tyr Gly Leu Val Ala
            20                  25                  30
Glu Ala Glu Ala Ala Glu Lys Lys Asp Ser Gly Pro Tyr Lys Met Glu
        35                  40                  45
His Phe Arg Trp Gly Ser Pro Pro Lys Asp
    50                  55

<210> 9
<211> 18
<212> PRT
<213> Homo sapiens

<400> 9
Asp Ser Gly Pro Tyr Lys Met Glu His Phe Arg Trp Gly Ser Pro Pro
1               5                   10                  15
Lys Asp

<210> 10
<211> 138
<212> PRT
<213> Homo sapiens

<400> 10
Met Thr Ala Leu Phe Leu Met Ser Met Leu Phe Gly Leu Ala Cys Gly
1               5                   10                  15
Gln Ala Met Ser Phe Cys Ile Pro Thr Glu Tyr Thr Met His Ile Glu
            20                  25                  30
Arg Arg Glu Cys Ala Tyr Cys Leu Thr Ile Asn Thr Thr Ile Cys Ala
        35                  40                  45
Gly Tyr Cys Met Thr Arg Asp Ile Asn Gly Lys Leu Phe Leu Pro Lys
    50                  55                  60
Tyr Ala Leu Ser Gln Asp Val Cys Thr Tyr Arg Asp Phe Ile Tyr Arg
65                  70                  75                  80
Thr Val Glu Ile Pro Gly Cys Pro Leu His Val Ala Pro Tyr Phe Ser
                85                  90                  95
Tyr Pro Val Ala Leu Ser Cys Lys Cys Gly Lys Cys Asn Thr Asp Tyr
            100                 105                 110
Ser Asp Cys Ile His Glu Ala Ile Lys Thr Asn Tyr Cys Thr Lys Pro
        115                 120                 125
Gln Lys Ser Tyr Leu Val Gly Phe Ser Val
    130                 135


<210> 11
<211> 226
<212> PRT
<213> Homo sapiens

<400> 11
Met Asn Ile Lys Gly Ser Pro Trp Lys Gly Ser Leu Leu Leu Leu Leu
1               5                   10                  15
Val Ser Asn Leu Leu Leu Cys Gln Ser Val Ala Pro Leu Pro Ile Cys
            20                  25                  30
Pro Gly Gly Ala Ala Arg Cys Gln Val Thr Leu Arg Asp Leu Phe Asp
        35                  40                  45
Arg Ala Val Val Leu Ser His Tyr Ile His Asn Leu Ser Ser Glu Met
    50                  55                  60
Phe Ser Glu Phe Asp Lys Arg Tyr Thr His Gly Arg Gly Phe Ile Thr
65                  70                  75                  80
Lys Ala Ile Asn Ser Cys His Thr Ser Ser Leu Ala Thr Pro Glu Asp
                85                  90                  95
Lys Glu Gln Ala Gln Gln Met Asn Gln Lys Asp Phe Leu Ser Leu Ile
            100                 105                 110
Val Ser Ile Leu Arg Ser Trp Asn Glu Pro Leu Tyr His Leu Thr Glu
        115                 120                 125
Val Arg Gly Met Gln Glu Ala Pro Glu Ala Ile Leu Ser Lys Ala Val
    130                 135                 140
Glu Ile Glu Glu Gln Thr Lys Arg Leu Leu Glu Gly Met Glu Leu Ile
145                 150                 155                 160
Val Ser Gln Val His Pro Glu Thr Lys Glu Asn Glu Ile Tyr Pro Val
                165                 170                 175
Trp Ser Gly Leu Pro Ser Leu Gln Met Ala Asp Glu Glu Ser Arg Leu
            180                 185                 190
Ser Ala Tyr Tyr Asn Leu Leu His Cys Leu Arg Arg Asp Ser His Lys
        195                 200                 205
Ile Asp Tyr Asn Leu Lys Leu Leu Lys Cys Arg Ile Ile His Asn Asn
    210                 215                 220

Asn Cys
225

<210> 12
<211> 48
<212> PRT
<213> Homo sapiens

<400> 12
Ser Pro Pro Arg Ile Thr Val Cys Cys Val Thr Cys Tyr Thr Thr Leu
1               5                   10                  15
Val Pro Val Tyr Asp Phe Ile Pro Cys Gly Pro Ala Leu Gln Ala Arg
            20                  25                  30
Thr Cys Gly Asp Leu Pro Gln Ser Lys Pro Leu Arg Gly Asp Pro Pro
        35                  40                  45

<210> 13
<211> 41
<212> PRT
<213> Homo sapiens

<400> 13
Ser Glu Glu Pro Pro Ile Ser Leu Asp Leu Thr Phe His Leu Leu Arg
1               5                   10                  15
Glu Val Leu Glu Met Ala Arg Ala Glu Gln Leu Ala Gln Gln Ala His
            20                  25                  30
Ser Asn Arg Lys Leu Met Glu Ile Ile
        35                  40

<210> 14
<211> 31
<212> PRT
<213> Homo sapiens

<400> 14
Ser Arg Thr His Arg His Ser Met Glu Ile Arg Thr Pro Asp Ile Asn
1               5                   10                  15
Pro Ala Trp Tyr Ala Ser Arg Gly Ile Arg Pro Val Gly Arg Phe
            20                  25                  30

<210> 15
<211> 92
<212> PRT
<213> Homo sapiens

<400> 15

<210> 16
<211> 44
<212> PRT
<213> Homo sapiens

<400> 16
Tyr Ala Asp Ala Ile Phe Thr Asn Ser Tyr Arg Lys Val Leu Gly Gln
1               5                   10                  15
Leu Ser Ala Arg Lys Leu Leu Gln Asp Ile Met Ser Arg Gln Gln Gly
            20                  25                  30
Glu Ser Asn Gln Glu Arg Gly Ala Arg Ala Arg Leu
        35                  40


<210> 17
<211> 28
<212> PRT
<213> Homo sapiens

<400> 17
Ser Ala Asn Ser Asn Pro Ala Met Ala Pro Arg Glu Arg Lys Ala Gly
1               5                   10                  15
Cys Lys Asn Phe Phe Trp Lys Thr Phe Thr Ser Cys
            20                  25


<210> 18
<211> 3
<212> PRT
<213> Homo sapiens

<400> 18
Glu His Pro
1


<210> 19
<211> 32
<212> PRT
<213> Homo sapiens

<400> 19
Cys Gly Asn Leu Ser Thr Cys Met Leu Gly Thr Tyr Thr Gln Asp Phe
1               5                   10                  15
Asn Lys Phe His Thr Phe Pro Gln Thr Ala Ile Gly Val Gly Ala Pro
            20                  25                  30


<210> 20
<211> 37
<212> PRT
<213> Homo sapiens

<400> 20
Ala Cys Asp Thr Ala Thr Cys Val Thr His Arg Leu Ala Gly Leu Leu
1               5                   10                  15
Ser Arg Ser Gly Gly Val Val Lys Asn Asn Phe Val Pro Thr Asn Val
            20                  25                  30
Gly Ser Lys Ala Phe
        35

<210> 21
<211> 34
<212> PRT
<213> Homo sapiens

<400> 21
Ser Val Ser Glu Ile Gln Leu Met His Asn Leu Gly Lys His Leu Asn
1               5                   10                  15
Ser Met Glu Arg Val Glu Trp Leu Arg Lys Lys Leu Gln Asp Val His
            20                  25                  30
Asn Phe

<210> 22
<211> 30
<212> PRT
<213> Homo sapiens

<400> 22
His Ala Glu Gly Thr Phe Thr Ser Asp Val Ser Ser Tyr Leu Glu Gly
1               5                   10                  15
Gln Ala Ala Lys Glu Phe Ile Ala Trp Leu Val Lys Gly Arg
            20                  25                  30

<210> 23
<211> 153
<212> PRT
<213> Homo sapiens

<400> 23
   Met Val Ala Thr Lys Thr Phe Ala Leu Leu Leu Leu Ser Leu Phe Leu
   1               5                   10                  15
   Ala Val Gly Leu Gly Glu Lys Lys Glu Gly His Phe Ser Ala Leu Pro
               20                  25                  30
   Ser Leu Pro Val Gly Ser His Ala Lys Val Ser Ser Pro Gln Pro Arg
           35                  40                  45
   Gly Pro Arg Tyr Ala Glu Gly Thr Phe Ile Ser Asp Tyr Ser Ile Ala
       50                  55                  60
   Met Asp Lys Ile His Gln Gln Asp Phe Val Asn Trp Leu Leu Ala Gln
   65                  70                  75                  80
   Lys Gly Lys Lys Asn Asp Trp Lys His Asn Ile Thr Gln Arg Glu Ala
                   85                  90                  95
   Arg Ala Leu Glu Leu Ala Ser Gln Ala Asn Arg Lys Glu Glu Glu Ala
               100                 105                 110
   Val Glu Pro Gln Ser Ser Pro Ala Lys Asn Pro Ser Asp Glu Asp Leu
           115                 120                 125
   Leu Arg Asp Leu Leu Ile Gln Glu Leu Leu Ala Cys Leu Leu Asp Gln
       130                 135                 140
   Thr Asn Leu Cys Arg Leu Arg Ser Arg
   145                 150

<210> 24
<211> 16
<212> PRT
<213> Homo sapiens

<400> 24
Gly Pro Trp Leu Glu Glu Glu Glu Glu Ala Tyr Gly Trp Met Asp Phe
1               5                   10                  15

<210> 25
<211> 27
<212> PRT
<213> Homo sapiens

<400> 25
His Ser Asp Gly Thr Phe Thr Ser Glu Leu Ser Arg Leu Arg Glu Gly
1               5                   10                  15
Ala Arg Leu Gln Arg Leu Leu Gln Gly Leu Val
            20                  25

<210> 26
<211> 33
<212> PRT
<213> Homo sapiens

<400> 26
Lys Ala Pro Ser Gly Arg Met Ser Ile Val Lys Asn Leu Gln Asn Leu
1               5                   10                  15
Asp Pro Ser His Arg Ile Ser Asp Arg Asp Tyr Met Gly Trp Met Asp
            20                  25                  30
Phe

<210> 27
<211> 22
<212> PRT
<213> Homo sapiens

<400> 27
Phe Val Pro Ile Phe Thr Tyr Gly Glu Leu Gln Arg Met Gln Glu Lys
1               5                   10                  15
Glu Arg Asn Lys Gly Gln
            20

<210> 28
<211> 28
<212> PRT
<213> Homo sapiens

<400> 28
His Ser Asp Ala Val Phe Thr Asp Asn Tyr Thr Arg Leu Arg Lys Gln
1               5                   10                  15
Met Ala Val Lys Lys Tyr Leu Asn Ser Ile Leu Asn
            20                  25

<210> 29
<211> 14
<212> PRT
<213> Homo sapiens

<400> 29
Ala Gly Cys Lys Asn Phe Phe Trp Lys Thr Phe Thr Ser Cys
1               5                   10

<210> 30
<211> 11

<212> PRT
<213> Homo sapiens

<400> 30
Arg Pro Lys Pro Gln Gln Phe Phe Gly Leu Met
1               5                   10


<210> 31
<211> 36
<212> PRT
<213> Homo sapiens

<400> 31
Ala Pro Leu Glu Pro Val Tyr Pro Gly Asp Asn Ala Thr Pro Glu Gln
1               5                   10                  15
Met Ala Gln Tyr Ala Ala Asp Leu Arg Arg Tyr Ile Asn Met Leu Thr
            20                  25                  30
Arg Pro Arg Tyr
        35


<210> 32
<211> 36
<212> PRT
<213> Homo sapiens

<400> 32
Tyr Pro Ile Lys Pro Glu Ala Pro Gly Glu Asp Ala Ser Pro Glu Glu
1               5                   10                  15
Leu Asn Arg Tyr Tyr Ala Ser Leu Arg His Tyr Leu Asn Leu Val Thr
            20                  25                  30
Arg Gln Arg Tyr
        35


<210> 33
<211> 36
<212> PRT
<213> Homo sapiens

<400> 33
Tyr Pro Ser Lys Pro Asp Asn Pro Gly Glu Asp Ala Pro Ala Glu Asp
1               5                   10                  15
Met Ala Arg Tyr Tyr Ser Ala Leu Arg His Tyr Ile Asn Leu Ile Thr
            20                  25                  30
Arg Gln Arg Tyr
        35


<210> 34
<211> 252
<212> PRT
<213> Homo sapiens

<400> 34
Met Arg Ala Pro Leu Leu Pro Pro Ala Pro Val Val Leu Ser Leu Leu
1               5                   10                  15
Ile Leu Gly Ser Gly His Tyr Ala Ala Gly Leu Asp Leu Asn Asp Thr
            20                  25                  30
Tyr Ser Gly Lys Arg Glu Pro Phe Ser Gly Asp His Ser Ala Asp Gly
        35                  40                  45
Phe Glu Val Thr Ser Arg Ser Glu Met Ser Ser Gly Ser Glu Ile Ser

50                  55                  60
Pro Val Ser Glu Met Pro Ser Ser Ser Glu Pro Ser Ser Gly Ala Asp
65                  70                  75                  80
Tyr Asp Tyr Ser Glu Glu Tyr Asp Asn Glu Pro Gln Ile Pro Gly Tyr
                85                  90                  95
Ile Val Asp Asp Ser Val Arg Val Glu Gln Val Val Lys Pro Pro Gln
            100                 105                 110
Asn Lys Thr Glu Ser Glu Asn Thr Ser Asp Lys Pro Lys Arg Lys Lys
        115                 120                 125
Lys Gly Gly Lys Asn Gly Lys Asn Arg Arg Asn Arg Lys Lys Lys Asn
    130                 135                 140
Pro Cys Asn Ala Glu Phe Gln Asn Phe Cys Ile His Gly Glu Cys Lys
145                 150                 155                 160
Tyr Ile Glu His Leu Glu Ala Val Thr Cys Lys Cys Gln Gln Glu Tyr
                165                 170                 175
Phe Gly Glu Arg Cys Gly Glu Lys Ser Met Lys Thr His Ser Met Ile
            180                 185                 190
Asp Ser Ser Leu Ser Lys Ile Ala Leu Ala Ala Ile Ala Ala Phe Met
        195                 200                 205
Ser Ala Val Ile Leu Thr Ala Val Ala Val Ile Thr Val Gln Leu Arg
    210                 215                 220
Arg Gln Tyr Val Arg Lys Tyr Glu Gly Glu Ala Glu Glu Arg Lys Lys
225                 230                 235                 240
Leu Arg Gln Glu Asn Gly Asn Val His Ala Ile Ala
                245                 250


<210> 35
<211> 180
<212> PRT
<213> Homo sapiens

<400> 35
Met Lys Ser Ile Tyr Phe Val Ala Gly Leu Phe Val Met Leu Val Gln
1               5                   10                  15
Gly Ser Trp Gln Gln Asp Thr Glu Glu Lys Ser Arg Ser Leu Arg Ser
            20                  25                  30
Phe Ser Ala Ser Gln Ala Asp Pro Leu Ser Asp Pro Asp Gln Met Asn
        35                  40                  45
Glu Asp Lys Arg His Ser Gln Gly Thr Phe Thr Ser Asp Tyr Ser Lys
    50                  55                  60
Tyr Leu Asp Ser Arg Arg Ala Gln Asp Phe Val Gln Trp Leu Met Asn
65                  70                  75                  80
Thr Lys Arg Asn Arg Asn Asn Ile Ala Lys Arg His Asp Glu Phe Glu
                85                  90                  95
Arg His Ala Glu Gly Thr Phe Thr Ser Asp Val Ser Ser Tyr Leu Glu
            100                 105                 110
Gly Gln Ala Ala Lys Glu Phe Ile Ala Trp Leu Val Lys Gly Arg Gly
        115                 120                 125
Arg Arg Asp Phe Pro Glu Glu Val Ala Ile Val Glu Glu Leu Gly Arg
    130                 135                 140
Arg His Ala Asp Gly Ser Phe Ser Asp Glu Met Asn Thr Ile Leu Asp
145                 150                 155                 160
Asn Leu Ala Ala Arg Asp Phe Ile Asn Trp Leu Ile Gln Thr Lys Ile
                165                 170                 175
Thr Asp Arg Lys
            180


<210> 36
<211> 36
<212> PRT
<213> Homo sapiens

<400> 36
Ala Pro Leu Glu Pro Val Tyr Pro Gly Asp Asn Ala Thr Pro Glu Gln
1               5                   10                  15
Met Ala Gln Tyr Ala Ala Asp Leu Arg Arg Tyr Ile Asn Met Leu Thr
            20                  25                  30
Arg Pro Arg Tyr
        35

<210> 37
<211> 167
<212> PRT
<213> Homo sapiens

<400> 37
Met Ala Ala Gly Ser Arg Thr Ser Leu Leu Leu Ala Phe Ala Leu Leu
1               5                   10                  15
Cys Leu Pro Trp Leu Gln Glu Ala Gly Ala Val Gln Thr Val Pro Leu
            20                  25                  30
Ser Arg Leu Phe Asp His Ala Met Leu Gln Ala His Arg Ala His Gln
        35                  40                  45
Leu Ala Ile Asp Thr Tyr Gln Glu Phe Glu Glu Thr Tyr Ile Pro Lys
    50                  55                  60
Asp Gln Lys Tyr Ser Phe Leu His Asp Ser Gln Thr Ser Phe Cys Phe
65                  70                  75                  80
Ser Asp Ser Ile Pro Thr Pro Ser Asn Met Glu Glu Thr Gln Gln Lys
                85                  90                  95
Ser Asn Leu Glu Leu Leu Arg Ile Ser Leu Leu Leu Ile Glu Ser Trp
            100                 105                 110
Leu Glu Pro Val Arg Phe Leu Arg Ser Met Phe Ala Asn Asn Leu Val
        115                 120                 125
Tyr Asp Thr Ser Asp Ser Asp Asp Tyr His Leu Leu Lys Asp Leu Glu
    130                 135                 140
Glu Gly Ile Gln Thr Leu Met Gly Val Arg Val Ala Pro Gly Val Ala
145                 150                 155                 160
Asn Pro Gly Thr Pro Leu Ala
                165

<210> 38
<211> 185
<212> PRT
<213> Homo sapiens

<400> 38
Met Pro Arg Leu Phe Leu Phe His Leu Leu Glu Phe Cys Leu Leu Leu
1               5                   10                  15
Asn Gln Phe Ser Arg Ala Val Ala Ala Lys Trp Lys Asp Asp Val Ile
            20                  25                  30
Lys Leu Cys Gly Arg Glu Leu Val Arg Ala Gln Ile Ala Ile Cys Gly
        35                  40                  45
Met Ser Thr Trp Ser Lys Arg Ser Leu Ser Gln Glu Asp Ala Pro Gln
    50                  55                  60
Thr Pro Arg Pro Val Ala Glu Ile Val Pro Ser Phe Ile Asn Lys Asp
65                  70                  75                  80
Thr Glu Thr Ile Ile Ile Met Leu Glu Phe Ile Ala Asn Leu Pro Pro
                85                  90                  95
Glu Leu Lys Ala Ala Leu Ser Glu Arg Gln Pro Ser Leu Pro Glu Leu
            100                 105                 110
Gln Gln Tyr Val Pro Ala Leu Lys Asp Ser Asn Leu Ser Phe Glu Glu
        115                 120                 125
Phe Lys Lys Leu Ile Arg Asn Arg Gln Ser Glu Ala Ala Asp Ser Asn

130                 135                 140
Pro Ser Glu Leu Lys Tyr Leu Gly Leu Asp Thr His Ser Gln Lys Lys
145                 150                 155                 160
Arg Arg Pro Tyr Val Ala Leu Phe Glu Lys Cys Cys Leu Ile Gly Cys
                165                 170                 175
Thr Lys Arg Ser Leu Ala Lys Tyr Cys
            180                 185

<210> 39
<211> 366
<212> PRT
<213> Homo sapiens

<400> 39
Met Val Leu His Leu Leu Leu Phe Leu Leu Leu Thr Pro Gln Gly Gly
1               5                   10                  15
His Ser Cys Gln Gly Leu Glu Leu Ala Arg Glu Leu Val Leu Ala Lys
            20                  25                  30
Val Arg Ala Leu Phe Leu Asp Ala Leu Gly Pro Pro Ala Val Thr Arg
        35                  40                  45
Glu Gly Gly Asp Pro Gly Val Arg Arg Leu Pro Arg Arg His Ala Leu
    50                  55                  60
Gly Gly Phe Thr His Arg Gly Ser Glu Pro Glu Glu Glu Glu Asp Val
65                  70                  75                  80
Ser Gln Ala Ile Leu Phe Pro Ala Thr Asp Ala Ser Cys Glu Asp Lys
                85                  90                  95
Ser Ala Ala Arg Gly Leu Ala Gln Glu Ala Glu Glu Gly Leu Phe Arg
            100                 105                 110
Tyr Met Phe Arg Pro Ser Gln His Thr Arg Ser Arg Gln Val Thr Ser
        115                 120                 125
Ala Gln Leu Trp Phe His Thr Gly Leu Asp Arg Gln Gly Thr Ala Ala
    130                 135                 140
Ser Asn Ser Ser Glu Pro Leu Leu Gly Leu Leu Ala Leu Ser Pro Gly
145                 150                 155                 160
Gly Pro Val Ala Val Pro Met Ser Leu Gly His Ala Pro Pro His Trp
                165                 170                 175
Ala Val Leu His Leu Ala Thr Ser Ala Leu Ser Leu Leu Thr His Pro
            180                 185                 190
Val Leu Val Leu Leu Leu Arg Cys Pro Leu Cys Thr Cys Ser Ala Arg
        195                 200                 205
Pro Glu Ala Thr Pro Phe Leu Val Ala His Thr Arg Thr Arg Pro Pro
    210                 215                 220
Ser Gly Gly Glu Arg Ala Arg Arg Ser Thr Pro Leu Met Ser Trp Pro
225                 230                 235                 240
Trp Ser Pro Ser Ala Leu Arg Leu Leu Gln Arg Pro Pro Glu Glu Pro
                245                 250                 255
Ala Ala His Ala Asn Cys His Arg Val Ala Leu Asn Ile Ser Phe Gln
            260                 265                 270
Glu Leu Gly Trp Glu Arg Trp Ile Val Tyr Pro Pro Ser Phe Ile Phe
        275                 280                 285
His Tyr Cys His Gly Gly Cys Gly Leu His Ile Pro Pro Asn Leu Ser
    290                 295                 300
Leu Pro Val Pro Gly Ala Pro Pro Thr Pro Ala Gln Pro Tyr Ser Leu
305                 310                 315                 320
Leu Pro Gly Ala Gln Pro Cys Cys Ala Ala Leu Pro Gly Thr Met Arg
                325                 330                 335
Pro Leu His Val Arg Thr Thr Ser Asp Gly Gly Tyr Ser Phe Lys Tyr
            340                 345                 350
Glu Thr Val Pro Asn Leu Leu Thr Gln His Cys Ala Cys Ile
        355                 360                 365

<210> 40
<211> 130
<212> PRT
<213> Homo sapiens

<400> 40
Ala Arg Gln Ser Glu Asp His Pro His Arg Arg Arg Arg Arg Gly Leu
1               5                   10                  15
Glu Cys Asp Gly Lys Val Asn Ile Cys Cys Lys Lys Gln Phe Phe Val
            20                  25                  30
Ser Phe Lys Asp Ile Gly Trp Asn Asp Trp Ile Ile Ala Pro Ser Gly
        35                  40                  45
Tyr His Ala Asn Tyr Cys Glu Gly Glu Cys Pro Ser His Ile Ala Gly
    50                  55                  60
Thr Ser Gly Ser Ser Leu Ser Phe His Ser Thr Val Ile Asn His Tyr
65                  70                  75                  80
Arg Met Arg Gly His Ser Pro Phe Ala Asn Leu Lys Ser Cys Cys Val
                85                  90                  95
Pro Thr Lys Leu Arg Pro Met Ser Met Leu Tyr Tyr Asp Asp Gly Gln
            100                 105                 110
Asn Ile Ile Lys Lys Asp Ile Gln Asn Met Ile Val Glu Glu Cys Gly
        115                 120                 125
Cys Ser
    130


<210> 41
<211> 31
<212> PRT
<213> Homo sapiens

<400> 41
Tyr Gly Gly Phe Met Thr Ser Glu Lys Ser Gln Thr Pro Leu Val Thr
1               5                   10                  15
Leu Phe Lys Asn Ala Ile Ile Lys Asn Ala Tyr Lys Lys Gly Glu
            20                  25                  30


<210> 42
<211> 8
<212> PRT
<213> Homo sapiens

<400> 42
Asp Arg Val Tyr Val His Pro Phe
1               5


<210> 43
<211> 9
<212> PRT
<213> Homo sapiens

<400> 43
His Trp Ser Tyr Gly Leu Arg Pro Gly
1               5


<210> 44
<211> 9994
<212> DNA
<213> SIV/HIV

<400> 44
tggaagggat ttattacagt gcaagaagac atagaatctt agacatgtac ttagaaaagg 60
aaaaaggcat cataccagat tggcaggatt acacctcagg accaggaatt agatacccaa 120
agacatttgg ctggctatgg aaattagtcc ctgtaaatgt atcagatgag gcacaggagg 180
atgaagagca ttatttaatg catccagctc aaacttccca gtgggatgac ccttggagag 240
aggttctagc atggaagttt gatccaactc tggcctacac ttatgaggca tatgttagat 300
acccagaaga gtttggaagc aagtcaggcc tgtcagagga agaggttaaa agaaggctaa 360
ccgcaagagg ccttcttaac atggctgaca agaaggaaac tcgctgaaac agcagggact 420
ttccacaagg ggatgttacg gggaggtact ggggaggagc cggtcgggaa cgcccacttt 480
cttgatgtat aaatatcact gcatttcgct ctgtattcag tcgctctgcg gagaggctgg 540
caggttgagc cctgggaggt tctctccagc actagcaggt agagcctggg tgttccctgc 600
tagactctca ccagcacttg gccggtgctg ggcagagtga ttccacgctt gcttgcttaa 660
agccctcttc aataaagctg ccattttaga agtaagctag tgtgtgttcc catctctcct 720
agccgccgcc tggtcaactc ggtactcaat aataagaaga ccctggtctg ttaggaccct 780
ttctgctttg ggaaaccgaa gcaggaaaat ccctagcaga ttggcgcccg aacagggacg 840
cgaaagcgaa agtagaacca gaggagctct ctcgacgcag gactcggctt gctgaagcgc 900
gcacggcaag aggcgagggg cggcgactgg tgagtacgcc atttttgact agcggaggct 960
agaaggagag agatgggtgc gagagcgtca atattaagcg ggggacaatt agatagatgg 1020
gaaaaaattc ggttacggcc agggggaaag aaaagatata agttaaaaca tatagtatgg 1080
gcaagcagag agctagaacg attcgcagtt aaccctggcc tgttagaaac agcagaaggc 1140
tgtagacaaa tactgggaca gctacaacca tcccttcaga caggatcaga ggaacttaaa 1200
tcattattta atacaatagc taccctctat tgtgtacatc aaagaataga gataaaagac 1260
accaaggaag ctttagataa gatagaggaa gagcaaaaca aaagtaagaa aaaagcacag 1320
caagcagcag ctgacacagg aaacagcagc agccaagtca gccaaaatta ccctatagtg 1380
cagaacgctc agggacaaat ggtacatcag gccatatcac ctagaacttt aaatgcatgg 1440
gtaaaagtag tagaagaaaa ggcttttaac ccagaagtaa tacccatgtt tgcagcattg 1500
tcagaaggag ccaccccaca agatttaaac accatgctaa acacagtggg gggacatcaa 1560
gcagccatgc aaatattaaa agagactatc aatgaggaag ctgcagaatg ggatagattg 1620
catccagtac atgcagggcc tattgcacca ggccaaatga gagaaccaag gggaagtgac 1680
atagcaggaa ctactagtac ccttcaggaa caaataggat ggatgacaaa taatccacct 1740
atcccagtag gagaaatcta taaaaaatgg ataatcatgg gattaaataa aattgtaagg 1800
atgtatagcc ctaccagtat tctggacata agacaaggac caaaggaacc ctttagagac 1860
tatgtagacc ggttctataa aactctaaga gccgagcaag cttcacagga agtaaaaaat 1920
tggatgacag aaaccttgtt ggtccaaaat tcaaaccccg attgtaagac tattttaaaa 1980
gcattaggac caggagctac actagaagaa atgatgacag catgccaggg agtgggagga 2040
cctggccata aagcaagagt tttggcagaa gcaatgagcc aagtaacaaa tccaacggcc 2100
gtgatgatgc agaaaagcaa ttttaggggc caaagaaaaa ttgttaagtg ttttaattgt 2160
ggcaaagaag ggcacatagc caaaaattgc agggctccta gaaaaaaggg ctgttggaaa 2220
tgtggaaagg aaggacacca aatgaaagat tgtactgaaa gacaggctaa ttttttaggg 2280
aagatctggc cttcctacaa gggaaggcca gggaattttc ctcaaagcag gctagaacca 2340
acagccccac cagaagcgag cttcaggttt ggggaggaga caacaactcc ccctcagaag 2400
caggagacga tagacaagga ggtgtatcct ttaacctccc tcagatcact ctttggcaac 2460
gacccctcgt cacaataaag ataggggggc aactaaaaga agctctatta gatacaggag 2520
cagatgatac agtgttagaa gacatgaatt tgccaggaaa atggaaacca aaaatgatag 2580
ggggaattgg aggatttatc aaagtaaaac agtatgatca gatacccata gaaatctgtg 2640
gacataaaac tataggtaca gtattaatag gacctacacc tgtcaacata attggaagga 2700
atttgttgac tcagcttggt tgcactttaa attttcccat tagtcctatt gaaactgtac 2760
cagtaaaatt aaagccagga atggatggcc caaaagttaa gcaatggcca ttgacagaag 2820
aaaaaataaa agcattaatg gagatatgca cagaaatgga aaaggaaggg aaaatttcaa 2880
aaattgggcc tgaaaatcca tacaatactc cagtgtttgc cataaagaaa aaagacagta 2940
ctaagtggag aaaattagta gatttcagag aacttaataa gaaaactcaa gacttctggg 3000
aggttcaatt aggaatacca catcccgcgg ggttaaaaaa gaaaaagtca gtaacagtac 3060
tggatgtggg tgatgcatac ttctcagttc ccttagatga agattttagg aagtatactg 3120
catttaccat acctagtata aacaatgaga catcaggaat tagatatcag tacaatgtgc 3180
ttccacaggg atggaaaggg tcaccatgtc agatcccagg gagagaatcc cacctggaaa 3240
cagtggagaa gagacaatag gagaggcctt cgaatggcta aacagaacag tagaggagat 3300
aaacagagag gcggtaaacc acctaccaag ggagctaatt ttccaggttt ggcaaaggtc 3360
ttgggaatac tggcatgatg aacaagggat gtcaccaagc tatgtaaaat acagatactt 3420
gtgtttaata caaaaggctt tatttatgca ttgcaagaaa ggctgtagat gtctagggga 3480
aggacatggg gcagggggat ggagaccagg acctcctcct cctccccctc caggactagc 3540
ataaatggaa gaaagacctc cagaaaatga aggaccacaa agggaaccat gggatgaatg 3600
ggtagtggag gttttggaag aactgaaaga agaagcttta aaacattttg atcctcgctt 3660
gctaactgcc cttggtaatc atatctataa tcgtcacgga gacactctag agggagcagg 3720

agaactcatt agaatcctcc aacgagcgct cttcatgcat ttcagaggcg gatgcatcca 3780
ctccagaatc ggccaacctg agggaggaaa tcctctctca gctataccgc cctctagaag 3840
cattctgtag agcaagaaat ggagccagta gatcctagac tagagccctg gaagcatcca 3900
ggaagtaagc ctaaaactgc ttgtaccaat tgctattgta aaaagtgttg ctttcattgc 3960
caagtttgtt tcataacaaa agccttaggc atctcctatg gcaggaagaa gcggagacag 4020
cgacgaagag ctcatcagaa cagtcagact catcaagctt ctctatcaaa gcagtaagta 4080
gtacatgtaa tgcaacctat accaatagta gcaatagtag cattagtagt agcaataata 4140
atagcaatag ttgtgtggtc catagtaatc atagaatata ggaaaatatt aagacaaaga 4200
aaaatagaca ggttaattga tagactaata gaaagagcag aagacagtgg caatgagagt 4260
gaaggagaga tatcagaatt atcagcactt gtggagagag ggcatcttgc tccttgggat 4320
attaatgata tgtagcactg caggacaatt gtgggtcaca gtctattatg gggtacctgt 4380
gtggagagaa gcaaccacca ctctattttg tgcatcagat gctaaagcat atgatacaga 4440
ggtgcataat gtctgggcca cacatgcctg tgtacccaca gaccccagcc cacaagaaat 4500
ggcattggaa aatgtgacag aaaattttga catgtggaaa aataatatgg tagaacagat 4560
gcatgaagat ataatcagct tatgggatca aagcctaaag ccttgtgtaa aattaactcc 4620
actatgtgtt actttaaatt gcactgatgt aaagagaaat gctactagta acactagtag 4680
tagctgggaa aggatggaac caggagaaat aaaaaactgc tctttcaatg tcacctcaaa 4740
tataagagat aagatgcgga aagaatatgc actcttttat aaacttgatg taataccaat 4800
aaataatact agtgataata gtgctaaata tagattgata agttgtaaca cctcagtcct 4860
tacacaagct tgtccaaaaa tatcctttga gccaattcca atacattatt gtaccccggc 4920
tggttttgcg cttctgaagt gtaatgataa ggagttcaat ggaacgggac catgtaaaaa 4980
tgtcagcaca gtacaatgta cacatggaat caagccagta gtatcaactc aactgctgtt 5040
aaatggcagt ctatcagaag gaggggttgt aattagatct caaaatttca caaacaatgc 5100
taaaaccata atagtacagc tgaatgaaac tgtagaaatt aattgtacaa ggcccaacaa 5160
caatacaaga agaagtataa atataggacc agggagagca ttttatgcag cagaacaaat 5220
aataggagat ataagacaag cacattgtaa cattagtaga gcaaaatgga ataacacttt 5280
aaaactgata gttggaaaat tacaagaaca atttgggaag aaaacaataa tctttaatca 5340
atcctcagga ggagaccctg agattgtaac acacagtttt aattgtggag gggaattttt 5400
ctactgtgat tcaacacaac tgtttaacag tacttggacg aatgaaaata acgggtccaa 5460
cactaaaggg aatgacacaa tcatactacc atgcagaata aaacaaattg taaacctgtg 5520
gcaggaagta ggaaaagcaa tgtatgcccc tcccatcaga agaccaatta gatgctcatc 5580
aaatattaca gggctgctac taacaagaga tggtggtcct aataggacga acgagacatt 5640
cagacctgga ggaggagata tgagggacaa ttggagaagt gaattataca aatataaagt 5700
agtaaaaatt gaaccattag gagtagcacc caccaaggca aagagaagag tggtgcaaag 5760
agaaaaaaga gcagtgggaa taggagctct gttccttggg ttcttgggaa cagcaggaag 5820
cactatgggc gcagcgtcac tgacgctgac ggtacaggcc agacaattat tgtctggtat 5880
agtgcaacag cagaacaatt tgctgagagc tattgaagcg caacaacatc tgttgcagct 5940
cacagtctgg ggcatcaagc agctccaggc aagagtcctg gctgtggaaa gatacctaag 6000
ggatcaacag ctcctgggaa tttggggttg ctctggaaaa ctcatttgca ccactgctgt 6060
gccttggaac actagttgga gtaataaatc tctagatgac atttggaaca acatgacttg 6120
gatgcagtgg gaaagagaaa ttgacaatta cacaaacaca atatacacct tacttcagga 6180
atcacaactc caacaagaac agaatgaaaa agaactattg gaattggata aatgggcaag 6240
tttgtggaat tggttcgata taacaagttg gctgtggtat ataaaaatat tcataatgat 6300
agtaggaggc ttgataggtt taagaatagt ttttactgta ctttctatag tgaatagagt 6360
taggaaggga tactcaccat tatcgttcca gacccaccgc ccagctccag ggggacccga 6420
caggcccgaa ggaatcgaag aagaaggtgg agagagagac agagaaagat ccaatcaatt 6480
agtggatgga ttcttagcaa ttatctgggt cgacctgcgg aacctgtgcc tcttcagcta 6540
ccaccgcttg agagacttac tcttgattgc aacgaggatt gtggaacttc tgggacgcag 6600
ggggtgggaa gccctcaaat attggtggaa tctcctgcag tattggagtc aggaactgaa 6660
gaatagtgct gttagcttgc ttaatgccat agccatagca gtagctgagg ggacagatag 6720
aattatagaa gtagtacaaa ggggggttag agctgttctt aacataccca caagaataag 6780
acagggagcg gaaaggcttc ttgtataaga tgggtggcaa gttgtcaaaa agtaagatgc 6840
ctggatggtc tactataagg gaaagaatga gacgagctca gccagcagca gagccagcag 6900
cagttggggt gggagcagca tctcgagacc tggaaagaca tggagcactc acaagtagca 6960
atacagcagc taacaatgct gattgtgcct ggctagaagc acaagaggac gaggaagtgg 7020
gttttccagt cagacctcag gtacctctta ggccaatgac ttacaaggga gctgtagatc 7080
ttagccactt tttaaaagaa aaggggggac tggaagggtt agtttactcc caaaaaagac 7140
aagacatcct tgatctgtgg gtctaccaca cacaaggcta cttccctgat tggcagaact 7200
acacaccagg gccagggatc agatatcccc tgacctttgg atggtgcttc aagctagtac 7260
cagttgatcc agataaggta gaagaggcca atgaaggaga gaacaactgc ttattacacc 7320
ctatggccca gcatgggatg gatgacccag agaaagaagt gttagtgtgg aagtttgaca 7380
gccgcctagc atttcatcac atggcccgag agctgcatcc ggagtactac aaagactgct 7440
gagcggccgc cctgcaggtc gacctcgagg gggggcccgg taccttaatt aattaaggta 7500

ccaggtaagt gtacccaatt cgccctatag tgagtcgtat tacaattcac tcgatcgccc 7560
ttcccaacag ttgcgcagcc tgaatggcga atggagatcc aatttttaag tgtataatgt 7620
gttaaactac tgattctaat tgtttgtgta ttttagattc acagtcccaa ggctcatttc 7680
aggcccctca gtcctcacag tctgttcatg atcataatca gccataccac atttgtagag 7740
gttttacttg ctttaaaaaa cctcccacac ctccccctga acctgaaaca taaaatgaat 7800
gcaattgttg ttgttaactt gtttattgca gcttataatg gttacaaata aagcaatagc 7860
atcacaaatt tcacaaataa agcatttttt tcactgcatt ctagttgtgg tttgtccaaa 7920
ctcatcaatg tatcttaacg cgtaaattgt aagcgttaat gcttcacgac cacgctgatg 7980
agctttaccg cagctgcctc gcgcgtttcg gtgatgacgg tgaaaacctc tgacacatgc 8040
agctcccgga gacggtcaca gcttgtctgt aagcggatgc cgggagcaga caagcccgtc 8100
agggcgcgtc agcgggtgtt ggcgggtgtc ggggcgcagc catgacccag tcacgtagcg 8160
atagcggagt gtatactggc ttaactatgc ggcatcagag cagattgtac tgagagtgca 8220
ccatatatgc ggtgtgaaat accgcacaga tgcgtaagga gaaaataccg catcaggcgc 8280
tcttccgctt cctcgctcac tgactcgctg cgctcggtcg ttcggctgcg gcgagcggta 8340
tcagctcact caaaggcggt aatacggtta tccacagaat caggggataa cgcaggaaag 8400
aacatgtgag caaaaggcca gcaaaaggcc aggaaccgta aaaaggccgc gttgctggcg 8460
tttttccata ggctccgccc ccctgacgag catcacaaaa atcgacgctc aagtcagagg 8520
tggcgaaacc cgacaggact ataaagatac caggcgtttc cccctggaag ctccctcgtg 8580
cgctctcctg ttccgaccct gccgcttacc ggatacctgt ccgcctttct cccttcggga 8640
agcgtggcgc tttctcatag ctcacgctgt aggtatctca gttcggtgta ggtcgttcgc 8700
tccaagctgg gctgtgtgca cgaacccccc gttcagcccg accgctgcgc cttatccggt 8760
aactatcgtc ttgagtccaa cccggtaaga cacgacttat cgccactggc agcagccact 8820
ggtaacagga ttagcagagc gaggtatgta ggcggtgcta cagagttctt gaagtggtgg 8880
cctaactacg gctacactag aaggacagta tttggtatct gcgctctgct gaagccagtt 8940
accttcggaa aaagagttgg tagctcttga tccggcaaac aaaccaccgc tggtagcggt 9000
ggtttttttg tttgcaagca gcagattacg cgcagaaaaa aaggatctca agaagatcct 9060
ttgatctttt ctacggggtc tgacgctcag tggaacgaaa actcacgtta agggattttg 9120
gtcatgaaca ataaaactgt ctgcttacat aaacagtaat acaaggggtg ttatgagcca 9180
tattcaacgg gaaacgtctt gctcgaggcc gcgattaaat tccaacatgg atgctgattt 9240
atatgggtat aaatgggctc gcgataatgt cgggcaatca ggtgcgacaa tctatcgatt 9300
gtatgggaag cccgatgcgc cagagttgtt tctgaaacat ggcaaaggta gcgttgccaa 9360
tgatgttaca gatgagatgg tcagactaaa ctggctgacg gaatttatgc ctcttccgac 9420
catcaagcat tttatccgta ctcctgatga tgcatggtta ctcaccactg cgatccccgg 9480
gaaaacagca ttccaggtat tagaagaata tcctgattca ggtgaaaata ttgttgatgc 9540
gctggcagtg ttcctgcgcc ggttgcattc gattcctgtt tgtaattgtc cttttaacag 9600
cgatcgcgta tttcgtctcg ctcaggcgca atcacgaatg aataacggtt tggttgatgc 9660
gagtgatttt gatgacgagc gtaatggctg gcctgttgaa caagtctgga aagaaatgca 9720
taagcttttg ccattctcac cggattcagt cgtcactcat ggtgatttct cacttgataa 9780
ccttattttt gacgagggga aattaatagg ttgtattgat gttggacgag tcggaatcgc 9840
agaccgatac caggatcttg ccatcctatg gaactgcctc ggtgagtttt ctccttcatt 9900
acagaaacgg ctttttcaaa aatatggtat tgataatcct gatatgaata aattgcagtt 9960
tcatttgatg ctcgatgagt ttttctaaga attc 9994

<210> 45
<211> 4980
<212> DNA
<213> SIV/HIV

<400> 45
cgcccgaaca gggacttgaa ggagagtgag agactcctga gtacggctga gtgaaggcag 60
taagggcggc aggaaccaac cacgacggag tgctcctata aaggcgcggg tcggtaccag 120
acggcgtgag gagcgggaga ggaagaggcc tccggttgca ggtgagtgca acacaaaaaa 180
gaaatagctg tcttttatcc aggaaggggt aataagatag agtgggagat gggcgtgaga 240
aactccgtct tgtcagggaa gaaagcagat gaattagaaa aaattaggct acgacccaac 300
ggaaagaaaa agtacatgtt gaagcatgta gtatgggcag caaatgaatt agatagattt 360
ggattagcag aaagcctgtt ggagaacaaa gaaggatgtc aaaaaatact ttcggtctta 420
gctccattag tgccaacagg ctcagaaaat ttaaaaagcc tttataatac tgtctgcgtc 480
atctggtgca ttcacgcaga agagaaagtg aaacacactg aggaagcaaa acagatagtg 540
cagagacacc tagtggtgga aataggaaca acagaaacta tgccaaaaac aagtagacca 600
acagcaccat ctagcggcag aggaggaaat tacccagtac aacaaatagg tggtaactat 660
gtccacctgc cattaagccc gagaacatta aatgcctggg taaaattgat agaggaaaag 720
aaatttggag cagaagtagt gccaggattt caggcactgt cagaaggttg caccccctat 780
gacattaatc agatgttaaa ttgtgtggga gaccatcaag cggctatgca gattatcaga 840

gatattataa acgaggaggc tgcagattgg gacttgcagc acccacaacc agctccacaa 900
caaggacaac ttagggagcc gtcaggatca gatattgcag gaacaactag ttcagtagat 960
gaacaaatcc agtggatgta cagacaacag aaccccatac cagtaggcaa catttacagg 1020
agatggatcc aactggggtt gcaaaaatgt gtcagaatgt ataacccaac aaacattcta 1080
gatgtaaaac aagggccaaa agagccattt cagagctatg tagacaggtt ctacaaaagt 1140
ttaagagcag aacagacaga tgcagcagta aagaattgga tgactcaaac actgctgatt 1200
caaaatgcta acccagattg caagctagtg ctgaaggggc tgggtgtgaa tcccacccta 1260
gaagaaatgc tgacggcttg tcaaggagta ggggggccgg gacagaaggc tagattaatg 1320
gcagaagccc tgaaagaggc cctcgcacca gtgcctatcc cttttgcagc agcccaacag 1380
aggggaccaa gaaagccaat taagtgttgg aattgtggga aagagggaca ctctgcaagg 1440
caatgcagag ccccaagaag acagggatgc tggaaatgtg gaaaaatgga ccatgttatg 1500
gccaaatgcc cagacagaca ggcgggtttt ttaggccttg gtccatgggg aaagaagccc 1560
cgcaatttcc ccatggctca agtgcatcag gggctgatgc caactgctcc cccagaggac 1620
ccagctgtgg atctgctaaa gaactacatg cagttgggca agcagcagag agaaaagcag 1680
agagaaagca gagagaagcc ttacaaggag gtgacagagg atttgctgca cctcaattct 1740
ctctttggag gagaccagta gtcactgctc atattgaagg acagcctgta gaagtattac 1800
tggatacagg ggctgatgat tctattgtaa caggaataga gttaggtcca cattataccc 1860
caaaaatagt aggaggaata ggaggtttta ttaatactaa agaatacaaa aatgtagaaa 1920
tagaagtttt aggcaaaagg attaaaggga caatcatgac aggggacacc ccgattaaca 1980
tttttggtag aaatttgcta acagctctgg ggatgtctct aaattttccc atagctaaag 2040
tagagcctgt aaaagtcgcc ttaaagccag gaaagaatgg accaaaattg aagcagtggc 2100
cattatcaaa agaaaagata gttgcattaa gagaaatctg ggaaaagatg gaaaaggatg 2160
gtcagttgga ggaagctccc ccgaccaatc catacaacac ccccacattt gctataaaga 2220
aaaaggataa gaacaaatgg agaatgctga tagattttag ggaactaaat agggtcactc 2280
aggactttac ggaagtccaa ttaggaatac cacaccctgc aggattagca aaaaggaaaa 2340
gaattacagt actggatata ggtgatgcat atttctccat acctctagat gaagaattta 2400
ggcagtacac tgcctttact ttaccatcag taaataatgc agagccagga aaacgataca 2460
tttataaggt tctgcctcag ggatggaagg ggtcaccagc catcttccaa tacactatga 2520
gacatgtgct agaacccttc aggaaggcaa atccagatgt gaccttagtc cagtatatgg 2580
atgacatctt aatagctagt gacaggacag acctggaaca tgacagggta gttttacagt 2640
caaaggaact cttgaatagc atagggtttt ctaccccaga agagaaattc caaaaagatc 2700
ccccatttca atggatgggg tacgaattgt ggccaacaaa atggaagttg caaaagatag 2760
agttgccaca aagagagacc tggacagtga atgatataca gaagttagta ggagtattaa 2820
attgggcagc tcaaatttat ccaggtataa aaaccaaaca tctctgtagg ttaattagag 2880
gaaaaatgac tctaacagag gaagttcagt ggactgagat ggcagaagca gaatatgagg 2940
aaaataaaat aattctcagt caggaacaag aaggatgtta ttaccaagaa ggcaagccat 3000
tagaagccac ggtaataaag agtcaggaca atcagtggtc ttataaaatt caccaagaag 3060
acaaaatact gaaagtagga aaatttgcaa agataaagaa tacacatacc aatggagtga 3120
gactattagc acatgtaata cagaaaatag gaaaggaagc aatagtgatc tggggacagg 3180
tcccaaaatt ccacttacca gttgagaagg atgtatggga acagtggtgg acagactatt 3240
ggcaggtaac ctggataccg gaatgggatt ttatctcaac accaccgcta gtaagattag 3300
tcttcaatct agtgaaggac cctatagagg gagaagaaac ctattataca gatggatcgt 3360
gtaataaaca gtcaaaagaa gggaaagcag gatatatcac agataggggc aaagacaaag 3420
taaaagtgtt agaacagact actaatcaac aagcagaatt ggaagcattt ctcatggcat 3480
tgacagactc agggccaaag gcaaatatta tagtagattc acaatatgtt atgggaataa 3540
taacaggatg ccctacagaa tcagagagca ggctagttaa tcaaataata gaagaaatga 3600
ttaaaaagtc agaaatttat gtagcatggg taccagcaca caaaggtata ggaggaaacc 3660
aagaaataga ccacctagtt agtcaaggga ttagacaagt tctcttcttg gaaaagatag 3720
agccagcaca agaagaacat gataaatacc atagtaatgt aaaagaattg gtattcaaat 3780
ttggattacc cagaatagtg gccagacaga tagtagacac ctgtgataaa tgccatcaga 3840
aaggagaggc tatacatggg caggtaaatt cagatctagg gacttggcaa atggattgta 3900
cccatctaga gggaaaaata atcatagttg cagtacatgt agctagtgga ttcatagaag 3960
cagaggtaat tccacaagag acaggaagac agacagcact atttctgtta aaattggcag 4020
gcagatggcc tattacacat ctacacacag ataatggtgc taactttgct tcgcaagaag 4080
taaagatggt tgcatggtgg gcagggatag agcacacctt tggggtacca tacaatccac 4140
agagtcaggg agtagtggaa gcaatgaatc accacctgaa aaatcaaata gatagaatca 4200
gggaacaagc aaattcagta gaaaccatag tattaatggc agttcattgc atgaatttta 4260
aaagaagggg aggaataggg gatatgactc cagcagaaag attaattaac atgatcacta 4320
cagaacaaga gatacaattt caacaatcaa aaaactcaaa atttaaaaat tttcgggtct 4380
attacagaga aggcagagat caactgtgga agggacccgg tgagctattg tggaaagggg 4440
aaggagcagt catcttaaag gtagggacag acattaaggt agtacccaga agaaaggcta 4500
aaattatcaa agattatgga ggaggaaaag aggtggatag cagttcccac atggaggata 4560
ccggagaggt tagagaggtg gcatagcctc ataaaatatc tgaaatataa aactaaagat 4620

ctacaaaagg tttgctatgt gccccatttt aaggtcggat gggcatggtg gacctgcagc 4680
agagtaatct tcccactaca ggaaggaagc catttagaag tacaagggta ttggcatttg 4740
acaccagaaa aagggtggct cagtacttat gcagtgagga taacctggta ctcaaagaac 4800
ttttggacag atgtaacacc aaactatgca gacattttac tgcatagcac ttatttccct 4860
tgctttacag cgggagaagt gagaagggcc atcaggggag aacaactgct gtcttgctgc 4920
aggttcccga gagctcataa gcaccaggta ccaagcctac agtacttagc actgaaagta 4980

<210> 46
<211> 472
<212> DNA
<213> SIV/HIV

<400> 46
atggaggagg aaaagaggtg gatagcagtt cccacatgga ggataccgga gaggttagag 60
aggtggcata gcctcataaa atatctgaaa tataaaacta aagatctaca aaaggtttgc 120
tatgtgcccc attttaaggt cggatgggca tggtggacct gcagcagagt aatcttccca 180
ctacaggaag gaagccattt agaagtacaa gggtattggc atttgacacc agaaaaaggg 240
tggctcagta cttatgcagt gaggataacc tggtactcaa agaacttttg gacagatgta 300
acaccaaact atgcagacat tttactgcat agcacttatt tcccttgctt tacagcggga 360
gaagtgagaa gggccatcag gggagaacaa ctgctgtctt gctgcaggtt cccgagagct 420
cataagcacc aggtaccaag cctacagtac ttagcactga aagtagtaag cg 472

<210> 47
<211> 2376
<212> DNA
<213> SIV/HIV

<400> 47
cgcccgaaca gggacgcgaa agcgaaagta gaaccagagg agctctctcg acgcaggact 60
cggcttgctg aagcgcgcac ggcaagaggc gaggggcggc gactggtgag tacgccattt 120
ttgactagcg gaggctagaa ggagagagat gggtgcgaga gcgtcaatat taagcggggg 180
acaattagat agatgggaaa aaattcggtt acggccaggg ggaaagaaaa gatataagtt 240
aaaacatata gtatgggcaa gcagagagct agaacgattc gcagttaacc ctggcctgtt 300
agaaacagca gaaggctgta gacaaatact gggacagcta caaccatccc ttcagacagg 360
atcagaggaa cttaaatcat tatttaatac aatagctacc ctctattgtg tacatcaaag 420
aatagagata aaagacacca aggaagcttt agataagata gaggaagagc aaaacaaaag 480
taagaaaaaa gcacagcaag cagcagctga cacaggaaac agcagcagcc aagtcagcca 540
aaattaccct atagtgcaga acgctcaggg acaaatggta catcaggcca tatcacctag 600
aactttaaat gcatgggtaa aagtagtaga agaaaaggct tttaacccag aagtaatacc 660
catgtttgca gcattgtcag aaggagccac cccacaagat ttaaacacca tgctaaacac 720
agtgggggga catcaagcag ccatgcaaat attaaaagag actatcaatg aggaagctgc 780
agaatgggat agattgcatc cagtacatgc agggcctatt gcaccaggcc aaatgagaga 840
accaagggga agtgacatag caggaactac tagtaccctt caggaacaaa taggatggat 900
gacaaataat ccacctatcc cagtaggaga aatctataaa aaatggataa tcatgggatt 960
aaataaaatt gtaaggatgt atagccctac cagtattctg gacataagac aaggaccaaa 1020
ggaacccttt agagactatg tagaccggtt ctataaaact ctaagagccg agcaagcttc 1080
acaggaagta aaaaattgga tgacagaaac cttgttggtc caaaattcaa accccgattg 1140
taagactatt ttaaaagcat taggaccagg agctacacta gaagaaatga tgacagcatg 1200
ccagggagtg ggaggacctg gccataaagc aagagttttg gcagaagcaa tgagccaagt 1260
aacaaatcca acggccgtga tgatgcagaa aagcaatttt aggggccaaa gaaaaattgt 1320
taagtgtttt aattgtggca aagaagggca catagccaaa aattgcaggg ctcctagaaa 1380
aaagggctgt tggaaatgtg gaaaggaagg acaccaaatg aaagattgta ctgaaagaca 1440
ggctaatttt ttagggaaga tctggccttc ctacaaggga aggccaggga attttcctca 1500
aagcaggcta gaaccaacag ccccaccaga agcgagcttc aggtttgggg aggagacaac 1560
aactccccct cagaagcagg agacgataga caaggaggtg tatcctttaa cctccctcag 1620
atcactcttt ggcaacgacc cctcgtcaca ataaagatag gggggcaact aaaagaagct 1680
ctattagata caggagcaga tgatacagtg ttagaagaca tgaatttgcc aggaaaatgg 1740
aaaccaaaaa tgataggggg aattggagga tttatcaaag taaaacagta tgatcagata 1800
cccatagaaa tctgtggaca taaaactata ggtacagtat taataggacc tacacctgtc 1860
aacataattg gaaggaattt gttgactcag cttggttgca ctttaaattt tcccattagt 1920
cctattgaaa ctgtaccagt aaaattaaag ccaggaatgg atggcccaaa agttaagcaa 1980
tggccattga cagaagaaaa aataaaagca ttaatggaga tatgcacaga aatggaaaag 2040

gaagggaaaa tttcaaaaat tgggcctgaa aatccataca atactccagt gtttgccata 2100
aagaaaaaag acagtactaa gtggagaaaa ttagtagatt tcagagaact taataagaaa 2160
actcaagact tctgggaggt tcaattagga ataccacatc ccgcggggtt aaaaaagaaa 2220
aagtcagtaa cagtactgga tgtgggtgat gcatacttct cagttccctt agatgaagat 2280
tttaggaagt atactgcatt taccatacct agtataaaca atgagacatc aggaattaga 2340
tatcagtaca atgtgcttcc acagggatgg aaaggg 2376

<210> 48
<211> 411
<212> DNA
<213> HIV

<400> 48
aacagcaggg actttccaca aggggatgtt acggggaggt actggggagg agccggtcgg 60
gaacgcccac tttcttgatg tataaatatc actgcatttc gctctgtatt cagtcgctct 120
gcggagaggc tggcaggttg agccctggga ggttctctcc agcactagca ggtagagcct 180
gggtgttccc tgctagactc tcaccagcac ttggccggtg ctgggcagag tgattccacg 240
cttgcttgct taaagccctc ttcaataaag ctgccatttt agaagtaagc tagtgtgtgt 300
tcccatctct cctagccgcc gcctggtcaa ctcggtactc aataataaga agaccctggt 360
ctgttaggac cctttctgct ttgggaaacc gaagcaggaa aatccctagc a 411

<210> 49
<211> 481
<212> DNA
<213> SV40

<400> 49
tcgagggggg gcccggtacc ttaattaatt aaggtaccag gtaagtgtac ccaattcgcc 60
ctatagtgag tcgtattaca attcactcga tcgcccttcc caacagttgc gcagcctgaa 120
tggcgaatgg agatccaatt tttaagtgta taatgtgtta aactactgat tctaattgtt 180
tgtgtatttt agattcacag tcccaaggct catttcaggc ccctcagtcc tcacagtctg 240
ttcatgatca taatcagcca taccacattt gtagaggttt tacttgcttt aaaaaacctc 300
ccacacctcc ccctgaacct gaaacataaa atgaatgcaa ttgttgttgt taacttgttt 360
attgcagctt ataatggtta caaataaagc aatagcatca caaatttcac aaataaagca 420
tttttttcac tgcattctag ttgtggtttg tccaaactca tcaatgtatc ttaacgcgta 480
a 481

<210> 50
<211> 818
<212> DNA
<213> SIV

<400> 50
tggaagggat ttattacagt gcaagaagac atagaatctt agacatgtac ttagaaaagg 60
aaaaaggcat cataccagat tggcaggatt acacctcagg accaggaatt agatacccaa 120
agacatttgg ctggctatgg aaattagtcc ctgtaaatgt atcagatgag gcacaggagg 180
atgaagagca ttatttaatg catccagctc aaacttccca gtgggatgac ccttggagag 240
aggttctagc atggaagttt gatccaactc tggcctacac ttatgaggca tatgttagat 300
acccagaaga gtttggaagc aagtcaggcc tgtcagagga agaggttaaa agaaggctaa 360
ccgcaagagg ccttcttaac atggctgaca agaaggaaac tcgctgaaac agcagggact 420
ttccacaagg ggatgttacg gggaggtact ggggaggagc cggtcgggaa cgcccacttt 480
cttgatgtat aaatatcact gcatttcgct ctgtattcag tcgctctgcg gagaggctgg 540
caggttgagc cctgggaggt tctctccagc actagcaggt agagcctggg tgttccctgc 600
tagactctca ccagcacttg gccggtgctg ggcagagtga ttccacgctt gcttgcttaa 660
agccctcttc aataaagctg ccattttaga agtaagctag tgtgtgttcc catctctcct 720
agccgccgcc tggtcaactc ggtactcaat aataagaaga ccctggtctg ttaggaccct 780
ttctgctttg ggaaaccgaa gcaggaaaat ccctagca 818

<210> 51
<211> 1565
<212> DNA
<213> Influenza A virus

<400> 51
agcaaaagca gggtagataa tcactcactg agtgacatca aaatcatggc gtcccaaggc 60
accaaacggt cttacgaaca gatggagact gatggagaac gccagaatgc cactgaaatc 120
agagcatccg tcggaaaaat gattggtgga attggacgat tctacatcca aatgtgcacc 180
gaactcaaac tcagtgatta tgagggacgg ttgatccaaa acagcttaac aatagagaga 240
atggtgctct ctgcttttga cgaaaggaga aataaatacc tggaagaaca tcccagtgcg 300
gggaaagatc ctaagaaaac tggaggacct atatacagga gagtaaacgg aaagtggatg 360
agagaactca tcctttatga caaagaagaa ataaggcgaa tctggcgcca agctaataat 420
ggtgacgatg caacggctgg tctgactcac atgatgatct ggcattccaa tttgaatgat 480
gcaacttatc agaggacaag agctcttgtt cgcaccggaa tggatcccag gatgtgctct 540
ctgatgcaag gttcaactct ccctaggagg tctggagccg caggtgctgc agtcaaagga 600
gttggaacaa tggtgatgga attggtcagg atgatcaaac gtgggatcaa tgatcggaac 660
ttctggaggg gtgagaatgg acgaaaaaca agaattgctt atgaaagaat gtgcaacatt 720
ctcaaaggga aatttcaaac tgctgcacaa aaagcaatga tggatcaagt gagagagagc 780
cggaacccag ggaatgctga gttcgaagat ctcacttttc tagcacggtc tgcactcata 840
ttgagagggt cggttgctca caagtcctgc ctgcctgcct gtgtgtatgg acctgccgta 900
gccagtgggt acgactttga aagagaggga tactctctag tcggaataga ccctttcaga 960
ctgcttcaaa acagccaagt gtacagccta atcagaccaa atgagaatcc agcacacaag 1020
agtcaactgg tgtggatggc atgccattct gccgcatttg aagatctaag agtattaagc 1080
ttcatcaaag ggacgaaggt gctcccaaga gggaagcttt ccactagagg agttcaaatt 1140
gcttccaatg aaaatatgga gactatggaa tcaagtacac ttgaactgag aagcaggtac 1200
tgggccataa ggaccagaag tggaggaaac accaatcaac agagggcatc tgcgggccaa 1260
atcagcatac aacctacgtt ctcagtacag agaaatctcc cttttgacag aacaaccatt 1320
atggcagcat tcaatgggaa tacagaggga agaacatctg acatgaggac cgaaatcata 1380
aggatgatgg aaagtgcaag accagaagat gtgtctttcc aggggcgggg agtcttcgag 1440
ctctcggacg aaaaggcagc gagcccgatc gtgccttcct ttgacatgag taatgaagga 1500
tcttatttct tcggagacaa tgcagaggag tacgacaatt aaagaaaaat acccttgttt 1560
ctact 1565

<210> 52
<211> 719
<212> DNA
<213> HIV-1

<400> 52
catggatgca atgaagagag ggctctgctg tgtgctgctg ctgtgtggag cagtcttcgt 60
ttcgcccagc gagatctcct ccaagaggtc cgtgcccggc tggtccaccg tgagggagag 120
gatgaggagg gccgagcccg ccgccgacag ggtgaggagg accgagcccg ccgccgtggg 180
cgtgggcgcc gtgtccaggg acctggagaa gcacggcgcc atcacctcct ccaacaccgc 240
cgccaccaac gccgactgcg cctggctgga ggcccaggag gacgaggagg tgggcttccc 300
cgtgaggccc caggtgcccc tgaggcccat gacctacaag ggcgccgtgg acctgtccca 360
cttcctgaag gagaagggcg gcctggaggg cctgatccac tcccagaaga ggcaggacat 420
cctggacctg tgggtgtacc acacccaggg ctacttcccc gactggcaga actacacccc 480
cggccccggc atcaggttcc ccctgacctt cggctggtgc ttcaagctgg tgcccgtgga 540
gcccgagaag gtggaggagg ccaacgaggg cgagaacaac tgcctgctgc accccatgtc 600
ccagcacggc atcgaggacc ccgagaagga ggtgctggag tggaggttcg actccaagct 660
ggccttccac cacgtggcca gggagctgca ccccgagtac tacaaggact gctaaagcc 719

<210> 53
<211> 671
<212> DNA
<213> HIV-1

<400> 53
gatctgccac catggccggc aagtggtcca agaggtccgt gcccggctgg tccaccgtga 60
gggagaggat gaggagggcc gagcccgccg ccgacagggt gaggaggacc gagcccgccg 120
ccgtgggcgt gggcgccgtg tccagggacc tggagaagca cggcgccatc acctcctcca 180
acaccgccgc caccaacgcc gactgcgcct ggctggaggc ccaggaggac gaggaggtgg 240
gcttccccgt gaggccccag gtgcccctga ggcccatgac ctacaagggc gccgtggacc 300
tgtcccactt cctgaaggag aagggcggcc tggagggcct gatccactcc cagaagaggc 360
aggacatcct ggacctgtgg gtgtaccaca cccagggcta cttccccgac tggcagaact 420
acacccccgg ccccggcatc aggttccccc tgaccttcgg ctggtgcttc aagctggtgc 480
ccgtggagcc cgagaaggtg gaggaggcca acgagggcga gaacaactgc ccgcccaccc 540

ccatgtccca gcacggcatc gaggaccccg agaaggaggt gctggagtgg aggttcgact 600
ccaagctggc cttccaccac gtggccaggg agctgcaccc cgagtactac aaggactgct 660
aaagcccggg c 671

<210> 54
<211> 720
<212> DNA
<213> HIV-1

<400> 54
catggatgca atgaagagag ggctctgctg tgtgctgctg ctgtgtggag cagtcttcgt 60
ttcgcccagc gagatctcct ccaagaggtc cgtgcccggc tggtccaccg tgagggagag 120
gatgaggagg gccgagcccg ccgccgacag ggtgaggagg accgagcccg ccgccgtggg 180
cgtgggcgcc gtgtccaggg acctggagaa gcacggcgcc atcacctcct ccaacaccgc 240
cgccaccaac gccgactgcg cctggctgga ggcccaggag gacgaggagg tgggcttccc 300
cgtgaggccc caggtgcccc tgaggcccat gacctacaag ggcgccgtgg acctgtccca 360
cttcctgaag gagaagggcg gcctggaggg cctgatccac tcccagaaga ggcaggacat 420
cctggacctg tgggtgtacc acacccaggg ctacttcccc gactggcaga actacacccc 480
cggccccggc atcaggttcc ccctgacctt cggctggtgc ttcaagctgg tgcccgtgga 540
gcccgagaag gtggaggagg ccaacgaggg cgagaacaac tgcgccgccc accccatgtc 600
ccagcacggc atcgaggacc ccgagaagga ggtgctggag tggaggttcg actccaagct 660
ggccttccac cacgtggcca gggagctgca ccccgagtac tacaaggact gctaaagccc 720

<210> 55
<211> 9599
<212> DNA
<213> HCV

<400> 55
gccagccccc tgatgggggc gacactccac catgaatcac tcccctgtga ggaactactg 60
tcttcacgca gaaagcgtct agccatggcg ttagtatgag tgtcgtgcag cctccaggac 120
cccccctccc gggagagcca tagtggtctg cggaaccggt gagtacaccg gaattgccag 180
gacgaccggg tcctttcttg gataaacccg ctcaatgcct ggagatttgg gcgtgccccc 240
gcaagactgc tagccgagta gtgttgggtc gcgaaaggcc ttgtggtact gcctgatagg 300
gtgcttgcga gtgccccggg aggtctcgta gaccgtgcac catgagcacg aatcctaaac 360
ctcaaagaaa aaccaaacgt aacaccaacc gtcgcccaca ggacgtcaag ttcccgggtg 420
gcggtcagat cgttggtgga gtttacttgt tgccgcgcag gggccctaga ttgggtgtgc 480
gcgcgacgag gaagacttcc gagcggtcgc aacctcgagg tagacgtcag cctatcccca 540
aggcacgtcg gcccgagggc aggacctggg ctcagcccgg gtacccttgg cccctctatg 600
gcaatgaggg ttgcgggtgg gcgggatggc tcctgtctcc ccgtggctct cggcctagct 660
ggggccccac agacccccgg cgtaggtcgc gcaatttggg taaggtcatc gataccctta 720
cgtgcggctt cgccgacctc atggggtaca taccgctcgt cggcgcccct cttggaggcg 780
ctgccagggc cctggcgcat ggcgtccggg ttctggaaga cggcgtgaac tatgcaacag 840
ggaaccttcc tggttgctct ttctctatct tccttctggc cctgctctct tgcctgactg 900
tgcccgcttc agcctaccaa gtgcgcaatt cctcggggct ttaccatgtc accaatgatt 960
gccctaactc gagtattgtg tacgaggcgg ccgatgccat cctgcacact ccggggtgtg 1020
tcccttgcgt tcgcgagggt aacgcctcga ggtgttgggt ggcggtgacc cccacggtgg 1080
ccaccaggga cggcaaactc cccacaacgc agcttcgacg tcatatcgat ctgcttgtcg 1140
ggagcgccac cctctgctcg gccctctacg tgggggacct gtgcgggtct gtctttcttg 1200
ttggtcaact gtttaccttc tctcccaggc gccactggac gacgcaagac tgcaattgtt 1260
ctatctatcc cggccatata acgggtcatc gcatggcatg ggatatgatg atgaactggt 1320
cccctacggc agcgttggtg gtagctcagc tgctccggat cccacaagcc atcatggaca 1380
tgatcgctgg tgctcactgg ggagtcctgg cgggcatagc gtatttctcc atggtgggga 1440
actgggcgaa ggtcctggta gtgctgctgc tatttgccgg cgtcgacgcg gaaacccacg 1500
tcaccggggg aaatgccggc cgcaccacgg ctgggcttgt tggtctcctt acaccaggcg 1560
ccaagcagaa catccaactg atcaacacca acggcagttg gcacatcaat agcacggcct 1620
tgaattgcaa tgaaagcctt aacaccggct ggttagcagg gctcttctat caacacaaat 1680
tcaactcttc aggctgtcct gagaggttgg ccagctgccg acgccttacc gattttgccc 1740
agggctgggg tcctatcagt tatgccaacg gaagcggcct cgacgaacgc ccctactgct 1800
ggcactaccc tccaagacct tgtggcattg tgcccgcaaa gagcgtgtgt ggcccggtat 1860
attgcttcac tcccagcccc gtggtggtgg gaacgaccga caggtcgggc gcgcctacct 1920
acagctgggg tgcaaatgat acggatgtct tcgtccttaa caacaccagg ccaccgctgg 1980

gcaattggtt cggttgtacc tggatgaact caactggatt caccaaagtg tgcggagcgc 2040
ccccttgtgt catcggaggg gtgggcaaca acaccttgct ctgccccact gattgcttcc 2100
gcaaacatcc ggaagccaca tactctcggt gcggctccgg tccctggatt acacccaggt 2160
gcatggtcga ctacccgtat aggctttggc actatccttg taccatcaat tacaccatat 2220
tcaaagtcag gatgtacgtg ggaggggtcg agcacaggct ggaagcggcc tgcaactgga 2280
cgcggggcga acgctgtgat ctggaagaca gggacaggtc cgagctcagc ccgttgctgc 2340
tgtccaccac acagtggcag gtccttccgt gttctttcac gaccctgcca gccttgtcca 2400
ccggcctcat ccacctccac cagaacattg tggacgtgca gtacttgtac ggggtagggt 2460
caagcatcgc gtcctgggcc attaagtggg agtacgtcgt tctcctgttc cttctgcttg 2520
cagacgcgcg cgtctgctcc tgcttgtgga tgatgttact catatcccaa gcggaggcgg 2580
ctttggagaa cctcgtaata ctcaatgcag catccctggc cgggacgcac ggtcttgtgt 2640
ccttcctcgt gttcttctgc tttgcgtggt atctgaaggg taggtgggtg cccggagcgg 2700
tctacgccct ctacgggatg tggcctctcc tcctgctcct gctggcgttg cctcagcggg 2760
catacgcact ggacacggag gtggccgcgt cgtgtggcgg cgttgttctt gtcgggttaa 2820
tggcgctgac tctgtcgcca tattacaagc gctatatcag ctggtgcatg tggtggcttc 2880
agtattttct gaccagagta gaagcgcaac tgcacgtgtg ggttcccccc ctcaacgtcc 2940
ggggggggcg cgatgccgtc atcttactca tgtgtgtagt acacccgacc ctggtatttg 3000
acatcaccaa actactcctg gccatcttcg gacccctttg gattcttcaa gccagtttgc 3060
ttaaagtccc ctacttcgtg cgcgttcaag gccttctccg gatctgcgcg ctagcgcgga 3120
agatagccgg aggtcattac gtgcaaatgg ccatcatcaa gttaggggcg cttactggca 3180
cctatgtgta taaccatctc acccctcttc gagactgggc gcacaacggc ctgcgagatc 3240
tggccgtggc tgtggaacca gtcgtcttct cccgaatgga gaccaagctc atcacgtggg 3300
gggcagatac cgccgcgtgc ggtgacatca tcaacggctt gcccgtctct gcccgtaggg 3360
gccaggagat actgcttggg ccagccgacg gaatggtctc caaggggtgg aggttgctgg 3420
cgcccatcac ggcgtacgcc cagcagacga gaggcctcct agggtgtata atcaccagcc 3480
tgactggccg ggacaaaaac caagtggagg gtgaggtcca gatcgtgtca actgctaccc 3540
aaaccttcct ggcaacgtgc atcaatgggg tatgctggac tgtctaccac ggggccggaa 3600
cgaggaccat cgcatcaccc aagggtcctg tcatccagat gtataccaat gtggaccaag 3660
accttgtggg ctggcccgct cctcaaggtt cccgctcatt gacaccctgt acctgcggct 3720
cctcggacct ttacctggtc acgaggcacg ccgatgtcat tcccgtgcgc cggcgaggtg 3780
atagcagggg tagcctgctt tcgccccggc ccatttccta cttgaaaggc tcctcggggg 3840
gtccgctgtt gtgccccgcg ggacacgccg tgggcctatt cagggccgcg gtgtgcaccc 3900
gtggagtggc taaagcggtg gactttatcc ctgtggagaa cctagggaca accatgagat 3960
ccccggtgtt cacggacaac tcctctccac cagcagtgcc ccagagcttc caggtggccc 4020
acctgcatgc tcccaccggc agcggtaaga gcaccaaggt cccggctgcg tacgcagccc 4080
agggctacaa ggtgttggtg ctcaacccct ctgttgctgc aacgctgggc tttggtgctt 4140
acatgtccaa ggcccatggg gttgatccta atatcaggac cggggtgaga acaattacca 4200
ctggcagccc catcacgtac tccacctacg gcaagttcct tgccgacggc gggtgctcag 4260
gaggtgctta tgacataata atttgtgacg agtgccactc cacggatgcc acatccatct 4320
tgggcatcgg cactgtcctt gaccaagcag agactgcggg ggcgagactg gttgtgctcg 4380
ccactgctac ccctccgggc tccgtcactg tgtcccatcc taacatcgag gaggttgctc 4440
tgtccaccac cggagagatc cccttttacg gcaaggctat ccccctcgag gtgatcaagg 4500
ggggaagaca tctcatcttc tgccactcaa agaagaagtg cgacgagctc gccgcgaagc 4560
tggtcgcatt gggcatcaat gccgtggcct actaccgcgg tcttgacgtg tctgtcatcc 4620
cgaccagcgg cgatgttgtc gtcgtgtcga ccgatgctct catgactggc tttaccggcg 4680
acttcgactc tgtgatagac tgcaacacgt gtgtcactca gacagtcgat ttcagccttg 4740
accctacctt taccattgag acaaccacgc tcccccagga tgctgtctcc aggactcaac 4800
gccggggcag gactggcagg gggaagccag gcatctatag atttgtggca ccgggggagc 4860
gcccctccgg catgttcgac tcgtccgtcc tctgtgagtg ctatgacgcg ggctgtgctt 4920
ggtatgagct cacgcccgcc gagactacag ttaggctacg agcgtacatg aacaccccgg 4980
ggcttcccgt gtgccaggac catcttgaat tttgggaggg cgtctttacg ggcctcactc 5040
atatagatgc ccacttttta tcccagacaa agcagagtgg ggagaacttt ccttacctgg 5100
tagcgtacca agccaccgtg tgcgctaggg ctcaagcccc tcccccatcg tgggaccaga 5160
tgtggaagtg tttgatccgc cttaaaccca ccctccatgg gccaacaccc ctgctataca 5220
gactgggcgc tgttcagaat gaagtcaccc tgacgcaccc aatcaccaaa tacatcatga 5280
catgcatgtc ggccgacctg gaggtcgtca cgagcacctg ggtgctcgtt ggcggcgtcc 5340
tggctgctct ggccgcgtat tgcctgtcaa caggctgcgt ggtcatagtg ggcaggatcg 5400
tcttgtccgg gaagccggca attatacctg acagggaggt tctctaccag gagttcgatg 5460
agatggaaga gtgctctcag cacttaccgt acatcgagca agggatgatg ctcgctgagc 5520
agttcaagca gaaggccctc ggcctcctgc agaccgcgtc ccgccatgca gaggttatca 5580
cccctgctgt ccagaccaac tggcagaaac tcgaggtctt ttgggcgaag cacatgtgga 5640
atttcatcag tgggatacaa tacttggcgg gcctgtcaac gctgcctggt aaccccgcca 5700
ttgcttcatt gatggctttt acagctgccg tcaccagccc actaaccact ggccaaaccc 5760

tcctcttcaa catattgggg gggtgggtgg ctgcccagct cgccgccccc ggtgccgcta 5820
ctgcctttgt gggtgctggc ctagctggcg ccgccatcgg cagcgttgga ctggggaagg 5880
tcctcgtgga cattcttgca gggtatggcg cgggcgtggc gggagctctt gtagcattca 5940
agatcatgag cggtgaggtc ccctccacgg aggacctggt caatctgctg cccgccatcc 6000
tctcgcctgg agcccttgta gtcggtgtgg tctgcgcagc aatactgcgc cggcacgttg 6060
gcccgggcga gggggcagtg caatggatga accggctaat agccttcgcc tcccggggga 6120
accatgtttc ccccacgcac tacgtgccgg agagcgatgc agccgcccgc gtcactgcca 6180
tactcagcag cctcactgta acccagctcc tgaggcgact gcatcagtgg ataagctcgg 6240
agtgtaccac tccatgctcc ggttcctggc taagggacat ctgggactgg atatgcgagg 6300
tgctgagcga ctttaagacc tggctgaaag ccaagctcat gccacaactg cctgggattc 6360
cctttgtgtc ctgccagcgc gggtataggg gggtctggcg aggagacggc attatgcaca 6420
ctcgctgcca ctgtggagct gagatcactg gacatgtcaa aaacgggacg atgaggatcg 6480
tcggtcctag gacctgcagg aacatgtgga gtgggacgtt ccccattaac gcctacacca 6540
cgggcccctg tactcccctt cctgcgccga actataagtt cgcgctgtgg agggtgtctg 6600
cagaggaata cgtggagata aggcgggtgg gggacttcca ctacgtatcg ggtatgacta 6660
ctgacaatct taaatgcccg tgccagatcc catcgcccga atttttcaca gaattggacg 6720
gggtgcgcct acacaggttt gcgccccctt gcaagccctt gctgcgggag gaggtatcat 6780
tcagagtagg actccacgag tacccggtgg ggtcgcaatt accttgcgag cccgaaccgg 6840
acgtagccgt gttgacgtcc atgctcactg atccctccca tataacagca gaggcggccg 6900
ggagaaggtt ggcgagaggg tcaccccctt ctatggccag ctcctcggct agccagctgt 6960
ccgctccatc tctcaaggca acttgcaccg ccaaccatga ctcccctgac gccgagctca 7020
tagaggctaa cctcctgtgg aggcaggaga tgggcggcaa catcaccagg gttgagtcag 7080
agaacaaagt ggtgattctg gactccttcg atccgcttgt ggcagaggag gatgagcggg 7140
aggtctccgt acctgcagaa attctgcgga agtctcggag attcgcccgg gccctgcccg 7200
tctgggcgcg gccggactac aaccccccgc tagtagagac gtggaaaaag cctgactacg 7260
aaccacctgt ggtccatggc tgcccgctac cacctccacg gtcccctcct gtgcctccgc 7320
ctcggaaaaa gcgtacggtg gtcctcaccg aatcaaccct atctactgcc ttggccgagc 7380
ttgccaccaa aagttttggc agctcctcaa cttccggcat tacgggcgac aatacgacaa 7440
catcctctga gcccgcccct tctggctgcc cccccgactc cgacgttgag tcctattctt 7500
ccatgccccc cctggagggg gagcctgggg atccggatct cagcgacggg tcatggtcga 7560
cggtcagtag tggggccgac acggaagatg tcgtgtgctg ctcaatgtct tattcctgga 7620
caggcgcact cgtcaccccg tgcgctgcgg aagaacaaaa actgcccatc aacgcactga 7680
gcaactcgtt gctacgccat cacaatctgg tgtattccac cacttcacgc agtgcttgcc 7740
aaaggcagaa gaaagtcaca tttgacagac tgcaagttct ggacagccat taccaggacg 7800
tgctcaagga ggtcaaagca gcggcgtcaa aagtgaaggc taacttgcta tccgtagagg 7860
aagcttgcag cctgacgccc ccacattcag ccaaatccaa gtttggctat ggggcaaaag 7920
acgtccgttg ccatgccaga aaggccgtag cccacatcaa ctccgtgtgg aaagaccttc 7980
tggaagacag tgtaacacca atagacacta ccatcatggc caagaacgag gttttctgcg 8040
ttcagcctga gaaggggggt cgtaagccag ctcgtctcat cgtgttcccc gacctgggcg 8100
tgcgcgtgtg cgagaagatg gccctgtacg acgtggttag caagctcccc ctggccgtga 8160
tgggaagctc ctacggattc caatactcac caggacagcg ggttgaattc ctcgtgcaag 8220
cgtggaagtc caagaagacc ccgatggggt tctcgtatga tacccgctgt tttgactcca 8280
cagtcactga gagcgacatc cgtacggagg aggcaattta ccaatgttgt gacctggacc 8340
cccaagcccg cgtggccatc aagtccctca ctgagaggct ttatgttggg ggccctctta 8400
ccaattcaag gggggaaaac tgcggctacc gcaggtgccg cgcgagcggc gtactgacaa 8460
ctagctgtgg taacaccctc acttgctaca tcaaggcccg ggcagcctgt cgagccgcag 8520
ggctccagga ctgcaccatg ctcgtgtgtg gcgacgactt agtcgttatc tgtgaaagtg 8580
cgggggtcca ggaggacgcg gcgagcctga gagccttcac ggaggctatg accaggtact 8640
ccgccccccc cggggacccc ccacaaccag aatacgactt ggagcttata acatcatgct 8700
cctccaacgt gtcagtcgcc cacgacggcg ctggaaagag ggtctactac cttacccgtg 8760
accctacaac ccccctcgcg agagccgcgt gggagacagc aagacacact ccagtcaatt 8820
cctggctagg caacataatc atgtttgccc ccacactgtg ggcgaggatg atactgatga 8880
cccatttctt tagcgtcctc atagccaggg atcagcttga acaggctctt aactgtgaga 8940
tctacggagc ctgctactcc atagaaccac tggatctacc tccaatcatt caaagactcc 9000
atggcctcag cgcattttca ctccacagtt actctccagg tgaaatcaat agggtggccg 9060
catgcctcag aaaacttggg gtcccgccct tgcgagcttg gagacaccgg gcccggagcg 9120
tccgcgctag gcttctgtcc agaggaggca gggctgccat atgtggcaag tacctcttca 9180
actgggcagt aagaacaaag ctcaaactca ctccaatagc ggccgctggc cggctggact 9240
tgtccggttg gttcacggct ggctacagcg ggggagacat ttatcacagc gtgtctcatg 9300
cccggccccg ctggttctgg ttttgcctac tcctgctcgc tgcaggggta ggcatctacc 9360
tcctccccaa ccgatgaagg ttggggtaaa cactccggcc tcttaagcca tttcctgttt 9420
tttttttttt tttttttttt tttttctttt tttttttctt tcctttcctt ctttttttcc 9480
tttctttttc ccttctttaa tggtggctcc atcttagccc tagtcacggc tagctgtgaa 9540

aggtccgtga gccgcatgac tgcagagagt gctgatactg gcctctctgc agatcatgt 9599

<210> 56
<211> 340
<212> DNA
<213> HCV

<400> 56
ctccacagtc actgagagcg acatccgtac ggaggaggca atctaccaat gttgtgacct 60
cgacccccaa gcccgcgtgg ccatcaagtc cctcaccgag aggctttatg ttgggggccc 120
tcttaccaat tcaagggggg agaactgcgg ctatcgcagg tgccgcgcga gcggcgtact 180
gacaactagc tgtggtaaca ccctcacttg ctacatcaag gcccgggcag cctgtcgagc 240
cgcagggctc caggactgca ccatgctcgt gtgtggcgac gacttagtcg ttatctgtga 300
aagcgcgggg gtccaggagg acgcggcgag cctgagagcc 340

<210> 57
<211> 340
<212> DNA
<213> HCV

<400> 57
ctccacagtc actgagagcg acatccgtac ggaggaggca atttaccaat gttgtgacct 60
ggacccccaa gcccgcatgg ccatcaagtc cctcactgag aggctttatg tcgggggccc 120
tcttaccaat tcaagggggg agaactgcgg ctaccgcagg tgccgcgcga gcggcgtact 180
gacaactagc tgtggtaaca ccctcacttg ctacatcaag gcccgggcag cctgtcgagc 240
cgcagggctc caggactgca ccatgcttgt gtgtggcgac gacttagtcg ttatctgtga 300
aagtgcgggg gtccaggagg acgcggcgag cctgagagcc 340

<210> 58
<211> 340
<212> DNA
<213> HCV

<400> 58
ctccacagtc actgagagcg acatccgtac ggaggaggca atctaccaat gttgtgatct 60
ggacccccaa gcccgcgtgg ccatcaagtc cctcactgag aggctttacg ttgggggccc 120
tcttaccaat tcaagggggg agaactgcgg ctaccgcagg tgccgggcga gcggcgtact 180
gacaactagc tgtggtaata ccctcacttg ctacatcaag gcccgggcag cctgtcgagc 240
cgcagggctc cgggactgca ccatgctcgt gtgtggtgac gacttggtcg ttatctgtga 300
gagtgcgggg gtccaggagg acgcggcgag cctgagagcc 340

<210> 59
<211> 340
<212> DNA
<213> HCV

<400> 59
ctctacagtc actgagaacg acatccgtac ggaggaggca atttaccaat gttgtgacct 60
ggacccccaa gcccgcgtgg ccatcaagtc cctcactgag aggctttatg ttgggggccc 120
ccttaccaat tcaagggggg aaaactgcgg ctatcgcagg tgccgcgcga gcggcgtact 180
gacaactagc tgtggtaaca ccctcacttg ctacattaag gcccgggcag cctgtcgagc 240
cgcagggctc caggactgca ccatgctcgt gtgtggcgac gacttagtcg ttatctgtga 300
gagtgcggga gtccaggagg acgcggcgaa cttgagagcc 340

<210> 60
<211> 340
<212> DNA
<213> HCV

<400> 60
ctccacagtc actgagagcg atatccgtac ggaggaggca atctaccagt gttgtgacct 60
ggacccccaa gcccgcgtgg ccatcaagtc cctcaccgag aggctttatg tcgggggccc 120
tcttaccaat tcaagggggg aaaactgcgg ctatcgcagg tgccgcgcaa gcggcgtact 180

gacaactagc tgtggtaaca ccctcacttg ttacatcaag gcccaagcag cctgtcgagc 240
cgcagggctc cgggactgca ccatgctcgt gtgtggcgac gacttagtcg ttatctgtga 300
aagtcaggga gtccaggagg atgcagcgaa cctgagagcc 340

<210> 61
<211> 340
<212> DNA
<213> HCV

<400> 61
ctctacagtc actgagagcg atatccgtac ggaggaggca atctaccaat gttgtgacct 60
ggaccccgaa gcccgtgtgg ccatcaagtc cctcactgag aggctttatg ttgggggccc 120
tcttaccaat tcaagggggg agaactgcgg ctaccgcagg tgccgcgcaa gcggcgtact 180
gacgactagc tgtggtaata ccctcacttg ttacatcaag gcccgggcag cctgtcgagc 240
cgcagggctc caggactgca ccatgctcgt gtgtggcgac gacctagtcg ttatctgcga 300
aagtgcgggg gtccaggagg acgcggcgag cctgagagcc 340

<210> 62
<211> 340
<212> DNA
<213> HCV

<400> 62
ctccacagtc actgagaatg acacccgtgt tgaggagtca atttaccaat gttgtgactt 60
ggcccccgaa gccagacagg ccataaggtc gctcacagag cggctctatg tcgggggtcc 120
tatgactaac tccaaagggc agaactgcgg ctatcgccgg tgccgcgcga gcggcgtgct 180
gacgactagc tgcggtaata ccctcacatg ctacctgaag gccacagcgg cctgtcgagc 240
tgccaagctc caggactgca cgatgctcgt gaacggagac gaccttgtcg ttatctgtga 300
aagcgcgggg aaccaagagg acgcggcaag cctacgagcc 340

<210> 63
<211> 340
<212> DNA
<213> HCV

<400> 63
ctcaacggtc actgagaatg acatccgtgt tgaggagtca atttaccaaa gttgtgactt 60
ggcccccgag gccagacaag ccataaggtc gctcacagag cggctttaca tcgggggccc 120
cctgactaat tcaaaagggc agaactgcgg ctatcgccga tgccgcgcca gcggtgtgct 180
gacgactagc tgcggtaata ccctcacatg ttacttgaag gccactgcgg cctgtagagc 240
tgcgaagctc caggactgca cgatgctcgt gtgcggagac gaccttgtcg ttatctgtga 300
aagcgcggga acccaggagg atgcggcgag cctacgagtc 340

<210> 64
<211> 340
<212> DNA
<213> HCV

<400> 64
ctcaacggtc accgagaatg acatccgtgt tgaggagtca atttatcaat gttgtgcctt 60
ggcccccgag gctagacagg ccataaggtc gctcacagag cggctttata tcgggggccc 120
cctgaccaat tcaaaggggc agaactgcgg ttatcgccgg tgccgcgcca gcggcgtact 180
gacgaccagc tgcggtaata cccttacatg ttacttgaag gcctctgcag cctgtcgagc 240
cgcgaagctc caggactgca cgatgctcgt gtgtggggac gaccttgtcg ttatctgtga 300
aagcgcggga acccaggagg acgcggcgaa cctacgagtc 340

<210> 65
<211> 340
<212> DNA
<213> HCV

<400> 65
ctcaacggtc actgagagtg acatccgtgt cgaggagtcg atttaccaat gttgtgactt 60

ggcccccgaa gccaggcagg ccataaggtc gctcaccgag cgactttata tcgggggccc 120
cctgactaat tcaaaagggc agaactgcgg ttatcgccgg tgccgcgcga gcggcgtgct 180
gacgactagc tgcggtaata ccctcacatg ttacttgaag gcctctgcag cctgtcgagc 240
tgcaaagctc caggactgca cgatgctcgt gaacggggac gaccttgtcg ttatctgcga 300
gagcgcggga acccaagagg acgcggcgag cctacgagtc 340

<210> 66
<211> 340
<212> DNA
<213> HCV

<400> 66
ctccacagtc actgagagtg acatccgtgt tgaggagtca atttaccaat gttgtgactt 60
ggcccccgaa gccagacagg ctataaggtc gctcacagag cggctgtaca tcgggggtcc 120
cctgactaat tcaaaagggc agaactgcgg ctatcgccgg tgccgcgcaa gcggcgtgct 180
gacgactagc tgcggtaaca ccctcacatg ttacttgaag gcctctgcgg cctgtcgagc 240
tgcgaagctc caggactgca cgatgctcgt gtgcggtgac gaccttgtcg ttatctgtga 300
gagcgcggga acccaagagg acgcggcgag cctacgagtc 340

<210> 67
<211> 340
<212> DNA
<213> HCV

<400> 67
ctcaacagtc actgagagtg acatccgtgt tgaggagtca atctaccaat gttgtgactt 60
ggcccccgaa gccagacagg ctataaggtc gctcacagag cggctttaca tcgggggtcc 120
cctgactaat tcaaaagggc agaactgcgg ctatcgccgg tgccgcgcaa gcggcgtgct 180
gacgactagc tgcggtaata ccctcacatg ttacctgaag gccagtgcgg cctgtcgagc 240
tgcgaagctc caggactgca caatgctcgt gtgcggtgac gaccttgtcg ttatctgtga 300
gagcgcgggg acccaagagg acgcggcgag cctacgagtc 340

<210> 68
<211> 340
<212> DNA
<213> HCV

<400> 68
ctcaaccgtc actgagagag acatcagaac tgaggagtcc atataccgag cctgctccct 60
gcctgaggag gctcacattg ccatacactc gctgactgag aggctctacg tgggagggcc 120
catgttcaac agcaagggcc agacctgcgg gtacaggcgt tgccgcgcca gcggggtgct 180
caccactagc atggggaaca ccatcacatg ctatgtaaaa gccctagcgg cttgcaaggc 240
tgcagggata gttgcaccct caatgctggt atgcggcgac gacttagttg tcatctcaga 300
aagccagggg actgaggagg acgagcggaa cctgagagct 340

<210> 69
<211> 340
<212> DNA
<213> HCV

<400> 69
ctctacagtc acgtaaaagg acatcacatc ctaggagtcc atctaccagt cctgttcact 60
gcccgaggag gctcgaactg ctatacactc actgactgag agactatacg taggggggcc 120
catgacaaac agcaagggcc aatcctgcgg gtacaggcgt tgccgcgcga gcgcagtgct 180
caccaccagc atgggcaaca cactcacgtg ctacgtaaaa gccagggcgg cgtgtaacgc 240
cgcggggatt gttgctccca ccatgctggt gtgcggtgac gacctggtcg tcatctcaga 300
gagtcaaggg gctgaggagg acgagcagaa cctgagagtc 340

<210> 70
<211> 340
<212> DNA
<213> HCV

<400> 70
ctctacagtc acagagaggg acatcagaac cgaggagtcc atctatctgt cctgctcact 60
gcctgaggag gcccgaactg ctatacactc actgactgag agactgtacg taggggggcc 120
catgacaaac agcaaggggc aatcctgcgg gtacaggcgt tgccgcgcga gcggagtgct 180
caccaccagc atgggcaaca cgctcacgtg ctacgtgaaa gccagagcgg cgtgtaacgc 240
cgcgggcatt gttgctccca ccatgttggt gtgcggcgac gacctggttg tcatctcaga 300
gagtcagggg gtcgaggaag atgagcggaa cctgagagtc 340

<210> 71
<211> 340
<212> DNA
<213> HCV

<400> 71
ctctacagtc acggagaggg acatcagaac cgaggagtcc atctatctgt cctgttcact 60
gcctgaggag gctcgaactg ccatacactc actgactgag aggctgtacg taggggggcc 120
catgacaaac agcaaagggc aatcctgcgg gtacaggcgt tgccgcgcga gcggagtgct 180
caccaccagc atgggtaaca cactcacgtg ctacgtgaaa gctaaagcgg catgtaacgc 240
cgcgggcatt gttgccccca ccatgttggt gtgcggcgac gacctagtcg tcatctcaga 300
gagtcaaggg gtcgaggagg atgagcgaaa cctgagagct 340

<210> 72
<211> 340
<212> DNA
<213> HCV

<400> 72
ctcaaccgtc acggagaggg acataagaac agaagaatcc atatatcagg gttgttccct 60
gcctcaggag gctagaactg ctatccactc gctcactgag agactctacg taggagggcc 120
catgacaaac agcaagggac aatcctgcgg ttacaggcgt tgccgcgcca gcggggtctt 180
caccaccagc atggggaata ccatgacatg ctacatcaaa gcccttgcag cgtgcaaagc 240
tgcagggatc gtggacccta tcatgctggt gtgtggagac gacctggtcg tcatctcgga 300
gagcgaaggt aacgaggagg acgagcgaaa cctgagagct 340

<210> 73
<211> 340
<212> DNA
<213> HCV

<400> 73
ctcgaccgtt accgaacatg acataatgac tgaagagtct atttaccaat cattgtactt 60
gcagcctgag gcgcgtgtgg caatacggtc actcacccaa cgcctgtact gtggaggccc 120
catgtataac agcaaggggc aacaatgtgg ttatcgtaga tgccgcgcca gcggcgtctt 180
caccactagt atgggcaaca ccatgacgtg ctacattaag gctttagcct cctgtagagc 240
cgcaaagctc caggactgca cgctcctggt gtgtggtgat gatcttgtgg ccatttgcga 300
gagccagggg acgcacgagg ataaagcgag cctgagagcc 340

<210> 74
<211> 340
<212> DNA
<213> HCV

<400> 74
ctcgaccgtt accgaacatg acataatgac tgaagagtcc atttaccaat cattgtactt 60
gcagcctgag gcacgcgcgg caatacggtc actcacccaa cgcctgtact gtggaggccc 120
catgtataac agcaaggggc aacaatgtgg ttaccgtaga tgccgcgcca gcggcgtctt 180
caccaccagt atgggcaaca ccatgacgtg ctacatcaag gcttcagccg cctgtagagc 240
tgcaaagctc caggactgca cgctcctggt gtgtggtgtg accttggtgg ccatttgcga 300
gagccaaggg acgcacgagg atgaagcgtg cctgagagtc 340

<210> 75
<211> 340
<212> DNA

<213> HCV

<400> 75
ctctactgtc actgaacagg acatcagggt ggaagaggag atataccagt gctgtaacct 60
tgaaccggag gccaggaaag tgatctcctc cctcacggag cggctttact gcgggggccc 120
tatgttcaac agcaaggggg cccagtgtgg ttatcgccgt tgccgtgcta gtggagtcct 180
gcctaccagc ttcggcaaca caatcacttg ttacatcaag gctagagcgg cttcgaaggc 240
cgcaggcctc cggaacccgg actttcttgt ctgcggagat gatctggtcg tggtggctga 300
gagtgatggc gtcgacgagg atagagcagc cctgagagcc 340

<210> 76
<211> 340
<212> DNA
<213> HCV

<400> 76
ctcgactgtc actgaacagg acatcagggt ggaagaggag atataccaat gctgtaacct 60
tgaaccggag gccaggaaag tgatctcctc cctcacggag cggctttact gcgggggccc 120
tatgttcaat agcaaggggg cccagtgtgg ttatcgccgt tgccgtgcta gtggagttct 180
gcctaccagc ttcggcaaca caatcacttg ttacatcaag gctagagcgg ctgcgaaggc 240
cgcagggctc cggaccccgg actttctcgt ctgcggagat gatctggttg tggtggctga 300
gagtgatggc gtcgacgagg atagaacagc cctgcgagcc 340

<210> 77
<211> 340
<212> DNA
<213> HCV

<400> 77
ctcaactgtc actgaacagg acatcagggt ggaagaggag atataccaat gctgtaacct 60
tgaaccggag gccaggaaag tgatctcctc cctcacggaa cggctttact gcgggggccc 120
tatgttcaac agcaaggggg cccagtgtgg ttatcgccgt tgccgtgcca gtggagttct 180
gcctaccagc ttcggcaaca caatcacttg ttacatcaaa gctagagcgg ctgccgaagc 240
cgcaggcctc cggaacccgg actttcttgt ctgcggagat gatctggttg tggtggctga 300
gagtgatggc gtcaatgagg atagagcagc cctgggagcc 340

<210> 78
<211> 100
<212> DNA
<213> HCV

<400> 78
gacggcgttg gtaatggctc agctgctccg gatcccacaa gccatcttgg acatgatcgc 60
tggtgctcac tggggagtcc tggcgggcat agcgtatttc 100

<210> 79
<211> 100
<212> DNA
<213> HCV

<400> 79
gacggcgttg gtggtagctc aggtactccg gatcccacaa gccatcatgg acatgatcgc 60
tggagcccac tggggagtcc tggcgggcat agcgtatttc 100

<210> 80
<211> 100
<212> DNA
<213> HCV

<400> 80
aacggcgctg gtagtagctc agctgctcag ggtcccgcaa gccatcgtgg acatgatcgc 60
tggtgcccac tggggagtcc tagcgggcat agcgtatttt 100

<210> 81
<211> 100
<212> DNA
<213> HCV

<400> 81
gacagcccta gtggtatcgc agttactccg gatcccacaa gccgtcatgg atatggtggc 60
gggggcccac tggggagtcc tggcgggcct tgcctactat 100

<210> 82
<211> 100
<212> DNA
<213> HCV

<400> 82
agcagcccta gtggtgtcgc agttactccg gatcccacaa agcatcgtgg acatggtggc 60
gggggcccac tggggagtcc tggcgggcct tgcttactat 100

<210> 83
<211> 100
<212> DNA
<213> HCV

<400> 83
ggcagcccta gtggtgtcgc agttactccg gatcccgcaa gctgtcgtgg acatggtggc 60
gggggcccac tggggaatcc tagcgggtct tgcctactat 100

<210> 84
<211> 100
<212> DNA
<213> HCV

<400> 84
tgtgggtatg gtggtggcgc acgtcctgcg tttgccccag accttgttcg acataatagc 60
cggggcccat tggggcatct tggcgggctt ggcctattac 100

<210> 85
<211> 100
<212> DNA
<213> HCV

<400> 85
tgtgggtatg gtggtagcac acgtcctgcg tctgccccag accttgttcg acataatagc 60
cggggcccat tggggcatct tggcaggcct agcctattac 100

<210> 86
<211> 100
<212> DNA
<213> HCV

<400> 86
tgtgggtatg gtggtggcgc aagtcctgcg tttgccccag accttgttcg acgtgctagc 60
cggggcccat tggggcatct tggcgggcct ggcctattac 100

<210> 87
<211> 100
<212> DNA
<213> HCV

<400> 87
taccactatg ctcctggcat acttggtgcg catcccggag gtcatcctgg acattatcac 60
gggaggacac tggggcgtga tgtttggcct ggcttatttc 100

<210> 88
<211> 252
<212> DNA
<213> HCV

<400> 88
gttagtatga gtgtcgtgca gcctccagga ccccccctcc cgggagagcc atagtggtct 60
gcggaaccgg tgagtacacc ggaattgcca ggacgaccgg gtcctttctt ggatcaaccc 120
gctcaatgcc tggagatttg ggcgtgcccc cgcaagactg ctagccgagt agtgttgggt 180
cgcgaaaggc cttgtggtac tgcctgatag ggtgcttgcg agtgccccgg gaggtctcgt 240
agaccgtgca cc 252

<210> 89
<211> 252
<212> DNA
<213> HCV

<400> 89
gttagtatga gtgtcgtgca gcctccagga ccccccctcc cgggagagcc atagtggtct 60
gcggaaccgg tgagtacacc ggaattgcca ggacgaccgg gtcctttctt ggatcaaccc 120
gctcaatgcc tggagatttg ggcgtgcccc cgcaagactg ctagccgagt agtgttgggt 180
cgcgaaaggc cttgtggtac tgcctgatag ggtgcttgcg agtgccccgg gaggtctcgt 240
agaccgtgca cc 252

<210> 90
<211> 252
<212> DNA
<213> HCV

<400> 90
gttagtatga gtgtcgtgca gcctccagga ccccccctcc cgggagagcc atagtggtct 60
gcggaaccgg tgagtacacc ggaattgcca ggacgaccgg gtcctttctt ggatcaaccc 120
gctcaatgcc tggagatttg ggcacgcccc cgcaagatca ctagccgagt agtgttgggt 180
cgcgaaaggc cttgtggtac tgcctgatag ggtgcttgcg agtgccccgg gaggtctcgt 240
agaccgtgca cc 252

<210> 91
<211> 252
<212> DNA
<213> HCV

<400> 91
gttagtatga gtgtcgtgca gcctccagga ccccccctcc cgggagagcc atagtggtct 60
gcggaaccgg tgagtacacc ggaattgcca ggacgaccgg gtcctttctt ggataaaccc 120
gctcaatgcc tggagatttg ggcgtgcccc cgcgagactg ctagccgagt agtgttgggt 180
cgcgaaaggc cttgtggtac tgcctgatag ggtgcttgcg agtgccccgg gaggtctcgt 240
agaccgtgca cc 252

<210> 92
<211> 252
<212> DNA
<213> HCV

<400> 92
gttagtatga gtgtcgtgca gcctccagga ccccccctcc cgggagagcc atagtggtct 60
gcggaaccgg tgagtacacc ggaattgcca ggacgaccgg gtcctttctt ggatcaaccc 120
gctcaatgcc tggagatttg ggcgtgcccc cgcaagactg ctagccgagt agtgttgggt 180
cgcgaaaggc cttgtggtac tgcctgatag ggtgcttgcg agtgccccgg gaggtctcgt 240
agaccgtgca cc 252

<210> 93
<211> 252
<212> DNA

<213> HCV

<400> 93
gttagtatga gtgtcgtgca gcctccagga ccccccctcc cgggagagcc atagtggtct 60
gcggaaccgg tgagtacacc ggaattgcca ggacgaccgg gtcctttctt ggataaaccc 120
gctcaatgcc tggagatttg ggcgtgcccc cgcaagactg ctagccgagt agtgttgggt 180
cgcgaaaggc cttgtggtac tgcctgatag ggtgcttgcg agtgccccgg gaggtctcgt 240
agaccgtgca cc 252

<210> 94
<211> 252
<212> DNA
<213> HCV

<400> 94
gttagtatga gtgtcgtgca gcctccagga ccccccctcc cgggagagcc atagtggtct 60
gcggaaccgg tgagtacacc ggaattgcca ggacgaccgg gtcctttctt ggatcaaccc 120
gctcaatgcc tggagatttg ggcgtgcccc cgcgagactg ctagccgagt agtgttgggt 180
cgcgaaaggc cttgtggtac tgcctgatag ggtgcttgcg agtgccccgg gaggtctcgt 240
agaccgtgca cc 252

<210> 95
<211> 252
<212> DNA
<213> HCV

<400> 95
gttagtatga gtgtcgtgca gcctccagga ccccccctcc cgggagagcc atagtggtct 60
gcggaaccgg tgagtacacc ggaattgcca ggacgaccgg gtcctttctt ggatcaaccc 120
gctcaatgcc tggagatttg ggcgtgcccc cgcgagactg ctagccgagt agtgttgggt 180
cgcgaaaggc cttgtggtac tgcctgatag ggtgcttgcg agtgccccgg gaggtctcgt 240
agaccgtgca tc 252

<210> 96
<211> 252
<212> DNA
<213> HCV

<400> 96
gttagtatga gtgtcgtgca gcctccagga ccccccctcc cgggagagcc atagtggtct 60
gcggaaccgg tgagtacacc ggaattgcca ggacgaccgg gtcctttctt ggatcaaccc 120
gctcaatgcc tggagatttg ggcgtgcccc cgcgagactg ctagccgagt agtgttgggt 180
cgcgaaaggc cttgtggtac tgcctgatag ggtgcttgcg agtgccccgg gaggtctcgt 240
agaccgtgca cc 252

<210> 97
<211> 252
<212> DNA
<213> HCV

<400> 97
gttagtatga gtgtcgtgca gcctccagga ccccccctcc cgggagagcc atagtggtct 60
gcggaaccgg tgagtacacc ggaattgcca ggacgaccgg gtcctttctt ggatcaaccc 120
gctcaatgcc tggagatttg ggcgtgcccc cgcgagactg ctagccgagt agtgttgggt 180
cgcgaaaggc cttgtggtac tgcctgatag ggtgcttgcg agtgccccgg gaggtctcgt 240
agaccgtgca cc 252

<210> 98
<211> 252
<212> DNA
<213> HCV

<400> 98

gttagtatga gtgtcgtgca gcctccagga ccccccctcc cgggagagcc atagtggtct 60
gcggaaccgg tgagtacacc ggaattgcca ggacgaccgg gtcctttctt ggatcaaccc 120
gctcaatgcc tggagatttg ggcgtgcccc cgcgagactg ctagccgagt agtgttgggt 180
cgcgaaaggc cttgtggtac tgcctgatag ggtgcttgcg agtgccccgg gaggtctcgt 240
agaccgtgca cc 252

<210> 99
<211> 252
<212> DNA
<213> HCV

<400> 99
gttagtatga gtgtcgtgca gcctccagga ccccccctcc cgggagagcc atagtggtct 60
gcggaaccgg tgagtacacc ggaattgcca ggacgaccgg gtcctttctt ggatcaaccc 120
gctcaatgcc tggagatttg ggcgtgcccc cgcgagactg ctagccgagt agtgttgggt 180
cgcgaaaggc cttgtggtac tgcctgatag ggtgcttgcg agtgccccgg gaggtctcgt 240
agaccgtgca cc 252

<210> 100
<211> 252
<212> DNA
<213> HCV

<400> 100
gttagtatga gtgtcgtgca gcctccagga ccccccctcc cgggagagcc atagtggtct 60
gcggaaccgg tgagtacacc ggaattgcca ggacgaccgg gtcctttctt ggatcaaccc 120
gctcaatgcc tggagatttg ggcgtgcccc cgcgagactg ctagccgagt agtgttgggt 180
cgcgaaaggc cttgtggtac tgcctgatag ggtgcttgcg agtgccccgg gaggtctcgt 240
agaccgtgca cc 252

<210> 101
<211> 252
<212> DNA
<213> HCV

<400> 101
gctagtatca gtgtcgtaca gcctccaggc ccccccctcc cgggagagcc atagtggtct 60
gcggaaccgg tgagtacacc ggaattgccg ggaagactgg gtcctttctt ggataaaccc 120
actctatgcc cggccatttg ggcgtgcccc cgcaagactg ctagccgagt agcgttgggt 180
tgcgaaaggc cttgtggtac tgcctgatag ggtgcttgcg agtgccccgg gaggtctcgt 240
agaccgtgca tc 252

<210> 102
<211> 252
<212> DNA
<213> HCV

<400> 102
gttagtatga gtctcgtaca gcctccaggc ccccccctcc cgggagagcc atagtggtct 60
gcggaaccgg tgagtacacc ggaattgctg ggaagactgg gtcctttctt ggataaaccc 120
actctatgcc cagccatttg ggcgtgcccc cgcaagactg ctagccgagt agcgttgggt 180
tgcgaaaggc cttgtggtac tgcctgatag ggtgcttgcg agtgccccgg gaggtctcgt 240
agaccgtgca tc 252

<210> 103
<211> 252
<212> DNA
<213> HCV

<400> 103
gttagtacga gtgtcgtgca gcctccagga ctccccctcc cgggagagcc atagtggtct 60
gcggaaccgg tgagtacacc ggaatcgctg gggtgaccgg gtcctttctt ggagcaaccc 120
gctcaatacc cagaaatttg ggcgtgcccc cgcgagatca ctagccgagt agtgttgggt 180

cgcgaaaggc cttgtggtac tgcctgatag ggtgcttgcg agtgccccgg gaggtctcgt 240
agaccgtgca ac 252

<210> 104
<211> 252
<212> DNA
<213> HCV

<400> 104
gttagtacga gtgtcgtgca gcctccagga ccccccctcc cgggagagcc atagtggtct 60
gcggaaccgg tgagtacacc ggaatcgctg gggtgaccgg gtcctttctt ggagtaaccc 120
gctcaatacc cagaaatttg ggcgtgcccc cgcgagatca ctagccgagt agtgttgggt 180
cgcgaaaggc cttgtggtac tgcctgatag ggtgcttgcg agtgccccgg gaggtctcgt 240
agaccgtgca ac 252

<210> 105
<211> 180
<212> DNA
<213> HCV

<400> 105
gttagtatga gtgtcgaaca gcctccagga ccccccctcc cgggagagcc atagtggtct 60
gcggaaccgg tgagtacacc ggaattgccg ggatgaccgg gtcctttctt ggataaaccc 120
gctcaatgcc cggagatttg ggcgtgcccc cgcgagactg ctagccgagt agtgttgggt 180

<210> 106
<211> 180
<212> DNA
<213> HCV

<400> 106
gttagtatga gtgtcgaaca gcctccagga ccccccctcc cgggagagcc atagtggtct 60
gcggaaccgg tgagtacacc ggaattgccg ggatgaccgg gtcctttctt ggataaaccc 120
gctcaatgcc cggagatttg ggcgtgcccc cgcgagactg ctagccgagt agtgttgggt 180

<210> 107
<211> 549
<212> DNA
<213> HCV

<400> 107
   atgagcacga atcctaaacc tcaaaaaaaa aacaaacgta acaccaaccg tcgcccacag 60
   gacgtcaagt tcccgggtgg cggtcagatc gttggtggag tttacttgtt gccgcgcagg 120
   ggccctagat tgggtgtgcg cgcgacgaga aagacttccg agcggtcgca acctcgaggt 180
   agacgtcagc ctatccccaa ggctcgtcgg cccgagggca ggacctgggc tcagcccggg 240
   tacccttggc ccctctatgg caatgagggc tgcgggtggg cgggatggct cctgtctccc 300
   cgtggctctc ggcctagctg gggccccaca gacccccggc gtaggtcgcg caatttgggt 360
   aaggtcatcg atacccttac gtgcggcttc gccgacctca tggggtacat accgctcgtc 420
   ggcgcccctc ttggaggcgc tgccagggcc ctggcgcatg gcgtccgggt tctggaagac 480
   ggcgtgaact atgcaacagg gaaccttcct ggttgctctt tctctatctt ccttctggcc 540
   ctgctctct 549

<210> 108
<211> 549
<212> DNA
<213> HCV

<400> 108
   atgagcacga atcctaaacc tcaaagaaaa accaaacgta acaccaaccg tcgcccacag 60
   gacgtcaagt tcccgggtgg cggtcagatc gttggtggag tttacttgtt gccgcgcagg 120
   ggccctagat tgggtgtgcg cgcgacgagg aagacttccg agcggtcgca acctcgaggt 180
   agacgtcagc ctatccccaa ggcgcgtcgg cccgagggca ggacctgggc tcagcccggg 240
   tacccttggc ccctctatgg caatgagggt tgcgggtggg cgggatggct cctgtctccc 300
   cgtggctctc ggcctagttg gggccccaca gacccccggc gtaggtcgcg caatttgggt 360
   aaggtcatcg atacccttac gtgcggcttc gccgaccaca tggggtacat accgctcgtc 420
   ggcgcccctc ttggaggcgc tgccagggct ctggcgcatg gcgtccgggt tctggaagac 480
   ggcgtgaact atgcaacagg gaaccttcct ggttgctctt tctctatctt ccttctggcc 540
   ctgctctct 549

<210> 109
<211> 549
<212> DNA
<213> HCV

<400> 109
atgagcacga atcctaaacc tcaaagaaaa accaaacgta acaccaaccg tcgcccacag 60
gacgttaagt tcccgggtgg cggtcagatc gttggtggag tttacttgtt gccgcgcagg 120
ggccctagat tgggtgtgcg cgcgacgagg aagacttccg agcggtcgca acctcgaggt 180
agacgtcagc ctatccctaa ggcgcgtcgg cccgagggca ggacctgggc tcagcccggg 240
tacccctggc ccctctatgg taatgagggt tgcggatggg cgggatggct cctgtccccc 300
cgtggctctc ggcctagttg gggccctaca gacccccggc gtaggtcgcg caatttgggt 360
aaggtcatcg ataccctcac gtgcggcttc gccgaccaca tggggtacat tccgctcgtt 420
ggcgcccctc ttgggggcgc tgccagggcc ctggcgcatg gcgtccgggt tctggaagac 480
ggcgtgaact atgcaacagg gaatcttcct ggttgctctt tctctatctt ccttctggcc 540
cttctctct 549

<210> 110
<211> 549
<212> DNA
<213> HCV

<400> 110
atgagcacga atcctaaacc tcaaagaaaa accaaacgta acaccaaccg tcgcccacag 60
gacgtcaagt tcccgggtgg cggtcagatc gttggtggag tttacttgtt gccgcgcagg 120
ggccctagat tgggtgtgcg cacgacgagg aagacttccg agcggtcgca acctcgaggt 180
agacgtcagc ccatccccaa ggctcgtcga cccgagggca ggacctgggc tcagcccggg 240
tacccttggc ccctctatgg caatgagggc tgcgggtggg cgggatggct cctgtctccc 300
cgtggctctc ggcctagctg gggccccaca gacccccggc gtaggtcgcg caatttgggt 360
aaggtcatcg atacccttac gtgcggcttc gccgacctca tggggtacat accgctcgtc 420
ggcgcccctc ttggaggcgc tgccagagcc ctggcgcatg gcgtccgggt tctggaagac 480
ggcgtgaact atgcaacagg gaaccttccc ggttgctctt tctctatctt ccttctggcc 540
ctgctctct 549

<210> 111
<211> 549
<212> DNA
<213> HCV

<400> 111
atgagcacga atcctaaacc tcaaagaaga accaaacgta acaccaaccg tcgcccacag 60
gacgtcaagt tcccgggtgg cggtcagatc gttggtggag tttacttgtt gccgcgcagg 120
ggccctagat tgggtgtgcg cgcgacgagg aagacttccg agcggtcgca acctcgaggt 180
agacgtcagc ctatccccaa ggcacgtcgg cccgagggta ggacctgggc tcagcccggg 240
tacccttggc ccctctatgg caatgagggt tgcgggtggg cgggatggct cctgtctccc 300
cgcggctctc ggcctaactg gggccccaca gacccccggc gtaggtcgcg caatttgggt 360
aaggtcatcg atacccttac gtgcggcttc gccgacctca tggggtacat accgctcgtc 420
ggcgcccctc ttggaggcgc tgccagggcc ctggcgcatg gcgtccgggt tctggaagac 480
ggcgtgaact atgcaacagg gaaccttcct ggttgctctt tctctatctt ccttctggcc 540
ctgctctct 549

<210> 112
<211> 549
<212> DNA
<213> HCV

<400> 112
atgagcacga atcctaaacc tcaaagaaaa accaaacgta acaccaaccg tcgcccacag 60
gacgtcaagt tcccgggtgg cggtcagatc gttggtggag tttacttgtt gccgcgcagg 120
ggccctagat tgggtgtgcg cgcgacgagg aagacttccg agcggtcgca acctcgtggt 180
agacgccagc ctatccccaa ggcgcgtcgg cccgagggca ggacctgggc tcagcccggg 240
tacccttggc ccctctatgg caatgagggt tgcgggtggg cgggatggct cctgtctccc 300
cgtggctctc ggcctagctg gggccccaca gacccccggc gtaggtcgcg caatttgggt 360
aaggtcatcg atacccttac gtgcggcttc gccgacctca tggggtacat accgctcgtc 420
ggcgcccctc ttggaggcgc tgccagggcc ctggcgcatg gcgtccgggt tctggaagac 480
ggcgtgaact atgcaacagg gaaccttcct ggttgctctt tttctatttt ccttctggcc 540
ctgctctct 549

<210> 113
<211> 549
<212> DNA
<213> HCV

<400> 113
atgagcacga atcctaaacc tcaaagaaaa accaaacgta acaccaaccg ccgcccacag 60
gacgttaagt tcccgggcgg tggccaggtc gttggtggag tttacctgtt gccgcgcagg 120
ggccccaggt tgggtgtgcg cgcgactagg aagacttccg agcggtcgca acctcgtgga 180
aggcgacaac ctatccccaa ggctcgccag cccgagggca gggcctgggc tcagcccggg 240
tacccttggc ccctctatgg caatgagggt atgggtgggc aggatggct cctgtcaccc 300
cgtggctctc ggcctagttg gggccccacg gacccccggc gtaggtcgcg taatttgggt 360
aaggtcatcg ataccctcac atgcggcttc gccgacctca tggggtacat tccgctcgtc 420
ggcgcccccc ttaggggcgc tgccagggcc ttggcgcatg gcgtccgggt tctggaggac 480
ggcgtgaact acgcaacagg gaatctgccc ggttgctcct tttctatctt cctcttggct 540
ctgctgtcc 549

<210> 114
<211> 549
<212> DNA
<213> HCV

<400> 114
atgagcacaa atcctaaacc tcaaagaaaa accaaacgta acaccaaccg ccgcccacag 60
gacgtcaagt tcccgggcgg tggtcagatc gttggtggag tttacctgtt gccgcgcagg 120
ggccccaggt tgggtgtgcg cgcgactagg aagacttccg agcggtcgca acctcgtgga 180
aggcgacaac ctatccccaa ggctcgccag cccgagggca gggcctgggc tcagcccggg 240
tacccttggc ccctctatgg caacgagggt atgggtgggc aggatggct cctgtcaccc 300
cgtggctctc ggcctagttg gggccccacg gacccccggc gtaggtcgcg taatttgggt 360
aaggtcatcg ataccctcac atgcggcttc gccgacctca tggggtacat tccgcttgtc 420
ggcgcccccc tagggggcgc tgccagggcc ctggcacatg gtgtccgggt tctggaggac 480
ggcgtgaact atgcaacagg gaatttgccc ggttgctctt tctctatctt cctcttggct 540
ctgctgtcc 549

<210> 115
<211> 549
<212> DNA
<213> HCV

<400> 115
atgagcacaa atcctaaacc ccaaagaaaa accaaacgta acaccaaccg tcgcccacag 60
gacgtcaagt tcccgggcgg tggtcagatc gttggtggag tttacctgtt gccgcgcagg 120
ggccccaggt tgggtgtgcg cgcgactagg aagacttccg agcggtcgca acctcgtgga 180
aggcgacaac ctatccccaa ggctcgccgg cccgagggca ggtcctgggc tcagcccggg 240
tacccttggc ccctctatgg caacgagggt atgggtgggc aggatggct cctgtcaccc 300
cgcggctccc ggcctagttg gggccccacg gacccccggc gtaggtcgcg taatttgggt 360
aaggtcatcg ataccctcac atgcggcttc gccgacctca tggggtacat tccgctcgtc 420
ggcgcccccc tagggggcgc tgccagggcc ctggcacatg gtgtccgggt tctggaggac 480
ggcgtgaact atgcaacagg gaatttgcct ggttgctctt tctctatctt cctcttggct 540
ctgctgtcc 549

<210> 116
<211> 549
<212> DNA
<213> HCV

<400> 116
atgagcacga atcctaaacc tcaaagaaaa accaaacgta acaccaaccg ccgcccacag 60
gacgtcaagt tcccgggcgg tggtcagatc gttggtggag tttacttgtt gccgcgcagg 120
ggccccaggt tgggtgtgcg cgcgactagg aagacttccg agcggtcgca acctcgtgga 180
aggcgacaac ctatccccaa ggctcgccag cccgagggta gggcctgggc tcagcccggg 240
tacccttggc ccctctatgg caatgagggt atgggtgggc cagggtggct cctgtccccc 300
cgcggctccc ggcctagttg gggccccaca gacccccggc gtaggtcgcg taatttgggt 360
aaggtcatcg ataccctcac atgcggcttc gccgacctca tggggtacat tccgctcgtc 420
ggcgcccccc tagggggcgc tgccagggcc ctggcgcatg gcgtccgggt tctggaggac 480
ggcgtgaact atgcaacagg gaatctgccc ggttgctctt tctctatctt cctcttggct 540
ttgctgtcc 549

<210> 117
<211> 549
<212> DNA
<213> HCV

<400> 117
atgagcacga atcctaaacc tcaaagaaaa accaaacgta acaccaaccg ccgcccacag 60
gacgtcaagt tcccgggcgg tggtcagatc gttggtggag tttacctgtt gccgcgcagg 120
ggccccaggt tgggtgtgcg cgcgactagg aagacttccg agcggtcgca acctcgtgga 180
aggcgacaac ctatccccaa ggctcgccgg cccgagggca gggcctgggc tcagcccggg 240
tatccttggc ccctctatgg caatgagggt ctgggtgggc caggatggct cctgtcaccc 300
cgcggctctc ggcctagctg gggccctacc gacccccggc gtaggtcgcg caacttgggt 360
aaggtcatcg atacccttac gtgcggcttc gccgacctca tggggtacat tccgctcgtc 420
ggcgcccccc ttaggggcgc tgccagggcc ctggcgcatg gcgtccgggt tctggaggac 480
ggcgtgaact atgcaacagg gaatttgccc ggttgctctt tctctatctt cctcttggct 540
ttgctgtcc 549

<210> 118
<211> 549
<212> DNA
<213> HCV

<400> 118
atgagcacga atcctaaacc tcaaagaaaa accaaacgta acaccaaccg ccgcccacag 60
gacgtcaagt tcccgggcgg tggtcagatc gttggtggag tttacttgtt gccgcgcagg 120
ggccccaggt tgggtgtgcg cgcgactagg aagacttccg agcggtcgca acctcgtgga 180
aggcgacaac ctatccccaa ggctcgccgg cccgagggca gggcctgggc tcagcccggg 240
tacccttggc ccctctatgg caatgagggt atgggtgggc caggatggct cctgtcaccc 300
cgtggttctc ggcctagttg gggccccacg gacccccggc gtaggtcgcg caatttgggt 360
aagatcatcg ataccctcac gtgcggcttc gccgacctca tggggtacat tccgctcgtc 420
ggcgcccccc tagggggcgc tgccagggcc ctggcgcatg gcgtccgggt tctggaggac 480
ggcgtgaact atgcaacagg gaatctgccc ggttgctcct tttctatctt ccttctggct 540
ttgctgtcc 549

<210> 119
<211> 549
<212> DNA
<213> HCV

<400> 119
atgagcacga atcctaaacc tcaaagaaaa accaaacgta acaccaaccg ccgcccacag 60
gacgtcaagt tcccgggcgg tggtcagatc gttggtggag tttacctgtt gccgcgcagg 120
ggccccaggt tgggtgtgcg cgcgactagg aagacttccg agcggtcgca acctcgtgga 180
aggcgacaac ctatccccaa ggctcgccag cccgagggca gggcctgggc tcagcccggg 240
taccccctggc ccctctatgg caatgagggt atgggtgggc caggatggct cctgtcaccc 300

cgcggctccc ggcctagttg gggccccaaa gacccccggc gtaggtcgcg taatttgggt 360
aaggtcatcg ataccctcac atgcggcttc gccgacctca tggggtacat tccgctcgtc 420
ggcgccccct tagggggcgc tgccagggcc ctggcgcatg gcgtccgggt tctggaggac 480
ggcgtgaact atgcaacagg gaatctaccc ggttgctctt tctctatctt cctcttggct 540
ttgctgtcc 549

<210> 120
<211> 549
<212> DNA
<213> HCV

<400> 120
atgagcacaa atcctaaacc tcaaagaaaa accaaaagaa acactaaccg ccgcccacag 60
gacgtcaagt tcccgggcgg tggccagatc gttggcggag tatacttgct gccgcgcagg 120
ggcccgagat tgggtgtgcg cgcgacgagg aaaacttccg aacgatccca gccacgcgga 180
aggcgtcagc ccatccctaa agatcgtcgc accgctggca agtcctgggg aaggccagga 240
tatccttggc ccctgtatgg gaatgagggt ctcggctggg cagggtggct cctgtccccc 300
cgtggctctc gcccttcatg gggccccact gacccccggc atagatcgcg caacttgggt 360
aaggtcatcg ataccctaac gtgcggtttt gccgacctca tggggtacat tcccgtcatc 420
ggcgcccccg ttggaggcgt tgccagagct ctcgcccacg gagtgagggt tctggaggat 480
ggggtaaatt atgcaacagg gaatttgccc ggttgctctt tctctatctt tctcttagcc 540
ctcttgtct 549

<210> 121
<211> 510
<212> DNA
<213> HCV

<400> 121
   atgagcacaa atcctcaacc tcaaagaaaa accaaaagaa acactaaccg ccgcccacag 60
   gacgtcaagt tcccgggcgg tggtcagatc gttggcggag tatacttgtt gccgcgcagg 120
   ggccccaggt tgggtgtgcg cgcgacgagg aaaacttccg aacggtccca gccacgtggg 180
   aggcgccagc ccatccccaa agatcggcgc accactggca agtcctgggg gaagccagga 240
   tacccttggc ccctgtatgg gaatgagggt ctcggctggg cagggtggct cctgtccccc 300
   cgcggttctc gcccttcatg gggccccact gacccccggc atagatcacg caacttgggt 360
   aaggtcatcg ataccctaac gtgtggtttt gccgacctca tggggtacat tcccgtcggt 420
   ggtgcccccg ttggtggtgt cgccagagcc cttgcccatg gggtgagggt tctggaagac 480
   gggataaatt atgcaacagg gaatctgccc 510

<210> 122
<211> 191
<212> PRT
<213> HCV

<400> 122
Met Ser Thr Asn Pro Lys Pro Gln Arg Lys Thr Lys Arg Asn Thr Asn
1               5                   10                  15
Arg Arg Pro Gln Asp Val Lys Phe Pro Gly Gly Gly Gln Ile Val Gly
            20                  25                  30
Gly Val Tyr Leu Leu Pro Arg Arg Gly Pro Arg Leu Gly Val Arg Ala
        35                  40                  45
Thr Arg Lys Thr Ser Glu Arg Ser Gln Pro Arg Gly Arg Arg Gln Pro
    50                  55                  60
Ile Pro Lys Ala Arg Arg Pro Glu Gly Arg Ala Trp Ala Gln Pro Gly
65                  70                  75                  80
Tyr Pro Trp Pro Leu Tyr Gly Asn Glu Gly Leu Gly Trp Ala Gly Trp
                85                  90                  95
Leu Leu Ser Pro Arg Gly Ser Arg Pro Ser Trp Gly Pro Thr Asp Pro
            100                 105                 110
Arg Arg Arg Ser Arg Asn Leu Gly Lys Val Ile Asp Thr Leu Thr Cys
        115                 120                 125
Gly Phe Ala Asp Leu Met Gly Tyr Ile Pro Leu Val Gly Ala Pro Leu
    130                 135                 140

Gly Gly Ala Ala Arg Ala Leu Ala His Gly Val Arg Val Leu Glu Asp
145                 150                 155                 160
Gly Val Asn Tyr Ala Thr Gly Asn Leu Pro Gly Cys Ser Phe Ser Ile
                165                 170                 175
Phe Leu Leu Ala Leu Leu Ser Cys Leu Thr Ile Pro Ala Ser Ala
            180                 185                 190

<210> 123
<211> 632
<212> PRT
<213> HCV

<400> 123
Met Ala Pro Ile Thr Ala Tyr Ser Gln Gln Thr Arg Gly Leu Leu Gly
 1               5                  10                  15
Cys Thr Ile Thr Ser Leu Thr Gly Arg Asp Lys Asn Gln Val Glu Gly
            20                  25                  30
Glu Val Gln Val Val Ser Thr Ala Thr Gln Ser Phe Leu Ala Thr Cys
        35                  40                  45
Ile Asn Gly Val Cys Trp Thr Val Tyr His Gly Ala Gly Ser Lys Thr
    50                  55                  60
Leu Ala Gly Pro Lys Gly Pro Ile Thr Gln Met Tyr Thr Asn Val Asp
65                  70                  75                  80
Gln Asp Leu Val Gly Trp Gln Ala Pro Pro Gly Ala Arg Ser Met Thr
                85                  90                  95
Pro Cys Thr Cys Gly Ser Ser Asp Leu Tyr Leu Val Thr Arg His Ala
            100                 105                 110
Asp Val Ile Pro Val Arg Arg Arg Gly Asp Ser Arg Gly Ser Leu Leu
        115                 120                 125
Ser Pro Arg Pro Val Ser Tyr Leu Lys Gly Ser Val Gly Gly Pro Leu
    130                 135                 140
Leu Cys Pro Ser Gly His Val Val Gly Ile Phe Arg Ala Ala Val Cys
145                 150                 155                 160
Thr Arg Gly Val Ala Lys Ala Val Asp Phe Ile Pro Val Glu Ser Met
                165                 170                 175
Glu Thr Thr Met Arg Ser Pro Val Phe Thr Asp Asn Ser Ser Pro Pro
            180                 185                 190
Ala Val Pro Gln Thr Phe Gln Val Ala His Leu His Ala Pro Thr Gly
        195                 200                 205
Ser Gly Lys Ser Thr Lys Val Pro Ala Ala Tyr Ala Ala Gln Gly Tyr
    210                 215                 220
Lys Val Leu Val Leu Asn Pro Ser Val Ala Ala Thr Leu Gly Phe Gly
225                 230                 235                 240
Ala Tyr Met Ser Lys Ala His Gly Ile Asp Pro Asn Ile Arg Thr Gly
                245                 250                 255
Val Arg Thr Ile Thr Thr Gly Ala Pro Ile Thr Tyr Ser Thr Tyr Gly
            260                 265                 270
Lys Phe Leu Ala Asp Gly Gly Cys Ser Gly Gly Ala Tyr Asp Ile Ile
        275                 280                 285
Ile Cys Gln Glu Cys His Ser Thr Asp Ser Thr Thr Ile Leu Gly Ile
    290                 295                 300
Gly Thr Val Leu Asp Gln Ala Glu Thr Ala Gly Ala Arg Leu Val Val
305                 310                 315                 320
Leu Ala Thr Ala Thr Pro Pro Gly Ser Val Thr Val Pro His Pro Asn
                325                 330                 335
Ile Glu Glu Val Ala Leu Ser Asn Asn Gly Glu Ile Pro Phe Tyr Gly
            340                 345                 350
Lys Ala Ile Pro Ile Glu Ala Ile Lys Gly Gly Arg His Leu Ile Phe
        355                 360                 365
Cys His Ser Lys Lys Lys Cys Asp Glu Leu Ala Ala Lys Leu Ser Gly
    370                 375                 380
Leu Gly Leu Asn Ala Val Ala Tyr Tyr Arg Gly Leu Asp Val Ser Val

385                 390                 395                 400
Ile Pro Thr Ser Gly Asp Val Val Val Val Ala Thr Asp Ala Leu Met
                405                 410                 415
Thr Gly Phe Thr Gly Asp Phe Asp Ser Val Ile Asp Cys Asn Thr Cys
            420                 425                 430
Val Thr Gln Thr Val Asp Phe Ser Leu Asp Pro Thr Phe Thr Ile Glu
        435                 440                 445
Thr Thr Thr Val Pro Gln Asp Ala Val Ser Arg Ser Gln Arg Arg Gly
    450                 455                 460
Arg Thr Gly Arg Gly Arg Ser Gly Ile Tyr Arg Phe Val Thr Pro Gly
465                 470                 475                 480
Glu Arg Pro Ser Gly Met Phe Asp Ser Ser Val Leu Cys Glu Cys Tyr
                485                 490                 495
Asp Ala Gly Cys Ala Trp Tyr Glu Leu Thr Pro Ala Glu Thr Ser Val
            500                 505                 510
Arg Leu Arg Ala Tyr Leu Asn Thr Pro Gly Leu Pro Val Cys Gln Asp
        515                 520                 525
His Leu Glu Phe Trp Glu Ser Val Phe Thr Gly Leu Thr His Ile Asp
    530                 535                 540
Ala His Phe Leu Ser Gln Thr Lys Gln Ala Gly Asp Asn Phe Pro Tyr
545                 550                 555                 560
Leu Val Ala Tyr Gln Ala Thr Val Cys Ala Arg Ala Gln Ala Pro Pro
                565                 570                 575
Pro Ser Trp Asp Gln Met Trp Lys Cys Leu Ile Arg Leu Lys Pro Thr
            580                 585                 590
Leu His Gly Pro Thr Pro Leu Leu Tyr Arg Leu Gly Ala Val Gln Asn
        595                 600                 605
Glu Val Thr Leu Thr His Pro Ile Thr Lys Tyr Ile Met Ala Cys Met
    610                 615                 620
Ser Ala Asp Leu Glu Val Val Thr
625                 630

<210> 124
<211> 214
<212> PRT
<213> HCV

<400> 124
Met Phe Trp Ala Lys His Met Trp Asn Phe Ile Ser Gly Ile Gln Tyr
 1               5                  10                  15
Leu Ala Gly Leu Ser Thr Leu Pro Gly Asn Pro Ala Ile Ala Ser Leu
            20                  25                  30
Met Ala Phe Thr Ala Ser Ile Thr Ser Pro Leu Thr Thr Gln Asn Thr
        35                  40                  45
Leu Leu Phe Asn Ile Leu Gly Gly Trp Val Ala Ala Gln Leu Ala Pro
    50                  55                  60
Pro Ser Ala Ala Ser Ala Phe Val Gly Ala Gly Ile Ala Gly Ala Ala
65                  70                  75                  80
Val Gly Ser Ile Gly Leu Gly Lys Val Leu Val Asp Ile Leu Ala Gly
                85                  90                  95
Tyr Gly Ala Gly Val Ala Gly Ala Leu Val Ala Phe Lys Val Met Ser
            100                 105                 110
Gly Glu Val Pro Ser Thr Glu Asp Leu Val Asn Leu Leu Pro Ala Ile
        115                 120                 125
Leu Ser Pro Gly Ala Leu Val Val Gly Val Val Cys Ala Ala Ile Leu
    130                 135                 140
Arg Arg His Val Gly Pro Gly Glu Gly Ala Val Gln Trp Met Asn Arg
145                 150                 155                 160
Leu Ile Ala Phe Ala Ser Arg Gly Asn His Val Ser Pro Thr His Tyr
                165                 170                 175
Val Pro Glu Ser Asp Ala Ala Ala Arg Val Thr Gln Ile Leu Ser Ser
            180                 185                 190

Leu Thr Ile Thr Gln Leu Leu Lys Arg Leu His Gln Trp Ile Asn Glu
        195                 200                 205
Asp Cys Ser Thr Pro Cys
    210

<210> 125
<211> 591
<212> PRT
<213> HCV

<400> 125
Met Ser Met Ser Tyr Thr Trp Thr Gly Ala Leu Ile Thr Pro Cys Ala
 1               5                  10                  15
Ala Glu Glu Ser Lys Leu Pro Ile Asn Pro Leu Ser Asn Ser Leu Leu
            20                  25                  30
Arg His His Asn Met Val Tyr Ala Thr Thr Ser Arg Ser Ala Ser Leu
        35                  40                  45
Arg Gln Lys Lys Val Thr Phe Asp Arg Leu Gln Val Leu Asp Asp His
    50                  55                  60
Tyr Arg Asp Val Leu Lys Glu Met Lys Ala Lys Ala Ser Thr Val Lys
65                  70                  75                  80
Ala Lys Leu Leu Ser Ile Glu Glu Ala Cys Lys Leu Thr Pro Pro His
                85                  90                  95
Ser Ala Lys Ser Lys Phe Gly Tyr Gly Ala Lys Asp Val Arg Asn Leu
            100                 105                 110
Ser Ser Arg Ala Val Asn His Ile Arg Ser Val Trp Glu Asp Leu Leu
        115                 120                 125
Glu Asp Thr Glu Thr Pro Ile Asp Thr Thr Ile Met Ala Lys Ser Glu
    130                 135                 140
Val Phe Cys Val Gln Pro Glu Lys Gly Gly Arg Lys Pro Ala Arg Leu
145                 150                 155                 160
Asn Phe Pro Asp Leu Gly Val Arg Val Cys Glu Lys Met Ala Leu Tyr
                165                 170                 175
Asp Val Val Ser Thr Leu Pro Gln Ala Val Met Gly Ser Ser Tyr Gly
            180                 185                 190
Phe Gln Tyr Ser Pro Lys Gln Arg Val Glu Phe Leu Val Asn Thr Trp
        195                 200                 205
Lys Ser Lys Lys Cys Pro Met Gly Phe Ser Tyr Gly Thr Arg Cys Phe
    210                 215                 220
Gly Ser Thr Val Thr Glu Ser Asp Ile Arg Val Glu Glu Ser Ile Tyr
225                 230                 235                 240
Gln Cys Cys Asp Leu Ala Pro Glu Ala Arg Gln Ala Ile Arg Ser Leu
                245                 250                 255
Thr Glu Arg Leu Tyr Ile Gly Gly Pro Leu Thr Asn Ser Lys Gly Gln
            260                 265                 270
Asn Cys Gly Tyr Arg Arg Cys Arg Ala Ser Gly Val Leu Thr Thr Ser
        275                 280                 285
Cys Gly Asn Thr Leu Thr Cys Tyr Leu Lys Ala Thr Ala Ala Cys Arg
    290                 295                 300
Ala Ala Lys Leu Gln Asp Cys Thr Met Leu Val Asn Gly Asp Asp Leu
305                 310                 315                 320
Val Val Ile Cys Glu Ser Ala Gly Thr Gln Glu Asp Ala Ala Ala Leu
                325                 330                 335
Arg Ala Phe Thr Glu Ala Met Thr Arg Tyr Ser Ala Pro Pro Gly Asp
            340                 345                 350
Pro Pro Gln Pro Glu Tyr Asp Leu Glu Leu Ile Thr Ser Cys Ser Ser
        355                 360                 365
Asn Val Ser Val Ala His Asp Ala Ser Gly Lys Arg Val Tyr Tyr Leu
    370                 375                 380
Thr Arg Asp Pro Thr Thr Pro Leu Ala Arg Ala Ala Trp Glu Thr Ala
385                 390                 395                 400
Arg His Thr Pro Val Asn Ser Trp Leu Gly Asn Ile Ile Met Tyr Ala

405                 410                 415
Pro Thr Leu Trp Ala Arg Met Ile Leu Met Thr His Phe Phe Ser Ile
            420                 425                 430
Leu Leu Ala Gln Glu Gln Leu Glu Lys Ala Leu Asp Cys Gln Ile Tyr
        435                 440                 445
Gly Ala Cys Tyr Ser Ile Glu Pro Leu Asp Leu Pro Gln Ile Ile Glu
    450                 455                 460
Arg Leu His Gly Leu Ser Ala Phe Ser Leu His Ser Tyr Ser Pro Gly
465                 470                 475                 480
Glu Ile Asn Arg Val Ala Ser Cys Leu Arg Lys Leu Gly Val Pro Pro
                485                 490                 495
Leu Arg Val Trp Arg His Arg Ala Arg Ser Val Arg Ala Lys Leu Leu
            500                 505                 510
Ser Gln Gly Gly Arg Ala Ala Thr Cys Gly Arg Tyr Leu Phe Asn Trp
        515                 520                 525
Ala Val Arg Thr Lys Leu Lys Leu Thr Pro Ile Pro Ala Ala Ser Gln
    530                 535                 540
Leu Asp Leu Ser Gly Trp Phe Val Ala Gly Tyr Ser Gly Gly Asp Ile
545                 550                 555                 560
Tyr His Ser Leu Ser Arg Ala Arg Pro Arg Trp Phe Pro Leu Cys Leu
                565                 570                 575
Leu Leu Leu Ser Val Gly Val Gly Ile Tyr Leu Leu Pro Asn Arg
            580                 585                 590

<210> 126
<211> 3651
<212> DNA
<213> Seol Hantavirus (SEOV)

<400> 126
tagtagtaga ctccgcaaga aacagcagtt aaagaacaat aggatcatgt ggagtttgct 60
attactggcc gctttagttg gccaaggctt tgcattaaaa aatgtatttg acatgagaat 120
tcagttgccc cactcagtca actttgggga aacaagtgtg tcaggctata cagaatttcc 180
cccactctca ttacaggagg cagaacagct agtgccagag agctcatgca acatggacaa 240
ccaccagtca ctctcaacaa taaataaatt aaccaaggtc atatggcgga aaaaagcaaa 300
tcaggaatca gcaaaccaga attcatttga agttgtggaa agtgaagtca gctttaaagg 360
gttgtgtatg ttaaagcata gaatggttga agaatcatat agaaatagga gatcagtaat 420
ctgttatgat ctagcctgta atagtacatt ctgtaaacca actgtttata tgattgttcc 480
tatacatgct tgcaacatga tgaaaagctg tttgattggc cttggcccct acagaatcca 540
ggttgtctat gaaaggacat actgcactac gggtatattg acagaaggaa aatgctttgt 600
ccctgacaag gctgttgtca gtgcattgaa aagaggcatg tatgctatag caagcataga 660
gacaatctgc ttttttattc atcagaaagg gaatacatat aagatagtga ctgccattac 720
atcagcaatg ggctccaaat gtaataatac agatactaaa gttcaaggat attatatctg 780
tattattggt ggaaactccg cccctgtata tgcccctgct ggtgaagact tcagagcaat 840
ggaggttttt tctgggatta ttacatcacc acatggagaa gaccatgacc tacccggcga 900
agaaatcgca acgtaccaga tttcagggca gatagaggca aaaatccctc atacagtgag 960
ctccaaaaac ttaaaattga ctgcttttgc aggtattcca tcatactcat caactagtat 1020
attggctgct tcagaagatg gtcgtttcat atttagtcct ggtttatttc ctaacctaaa 1080
tcagtcagtc tgtgacaaca atgcactccc tttaatctgg aggggcctaa ttgatttaac 1140
gggatactat gaggcagtcc acccttgcaa tgtgttctgt gtcttatcag gaccaggtgc 1200
ttcatgtgag gccttttcag aaggaggtag gggcaatatt acttctccaa tgtgtctggt 1260
gtctaagcaa aatagattta gagcagctga gcagcagatt agctttgtct gccaaagagt 1320
tgatatggat attatagtgt actgtaatgg tcagaaaaaa acaatcctaa caaaaacatt 1380
agttataggc caatgtattt atactattac aagtctcttt tcactgttac caggggttgc 1440
ccattctatt gctattgagt tgtgtgttcc agggtttcat ggctgggcca cagctgcact 1500
tttgattaca ttctgcttcg gctgggtatt gattcctgca tgtacattag ctattctttt 1560
agtccttaag ttctttgcaa atatccttca tacaagcaat caagagaacc gattcaaagc 1620
cattctacgg aaaataaagg aggagtttga aaaaacaaag ggttccatgg tttgtgagat 1680
ctgtaagtat gagtgtgaaa cattaaagga attgaaggca cataacctat catgtgttca 1740
aggagagtgc ccatattgct ttacccactg tgaaccgaca gaaactgcaa ttcaggcaca 1800
ttacaaagtt tgtcaagcca cccaccgatt cagagaagat ttaaaaaaga ctgtaactcc 1860
tcaaaatatt gggccaggct gttaccgaac actaaatctt tttaggtata aaagtaggtg 1920

ttatattctg acaatgtgga ctcttcttct cattattgaa tccatcctct gggcagcaag 1980
tgcagcagaa atcccccttg tccctctctg gacagataat gctcatggcg ttgggagtgt 2040
tcctatgcat acggatcttg aattagactt ctctttgcca tccagttcta agtacacata 2100
caaaagacat ctcacaaacc cagttaatga ccaacagagt gtctcattgc atatagaaat 2160
tgaaagtcaa ggcattggtg ctgctgttca tcatcttgga cattggtatg atgcaagatt 2220
gaatctaaaa acctcatttc attgttatgg tgcctgcaca aaatatcaat acccatggca 2280
cactgcaaaa tgccattttg agaaagatta tgagtatgaa aatagctggg cttgcaaccc 2340
cccagattgc ccaggggttg gtacaggttg tactgcttgt ggattatatc tagatcaatt 2400
gaagccggta ggaacagcct ttaaaattat aagtgtaaga tacagtagaa aagtgtgcgt 2460
gcagtttggt gaagaacacc tttgtaaaac aattgatatg aatgattgct ttgtgactag 2520
gcatgccaaa atatgtataa ttgggactgt atctaagttt tctcaaggtg acactctact 2580
atttctgggg cccatggaag gaggtggtat aatctttaaa cactggtgta catctacctg 2640
tcactttgga gaccctggtg atgtcatggg tccaaaagat aaaccattta tttgccctga 2700
atttccaggg caatttagga aaaaatgtaa ctttgccaca actccagttt gtgaatatga 2760
tggaaacatt atatcaggct ataagaaagt acttgcaaca attgattctt tccaatcatt 2820
taacacaagc aatatacact tcactgatga gagaattgaa tggagagacc ctgatggcat 2880
gcttcgggat catattaata ttgttatttc taaagatatt gattttgaaa atttggctga 2940
gaatccttgt aaagtagggc tccaggcagc aaacatagaa ggtgcctggg gttcaggtgt 3000
cgggtttaca ctcacatgca aggtgtctct cacagaatgc ccaacatttc ttacatcaat 3060
aaaggcctgt gacatggcaa tttgttatgg tgcagaaagt gtgacactct cacgaggaca 3120
aaatactgtc aaaattaccg ggaaaggtgg ccatagtggt tcttcattca aatgctgtca 3180
tgggaaagaa tgttcatcaa ctggcctcca agccagtgca ccacatctgg ataaggtaaa 3240
tggtatctct gagttagaaa acgagaaagt ttatgatgac ggtgcacctg aatgtggcat 3300
tacttgttgg tttaaaaaat caggtgaatg ggttatgggt ataatcaatg ggaactgggt 3360
tgtcctaatt gtcttgtgtg tactgctgct cttttctctt atcctgttga gcatcttgtg 3420
tcctgttaga aagcataaaa aatcataaat cccacctaac aatcttcaca tcatgtatcg 3480
attttcaaac actttatcat ttagaactta acttggcact actatctgat aactgacttt 3540
catttttatt tttatatgga ttaattacta aaaaaaatac tctcttctat ctcccaatct 3600
tttattgatt caccggggtg ctgtcttgac atctggcggc gtctactact a 3651

<210> 127
<211> 1769
<212> DNA
<213> Seol Hantavirus (SEOV)

<400> 127
tagtagtaga ctccctaaag agctactaca ctaacaagaa aaatggcaac tatggaagaa 60
atccagagag aaatcactgc tcacgagggg cagcttgtga tagcacgcca gaaggtcaag 120
gatgcagaaa agcagtatga gaaggatcct gatgacttaa acaagagggc actgcatgat 180
cgggagagtg tcgcagcttc aatacaatca aaaattgatg aattgaagcg ccaacttgcc 240
gacagattgc agcagggaag aacatccggg caggaccggg atcctacagg ggtagagcca 300
ggtgatcatc ttaaggaaag atcagcacta agctacggga atacactgga cctgaatagt 360
cttgacattg atgaacctac aggacagaca gctgattggc tgaccataat tgtctatctg 420
acatcattcg tggtcccgat catcttgaag gcactgtaca tgttaacaac acgaggtagg 480
cagacttcaa aggacaacaa ggggatgagg atcagattca aggatgacag ctcatatgag 540
gatgtcaatg gaatcagaaa gcccaaacat ctgtatgtgt caatgccaaa cgcccaatcc 600
agcatgaagg ctgaagagat aacaccagga agattccgca ctgcagtatg tggactatat 660
cctgcacaga taaaggcaag gaatatggta agccctgtca tgagtgtagt tgggttcttg 720
gcactggcaa aagactggac atcgagaatt gaagaatggc tcggtgcacc ctgcaaattc 780
atggcggagt ctcttattgc cgggagttta tctgggaatc ctgtgaatcg tgactatatc 840
agacagagac aaggtgcact tgcagggatg gagccaaagg aatttcaagc cctcaggcaa 900
cattcaaagg atgctggatg tacactagtt gaacatattg agtcaccatc atcaatatgg 960
gtgtttgctg gggcccctga taggtgtcca ccaacatgct tgtttgtcgg agggatggct 1020
gaattaggtg ccttcttttc tatacttcag gatatgagga acacaatcat ggcttcaaaa 1080
actgtgggca cagctgatga aaagcttcga aagaaatcat cattctatca atcatacctc 1140
agacgcacac aatcaatggg aatacaactg gaccagagga taattgttat gtttatggtt 1200
gcctggggaa aggaggcagt ggacaacttt catctcggtg atgacatgga tccagagctt 1260
cgtagcctgg ctcagatctt gattgaccag aaagtgaagg aaatctcaaa ccaggaacct 1320
atgaaattat aagtacataa atatataatc aatactaact ataggttaag aaatactaat 1380
cattagttaa taagaatata gatttattga ataatcatat taaataatta ggtaagttaa 1440
ctagtattta gttaagttag ctaattgatt tatatgattg tcacaattaa atgtaatcat 1500
aagcacaatc actgccatgt ataatcacgg gtatacgggt ggttttcata tggggaacag 1560
ggtgggctta gggccaggtc accttaagtg accttttttt gtatatatgg atgtagattt 1620

caattgatcg aatactaatc ctactgtcct cttttctttt cctttctcct tctttactaa 1680
caacaacaaa ctacctcaca ccttaatata tactacttta ttaagttgtt aagttgtgtc 1740
tttttgggga gtaagggagt ctactacta 1769

<210> 128
<211> 3011
<212> PRT
<213> HCV-1

<400> 128
Met Ser Thr Asn Pro Lys Pro Gln Lys Lys Asn Lys Arg Asn Thr Asn
1               5                   10                  15
Arg Arg Pro Gln Asp Val Lys Phe Pro Gly Gly Gly Gln Ile Val Gly
            20                  25                  30
Gly Val Tyr Leu Leu Pro Arg Arg Gly Pro Arg Leu Gly Val Arg Ala
        35                  40                  45
Thr Arg Lys Thr Ser Glu Arg Ser Gln Pro Arg Gly Arg Arg Gln Pro
    50                  55                  60
Ile Pro Lys Ala Arg Arg Pro Glu Gly Arg Thr Trp Ala Gln Pro Gly
65                  70                  75                  80
Tyr Pro Trp Pro Leu Tyr Gly Asn Glu Gly Cys Gly Trp Ala Gly Trp
                85                  90                  95
Leu Leu Ser Pro Arg Gly Ser Arg Pro Ser Trp Gly Pro Thr Asp Pro
            100                 105                 110
Arg Arg Arg Ser Arg Asn Leu Gly Lys Val Ile Asp Thr Leu Thr Cys
        115                 120                 125
Gly Phe Ala Asp Leu Met Gly Tyr Ile Pro Leu Val Gly Ala Pro Leu
    130                 135                 140
Gly Gly Ala Ala Arg Ala Leu Ala His Gly Val Arg Val Leu Glu Asp
145                 150                 155                 160
Gly Val Asn Tyr Ala Thr Gly Asn Leu Pro Gly Cys Ser Phe Ser Ile
                165                 170                 175
Phe Leu Leu Ala Leu Leu Ser Cys Leu Thr Val Pro Ala Ser Ala Tyr
            180                 185                 190
Gln Val Arg Asn Ser Thr Gly Leu Tyr His Val Thr Asn Asp Cys Pro
        195                 200                 205
Asn Ser Ser Ile Val Tyr Glu Ala Ala Asp Ala Ile Leu His Thr Pro
    210                 215                 220
Gly Cys Val Pro Cys Val Arg Glu Gly Asn Ala Ser Arg Cys Trp Val
225                 230                 235                 240
Ala Met Thr Pro Thr Val Ala Thr Arg Asp Gly Lys Leu Pro Ala Thr
                245                 250                 255
Gln Leu Arg Arg His Ile Asp Leu Leu Val Gly Ser Ala Thr Leu Cys
            260                 265                 270
Ser Ala Leu Tyr Val Gly Asp Leu Cys Gly Ser Val Phe Leu Val Gly
        275                 280                 285
Gln Leu Phe Thr Phe Ser Pro Arg Arg His Trp Thr Thr Gln Gly Cys
    290                 295                 300
Asn Cys Ser Ile Tyr Pro Gly His Ile Thr Gly His Arg Met Ala Trp
305                 310                 315                 320
Asp Met Met Met Asn Trp Ser Pro Thr Thr Ala Leu Val Met Ala Gln
                325                 330                 335
Leu Leu Arg Ile Pro Gln Ala Ile Leu Asp Met Ile Ala Gly Ala His
            340                 345                 350
Trp Gly Val Leu Ala Gly Ile Ala Tyr Phe Ser Met Val Gly Asn Trp
        355                 360                 365
Ala Lys Val Leu Val Val Leu Leu Leu Phe Ala Gly Val Asp Ala Glu
    370                 375                 380
Thr His Val Thr Gly Gly Ser Ala Gly His Thr Val Ser Gly Phe Val
385                 390                 395                 400
Ser Leu Leu Ala Pro Gly Ala Lys Gln Asn Val Gln Leu Ile Asn Thr
                405                 410                 415
Asn Gly Ser Trp His Leu Asn Ser Thr Ala Leu Asn Cys Asn Asp Ser

            420                 425                 430
Leu Asn Thr Gly Trp Leu Ala Gly Leu Phe Tyr His His Lys Phe Asn
        435                 440                 445
Ser Ser Gly Cys Pro Glu Arg Leu Ala Ser Cys Arg Pro Leu Thr Asp
    450                 455                 460
Phe Asp Gln Gly Trp Gly Pro Ile Ser Tyr Ala Asn Gly Ser Gly Pro
465                 470                 475                 480
Asp Gln Arg Pro Tyr Cys Trp His Tyr Pro Pro Lys Pro Cys Gly Ile
                485                 490                 495
Val Pro Ala Lys Ser Val Cys Gly Pro Val Tyr Cys Phe Thr Pro Ser
            500                 505                 510
Pro Val Val Val Gly Thr Thr Asp Arg Ser Gly Ala Pro Thr Tyr Ser
        515                 520                 525
Trp Gly Glu Asn Asp Thr Asp Val Phe Val Leu Asn Asn Thr Arg Pro
    530                 535                 540
Pro Leu Gly Asn Trp Phe Gly Cys Thr Trp Met Asn Ser Thr Gly Phe
545                 550                 555                 560
Thr Lys Val Cys Gly Ala Pro Pro Cys Val Ile Gly Gly Ala Gly Asn
                565                 570                 575
Asn Thr Leu His Cys Pro Thr Asp Cys Phe Arg Lys His Pro Asp Ala
            580                 585                 590
Thr Tyr Ser Arg Cys Gly Ser Gly Pro Trp Ile Thr Pro Arg Cys Leu
        595                 600                 605
Val Asp Tyr Pro Tyr Arg Leu Trp His Tyr Pro Cys Thr Ile Asn Tyr
    610                 615                 620
Thr Ile Phe Lys Ile Arg Met Tyr Val Gly Gly Val Glu His Arg Leu
625                 630                 635                 640
Glu Ala Ala Cys Asn Trp Thr Arg Gly Glu Arg Cys Asp Leu Glu Asp
                645                 650                 655
Arg Asp Arg Ser Glu Leu Ser Pro Leu Leu Leu Thr Thr Thr Gln Trp
            660                 665                 670
Gln Val Leu Pro Cys Ser Phe Thr Thr Leu Pro Ala Leu Ser Thr Gly
        675                 680                 685
Leu Ile His Leu His Gln Asn Ile Val Asp Val Gln Tyr Leu Tyr Gly
    690                 695                 700
Val Gly Ser Ser Ile Ala Ser Trp Ala Ile Lys Trp Glu Tyr Val Val
705                 710                 715                 720
Leu Leu Phe Leu Leu Leu Ala Asp Ala Arg Val Cys Ser Cys Leu Trp
                725                 730                 735
Met Met Leu Leu Ile Ser Gln Ala Glu Ala Ala Leu Glu Asn Leu Val
            740                 745                 750
Ile Leu Asn Ala Ala Ser Leu Ala Gly Thr His Gly Leu Val Ser Phe
        755                 760                 765
Leu Val Phe Phe Cys Phe Ala Trp Tyr Leu Lys Gly Lys Trp Val Pro
    770                 775                 780
Gly Ala Val Tyr Thr Phe Tyr Gly Met Trp Pro Leu Leu Leu Leu Leu
785                 790                 795                 800
Leu Ala Leu Pro Gln Arg Ala Tyr Ala Leu Asp Thr Glu Val Ala Ala
                805                 810                 815
Ser Cys Gly Gly Val Val Leu Val Gly Leu Met Ala Leu Thr Leu Ser
            820                 825                 830
Pro Tyr Tyr Lys Arg Tyr Ile Ser Trp Cys Leu Trp Trp Leu Gln Tyr
        835                 840                 845
Phe Leu Thr Arg Val Glu Ala Gln Leu His Val Trp Ile Pro Pro Leu
    850                 855                 860
Asn Val Arg Gly Gly Arg Asp Ala Val Ile Leu Leu Met Cys Ala Val
865                 870                 875                 880
His Pro Thr Leu Val Phe Asp Ile Thr Lys Leu Leu Leu Ala Val Phe
                885                 890                 895
Gly Pro Leu Trp Ile Leu Gln Ala Ser Leu Leu Lys Val Pro Tyr Phe
            900                 905                 910
Val Arg Val Gln Gly Leu Leu Arg Phe Cys Ala Leu Ala Arg Lys Met
        915                 920                 925

Ile Gly Gly His Tyr Val Gln Met Val Ile Ile Lys Leu Gly Ala Leu
    930                 935                 940
Thr Gly Thr Tyr Val Tyr Asn His Leu Thr Pro Leu Arg Asp Trp Ala
945                 950                 955                 960
His Asn Gly Leu Arg Asp Leu Ala Val Ala Val Glu Pro Val Val Phe
                965                 970                 975
Ser Gln Met Glu Thr Lys Leu Ile Thr Trp Gly Ala Asp Thr Ala Ala
            980                 985                 990
Cys Gly Asp Ile Ile Asn Gly Leu Pro Val Ser Ala Arg Arg Gly Arg
        995                 1000                1005
Glu Ile Leu Leu Gly Pro Ala Asp Gly Met Val Ser Lys Gly Trp Arg
    1010                1015                1020
Leu Leu Ala Pro Ile Thr Ala Tyr Ala Gln Gln Thr Arg Gly Leu Leu
1025                1030                1035                1040
Gly Cys Ile Ile Thr Ser Leu Thr Gly Arg Asp Lys Asn Gln Val Glu
                1045                1050                1055
Gly Glu Val Gln Ile Val Ser Thr Ala Ala Gln Thr Phe Leu Ala Thr
            1060                1065                1070
Cys Ile Asn Gly Val Cys Trp Thr Val Tyr His Gly Ala Gly Thr Arg
        1075                1080                1085
Thr Ile Ala Ser Pro Lys Gly Pro Val Ile Gln Met Tyr Thr Asn Val
    1090                1095                1100
Asp Gln Asp Leu Val Gly Trp Pro Ala Pro Gln Gly Ser Arg Ser Leu
1105                1110                1115                1120
Thr Pro Cys Thr Cys Gly Ser Ser Asp Leu Tyr Leu Val Thr Arg His
                1125                1130                1135
Ala Asp Val Ile Pro Val Arg Arg Arg Gly Asp Ser Arg Gly Ser Leu
            1140                1145                1150
Leu Ser Pro Arg Pro Ile Ser Tyr Leu Lys Gly Ser Ser Gly Gly Pro
        1155                1160                1165
Leu Leu Cys Pro Ala Gly His Ala Val Gly Ile Phe Arg Ala Ala Val
    1170                1175                1180
Cys Thr Arg Gly Val Ala Lys Ala Val Asp Phe Ile Pro Val Glu Asn
1185                1190                1195                1200
Leu Glu Thr Thr Met Arg Ser Pro Val Phe Thr Asp Asn Ser Ser Pro
                1205                1210                1215
Pro Val Val Pro Gln Ser Phe Gln Val Ala His Leu His Ala Pro Thr
            1220                1225                1230
Gly Ser Gly Lys Ser Thr Lys Val Pro Ala Ala Tyr Ala Ala Gln Gly
        1235                1240                1245
Tyr Lys Val Leu Val Leu Asn Pro Ser Val Ala Ala Thr Leu Gly Phe
    1250                1255                1260
Gly Ala Tyr Met Ser Lys Ala His Gly Ile Asp Pro Asn Ile Arg Thr
1265                1270                1275                1280
Gly Val Arg Thr Ile Thr Thr Gly Ser Pro Ile Thr Tyr Ser Thr Tyr
                1285                1290                1295
Gly Lys Phe Leu Ala Asp Gly Gly Cys Ser Gly Gly Ala Tyr Asp Ile
            1300                1305                1310
Ile Ile Cys Asp Glu Cys His Ser Thr Asp Ala Thr Ser Ile Leu Gly
        1315                1320                1325
Ile Gly Thr Val Leu Asp Gln Ala Glu Thr Ala Gly Ala Arg Leu Val
    1330                1335                1340
Val Leu Ala Thr Ala Thr Pro Pro Gly Ser Val Thr Val Pro His Pro
1345                1350                1355                1360
Asn Ile Glu Glu Val Ala Leu Ser Thr Thr Gly Glu Ile Pro Phe Tyr
                1365                1370                1375
Gly Lys Ala Ile Pro Leu Glu Val Ile Lys Gly Gly Arg His Leu Ile
            1380                1385                1390
Phe Cys His Ser Lys Lys Lys Cys Asp Glu Leu Ala Ala Lys Leu Val
        1395                1400                1405
Ala Leu Gly Ile Asn Ala Val Ala Tyr Tyr Arg Gly Leu Asp Val Ser
    1410                1415                1420
Val Ile Pro Thr Ser Gly Asp Val Val Val Val Ala Thr Asp Ala Leu

1425                1430                1435                1440
Met Thr Gly Tyr Thr Gly Asp Phe Asp Ser Val Ile Asp Cys Asn Thr
                1445                1450                1455
Cys Val Thr Gln Thr Val Asp Phe Ser Leu Asp Pro Thr Phe Thr Ile
            1460                1465                1470
Glu Thr Ile Thr Leu Pro Gln Asp Ala Val Ser Arg Thr Gln Arg Arg
        1475                1480                1485
Gly Arg Thr Gly Arg Gly Lys Pro Gly Ile Tyr Arg Phe Val Ala Pro
    1490                1495                1500
Gly Glu Arg Pro Ser Gly Met Phe Asp Ser Ser Val Leu Cys Glu Cys
1505                1510                1515                1520
Tyr Asp Ala Gly Cys Ala Trp Tyr Glu Leu Thr Pro Ala Glu Thr Thr
                1525                1530                1535
Val Arg Leu Arg Ala Tyr Met Asn Thr Pro Gly Leu Pro Val Cys Gln
            1540                1545                1550
Asp His Leu Glu Phe Trp Glu Gly Val Phe Thr Gly Leu Thr His Ile
        1555                1560                1565
Asp Ala His Phe Leu Ser Gln Thr Lys Gln Ser Gly Glu Asn Leu Pro
    1570                1575                1580
Tyr Leu Val Ala Tyr Gln Ala Thr Val Cys Ala Arg Ala Gln Ala Pro
1585                1590                1595                1600
Pro Pro Ser Trp Asp Gln Met Trp Lys Cys Leu Ile Arg Leu Lys Pro
                1605                1610                1615
Thr Leu His Gly Pro Thr Pro Leu Leu Tyr Arg Leu Gly Ala Val Gln
            1620                1625                1630
Asn Glu Ile Thr Leu Thr His Pro Val Thr Lys Tyr Ile Met Thr Cys
        1635                1640                1645
Met Ser Ala Asp Leu Glu Val Val Thr Ser Thr Trp Val Leu Val Gly
    1650                1655                1660
Gly Val Leu Ala Ala Leu Ala Ala Tyr Cys Leu Ser Thr Gly Cys Val
1665                1670                1675                1680
Val Ile Val Gly Arg Val Val Leu Ser Gly Lys Pro Ala Ile Ile Pro
                1685                1690                1695
Asp Arg Glu Val Leu Tyr Arg Glu Phe Asp Glu Met Glu Glu Cys Ser
            1700                1705                1710
Gln His Leu Pro Tyr Ile Glu Gln Gly Met Met Leu Ala Glu Gln Phe
        1715                1720                1725
Lys Gln Lys Ala Leu Gly Leu Leu Gln Thr Ala Ser Arg Gln Ala Glu
    1730                1735                1740
Val Ile Ala Pro Ala Val Gln Thr Asn Trp Gln Lys Leu Glu Thr Phe
1745                1750                1755                1760
Trp Ala Lys His Met Trp Asn Phe Ile Ser Gly Ile Gln Tyr Leu Ala
                1765                1770                1775
Gly Leu Ser Thr Leu Pro Gly Asn Pro Ala Ile Ala Ser Leu Met Ala
            1780                1785                1790
Phe Thr Ala Ala Val Thr Ser Pro Leu Thr Thr Ser Gln Thr Leu Leu
        1795                1800                1805
Phe Asn Ile Leu Gly Gly Trp Val Ala Ala Gln Leu Ala Ala Pro Gly
    1810                1815                1820
Ala Ala Thr Ala Phe Val Gly Ala Gly Leu Ala Gly Ala Ala Ile Gly
1825                1830                1835                1840
Ser Val Gly Leu Gly Lys Val Leu Ile Asp Ile Leu Ala Gly Tyr Gly
                1845                1850                1855
Ala Gly Val Ala Gly Ala Leu Val Ala Phe Lys Ile Met Ser Gly Glu
            1860                1865                1870
Val Pro Ser Thr Glu Asp Leu Val Asn Leu Leu Pro Ala Ile Leu Ser
        1875                1880                1885
Pro Gly Ala Leu Val Val Gly Val Val Cys Ala Ala Ile Leu Arg Arg
    1890                1895                1900
His Val Gly Pro Gly Glu Gly Ala Val Gln Trp Met Asn Arg Leu Ile
1905                1910                1915                1920
Ala Phe Ala Ser Arg Gly Asn His Val Ser Pro Thr His Tyr Val Pro
                1925                1930                1935

Glu Ser Asp Ala Ala Ala Arg Val Thr Ala Ile Leu Ser Ser Leu Thr
            1940                1945                1950
Val Thr Gln Leu Leu Arg Arg Leu His Gln Trp Ile Ser Ser Glu Cys
        1955                1960                1965
Thr Thr Pro Cys Ser Gly Ser Trp Leu Arg Asp Ile Trp Asp Trp Ile
    1970                1975                1980
Cys Glu Val Leu Ser Asp Phe Lys Thr Trp Leu Lys Ala Lys Leu Met
1985                1990                1995                2000
Pro Gln Leu Pro Gly Ile Pro Phe Val Ser Cys Gln Arg Gly Tyr Lys
                2005                2010                2015
Gly Val Trp Arg Val Asp Gly Ile Met His Thr Arg Cys His Cys Gly
            2020                2025                2030
Ala Glu Ile Thr Gly His Val Lys Asn Gly Thr Met Arg Ile Val Gly
        2035                2040                2045
Pro Arg Thr Cys Arg Asn Met Trp Ser Gly Thr Phe Pro Ile Asn Ala
    2050                2055                2060
Tyr Thr Thr Gly Pro Cys Thr Pro Leu Pro Ala Pro Asn Tyr Thr Phe
2065                2070                2075                2080
Ala Leu Trp Arg Val Ser Ala Glu Glu Tyr Val Glu Ile Arg Gln Val
                2085                2090                2095
Gly Asp Phe His Tyr Val Thr Gly Met Thr Thr Asp Asn Leu Lys Cys
            2100                2105                2110
Pro Cys Gln Val Pro Ser Pro Glu Phe Phe Thr Glu Leu Asp Gly Val
        2115                2120                2125
Arg Leu His Arg Phe Ala Pro Pro Cys Lys Pro Leu Leu Arg Glu Glu
    2130                2135                2140
Val Ser Phe Arg Val Gly Leu His Glu Tyr Pro Val Gly Ser Gln Leu
2145                2150                2155                2160
Pro Cys Glu Pro Glu Pro Asp Val Ala Val Leu Thr Ser Met Leu Thr
                2165                2170                2175
Asp Pro Ser His Ile Thr Ala Glu Ala Ala Gly Arg Arg Leu Ala Arg
            2180                2185                2190
Gly Ser Pro Pro Ser Val Ala Ser Ser Ser Ala Ser Gln Leu Ser Ala
        2195                2200                2205
Pro Ser Leu Lys Ala Thr Cys Thr Ala Asn His Asp Ser Pro Asp Ala
    2210                2215                2220
Glu Leu Ile Glu Ala Asn Leu Leu Trp Arg Gln Glu Met Gly Gly Asn
2225                2230                2235                2240
Ile Thr Arg Val Glu Ser Glu Asn Lys Val Val Ile Leu Asp Ser Phe
                2245                2250                2255
Asp Pro Leu Val Ala Glu Glu Asp Glu Arg Glu Ile Ser Val Pro Ala
            2260                2265                2270
Glu Ile Leu Arg Lys Ser Arg Arg Phe Ala Gln Ala Leu Pro Val Trp
        2275                2280                2285
Ala Arg Pro Asp Tyr Asn Pro Pro Leu Val Glu Thr Trp Lys Lys Pro
    2290                2295                2300
Asp Tyr Glu Pro Pro Val Val His Gly Cys Pro Leu Pro Pro Pro Lys
2305                2310                2315                2320
Ser Pro Pro Val Pro Pro Pro Arg Lys Lys Arg Thr Val Val Leu Thr
                2325                2330                2335
Glu Ser Thr Leu Ser Thr Ala Leu Ala Glu Leu Ala Thr Arg Ser Phe
            2340                2345                2350
Gly Ser Ser Ser Thr Ser Gly Ile Thr Gly Asp Asn Thr Thr Thr Ser
        2355                2360                2365
Ser Glu Pro Ala Pro Ser Gly Cys Pro Pro Asp Ser Asp Ala Glu Ser
    2370                2375                2380
Tyr Ser Ser Met Pro Pro Leu Glu Gly Glu Pro Gly Asp Pro Asp Leu
2385                2390                2395                2400
Ser Asp Gly Ser Trp Ser Thr Val Ser Ser Glu Ala Asn Ala Glu Asp
                2405                2410                2415
Val Val Cys Cys Ser Met Ser Tyr Ser Trp Thr Gly Ala Leu Val Thr
            2420                2425                2430
Pro Cys Ala Ala Glu Glu Gln Lys Leu Pro Ile Asn Ala Leu Ser Asn

2435                2440                2445
Ser Leu Leu Arg His His Asn Leu Val Tyr Ser Thr Thr Ser Arg Ser
    2450                2455                2460
Ala Cys Gln Arg Gln Lys Lys Val Thr Phe Asp Arg Leu Gln Val Leu
2465                2470                2475                2480
Asp Ser His Tyr Gln Asp Val Leu Lys Glu Val Lys Ala Ala Ala Ser
                2485                2490                2495
Lys Val Lys Ala Asn Leu Leu Ser Val Glu Glu Ala Cys Ser Leu Thr
            2500                2505                2510
Pro Pro His Ser Ala Lys Ser Lys Phe Gly Tyr Gly Ala Lys Asp Val
        2515                2520                2525
Arg Cys His Ala Arg Lys Ala Val Thr His Ile Asn Ser Val Trp Lys
    2530                2535                2540
Asp Leu Leu Glu Asp Asn Val Thr Pro Ile Asp Thr Thr Ile Met Ala
2545                2550                2555                2560
Lys Asn Glu Val Phe Cys Val Gln Pro Glu Lys Gly Gly Arg Lys Pro
                2565                2570                2575
Ala Arg Leu Ile Val Phe Pro Asp Leu Gly Val Arg Val Cys Glu Lys
            2580                2585                2590
Met Ala Leu Tyr Asp Val Val Thr Lys Leu Pro Leu Ala Val Met Gly
        2595                2600                2605
Ser Ser Tyr Gly Phe Gln Tyr Ser Pro Gly Gln Arg Val Glu Phe Leu
    2610                2615                2620
Val Gln Ala Trp Lys Ser Lys Lys Thr Pro Met Gly Phe Ser Tyr Asp
2625                2630                2635                2640
Thr Arg Cys Phe Asp Ser Thr Val Thr Glu Ser Asp Ile Arg Thr Glu
                2645                2650                2655
Glu Ala Ile Tyr Gln Cys Cys Asp Leu Asp Pro Gln Ala Arg Val Ala
            2660                2665                2670
Ile Lys Ser Leu Thr Glu Arg Leu Tyr Val Gly Gly Pro Leu Thr Asn
        2675                2680                2685
Ser Arg Gly Glu Asn Cys Gly Tyr Arg Arg Cys Arg Ala Ser Gly Val
    2690                2695                2700
Leu Thr Thr Ser Cys Gly Asn Thr Leu Thr Cys Tyr Ile Lys Ala Arg
2705                2710                2715                2720
Ala Ala Cys Arg Ala Ala Gly Leu Gln Asp Cys Thr Met Leu Val Cys
                2725                2730                2735
Gly Asp Asp Leu Val Val Ile Cys Glu Ser Ala Gly Val Gln Glu Asp
            2740                2745                2750
Ala Ala Ser Leu Arg Ala Phe Thr Glu Ala Met Thr Arg Tyr Ser Ala
        2755                2760                2765
Pro Pro Gly Asp Pro Pro Gln Pro Glu Tyr Asp Leu Glu Leu Ile Thr
    2770                2775                2780
Ser Cys Ser Ser Asn Val Ser Val Ala His Asp Gly Ala Gly Lys Arg
2785                2790                2795                2800
Val Tyr Tyr Leu Thr Arg Asp Pro Thr Thr Pro Leu Ala Arg Ala Ala
                2805                2810                2815
Trp Glu Thr Ala Arg His Thr Pro Val Asn Ser Trp Leu Gly Asn Ile
            2820                2825                2830
Ile Met Phe Ala Pro Thr Leu Trp Ala Arg Met Ile Leu Met Thr His
        2835                2840                2845
Phe Phe Ser Val Leu Ile Ala Arg Asp Gln Leu Glu Gln Ala Leu Asp
    2850                2855                2860
Cys Glu Ile Tyr Gly Ala Cys Tyr Ser Ile Glu Pro Leu Asp Leu Pro
2865                2870                2875                2880
Pro Ile Ile Gln Arg Leu His Gly Leu Ser Ala Phe Ser Leu His Ser
                2885                2890                2895
Tyr Ser Pro Gly Glu Ile Asn Arg Val Ala Ala Cys Leu Arg Lys Leu
            2900                2905                2910
Gly Val Pro Pro Leu Arg Ala Trp Arg His Arg Ala Arg Ser Val Arg
        2915                2920                2925
Ala Arg Leu Leu Ala Arg Gly Gly Arg Ala Ala Ile Cys Gly Lys Tyr
    2930                2935                2940

Leu Phe Asn Trp Ala Val Arg Thr Lys Leu Lys Leu Thr Pro Ile Ala
2945                2950                2955                2960
Ala Ala Gly Gln Leu Asp Leu Ser Gly Trp Phe Thr Ala Gly Tyr Ser
                2965                2970                2975
Gly Gly Asp Ile Tyr His Ser Val Ser His Ala Arg Pro Arg Trp Ile
            2980                2985                2990
Trp Phe Cys Leu Leu Leu Leu Ala Ala Gly Val Gly Ile Tyr Leu Leu
        2995                3000                3005
Pro Asn Arg
    3010

<210> 129
<211> 363
<212> PRT
<213> HCV-1

<400> 129
Glu Thr His Val Thr Gly Gly Ser Ala Gly His Thr Val Ser Gly Phe
1               5                   10                  15
Val Ser Leu Leu Ala Pro Gly Ala Lys Gln Asn Val Gln Leu Ile Asn
            20                  25                  30
Thr Asn Gly Ser Trp His Leu Asn Ser Thr Ala Leu Asn Cys Asn Asp
        35                  40                  45
Ser Leu Asn Thr Gly Trp Leu Ala Gly Leu Phe Tyr His His Lys Phe
    50                  55                  60
Asn Ser Ser Gly Cys Pro Glu Arg Leu Ala Ser Cys Arg Pro Leu Thr
65                  70                  75                  80
Asp Phe Asp Gln Gly Trp Gly Pro Ile Ser Tyr Ala Asn Gly Ser Gly
                85                  90                  95
Pro Asp Gln Arg Pro Tyr Cys Trp His Tyr Pro Pro Lys Pro Cys Gly
            100                 105                 110
Ile Val Pro Ala Lys Ser Val Cys Gly Pro Val Tyr Cys Phe Thr Pro
        115                 120                 125
Ser Pro Val Val Val Gly Thr Thr Asp Arg Ser Gly Ala Pro Thr Tyr
    130                 135                 140
Ser Trp Gly Glu Asn Asp Thr Asp Val Phe Val Leu Asn Asn Thr Arg
145                 150                 155                 160
Pro Pro Leu Gly Asn Trp Phe Gly Cys Thr Trp Met Asn Ser Thr Gly
                165                 170                 175
Phe Thr Lys Val Cys Gly Ala Pro Pro Cys Val Ile Gly Gly Ala Gly
            180                 185                 190
Asn Asn Thr Leu His Cys Pro Thr Asp Cys Phe Arg Lys His Pro Asp
        195                 200                 205
Ala Thr Tyr Ser Arg Cys Gly Ser Gly Pro Trp Ile Thr Pro Arg Cys
    210                 215                 220
Leu Val Asp Tyr Pro Tyr Arg Leu Trp His Tyr Pro Cys Thr Ile Asn
225                 230                 235                 240
Tyr Thr Ile Phe Lys Ile Arg Met Tyr Val Gly Gly Val Glu His Arg
                245                 250                 255
Leu Glu Ala Ala Cys Asn Trp Thr Arg Gly Glu Arg Cys Asp Leu Glu
            260                 265                 270
Asp Arg Asp Arg Ser Glu Leu Ser Pro Leu Leu Leu Thr Thr Thr Gln
        275                 280                 285
Trp Gln Val Leu Pro Cys Ser Phe Thr Thr Leu Pro Ala Leu Ser Thr
    290                 295                 300
Gly Leu Ile His Leu His Gln Asn Ile Val Asp Val Gln Tyr Leu Tyr
305                 310                 315                 320
Gly Val Gly Ser Ser Ile Ala Ser Trp Ala Ile Lys Trp Glu Tyr Val
                325                 330                 335
Val Leu Leu Phe Leu Leu Leu Ala Asp Ala Arg Val Cys Ser Cys Leu
            340                 345                 350
Trp Met Met Leu Leu Ile Ser Gln Ala Glu Ala

355                 360

<210> 130
<211> 366
<212> PRT
<213> HCV-1

<400> 130
Glu Thr His Val Thr Gly Gly Ser Ala Gly His Thr Val Ser Gly Phe
1               5                   10                  15
Val Ser Leu Leu Ala Pro Gly Ala Lys Gln Asn Val Gln Leu Ile Asn
            20                  25                  30
Thr Asn Gly Ser Trp His Leu Asn Ser Thr Ala Leu Asn Cys Asn Asp
        35                  40                  45
Ser Leu Asn Thr Gly Trp Leu Ala Gly Leu Phe Tyr His His Lys Phe
    50                  55                  60
Asn Ser Ser Gly Cys Pro Glu Arg Leu Ala Ser Cys Arg Pro Leu Thr
65                  70                  75                  80
Asp Phe Asp Gln Gly Trp Gly Pro Ile Ser Tyr Ala Asn Gly Ser Gly
                85                  90                  95
Pro Asp Gln Arg Pro Tyr Cys Trp His Tyr Pro Pro Lys Pro Cys Gly
            100                 105                 110
Ile Val Pro Ala Lys Ser Val Cys Gly Pro Val Tyr Cys Phe Thr Pro
        115                 120                 125
Ser Pro Val Val Val Gly Thr Thr Asp Arg Ser Gly Ala Pro Thr Tyr
    130                 135                 140
Ser Trp Gly Glu Asn Asp Thr Asp Val Phe Val Leu Asn Asn Thr Arg
145                 150                 155                 160
Pro Pro Leu Gly Asn Trp Phe Gly Cys Thr Trp Met Asn Ser Thr Gly
                165                 170                 175
Phe Thr Lys Val Cys Gly Ala Pro Pro Cys Val Ile Gly Gly Ala Gly
            180                 185                 190
Asn Asn Thr Leu His Cys Pro Thr Asp Cys Phe Arg Lys His Pro Asp
        195                 200                 205
Ala Thr Tyr Ser Arg Cys Gly Ser Gly Pro Trp Ile Thr Pro Arg Cys
    210                 215                 220
Leu Val Asp Tyr Pro Tyr Arg Leu Trp His Tyr Pro Cys Thr Ile Asn
225                 230                 235                 240
Tyr Thr Ile Phe Lys Ile Arg Met Tyr Val Gly Gly Val Glu His Arg
                245                 250                 255
Leu Glu Ala Ala Cys Asn Trp Thr Arg Gly Glu Arg Cys Asp Leu Glu
            260                 265                 270
Asp Arg Asp Arg Ser Glu Leu Ser Pro Leu Leu Leu Thr Thr Thr Gln
        275                 280                 285
Trp Gln Val Leu Pro Cys Ser Phe Thr Thr Leu Pro Ala Leu Ser Thr
    290                 295                 300
Gly Leu Ile His Leu His Gln Asn Ile Val Asp Val Gln Tyr Leu Tyr
305                 310                 315                 320
Gly Val Gly Ser Ser Ile Ala Ser Trp Ala Ile Lys Trp Glu Tyr Val
                325                 330                 335
Val Leu Leu Phe Leu Leu Leu Ala Asp Ala Arg Val Cys Ser Cys Leu
            340                 345                 350
Trp Met Met Leu Leu Ile Ser Gln Ala Glu Ala Ala Leu Glu
        355                 360                 365

<210> 131
<211> 426
<212> PRT
<213> HCV-1

<400> 131

Glu Thr His Val Thr Gly Gly Ser Ala Gly His Thr Val Ser Gly Phe
1               5                   10                  15
Val Ser Leu Leu Ala Pro Gly Ala Lys Gln Asn Val Gln Leu Ile Asn
            20                  25                  30
Thr Asn Gly Ser Trp His Leu Asn Ser Thr Ala Leu Asn Cys Asn Asp
        35                  40                  45
Ser Leu Asn Thr Gly Trp Leu Ala Gly Leu Phe Tyr His His Lys Phe
    50                  55                  60
Asn Ser Ser Gly Cys Pro Glu Arg Leu Ala Ser Cys Arg Pro Leu Thr
65                  70                  75                  80
Asp Phe Asp Gln Gly Trp Gly Pro Ile Ser Tyr Ala Asn Gly Ser Gly
                85                  90                  95
Pro Asp Gln Arg Pro Tyr Cys Trp His Tyr Pro Pro Lys Pro Cys Gly
            100                 105                 110
Ile Val Pro Ala Lys Ser Val Cys Gly Pro Val Tyr Cys Phe Thr Pro
        115                 120                 125
Ser Pro Val Val Val Gly Thr Thr Asp Arg Ser Gly Ala Pro Thr Tyr
    130                 135                 140
Ser Trp Gly Glu Asn Asp Thr Asp Val Phe Val Leu Asn Asn Thr Arg
145                 150                 155                 160
Pro Pro Leu Gly Asn Trp Phe Gly Cys Thr Trp Met Asn Ser Thr Gly
                165                 170                 175
Phe Thr Lys Val Cys Gly Ala Pro Pro Cys Val Ile Gly Gly Ala Gly
            180                 185                 190
Asn Asn Thr Leu His Cys Pro Thr Asp Cys Phe Arg Lys His Pro Asp
        195                 200                 205
Ala Thr Tyr Ser Arg Cys Gly Ser Gly Pro Trp Ile Thr Pro Arg Cys
    210                 215                 220
Leu Val Asp Tyr Pro Tyr Arg Leu Trp His Tyr Pro Cys Thr Ile Asn
225                 230                 235                 240
Tyr Thr Ile Phe Lys Ile Arg Met Tyr Val Gly Gly Val Glu His Arg
                245                 250                 255
Leu Glu Ala Ala Cys Asn Trp Thr Arg Gly Glu Arg Cys Asp Leu Glu
            260                 265                 270
Asp Arg Asp Arg Ser Glu Leu Ser Pro Leu Leu Leu Thr Thr Thr Gln
        275                 280                 285
Trp Gln Val Leu Pro Cys Ser Phe Thr Thr Leu Pro Ala Leu Ser Thr
    290                 295                 300
Gly Leu Ile His Leu His Gln Asn Ile Val Asp Val Gln Tyr Leu Tyr
305                 310                 315                 320
Gly Val Gly Ser Ser Ile Ala Ser Trp Ala Ile Lys Trp Glu Tyr Val
                325                 330                 335
Val Leu Leu Phe Leu Leu Leu Ala Asp Ala Arg Val Cys Ser Cys Leu
            340                 345                 350
Trp Met Met Leu Leu Ile Ser Gln Ala Glu Ala Ala Leu Glu Asn Leu
        355                 360                 365
Val Ile Leu Asn Ala Ala Ser Leu Ala Gly Thr His Gly Leu Val Ser
    370                 375                 380
Phe Leu Val Phe Phe Cys Phe Ala Trp Tyr Leu Lys Gly Lys Trp Val
385                 390                 395                 400
Pro Gly Ala Val Tyr Thr Phe Tyr Gly Met Trp Pro Leu Leu Leu Leu
                405                 410                 415
Leu Leu Ala Leu Pro Gln Arg Ala Tyr Ala
            420                 425

<210> 132
<211> 555
<212> PRT
<213> HCV-1

<400> 132
Tyr Gln Val Arg Asn Ser Thr Gly Leu Tyr His Val Thr Asn Asp Cys

1               5                   10                  15
Pro Asn Ser Ser Ile Val Tyr Glu Ala Ala Asp Ala Ile Leu His Thr
                20                  25                  30
Pro Gly Cys Val Pro Cys Val Arg Glu Gly Asn Ala Ser Arg Cys Trp
            35                  40                  45
Val Ala Met Thr Pro Thr Val Ala Thr Arg Asp Gly Lys Leu Pro Ala
    50                  55                  60
Thr Gln Leu Arg Arg His Ile Asp Leu Leu Val Gly Ser Ala Thr Leu
65                  70                  75                  80
Cys Ser Ala Leu Tyr Val Gly Asp Leu Cys Gly Ser Val Phe Leu Val
                85                  90                  95
Gly Gln Leu Phe Thr Phe Ser Pro Arg Arg His Trp Thr Thr Gln Gly
            100                 105                 110
Cys Asn Cys Ser Ile Tyr Pro Gly His Ile Thr Gly His Arg Met Ala
        115                 120                 125
Trp Asp Met Met Met Asn Trp Ser Pro Thr Thr Ala Leu Val Met Ala
    130                 135                 140
Gln Leu Leu Arg Ile Pro Gln Ala Ile Leu Asp Met Ile Ala Gly Ala
145                 150                 155                 160
His Trp Gly Val Leu Ala Gly Ile Ala Tyr Phe Ser Met Val Gly Asn
                165                 170                 175
Trp Ala Lys Val Leu Val Val Leu Leu Leu Phe Ala Gly Val Asp Ala
            180                 185                 190
Glu Thr His Val Thr Gly Gly Ser Ala Gly His Thr Val Ser Gly Phe
        195                 200                 205
Val Ser Leu Leu Ala Pro Gly Ala Lys Gln Asn Val Gln Leu Ile Asn
    210                 215                 220
Thr Asn Gly Ser Trp His Leu Asn Ser Thr Ala Leu Asn Cys Asn Asp
225                 230                 235                 240
Ser Leu Asn Thr Gly Trp Leu Ala Gly Leu Phe Tyr His His Lys Phe
                245                 250                 255
Asn Ser Ser Gly Cys Pro Glu Arg Leu Ala Ser Cys Arg Pro Leu Thr
            260                 265                 270
Asp Phe Asp Gln Gly Trp Gly Pro Ile Ser Tyr Ala Asn Gly Ser Gly
        275                 280                 285
Pro Asp Gln Arg Pro Tyr Cys Trp His Tyr Pro Pro Lys Pro Cys Gly
    290                 295                 300
Ile Val Pro Ala Lys Ser Val Cys Gly Pro Val Tyr Cys Phe Thr Pro
305                 310                 315                 320
Ser Pro Val Val Val Gly Thr Thr Asp Arg Ser Gly Ala Pro Thr Tyr
                325                 330                 335
Ser Trp Gly Glu Asn Asp Thr Asp Val Phe Val Leu Asn Asn Thr Arg
            340                 345                 350
Pro Pro Leu Gly Asn Trp Phe Gly Cys Thr Trp Met Asn Ser Thr Gly
        355                 360                 365
Phe Thr Lys Val Cys Gly Ala Pro Pro Cys Val Ile Gly Gly Ala Gly
    370                 375                 380
Asn Asn Thr Leu His Cys Pro Thr Asp Cys Phe Arg Lys His Pro Asp
385                 390                 395                 400
Ala Thr Tyr Ser Arg Cys Gly Ser Gly Pro Trp Ile Thr Pro Arg Cys
                405                 410                 415
Leu Val Asp Tyr Pro Tyr Arg Leu Trp His Tyr Pro Cys Thr Ile Asn
            420                 425                 430
Tyr Thr Ile Phe Lys Ile Arg Met Tyr Val Gly Gly Val Glu His Arg
        435                 440                 445
Leu Glu Ala Ala Cys Asn Trp Thr Arg Gly Glu Arg Cys Asp Leu Glu
    450                 455                 460
Asp Arg Asp Arg Ser Glu Leu Ser Pro Leu Leu Leu Thr Thr Thr Gln
465                 470                 475                 480
Trp Gln Val Leu Pro Cys Ser Phe Thr Thr Leu Pro Ala Leu Ser Thr
                485                 490                 495
Gly Leu Ile His Leu His Gln Asn Ile Val Asp Val Gln Tyr Leu Tyr
            500                 505                 510

Gly Val Gly Ser Ser Ile Ala Ser Trp Ala Ile Lys Trp Glu Tyr Val
        515                 520                 525
Val Leu Leu Phe Leu Leu Leu Ala Asp Ala Arg Val Cys Ser Cys Leu
    530                 535                 540
Trp Met Met Leu Leu Ile Ser Gln Ala Glu Ala
545                 550                 555

<210> 133
<211> 558
<212> PRT
<213> HCV-1

<400> 133
Tyr Gln Val Arg Asn Ser Thr Gly Leu Tyr His Val Thr Asn Asp Cys
 1               5                  10                  15
Pro Asn Ser Ser Ile Val Tyr Glu Ala Ala Asp Ala Ile Leu His Thr
            20                  25                  30
Pro Gly Cys Val Pro Cys Val Arg Glu Gly Asn Ala Ser Arg Cys Trp
        35                  40                  45
Val Ala Met Thr Pro Thr Val Ala Thr Arg Asp Gly Lys Leu Pro Ala
    50                  55                  60
Thr Gln Leu Arg Arg His Ile Asp Leu Leu Val Gly Ser Ala Thr Leu
65                  70                  75                  80
Cys Ser Ala Leu Tyr Val Gly Asp Leu Cys Gly Ser Val Phe Leu Val
                85                  90                  95
Gly Gln Leu Phe Thr Phe Ser Pro Arg Arg His Trp Thr Thr Gln Gly
            100                 105                 110
Cys Asn Cys Ser Ile Tyr Pro Gly His Ile Thr Gly His Arg Met Ala
        115                 120                 125
Trp Asp Met Met Met Asn Trp Ser Pro Thr Thr Ala Leu Val Met Ala
    130                 135                 140
Gln Leu Leu Arg Ile Pro Gln Ala Ile Leu Asp Met Ile Ala Gly Ala
145                 150                 155                 160
His Trp Gly Val Leu Ala Gly Ile Ala Tyr Phe Ser Met Val Gly Asn
                165                 170                 175
Trp Ala Lys Val Leu Val Val Leu Leu Leu Phe Ala Gly Val Asp Ala
            180                 185                 190
Glu Thr His Val Thr Gly Gly Ser Ala Gly His Thr Val Ser Gly Phe
        195                 200                 205
Val Ser Leu Leu Ala Pro Gly Ala Lys Gln Asn Val Gln Leu Ile Asn
    210                 215                 220
Thr Asn Gly Ser Trp His Leu Asn Ser Thr Ala Leu Asn Cys Asn Asp
225                 230                 235                 240
Ser Leu Asn Thr Gly Trp Leu Ala Gly Leu Phe Tyr His His Lys Phe
                245                 250                 255
Asn Ser Ser Gly Cys Pro Glu Arg Leu Ala Ser Cys Arg Pro Leu Thr
            260                 265                 270
Asp Phe Asp Gln Gly Trp Gly Pro Ile Ser Tyr Ala Asn Gly Ser Gly
        275                 280                 285
Pro Asp Gln Arg Pro Tyr Cys Trp His Tyr Pro Pro Lys Pro Cys Gly
    290                 295                 300
Ile Val Pro Ala Lys Ser Val Cys Gly Pro Val Tyr Cys Phe Thr Pro
305                 310                 315                 320
Ser Pro Val Val Val Gly Thr Thr Asp Arg Ser Gly Ala Pro Thr Tyr
                325                 330                 335
Ser Trp Gly Glu Asn Asp Thr Asp Val Phe Val Leu Asn Asn Thr Arg
            340                 345                 350
Pro Pro Leu Gly Asn Trp Phe Gly Cys Thr Trp Met Asn Ser Thr Gly
        355                 360                 365
Phe Thr Lys Val Cys Gly Ala Pro Pro Cys Val Ile Gly Gly Ala Gly
    370                 375                 380
Asn Asn Thr Leu His Cys Pro Thr Asp Cys Phe Arg Lys His Pro Asp

385                 390                 395                 400
Ala Thr Tyr Ser Arg Cys Gly Ser Gly Pro Trp Ile Thr Pro Arg Cys
                405                 410                 415
Leu Val Asp Tyr Pro Tyr Arg Leu Trp His Tyr Pro Cys Thr Ile Asn
            420                 425                 430
Tyr Thr Ile Phe Lys Ile Arg Met Tyr Val Gly Gly Val Glu His Arg
        435                 440                 445
Leu Glu Ala Ala Cys Asn Trp Thr Arg Gly Glu Arg Cys Asp Leu Glu
    450                 455                 460
Asp Arg Asp Arg Ser Glu Leu Ser Pro Leu Leu Leu Thr Thr Thr Gln
465                 470                 475                 480
Trp Gln Val Leu Pro Cys Ser Phe Thr Thr Leu Pro Ala Leu Ser Thr
                485                 490                 495
Gly Leu Ile His Leu His Gln Asn Ile Val Asp Val Gln Tyr Leu Tyr
            500                 505                 510
Gly Val Gly Ser Ser Ile Ala Ser Trp Ala Ile Lys Trp Glu Tyr Val
        515                 520                 525
Val Leu Leu Phe Leu Leu Leu Ala Asp Ala Arg Val Cys Ser Cys Leu
    530                 535                 540
Trp Met Met Leu Leu Ile Ser Gln Ala Glu Ala Ala Leu Glu
545                 550                 555

<210> 134
<211> 618
<212> PRT
<213> HCV-1

<400> 134
Tyr Gln Val Arg Asn Ser Thr Gly Leu Tyr His Val Thr Asn Asp Cys
1               5                   10                  15
Pro Asn Ser Ser Ile Val Tyr Glu Ala Ala Asp Ala Ile Leu His Thr
            20                  25                  30
Pro Gly Cys Val Pro Cys Val Arg Glu Gly Asn Ala Ser Arg Cys Trp
        35                  40                  45
Val Ala Met Thr Pro Thr Val Ala Thr Arg Asp Gly Lys Leu Pro Ala
    50                  55                  60
Thr Gln Leu Arg Arg His Ile Asp Leu Leu Val Gly Ser Ala Thr Leu
65                  70                  75                  80
Cys Ser Ala Leu Tyr Val Gly Asp Leu Cys Gly Ser Val Phe Leu Val
                85                  90                  95
Gly Gln Leu Phe Thr Phe Ser Pro Arg Arg His Trp Thr Thr Gln Gly
            100                 105                 110
Cys Asn Cys Ser Ile Tyr Pro Gly His Ile Thr Gly His Arg Met Ala
        115                 120                 125
Trp Asp Met Met Met Asn Trp Ser Pro Thr Thr Ala Leu Val Met Ala
    130                 135                 140
Gln Leu Leu Arg Ile Pro Gln Ala Ile Leu Asp Met Ile Ala Gly Ala
145                 150                 155                 160
His Trp Gly Val Leu Ala Gly Ile Ala Tyr Phe Ser Met Val Gly Asn
                165                 170                 175
Trp Ala Lys Val Leu Val Val Leu Leu Leu Phe Ala Gly Val Asp Ala
            180                 185                 190
Glu Thr His Val Thr Gly Gly Ser Ala Gly His Thr Val Ser Gly Phe
        195                 200                 205
Val Ser Leu Leu Ala Pro Gly Ala Lys Gln Asn Val Gln Leu Ile Asn
    210                 215                 220
Thr Asn Gly Ser Trp His Leu Asn Ser Thr Ala Leu Asn Cys Asn Asp
225                 230                 235                 240
Ser Leu Asn Thr Gly Trp Leu Ala Gly Leu Phe Tyr His His Lys Phe
                245                 250                 255
Asn Ser Ser Gly Cys Pro Glu Arg Leu Ala Ser Cys Arg Pro Leu Thr
            260                 265                 270

Asp Phe Asp Gln Gly Trp Gly Pro Ile Ser Tyr Ala Asn Gly Ser Gly
        275                 280                 285
Pro Asp Gln Arg Pro Tyr Cys Trp His Tyr Pro Pro Lys Pro Cys Gly
    290                 295                 300
Ile Val Pro Ala Lys Ser Val Cys Gly Pro Val Tyr Cys Phe Thr Pro
305                 310                 315                 320
Ser Pro Val Val Val Gly Thr Thr Asp Arg Ser Gly Ala Pro Thr Tyr
                325                 330                 335
Ser Trp Gly Glu Asn Asp Thr Asp Val Phe Val Leu Asn Asn Thr Arg
            340                 345                 350
Pro Pro Leu Gly Asn Trp Phe Gly Cys Thr Trp Met Asn Ser Thr Gly
        355                 360                 365
Phe Thr Lys Val Cys Gly Ala Pro Pro Cys Val Ile Gly Gly Ala Gly
    370                 375                 380
Asn Asn Thr Leu His Cys Pro Thr Asp Cys Phe Arg Lys His Pro Asp
385                 390                 395                 400
Ala Thr Tyr Ser Arg Cys Gly Ser Gly Pro Trp Ile Thr Pro Arg Cys
                405                 410                 415
Leu Val Asp Tyr Pro Tyr Arg Leu Trp His Tyr Pro Cys Thr Ile Asn
            420                 425                 430
Tyr Thr Ile Phe Lys Ile Arg Met Tyr Val Gly Gly Val Glu His Arg
        435                 440                 445
Leu Glu Ala Ala Cys Asn Trp Thr Arg Gly Glu Arg Cys Asp Leu Glu
    450                 455                 460
Asp Arg Asp Arg Ser Glu Leu Ser Pro Leu Leu Leu Thr Thr Thr Gln
465                 470                 475                 480
Trp Gln Val Leu Pro Cys Ser Phe Thr Thr Leu Pro Ala Leu Ser Thr
                485                 490                 495
Gly Leu Ile His Leu His Gln Asn Ile Val Asp Val Gln Tyr Leu Tyr
            500                 505                 510
Gly Val Gly Ser Ser Ile Ala Ser Trp Ala Ile Lys Trp Glu Tyr Val
        515                 520                 525
Val Leu Leu Phe Leu Leu Leu Ala Asp Ala Arg Val Cys Ser Cys Leu
    530                 535                 540
Trp Met Met Leu Leu Ile Ser Gln Ala Glu Ala Ala Leu Glu Asn Leu
545                 550                 555                 560
Val Ile Leu Asn Ala Ala Ser Leu Ala Gly Thr His Gly Leu Val Ser
                565                 570                 575
Phe Leu Val Phe Phe Cys Phe Ala Trp Tyr Leu Lys Gly Lys Trp Val
            580                 585                 590
Pro Gly Ala Val Tyr Thr Phe Tyr Gly Met Trp Pro Leu Leu Leu Leu
        595                 600                 605
Leu Leu Ala Leu Pro Gln Arg Ala Tyr Ala
    610                 615

<210> 135
<211> 211
<212> PRT
<213> HBV-1

<400> 135
Met Gln Leu Phe His Leu Cys Leu Ile Ile Ser Cys Ser Cys Pro Thr
 1               5                  10                  15
Val Gln Ala Ser Lys Leu Cys Leu Gly Trp Leu Asp Met Asp Ile Asp
            20                  25                  30
Pro Tyr Lys Glu Phe Gly Ala Thr Val Glu Leu Leu Ser Phe Leu Pro
        35                  40                  45
Ser Asp Phe Phe Pro Ser Val Arg Asp Leu Leu Asp Thr Ala Ser Ala
    50                  55                  60
Leu Tyr Arg Glu Ala Leu Glu Ser Pro Glu His Cys Ser Pro His His
65                  70                  75                  80
Thr Ala Leu Arg Gln Ala Ile Leu Cys Trp Gly Asp Leu Ile Thr Leu

85                      90                      95
Ser Thr Trp Val Gly Gly Asn Leu Glu Asp Pro Thr Ser Arg Asp Leu
            100                     105                     110
Val Val Ser Tyr Val Asn Thr Asn Met Gly Leu Lys Phe Arg Gln Leu
        115                     120                     125
Leu Trp Phe His Ile Ser Cys Leu Thr Phe Gly Arg Glu Thr Val Ile
    130                     135                     140
Glu Tyr Leu Val Ser Phe Gly Val Trp Ile Arg Thr Pro Pro Ala Tyr
145                     150                     155                     160
Arg Pro Pro Asn Ala Pro Ile Leu Ser Thr Leu Pro Glu Thr Thr Val
                165                     170                     175
Val Arg Arg Arg Gly Arg Ser Pro Arg Arg Arg Thr Pro Ser Pro Arg
            180                     185                     190
Arg Arg Arg Ser Gln Ser Pro Arg Arg Arg Arg Thr Gln Ser Arg Glu
        195                     200                     205
Ser Gln Cys
    210

<210> 136
<211> 142
<212> PRT
<213> Homo sapiens

<400> 136
Met Gly Ala Pro Thr Leu Pro Pro Ala Trp Gln Pro Phe Leu Lys Asp
 1               5                  10                  15
His Arg Ile Ser Thr Phe Lys Asn Trp Pro Phe Leu Glu Gly Cys Ala
            20                  25                  30
Cys Thr Pro Glu Arg Met Ala Glu Ala Gly Phe Ile His Cys Pro Thr
        35                  40                  45
Glu Asn Glu Pro Asp Leu Ala Gln Cys Phe Phe Cys Phe Lys Glu Leu
    50                  55                  60
Glu Gly Trp Glu Pro Asp Asp Asp Pro Ile Glu Glu His Lys Lys His
65                  70                  75                  80
Ser Ser Gly Cys Ala Phe Leu Ser Val Lys Lys Gln Phe Glu Glu Leu
                85                  90                  95
Thr Leu Gly Glu Phe Leu Lys Leu Asp Arg Glu Arg Ala Lys Asn Lys
            100                 105                 110
Ile Ala Lys Glu Thr Asn Asn Lys Lys Lys Glu Phe Glu Glu Thr Ala
        115                 120                 125
Lys Lys Val Arg Arg Ala Ile Glu Gln Leu Ala Ala Met Asp
    130                 135                 140

<210> 137
<211> 165
<212> PRT
<213> Homo sapiens

<400> 137
Met Gly Ala Pro Thr Leu Pro Pro Ala Trp Gln Pro Phe Leu Lys Asp
 1               5                  10                  15
His Arg Ile Ser Thr Phe Lys Asn Trp Pro Phe Leu Glu Gly Cys Ala
            20                  25                  30
Cys Thr Pro Glu Arg Met Ala Glu Ala Gly Phe Ile His Cys Pro Thr
        35                  40                  45
Glu Asn Glu Pro Asp Leu Ala Gln Cys Phe Phe Cys Phe Lys Glu Leu
    50                  55                  60
Glu Gly Trp Glu Pro Asp Asp Asp Pro Ile Gly Pro Gly Thr Val Ala
65                  70                  75                  80
Tyr Ala Cys Asn Thr Ser Thr Leu Gly Gly Arg Gly Gly Arg Ile Thr
                85                  90                  95

Arg Glu Glu His Lys Lys His Ser Ser Gly Cys Ala Phe Leu Ser Val
            100                 105                 110
Lys Lys Gln Phe Glu Glu Leu Thr Leu Gly Glu Phe Leu Lys Leu Asp
        115                 120                 125
Arg Glu Arg Ala Lys Asn Lys Ile Ala Lys Glu Thr Asn Asn Lys Lys
    130                 135                 140
Lys Glu Phe Glu Glu Thr Ala Lys Lys Val Arg Arg Ala Ile Glu Gln
145                 150                 155                 160
Leu Ala Ala Met Asp
                165

<210> 138
<211> 298
<212> PRT
<213> Homo sapiens

<400> 138
Met Gly Pro Lys Asp Ser Ala Lys Cys Leu His Arg Gly Pro Gln Pro
 1               5                  10                  15
Ser His Trp Ala Ala Gly Asp Gly Pro Thr Gln Glu Arg Cys Gly Pro
            20                  25                  30
Arg Ser Leu Gly Ser Pro Val Leu Gly Leu Asp Thr Cys Arg Ala Trp
        35                  40                  45
Asp His Val Asp Gly Gln Ile Leu Gly Gln Leu Arg Pro Leu Thr Glu
    50                  55                  60
Glu Glu Glu Glu Glu Gly Ala Gly Ala Thr Leu Ser Arg Gly Pro Ala
65                  70                  75                  80
Phe Pro Gly Met Gly Ser Glu Glu Leu Arg Leu Ala Ser Phe Tyr Asp
                85                  90                  95
Trp Pro Leu Thr Ala Glu Val Pro Pro Glu Leu Leu Ala Ala Ala Gly
            100                 105                 110
Phe Phe His Thr Gly His Gln Asp Lys Val Arg Cys Phe Phe Cys Tyr
        115                 120                 125
Gly Gly Leu Gln Ser Trp Lys Arg Gly Asp Asp Pro Trp Thr Glu His
    130                 135                 140
Ala Lys Trp Phe Pro Ser Cys Gln Phe Leu Leu Arg Ser Lys Gly Arg
145                 150                 155                 160
Asp Phe Val His Ser Val Gln Glu Thr His Ser Gln Leu Leu Gly Ser
                165                 170                 175
Trp Asp Pro Trp Glu Glu Pro Glu Asp Ala Ala Pro Val Ala Pro Ser
            180                 185                 190
Val Pro Ala Ser Gly Tyr Pro Glu Leu Pro Thr Pro Arg Arg Glu Val
        195                 200                 205
Gln Ser Glu Ser Ala Gln Glu Pro Gly Gly Val Ser Pro Ala Glu Ala
    210                 215                 220
Gln Arg Ala Trp Trp Val Leu Glu Pro Pro Gly Ala Arg Asp Val Glu
225                 230                 235                 240
Ala Gln Leu Arg Arg Leu Gln Glu Glu Arg Thr Cys Lys Val Cys Leu
                245                 250                 255
Asp Arg Ala Val Ser Ile Val Phe Val Pro Cys Gly His Leu Val Cys
            260                 265                 270
Ala Glu Cys Ala Pro Gly Leu Gln Leu Cys Pro Ile Cys Arg Ala Pro
        275                 280                 285
Val Arg Ser Arg Val Arg Thr Phe Leu Ser
    290                 295

<210> 139
<211> 280
<212> PRT
<213> Homo sapiens

<400> 139
Met Gly Pro Lys Asp Ser Ala Lys Cys Leu His Arg Gly Pro Gln Pro
1               5                   10                  15
Ser His Trp Ala Ala Gly Asp Gly Pro Thr Gln Glu Arg Cys Gly Pro
            20                  25                  30
Arg Ser Leu Gly Ser Pro Val Leu Gly Leu Asp Thr Cys Arg Ala Trp
        35                  40                  45
Asp His Val Asp Gly Gln Ile Leu Gly Gln Leu Arg Pro Leu Thr Glu
    50                  55                  60
Glu Glu Glu Glu Glu Gly Ala Gly Ala Thr Leu Ser Arg Gly Pro Ala
65                  70                  75                  80
Phe Pro Gly Met Gly Ser Glu Glu Leu Arg Leu Ala Ser Phe Tyr Asp
                85                  90                  95
Trp Pro Leu Thr Ala Glu Val Pro Pro Glu Leu Leu Ala Ala Ala Gly
            100                 105                 110
Phe Phe His Thr Gly His Gln Asp Lys Val Arg Cys Phe Phe Cys Tyr
        115                 120                 125
Gly Gly Leu Gln Ser Trp Lys Arg Gly Asp Asp Pro Trp Thr Glu His
    130                 135                 140
Ala Lys Trp Phe Pro Ser Cys Gln Phe Leu Leu Arg Ser Lys Gly Arg
145                 150                 155                 160
Asp Phe Val His Ser Val Gln Glu Thr His Ser Gln Leu Leu Gly Ser
                165                 170                 175
Trp Asp Pro Trp Glu Glu Pro Glu Asp Ala Ala Pro Val Ala Pro Ser
            180                 185                 190
Val Pro Ala Ser Gly Tyr Pro Glu Leu Pro Thr Pro Arg Arg Glu Val
        195                 200                 205
Gln Ser Glu Ser Ala Gln Glu Pro Gly Ala Arg Asp Val Glu Ala Gln
    210                 215                 220
Leu Arg Arg Leu Gln Glu Glu Arg Thr Cys Lys Val Cys Leu Asp Arg
225                 230                 235                 240
Ala Val Ser Ile Val Phe Val Pro Cys Gly His Leu Val Cys Ala Glu
                245                 250                 255
Cys Ala Pro Gly Leu Gln Leu Cys Pro Ile Cys Arg Ala Pro Val Arg
            260                 265                 270
Ser Arg Val Arg Thr Phe Leu Ser
        275                 280

<210> 140
<211> 750
<212> DNA
<213> HCV

<400> 140
caactgatca acaccaacgg cagttggcac atcaatagca cggccttgaa ttgcaatgaa 60
agccttaaca ccggctggtt agcagggctc ttctatcaac acaaattcaa ctcttcaggc 120
tgtcctgaga ggttggccag ctgccgacgc cttaccgatt ttgcccaggg ctggggtcct 180
atcagttatg ccaacggaag cggcctcgac gaacgcccct actgctggca ctaccctcca 240
agaccttgtg gcattgtgcc cgcaaagagc gtgtgtggcc cggtatattg cttcactccc 300
agccccgtgg tggtgggaac gaccgacagg tcgggcgcgc ctacctacag ctggggtgca 360
aatgatacgg atgtcttcgt ccttaacaac accaggccac cgctgggcaa ttggttcggt 420
tgtacctgga tgaactcaac tggattcacc aaagtgtgcg gagcgccccc ttgtgtcatc 480
ggaggggtgg gcaacaacac cttgctctgc cccactgatt gcttccgcaa acatccggaa 540
gccacatact ctcggtgcgg ctccggtccc tggattacac ccaggtgcat ggtcgactac 600
ccgtataggc tttggcacta tccttgtacc atcaattaca ccatattcaa agtcaggatg 660
tacgtgggag gggtcgagca caggctggaa gcggcctgca actggacgcg gggcgaacgc 720
tgtgatctgg aagacaggga caggtccgag                                  750

<210> 141
<211> 2359
<212> DNA
<213> Murine rotavirus

<400> 141
ggctataaaa tggcttcact catttataga caactgctca cgaattcctt taccgtacat 60
atatctgatg aaattgaaac tattggagca gagaagacac aaaatgttac agtgaatccc 120
ggtccattcg cgcaaacggg atacgcccca gcaaactggg ggccaggcga aactaacgac 180
tcaacaacag tagaaccaat gcttgatgga ccataccaac caatagcgtt cagtccgccg 240
ccagagtact atatcatcct ctccccgact gcacccggag taatcgctga atgtacgaat 300
actgtcaacc gctggatagc aatcatagct atagagccaa acgtgtcaac aacaaatcgt 360
acctacacat tgttcggaat tactgaacag ctaacagtag aaaacagctc cgtggataaa 420
tggaagttta tagacttcat gaaaactcca acaactggca gctacgtccg ttataacatt 480
ttgttgtcta gcactaagct atgcgcagtg gcgaacgaca cggacaattt atactcctat 540
gttggagaaa cgcctactgc aggtcaggca tactactctt ctttcaatat atttaaccta 600
accgcgcact gtgacttcta cattatacca tggtcgcagc aatcgttgtg cacgcaatac 660
gttaataacg gattaccgcc gatccagaat acaagaaatg tagtgccaag acatctgtca 720
gcgagatcaa tcatcacaca aagagcgcaa cagaatgaag acattgttgt gtcaaagaca 780
tccttatgga aagaaatgca gtttaatagg gacataacaa tacgtttcaa attcgcgaat 840
gcaataataa agtctggcgg cttgggatat aattggtcag agatctcttt caaaccagcg 900
aactaccaat acacgtacac acgtgatggt gaagaagtaa ctgcgcatac tacgtgctcg 960
gtaaacggtg tgaacaactt cgatttcttt ggcggtacgc tccctacgga tttcggtatt 1020
tcgcggtacg aagtgattaa ggagaattca ttcgtgtaca tagactattg ggacgactct 1080
caggctttca gaaatatggt ctatgtgcgc tcactagcgg ctgatttgaa cactgtcgaa 1140
tgcactgggg gggcgtacag cttttcacta ccagttgggc aatggccggt gatgacgggt 1200
ggtgcagtgt ctttgcgagc tgccggagtt acactatcta cacagttcac agacttcgtg 1260
tcgctaaatt cgttgagatt taggtttcgt ttgtcagtgg aagaaccgtc attcagtata 1320
acgagaacaa gagtgtcagg gctatacggc ttgccagagc gggatcctaa caacggcaga 1380
gaatattacg aaattgcagg tagattttcg ttaatatcat tagtgccgtc caacgataac 1440
tatcaaacac cgataatgaa ttcagttacg gtgcggcaag atctggagag acagctaggc 1500
gaactacgac gagaattcaa cgcgctgtcg caggaaatag cgctgtcaca gttggtggat 1560
ttagcgctac tgccattaga tatgttctca atgttttcag gcatcaaagc aacgctcgac 1620
gtggcaaagt caatggcaac gaacgtgatg aaaaaattca aaaaatcggg actggccacg 1680
tcgatttcac gcatgactga gtcactatca gatgcagctt cctcagtgtc tcggagtgag 1740
ctgcatacgc tcagtcagtt ccacgtcatc agcttggaca gacgtttcgt agctgctgtg 1800
gccaacgtgg aaaatgccgc ctcaacagtt tcaacacaga cggccacaat cagcagacgg 1860
ttgagactga aggaaatcac aacgcagact gaaggcatga acttcgatga catctcagcc 1920
gctgtactta aaactaagct tgataaatca gtacgaatcg cgccgaacac gctaccagac 1980
atagtaacag aagcgtcaga gaagttcatt ccgaacagat catacagagt tataaacaac 2040
aatgaagcat tcgaaactgg aactgacgga cgcttcttcg cataccgagt tgacactctt 2100
gaggaactgc cattcgacgt tcagaaattc gcatgccatg ctgcagagtc cccagtaatc 2160
tcagccatca ctgacttcaa gactttgaaa aatttgaacg ataactacgg aatctcgaaa 2220
gaacaggcct tcagtttatt acgctcagat ccgcgagtac tccgtgaatt tattaatcag 2280
gggaatccaa taatacgtaa tagaatagaa cagttaatta tgcagtgtag actgtgagca 2340
gtgtctagag gatgtgacc 2359

<210> 142
<211> 1356
<212> DNA
<213> Human rotavirus

<400> 142
ggctttaaaa cgaagtcttc gacatggagg ttctgtattc attgtcaaaa actcttaaag 60
atgctaggga taagattgtt gaaggtacat tatattctaa tgttagtgat ctcattcagc 120
aatttaatca aatgatagta accatgaatg gaaatgactt tcaaactgga ggaattggca 180
atttacctat tagaaattgg acatttgact ttggtctact aggtactacg ctgttaaacc 240
ttgatgctaa ttacgttgag actgcaagaa ctacaattaa gtattttatt gactttattg 300
ataatgtatg tatggatgaa atggcaagag agtctcaaag aaatggagta gctccacaat 360
ctgaggcatt gaggaagcta gccggtatta aatttaaaag aataaatttt aataattcat 420
cagaatatat agaaaattgg aatttacaaa atagaagaca gcgtaccgga tttgttttcc 480
ataaacctaa tatatttcca tactcagcat catttacttt aaataggtct caaccaatgc 540
atgacaattt aatgggaacc atgtggctta acgctggatc agaaattcaa gtggctggat 600
ttgactactc gtgtgcccta aatgctccag caaatattca gcagtttgaa catattgtcc 660
agcttaggcg tgcgctaact acagctacta taactttgct acctgatgca gaaagattta 720
gttttccaag agttattaat tcagcagatg gcgcaaccac atggttcttt aatccaatta 780
tcctaagacc aaacaatgta gaggtagaat ttttactgaa tggacaaatt attaatacat 840

atcaagctag atttggaact attatcgcaa gaaattttga tacaattcgt ctatcattcc 900
aattaatgcg tccaccaaac atgacgccag ccgtaaatgc attatttccg caagcacaac 960
cttttcaaca tcatgcaaca gttggactta cgttacgtat tgagtctgca gtttgtgaat 1020
cagtgcttgc ggatgcaaat gaaactttat tggcgaatgt tactgcagta cgtcaagagt 1080
atgctatagg cgttggacca gtatttccac caggcatgaa ttggactgag ctgattacta 1140
actattcacc atccagggaa gataatttgc aacgtgtctt tacagtagcc tctatcagaa 1200
gcatgttaat taagtgagga ccagactaac catctggtat ccaatcttaa ttagcatgta 1260
gctatgtcaa gtcattcaga ctctacaagt aaggacatga tttcatgttc gctacgtaga 1320
gtaactgcat gaatgatcta gtgagaggat gtgacc 1356

<210> 143
<211> 1356
<212> DNA
<213> Bovine rotavirus

<400> 143
ggcttttaaa cgaagtcttc aacatggatg tcctgtactc cttgtcaaaa actcttaaag 60
atgctagaga caaaattgtc gaaggcacat tatactccaa tgtaagtgat ctaattcaac 120
aatttaatca aatgataatt actatgaatg gaaatgagtt ccaaactgga ggaattggta 180
atctaccgat tagaaattgg aattttgatt ttggattact cggaacaact ctactaaatt 240
tggatgccaa ctacgtcgaa acggcccgca atacaattga ttattttgta gattttgtag 300
ataatgtatg tatggatgaa atggttagag aatcacaaag aaatggaatt gcaccacaat 360
cagattcact tagaaagttg tcaggtatta aattcaaaag aataaatttt gacaattcat 420
cagaatacat agagaactgg aatttgcaaa acagaagaca aagaacgggt tttacatttc 480
ataaaccaaa cattttccct tactcagcgt cattcacact gaacagatca caaccagctc 540
atgataactt gatgggtacg atgtggctca atgcgggatc agaaattcag gtcgctggat 600
tcgattattc atgtgcaatc aatgcgccag ccaatacaca acaatttgag catattgtac 660
agctccgaag agtgttgact acagctacaa taactctttt accagatgca gaaagattta 720
gttttccaag agtgattaat tcagctgacg gagctactac atggtacttc aacccagtga 780
ttcttagacc aaataacgtt gaagtagagt ttctactaaa cgggcagata ataaatactt 840
accaagcaag atttggaacg atcatagcta gaaattttga tacaattaga ttgtcatttc 900
agttgatgag accaccaaat atgacaccag cggtagcggc gttatttcca aatgcgcagc 960
catttgaaca tcaggcaaca gtaggactca cgcttagaat tgaatctgca gtttgtgaat 1020
cagtgcttgc cgacgcaagt gaaacaatgc tagcaaatgt gacatctgtt agacaagaat 1080
acgcgatacc agttggacca gtttttccac caggtatgaa ttggactgat ttgatcacta 1140
actattcacc atctagagag gataatttgc agcgtgtatt tacagtggct tccattagaa 1200
gcatgcttgt caaatgagga ccaagctaac cacttggtat ccgactttgg tgagtatgta 1260
gctacgtcaa gctgtttgaa ctctgtaagt aaggatgcgt ctacgtattc gctacacaga 1320
gtaatcactc agatggcgta gtgagaggat gtgacc 1356

<210> 144
<211> 1061
<212> DNA
<213> Murine rotavirus

<400> 144
ggctttaaaa gagagaattt ccgtttggct agcggttagc tccttttaat gtatggtatt 60
gaatatacca cagctttaac tttcctgata tcatttcttt tattgcgcta catactaaaa 120
tcagtagtta aaattatgga ctttatagtt tacaggtttt tgtttgtaat tctaattttg 180
tcgccatgta ttaaagctca aaactacggc attaatcttc caattactgg ttcaatggac 240
actgcgtatg caaactcaac tcaaccggag acatttctga cttccactct atgcctttac 300
tatccaacag aagcagctac tgagataaag gataactcgt ggaaagacac gttatcgcaa 360
ctattcttaa cgaaaggatg gccaataggg tcagtctatt ttaaagaata caccgacata 420
gcagcgttct caatcgatcc acaactatac tgtgattaca acgtagtgct gatgaaatat 480
gacgcttcat tacaaatgga tatgtcggaa cttgcagact tgatactgaa tgaatggctt 540
tgtaatccaa tggacatcac gctatactac taccagcaaa cagacgaagc gaacaaatgg 600
atctccatgg gctcttcatg taccatcaga gtatgtccac ttaacactca gacactggga 660
ataggctgtc tcactaccga tgttacgacc ttcgaagaaa ttgcgactgc ggagaaatta 720
gcgataacgg acgtcgtaga tggcgtgagt cacaagctta acgttacaac cgcgacttgt 780
acaattcgta actgtaagaa acttggtccg cgagaaaatg tagcagttat acaagtaggt 840
ggctctgaca taatagacat aactgcagat ccaacaactg caccacaaac cgagagaatg 900
atgcgcatta attggaaaaa atggtggcaa gtgttctaca ccgtcgttga ttatgtaaat 960
cagataatct caacaatgtc caaacgatct agatcactga actcagcagc tttttattat 1020

agagtgtagg tataactgaa gttacagctg atgatgtgac c 1061

<210> 145
<211> 2359
<212> DNA
<213> Human rotavirus

<400> 145
ggctataaaa tggcttcgct catttataga cagcttctca ctaattcata ttcagtagat 60
ttacatgatg aaatagagca aattgggtca gaaaaaactc aaaacgtaac tgtaaatcca 120
ggtccatttg cccaaactag atatgctcca gtaaattggg gtcatggaga gataaatgat 180
tcaaccacag tagaaccaat tttagatggt ccttatcagc ctactacatt taaaccactt 240
actgattatt ggatacttat taactcaaat acaaatggag tggtatacga gagtacgaat 300
aatagtgact tttggactgc agtagttgct attgaaccgc acgttatcca agtagataga 360
caatatactg tatttggtga aaataaacaa tttaatgtaa gaaatgattc agataaatgg 420
aagtttttag aaatgtttag aggcagtagt caaaatgaat tttataatag acgtacacta 480
acttctgata ctaaactcgt aggaatatta aaatatggtg gaaggatatg gacatttcat 540
ggtgaaacac cgagagctac tactgatagt tcaaatactg caaatttaaa cgatatatca 600
attataatac attcagaatt ttatattatc ccaaggtccc aagaatctaa gtgtaatgaa 660
tatattaaca atggtttgcc accaattcaa aatactagaa atgtagtacc attatcatta 720
tcatctagat ccatacagta taaaagagca caagttaatg aagatattac aatttcaaaa 780
acctcattat ggaaagaaat gcaatgtaat agggatatta taattagatt taaatttggt 840
aatagtattg taaaactggg gggactaggt tataaatggt ccgaaatatc atataaagca 900
gcaaattatc aatataatta tctacgtgat ggcgaacaag taactgcaca tactacttgc 960
tcagtaaatg gagtaaataa ttttagctac aacggaggat ctttacctac tgattttagt 1020
gtctcaaggt atgaagttat taaagaaaat tcttatgtat atgtagatta ttgggatgat 1080
tcaaaagcat ttagaaatat ggtatatgtc agatcattag cagctaattt gaactcagtg 1140
aaatgtacag gtggaagtta tgactttagt atacctgtag gtgcatggcc agtcatgaat 1200
ggtggcgctg tttcgttgca ttttgctgga gttacattat ctacgcaatt cacagatttc 1260
gtatcattga attcactacg atttagattt agtttgacag tggatgagcc atctttttca 1320
atattgagaa cacgtacggt gaatttgtac ggattaccag ctgcaaatcc aaataatgga 1380
aatgaatact atgaaatatc aggaaggttt tcgctcattt ctttagttcc aactaatgat 1440
gattatcaga ctccaattat gaattcagta acagtaagac aagatttaga acgtcaactt 1500
actgatttac gagaggaatt taattcatta tcacaagaaa tagctatgtc acaattaatt 1560
gatttagcgt tattaccttt agatatgttt tctatgtttt cggaattaaa aagtacaatt 1620
gatttgacta aatcaatggc aactagtgta atgaaaaaat ttagaaaatc aaaattagct 1680
acatcaattt cagaaatgac tcattcattg tcagacgcag catcatcagc atcaagaagc 1740
gtttctatca gatcgaatat atccacaatt tcgaattgga ctaatgtttc aaatgatgta 1800
tcaaatgtga ctaattcgtt gagtgatatt tcaacacaaa cgtctacaat cagtaagaac 1860
cttagattaa aagaaatgat tactcaaact gaaggaatga gttttgatga tatttcagcg 1920
gcagtattaa aaacaaaaat agatatgtct actcaaattg gaaagaatac tttacccgat 1980
atagtcacag aggcatctga gaaatttatt ccaaaacgat cgtatcgaat attgaaagat 2040
gatgaagtaa tggaaattaa tactgaaggg aaagtctttg catataaaat cgacacactt 2100
aatgaagtgc catttgatgt aaataaattt gctgaacttg taacaaattc tccagttata 2160
tcagcaataa tcgattttaa aacattaaaa aatttgaatg ataattatgg aattactcga 2220
atagaagcat taaatttaat taaatcgaat ccaaatgtat tacgtaattt cattaaccaa 2280
aataatccaa ttataaggaa tagaattgaa cagctaattc tacaatgtaa attgtgagaa 2340
cgctattgag gatgtgacc 2359

<210> 146
<211> 1062
<212> DNA
<213> Murine rotavirus

<400> 146
   ggctttaaaa gagagaattt ccgtctggct agcggatagc tccttttaat gtatggtatt 60
   gaatatacca cagttctatt ttatttgata tcgttcgttc ttgtgagtta tattctgaaa 120
   accataataa agataatgga ctatattatt tatagaatag catttgtaat tgtagtatta 180
   tcagtattat cgaatgcaca aaattatgga ataaatttgc caattactgg atctatggat 240
   acagcatatg ctaactcaac acaagacaat aattttttag tttcaacttt atgtctatat 300
   tatccatcag aagctccaac tcaaattagt gacactgaat ggaaagatac actatctcag 360
   ctgtttttaa ccaaaggatg gccgacaggt tcagtttatt ttaatgaata ttcaaacgtt 420
   ttagaatttt ccatcgaccc aaagctatac tgtgattata atgttgtgct aattagattc 480
   gtttctggtg aggagttgga catatctgaa ttagctgatc taatactgaa tgagtggtta 540
   tgtaatccaa tggatataac attatattat taccaacaaa ctggagaggc aaacaaatgg 600
   atatcaatgg gatcatcatg taccgttaaa gtgtgtccat taaatactca gacattagga 660
   attggatgtc aaacgacaaa tacagctact tttgaaacag ttgctgatag cgaaaaattg 720
   gcaataattg atgttgtcta catcgtaaat cataaattaa atatcacatc tactacatgt 780
   acaatacgga attgtaataa actaggaccg agagaaaatg tggctataat acaggttggc 840
   ggttctaata tattagatat aacagctgat cccacaactt ctccacaaac agaacgaatg 900
   atgcgcgtaa actggaaaaa atggtggcaa gtattctaca ctgtagttga ttacattaat 960
   cagatagtac aagtaatgtc caaaagatca agatcgttag attcgtcagc tttctattat 1020
   agagtgtaga tatatcctaa aatagaactg tttgatgtga cc 1062

<210> 147
<211> 2382
<212> DNA
<213> SARS coronavirus

<400> 147
gctagcgccg ccaccatgga gaccgacacc ctgctgctct gggtgctgct gctctgggtg 60
cccggcagca ccggcgacgc cgccagcgac ctggaccgct gcaccacctt cgacgacgtg 120
caggccccca actacaccca gcacaccagc agcatgcgcg gcgtgtacta ccccgacgag 180
atcttccgca gcgacaccct gtacctgacc caggacctgt tcctgccctt ctactccaac 240
gtgaccggct tccacaccat caaccacacc ttcgacaacc ccgtgatccc cttcaaggac 300
ggcatctact tcgccgccac cgagaagagc aacgtggtgc gcggctgggt gttcggcagc 360
accatgaaca acaagagcca gagcgtgatc atcatcaaca acagcaccaa cgtggtgatc 420
cgcgcctgca acttcgagct gtgcgacaac cccttcttcg ccgtgagcaa gcccatgggc 480
acccagaccc acaccatgat cttcgacaac gccttcaact gcaccttcga gtacatcagc 540
gacgccttca gcctggacgt gagcgagaag agcggcaact tcaagcacct gcgcgagttc 600
gtgttcaaga acaaggacgg cttcctgtac gtgtacaagg gctaccagcc catcgacgtg 660
gtgcgcgacc tgcccagcgg cttcaacacc ctgaagccca tcttcaagct gcccctgggc 720
atcaacatca ccaacttccg cgccatcctg accgccttca gccccgccca ggacacctgg 780
ggcaccagcg ccgccgccta cttcgtgggc tacctgaagc ccaccacctt catgctgaag 840
tacgacgaga acggcaccat caccgacgcc gtggactgca gccagaaccc cctggccgag 900
ctgaagtgca gcgtgaagag cttcgagatc gacaagggca tctaccagac cagcaacttc 960
cgcgtggtgc ccagcggcga cgtggtgcgc ttccccaaca tcaccaacct gtgccccttc 1020
ggcgaggtgt tcaacgccac caagttcccc agcgtgtacg cctgggagcg caagaagatc 1080
agcaactgcg tggccgacta cagcgtgctg tacaacagca ccttcttcag caccttcaag 1140
tgctacggcg tgagcgccac caagctgaac gacctgtgct tcagcaacgt gtacgccgac 1200
agcttcgtgg tgaagggcga cgacgtgcgc cagatcgccc ccggccagac cggcgtgatc 1260
gccgactaca actacaagct gcccgacgac ttcatgggct gcgtgctggc ctggaacacc 1320
cgcaacatcg acgccaccag caccggcaac tacaactaca agtaccgcta cctgcgccac 1380
ggcaagctgc gccccttcga gcgcgacatc agcaacgtgc ccttcagccc cgacggcaag 1440
ccctgcaccc cccccgccct gaactgctac tggcccctga acgactatgg cttctacacc 1500
accaccggca tcggctacca gccctaccgc gtggtggtgc tgagcttcga gctgctgaac 1560
gcccccgcca ccgtgtgcgg ccccaagctg agcaccgacc tgatcaagaa ccagtgcgtg 1620
aacttcaact tcaacggcct gaccggcacc ggcgtgctga cccccagcag caagcgcttc 1680
cagcccttcc agcagttcgg ccgcgacgtg agcgacttca ccgacagcgt gcgcgacccc 1740
aagaccagcg agatcctgga catcagcccc tgcagcttcg gcggcgtgag cgtgatcacc 1800
cccggcacca acgccagcag cgaggtggcc gtgctgtacc aggacgtgaa ctgcaccgac 1860
gtgagcaccg ccatccacgc cgaccagctg acccccgcct ggcgcatcta cagcaccggc 1920
aacaacgtgt tccagaccca ggccggctgc ctgatcggcg ccgagcacgt ggacaccagc 1980
tacgagtgcg acatccccat cggcgccggc atctgcgcca gctaccacac cgtgagcctg 2040
ctgcgcagca ccagccagaa gagcatcgtg gcctacacca tgagcctggg cgccagcggc 2100
ggcgggggca gcggcggcgg cggggagctg taccactacc aggagtgcgt gcgcggcacc 2160
accgtgctgc tgaaggagcc ctgccccagc ggcacctacg agggcaacag ccccttccac 2220
cccctggccg acaacaagtt cgccctgacc tgcaccagca cccacttcgc cttcgcctgc 2280
gccgacggca cccgccacac ctaccagctg cgcgcccgca gcgtgagccc caagctgttc 2340
atccgccagg aggaggtgca gcaggagctg tactgagaat tc 2382

<210> 148
<211> 784
<212> PRT
<213> SARS coronavirus

<400> 148
Met Glu Thr Asp Thr Leu Leu Leu Trp Val Leu Leu Leu Trp Val Pro
1               5                   10                  15
Gly Ser Thr Gly Asp Ala Ala Ser Asp Leu Asp Arg Cys Thr Thr Phe
            20                  25                  30
Asp Asp Val Gln Ala Pro Asn Tyr Thr Gln His Thr Ser Ser Met Arg
        35                  40                  45
Gly Val Tyr Tyr Pro Asp Glu Ile Phe Arg Ser Asp Thr Leu Tyr Leu
    50                  55                  60
Thr Gln Asp Leu Phe Leu Pro Phe Tyr Ser Asn Val Thr Gly Phe His
65                  70                  75                  80
Thr Ile Asn His Thr Phe Gly Asn Pro Val Ile Pro Phe Lys Asp Gly
                85                  90                  95
Ile Tyr Phe Ala Ala Thr Glu Lys Ser Asn Val Val Arg Gly Trp Val
            100                 105                 110
Phe Gly Ser Thr Met Asn Asn Lys Ser Gln Ser Val Ile Ile Ile Asn
        115                 120                 125
Asn Ser Thr Asn Val Val Ile Arg Ala Cys Asn Phe Glu Leu Cys Asp
    130                 135                 140
Asn Pro Phe Phe Ala Val Ser Lys Pro Met Gly Thr Gln Thr His Thr
145                 150                 155                 160
Met Ile Phe Asp Asn Ala Phe Asn Cys Thr Phe Glu Tyr Ile Ser Asp
                165                 170                 175
Ala Phe Ser Leu Asp Val Ser Glu Lys Ser Gly Asn Phe Lys His Leu
            180                 185                 190
Arg Glu Phe Val Phe Lys Asn Lys Asp Gly Phe Leu Tyr Val Tyr Lys
        195                 200                 205
Gly Tyr Gln Pro Ile Asp Val Val Arg Asp Leu Pro Ser Gly Phe Asn
    210                 215                 220
Thr Leu Lys Pro Ile Phe Lys Leu Pro Leu Gly Ile Asn Ile Thr Asn
225                 230                 235                 240
Phe Arg Ala Ile Leu Thr Ala Phe Ser Pro Ala Gln Asp Ile Trp Gly
                245                 250                 255
Thr Ser Ala Ala Ala Tyr Phe Val Gly Tyr Leu Lys Pro Thr Thr Phe
            260                 265                 270
Met Leu Lys Tyr Asp Glu Asn Gly Thr Thr Asp Ala Val Asp Cys Ser
        275                 280                 285
Gln Asn Pro Leu Ala Glu Leu Lys Cys Ser Val Lys Ser Phe Glu Ile
    290                 295                 300
Asp Lys Gly Ile Tyr Gln Thr Ser Asn Phe Arg Val Val Pro Ser Gly
305                 310                 315                 320
Asp Val Val Arg Phe Pro Asn Ile Thr Asn Leu Cys Pro Phe Gly Glu
                325                 330                 335
Val Phe Asn Ala Thr Lys Phe Pro Ser Val Tyr Ala Trp Glu Arg Lys
            340                 345                 350
Lys Ile Ser Asn Cys Val Ala Asp Tyr Ser Val Leu Tyr Asn Ser Thr
        355                 360                 365
Phe Phe Ser Thr Phe Lys Cys Tyr Gly Val Ser Ala Thr Lys Leu Asn
    370                 375                 380
Asp Leu Cys Phe Ser Asn Val Tyr Ala Asp Ser Phe Val Val Lys Gly
385                 390                 395                 400
Asp Asp Val Arg Gln Ile Ala Pro Gly Gln Thr Gly Val Ile Ala Asp
                405                 410                 415
Tyr Asn Tyr Lys Leu Pro Asp Asp Phe Met Gly Cys Val Leu Ala Trp
            420                 425                 430
Asn Thr Arg Asn Ile Asp Ala Thr Ser Thr Gly Asn Tyr Asn Tyr Lys
        435                 440                 445
Tyr Arg Tyr Leu Arg His Gly Lys Leu Arg Pro Phe Glu Arg Asp Ile
    450                 455                 460
Ser Asn Val Pro Phe Ser Pro Asp Gly Lys Pro Cys Thr Pro Pro Ala
465                 470                 475                 480
Leu Asn Cys Tyr Trp Pro Leu Asn Asp Tyr Gly Phe Tyr Thr Thr Thr
                485                 490                 495

Gly Ile Gly Tyr Gln Pro Tyr Arg Val Val Val Leu Ser Phe Glu Leu
            500                 505                 510
Leu Asn Ala Pro Ala Thr Val Cys Gly Pro Lys Leu Ser Thr Asp Leu
        515                 520                 525
Ile Lys Asn Gln Cys Val Asn Phe Asn Phe Asn Gly Leu Thr Gly Thr
    530                 535                 540
Gly Val Leu Thr Pro Ser Ser Lys Arg Phe Gln Pro Phe Gln Gln Phe
545                 550                 555                 560
Gly Arg Asp Val Ser Asp Phe Thr Asp Ser Val Arg Asp Pro Lys Thr
                565                 570                 575
Ser Glu Ile Leu Asp Ile Ser Pro Cys Ala Phe Gly Gly Val Ser Val
            580                 585                 590
Ile Thr Pro Gly Thr Asn Ala Ser Ser Glu Val Ala Val Leu Tyr Gln
        595                 600                 605
Asp Val Asn Cys Thr Asp Val Ser Thr Ala Ile His Ala Asp Gln Leu
    610                 615                 620
Thr Pro Ala Trp Arg Tyr Ser Thr Gly Asn Asn Val Phe Gln Thr Gln
625                 630                 635                 640
Ala Gly Cys Leu Ile Gly Ala Glu His Val Asp Thr Ser Tyr Glu Cys
                645                 650                 655
Asp Ile Pro Ile Gly Ala Gly Ile Cys Ala Ser Tyr His Thr Val Ser
            660                 665                 670
Leu Leu Arg Ser Thr Ser Gln Lys Ser Ile Val Ala Tyr Thr Met Ser
        675                 680                 685
Leu Gly Ala Ser Gly Gly Gly Gly Ser Gly Gly Gly Gly Glu Leu Tyr
    690                 695                 700
His Tyr Gln Glu Cys Val Arg Gly Thr Thr Val Leu Leu Lys Glu Pro
705                 710                 715                 720
Cys Pro Ser Gly Thr Tyr Glu Gly Asn Ser Pro Phe His Pro Leu Ala
                725                 730                 735
Asp Asn Lys Phe Ala Leu Thr Cys Thr Ser Thr His Phe Ala Phe Ala
            740                 745                 750
Cys Ala Asp Gly Thr Arg His Thr Tyr Gln Leu Arg Ala Arg Ser Val
        755                 760                 765
Ser Pro Lys Leu Phe Ile Arg Gln Glu Glu Val Gln Gln Glu Leu Tyr
    770                 775                 780


<210> 149
<211> 1295
<212> DNA
<213> SARS coronavirus

<400> 149
tctagagccg ccaccatggc cagcgacaac ggcccccaga gcaaccagcg cagcgccccc 60
cgcatcacct tcggcggccc caccgacagc accgacaaca accagaatgg cggccggaac 120
ggcgcccgcc ctaagcagcg ccgcccccag ggcctgccca acaacaccgc cagctggttc 180
accgccctga cccagcacgg caaggaggag ctgcgcttcc cccgcggcca gggcgtgccc 240
atcaacacca acagcggccc cgacgatcag atcggctact atcgccgcgc caccaggcgc 300
gtgcgcggcg gcgacggcaa gatgaaggag ctgagccccc gctggtactt ctactacctg 360
ggcaccggcc ccgaggccag cctgccctac ggcgccaaca aggagggcat cgtgtgggtg 420
gccaccgagg gcgccctgaa cacccccaag gaccacatcg gcacccgcaa ccccaacaac 480
aatgccgcca ccgtgctgca gctgccccag ggcaccaccc tgcccaaggg cttctacgcc 540
gagggcagcc gcggcggcag ccaggccagc agccgctcca gcagccgcag ccgcggcaac 600
agccgcaaca gcacccccgg cagcagccgc ggcaacagcc ctgcccgcat ggccagcggc 660
ggcggggaga ccgccctggc cctgctgctc ctggaccgcc tgaaccagct ggagagcaag 720
gtgtccggca agggccagca gcagcagggc cagaccgtga ccaagaagag cgccgccgag 780
gccagcaaga agccccgcca gaagcgcacc gccaccaagc agtacaacgt gacccaggcc 840
ttcggccgca ggggccccga gcagacccag ggcaacttcg gcgaccagga cctgatccgc 900
cagggcaccg actacaagca ctggccccag atcgcccagt tcgcccccag cgccagcgcc 960
ttcttcggca tgagccgcat cggcatggag gtgaccccca gcggcacctg gctgacctac 1020
cacggcgcca tcaagctgga cgataaggac ccccagttca aggacaacgt gatcctgctg 1080
aacaagcaca tcgacgccta caagaccttc ccccccaccg agcccaagaa ggacaagaaa 1140

aagaagaccg acgaggccca gcccctgcct cagcgccaga agaagcagcc caccgtgacc 1200
ctgctgcccg ccgccgacat ggacgacttc agccgccagc tgcagaacag catgagcggc 1260
gccagcgccg acagcaccca ggcctgagcg gccgc 1295

<210> 150
<211> 421
<212> PRT
<213> SARS coronavirus

<400> 150
Met Ser Asp Asn Gly Pro Gln Ser Asn Gln Arg Ser Ala Pro Ile Asp
1               5                   10                  15
Thr Phe Gly Gly Pro Thr Asp Ser Thr Asp Asn Asn Gln Asn Gly Gly
            20                  25                  30
Arg Asn Gly Ala Arg Pro Lys Gln Arg Arg Pro Gln Gly Leu Pro Asn
        35                  40                  45
Asn Thr Ala Ser Trp Phe Thr Ala Leu Thr Gln His Gly Lys Glu Glu
    50                  55                  60
Leu Arg Phe Pro Arg Gly Gln Gly Val Pro Ile Asn Thr Asn Ser Gly
65                  70                  75                  80
Pro Asp Asp Gln Ile Gly Tyr Tyr Arg Arg Ala Thr Arg Arg Val Arg
                85                  90                  95
Gly Gly Asp Gly Lys Met Lys Glu Leu Ser Pro Arg Trp Tyr Phe Tyr
            100                 105                 110
Tyr Leu Gly Thr Gly Pro Glu Ala Ser Leu Pro Tyr Gly Ala Asn Lys
        115                 120                 125
Glu Gly Ile Val Trp Val Ala Thr Glu Gly Ala Leu Asn Thr Pro Lys
    130                 135                 140
Asp His Ile Gly Thr Arg Asn Pro Asn Asn Asn Ala Ala Thr Val Leu
145                 150                 155                 160
Gln Leu Pro Gln Gly Thr Thr Leu Pro Lys Gly Phe Tyr Ala Glu Gly
                165                 170                 175
Ser Arg Gly Gly Ser Gln Ala Ser Ser Arg Ser Ser Ser Arg Ser Arg
            180                 185                 190
Gly Asn Ser Arg Asn Ser Thr Pro Gly Ser Ser Arg Gly Asn Ser Pro
        195                 200                 205
Ala Arg Met Ala Ser Gly Gly Gly Glu Thr Ala Leu Ala Leu Leu Leu
    210                 215                 220
Leu Asp Arg Leu Asn Gln Leu Glu Ser Lys Val Ser Gly Lys Gly Gln
225                 230                 235                 240
Gln Gln Gln Gly Gln Thr Val Thr Lys Lys Ser Ala Ala Glu Ala Ser
                245                 250                 255
Lys Lys Pro Arg Gln Lys Arg Thr Ala Thr Lys Gln Tyr Asn Val Thr
            260                 265                 270
Gln Ala Phe Gly Arg Arg Gly Pro Glu Gln Thr Gln Gly Asn Phe Gly
        275                 280                 285
Asp Gln Asp Leu Ile Arg Gln Gly Thr Asp Tyr Lys His Trp Pro Gln
    290                 295                 300
Ile Ala Gln Phe Ala Pro Ser Ala Ser Ala Phe Phe Gly Met Ser Ile
305                 310                 315                 320
Asp Gly Met Glu Val Thr Pro Ser Gly Thr Trp Leu Thr Tyr His Gly
                325                 330                 335
Ala Lys Leu Asp Asp Lys Asp Pro Gln Phe Lys Asp Asn Val Ile Leu
            340                 345                 350
Leu Asn Lys His Ile Asp Ala Tyr Lys Thr Phe Pro Pro Thr Glu Pro
        355                 360                 365
Lys Lys Asp Lys Lys Lys Lys Thr Asp Glu Ala Gln Pro Leu Pro Gln
    370                 375                 380
Arg Gln Lys Lys Gln Pro Thr Val Thr Leu Leu Pro Ala Ala Asp Met
385                 390                 395                 400
Asp Asp Phe Ser Arg Gln Leu Gln Asn Ser Met Ser Gly Ala Ser Ala
                405                 410                 415
Asp Ser Thr Gln Ala

            420

<210> 151
<211> 2743
<212> DNA
<213> Human HBV

<400> 151
ggggggcttt ttcacctctg cctaatcatc tcttgttcat gtcctactgt tcaagcctcc 60
aagctgtgcc ttgggtggct ttagggcatg gacattgacc cttataaaga atttggagct 120
actgtggagt tactctcgtt tttgccttct gacttctttc cttccgtacg agatcttcta 180
gataccgccg cagctctgta tcgggatgcc ttagagtctc ctgagcattg ttcacctcac 240
catactgcac tcaggcaagc aattctttgc tggggagact taatgactct agctacctgg 300
gtgggtacta atttagaaga tccagcatct agggacctag tagtcagtta tgtcaacact 360
aatgtgggcc taaagttcag acaattattg tggtttcaca tttcttgtct cacttttgga 420
agagaaacgg ttctagagta tttggtgtct tttggagtgt ggattcgcac tcctccagct 480
tatagaccac caaatgcccc tatcctatca acgcttccgg agactactgt tgttagacga 540
cgaggcaggt cccctagaag aagaactccc tcgcctcgca gacgaagatc tcaatcgccg 600
cgtcgcagaa gatctcaatc tcgggaatct caatgttagt atcccttgga ctcataaggt 660
gggaaatttt actgggcttt attcttctac tgtacctgtc tttaaccctc attggaaacc 720
gccctctttt cctaatatac atttacacca agatattatc aaaaaatgtg aacagtttgt 780
agggccgctc acagtcaatg agaaaagaag gttgaaattg atcatgcctg ctaggtttta 840
tcctaatttt accaaatatt tgcccttgga taagggtatt aaaccttatt atccagaaca 900
tctagttaat cattacttcc aaactagaca ctatttacac actctatgga aggcgggtgt 960
tttatataag agagtatcaa cacatagcgc ctcattttgt gggtcaccat attcttggga 1020
acaagagcta cagcatgggg cagaatcttt ccaccagcaa tcctctggga ttctttcccg 1080
accaccagtt ggatccagcc ttcagagcaa acaccaacaa tccagattgg gacttcaatc 1140
ccaacaagga cacctggcca gacgccaaca aggtaggagc tggagcattc gggctagggt 1200
tcaccccacc gcacggaggc cttttggggt ggagccctca ggctcagggc ataatgcaaa 1260
ccttgccagc aaatccgcct cctgcctcta ccaatcgcca gtcaggacgg cagcctaccc 1320
cgctgtctcc acctctgaga aacactcatc ctcaggccat gcactggaac tccacaacct 1380
tccaccaaac tctgcaagat cccagagtga gaggcctgta tttccctgct ggtggctcca 1440
gttcagggac agtaaaccct gttccgacta ctacctctcc catatcgtca atcttctcga 1500
ggattgggga ccctgcgctg aacatggaga acatcacatc aggattccta ggacccctgc 1560
tcgtgttaca ggcggggttt ttcttgttga caagaatcct cacaataccg cagagtctag 1620
actcgtggtg gacttctctc aattttctag ggggaactac cgtgtgtctt ggccaaaatt 1680
cgcagtcccc aatctccaat cactcaccaa cctcctgtcc tccaacttgt cctggttatc 1740
gctggatgtg tctgcggcgt tttatcatct tcctcttcat cctgctgcta tgcctcatct 1800
tcttgttggt tcttctggac tatcaaggta tgttgcccgt ttgtcctcta attccaggat 1860
catcaaccac cagcacggga tcctgcagaa cctgcacgac tcctgctcaa ggaatctcta 1920
tgtatccctc ctgttgctgt acaaaacctt cggatggaaa ctgcacctgt attcccatcc 1980
catcatcctg ggctttcgga aaattcctat gggagtgggc ctcagcccgt ttctcttggc 2040
tcagtttact agtgccattt gttcagtggt tcgtagggct ttcccccatt gtttggcttt 2100
cagttatatg gatgatgtgg tattgggggc caagtctgta cagcatcttg agtccctttt 2160
taccgctgtt accaattttc ttttgtcttt gggcatacat ttaaacccta acaaaacaaa 2220
aagatggggt tattctctaa atttcatggg ctatgtcatt ggatgttggg gatcattgcc 2280
acaagatcac atcatacaca aaatcaaaga atgttttaga aaactccctg ttcacaggcc 2340
tattgattgg aaagtgtgtc aacgaattgt gggtcttctg ggttttgctg cccctttcac 2400
acaatgtggt tatcctgctt taatgccctt gtatgcatgt attcaatcta agcaggcttt 2460
cactttttcg ccaacttaca aggcctttct gtgtaaacaa tacctgaacc tttaccccgt 2520
tgccgagcaa cggccaggtc tgtgccaagt gtttgctgat gcaaccccca ctggctgggg 2580
cttggtcatg ggccatcagc gcatgcgtgg aacctttctg gctcctctgc cgatccatac 2640
tgcggaactc ctagccgctt gttttgctcg cagcaggtct ggagcaaaca ttctcggaac 2700
tgacaactct gttgtcctct cccgcaaata tacatcgttt cca 2743

<210> 152
<211> 1485
<212> DNA
<213> Human HBV

<400> 152
atgggagcca gagccagcat cctgagagga ggcaaactgg acaagtggga gaagattaga 60

ctgcggcctg gaggcaagaa acggtacatg atcaagcacc tggtgtgggc cagcagagag 120
ctggagcggt tcgcactgaa tcctggcctc ctggagacca gcgaaggatg caaacagatc 180
atgaagcagc tccaaccagc tctgcagacc ggcactgagg aactgagaag cctgtacaac 240
accgtggcca ccctgtactg cgtgcacgag ggcgtggaag tgcgggacac caaggaggcc 300
ctggaccgga tcgaggaaga gcagaacaag atccagcaaa agatccagca gaagacccaa 360
caggccgctg atggaaaggt gagccagaac taccccatcg tccagaacct ccagggccag 420
atggtgcacc agaagctgag ccctcggaca ctgaacgcct gggtcaaggt gatcgaagag 480
aaggccttca gccctgaagt gatccccatg ttcacagctc tgagcgaagg cgccactcct 540
caggacctga acaccatgct gaacaccgtg ggaggccacc aagctgcaat gcagatgctg 600
aaggacacca tcaacgagga agctgccgag tgggacagac tgcatccagt ccacgccgga 660
cccatcgctc ctggccagat gcgggaacct agaggaagcg atatcgctgg cactacctcc 720
accctgcaag agcagatcgc ttggatgacc agcaaccccc ctatccccgt cggcgacatc 780
tacaagcggt ggatcatcct gggcctgaac aagatcgtga gaatgtacag ccccgtgagc 840
atcctggaca tcaagcaagg acctaaggag cccttcagag actacgtcga ccggttcttt 900
aagactctga gagccgagca ggcaacccag gaggtgaaga actggatgac cgacacactg 960
ctggtccaga acgccaaccc cgactgcaag accatcctga aggctctggg acccggcgcc 1020
acactggaag agatgatgac agcatgccag ggcgtcggag gaccaagcca caaagcaaga 1080
gtgctcgccg aggccatgag ccagaccaac agcgtgaata tcctgatgca gaagagcaac 1140
ttcaaaggca acaagcggat ggtcaagtgc ttcaactgtg gcaaggaagg acacatcgca 1200
cggaactgca gagctccacg gaagaaaggc tgctggaagt gcggcaagga aggacaccag 1260
atgaaggact gcacagagcg gcaagcaaac ttcctcggaa agatctggcc aagccacaag 1320
ggaagacccg gcaatttcct gcagaacaga cctgagccca ccgccccacc tgctgagagc 1380
ttccggttcg aagagaccac acccgccccc aagcaggaga gcaaggacag agaagcactg 1440
accagcctga agagcctgtt cggcagcgat cccctgagcc agtga 1485

<210> 153
<211> 1422
<212> DNA
<213> Human HBV

<400> 153
ttgtgggtca cagtctatta tggggtacct gtgtggaaag aagcaaccac cactctattt 60
tgtgcatcag atgctaaagc atatgataca gaggtacata atgtttgggc cacacatgcc 120
tgtgtaccca cagaccccaa cccacaagaa gtagaattgg aaaatgtgac agaaaatttt 180
aacatgtgga aaaataacat ggtagaacag atgcatgagg atataatcag tttatgggat 240
caaagcctaa agccatgtgt aaaattaact ccactctgtg ttactttaaa ttgcactgat 300
ttgaggaatg ctactaatgg gaatgacact aataccacta gtagtagcag ggaaatgatg 360
gggggaggag aaatgaaaaa ttgctctttc aaaatcacca caaacataag aggtaaggtg 420
cagaaagaat atgcactttt ttatgaactt gatatagtac caatagataa taatagtaat 480
aatagatata ggttgataag ttgtaacacc tcagtcatta cacaggcctg tccaaagata 540
tcctttgagc caattcccat acattattgt gccccggctg gttttgcgat tctaaagtgt 600
aaagataaga agttcaatgg aaaaggacca tgttcaaatg tcagcacagt acaatgtaca 660
catgggatta ggccagtagt atcaactcaa ctgctgttaa atggcagtct agcagaagaa 720
gaggtagtaa ttagatccga aaatttcgcg gacaatgcta aaaccataat agtacagctg 780
aatgaatctg tagaaattaa ttgtacaaga cccaacaaca atacaagaaa aagtatacat 840
ataggaccag gcagagcatt atatacaaca ggagaaataa taggagatat aagacaagca 900
cattgtaacc ttagtagagc aaaatggaat gacactttaa ataagatagt tataaaatta 960
agagaacaat ttgggaataa aacaatagtc tttaagcatt cctcaggagg ggacccagaa 1020
attgtgacgc acagttttaa ttgtggaggg gaatttttct actgtaattc aacacaactg 1080
tttaatagta cttggaatgt tactgaagag tcaaataaca ctgtagaaaa taacacaatc 1140
acactcccat gcagaataaa acaaattata aacatgtggc agaaagtagg aagagcaatg 1200
tatgcccctc ccatcagagg acaaattaga tgttcatcaa atattacagg gctgctatta 1260
acaagagatg gtggtccaga ggcaaacaag accgaggtct tcagacctgg aggaggagat 1320
atgagggaca attggagaag tgaattatat aaatataaag tagtaaaaat tgaaccatta 1380
ggagtagcac ccaccaaggc aaagagaaga gtggtggagt aa 1422

<210> 154
<211> 1422
<212> DNA
<213> Human HBV

<400> 154
ctgtgggtga ccgtgtacta cggcgtgccc gtgtggaagg aggccaccac caccctgttc 60

tgcgccagcg accgcaaggc ctacgacacc gaggtgcaca acgtgtgggc cacccacgcc 120
tgcgtgccca ccgaccccaa cccccaggag gtggagctga agaacgtgac cgagaacttc 180
aacatgtgga agaacaacat ggtggagcag atgcacgagg acatcatcag cctgtgggac 240
cagagcctga agccctgcgt gaagctgacc cccctgtgcg tgaccctgaa ctgcaccgac 300
ctgcgcaacg ccaccaacgg caacgacacc aacaccacta gtagcagccg cggcatggtg 360
ggcggcggcg agatgaagaa ctgcagcttc aacatcacca ccaacatccg cggcaaggtg 420
cagaaggagt acgccctgtt ctacaagctg gacatcgccc ccatcgacaa caacagcaac 480
aaccgctacc gcctgatcag ctgcaacacc agcgtgatca cccaggcctg ccccaaggtg 540
agcttcgagc ccatccccat ccactactgc gcccccgccg gcttcgccat cctgaagtgc 600
aaggacaaga agttcaacgg caagggcccc tgcaccaacg tgagcaccgt gcagtgcacc 660
cacggcatcc gccccgtggt gagcacccag ctgctgctga acggcagcct ggccgaggag 720
gaggtggtga tccgcagcgc caacttcgcc gacaacgcca aggtgatcat cgtgcagctg 780
aacgagagcg tggagatcaa ctgcacccgc cccaacaaca acacccgcaa gtccatccac 840
atcggccccg gccgcgcctt ctacaccacc ggcgagatca tcggcgacat ccgccaggcc 900
cactgcaacc tgagccgcgc caagtggaac gacaccctga acaagatcgt gatcaagctg 960
cgcgagcagt tcggcaacaa gaccatcgtg ttcaagcaca gcagcggcgg cgaccccgag 1020
atcgtgaccc acagcttcaa ttgcggcggc gagttcttct actgcaacag cacccagctg 1080
ttcaacagca cctggaacgt gaccgaggag agcaacaaca ccgtggagaa caacaccatc 1140
accctgccct gccgcatcaa gcagatcatc aacatgtggc aggaggtggg ccgcgccatg 1200
tacgcccccc ccatccgcgg ccagatccgc tgcagttcga acatcaccgg cctgctgctg 1260
acccgcgacg gcggccccga ggacaacaag accgaggtgt tccgccccgg cggcggcgac 1320
atgcgcgaca actggcgcag cgagctgtac aagtacaagg tggtgaagat cgagcccctg 1380
ggcgtggccc ccaccaaggc caagcgccgc gtggtggagt aa 1422

<210> 155
<211> 1421
<212> DNA
<213> Human HBV

<400> 155
ttgtgggtca cagtctatta tggggtacct gtgtggaaag aagcaaacac cactctattt 60
tgtgcatcag atgctaaagc atatgataca gaggtacata atgtttgggc cacacatgcc 120
tgtgtaccca cagaccccga tccacaagaa gtagaattgg aaaatgtgac agaaaatttt 180
aacatgtgga aaaataacat ggtagaacag atgcatgagg atataattag tttatgggat 240
caaagcctaa agccatgtgt aaaattaacc ccactctgtg ttactctaaa ttgcaccaat 300
ctgaggaatg atactaatac cacgaggaat gctactaata ccacgagtag tgagacaatg 360
atggaggagg gagaaataaa aaattgctct ttcaatatca ccacaagcat aagagataag 420
gtgcaaaaag aatttgcact tttttataaa cttgatgtag taccaataga aaatgatact 480
actagctata ggttgataag ttgtaatacc tcagtcctta cacaggcctg cccaaaggta 540
tcctttgagc caattcccat acatttttgt gccccggctg gttttgcaat tctaaagtgt 600
aaagataaga agttcaatgg aacaggacca tgtacaaatg tcagcacagt acaatgcaca 660
catggaatta agccagtagt atcaactcaa ctgctgttaa atggcagtct agcagaagaa 720
gaggtagtaa ttaggtccgc caatctctcg gacaatgcta aaaccataat agtacagctg 780
aatgaatctg tacaaatgaa ttgtacgaga cccaacaaca atacaagaaa aagtatacat 840
ataggaccag gcagagcatt ttatacaaca ggagaaataa taggagatat aagacaagca 900
cattgtaacc ttagtagaac aaaatggaat gaaactttaa aaaggatagt tataaaatta 960
agagagcaat atgagaataa aacatagtct ttaatcaatc ctcaggaggg gacccagaaa 1020
ttgtaatgct cagctttaat tgtggagggg aatttttcta ctgtaattca acaaaactgt 1080
ttaatagtac ttggaatggt actgagtcaa ataacacagg agatgaccca atcgtactcc 1140
catgcagaat aaaacaagtt ataaacatgt ggcaagaagt aggaaaagca atgtatgccc 1200
ctcccatcag aggacaaatt agatgctcat caaatattac aggactgcta ttaacaagag 1260
atggtggtaa cagtaacgag accaatacca ccgagatctt cagacctggg ggaggaaata 1320
tgaaggacaa ttggagaagt gaattatata aatataaagt agtaagaatt gaaccattag 1380
gaatagcacc caccagggca aagagaagag tggtggagta a 1421

<210> 156
<211> 1421
<212> DNA
<213> Human HBV

<400> 156
ctgtgggtga ccgtctacta tggggtgcct gtgtggaagg aggccaacac cactctgttc 60
tgcgcttctg acgctaaggc ctacgatacc gaggtgcaca atgtgtgggc cacccacgcc 120

tgtgtgccca ccgaccccga ccctcaggag gtggagctgg agaacgtgac cgaaaacttc 180
aacatgtgga agaataacat ggtggagcag atgcatgagg atatcattag cctgtgggac 240
cagagcctaa agccctgcgt gaagctgacc cccctgtgtg tgactctgaa ctgcaccaac 300
ctgaggaatg atactaacac caccaggaac gccactaata cgaccagcag cgagaccatg 360
atggaggagg gcgagatcaa gaactgctct ttcaacatca ccacgagcat cagagacaag 420
gtgcagaagg agtttgccct tttctataaa cttgatgtgg tgcctatcga gaatgacact 480
actagctaca ggctgatcag ctgcaacacc agcgtcctga cacaggcctg ccccaaggtg 540
tccttcgagc caattcccat ccacttttgt gccccggctg gtttcgccat tctaaagtgc 600
aaggataaga agttcaacgg caccggtcct tgtaccaatg tcagcaccgt acaatgcacc 660
acggcattaa gcccgtggtg agcactcagc tgctgctgaa cggcagcctg gccgaggaag 720
aggtggtgat tcgctccgcc aacctctctg acaatgctaa gaccataatc gtgcagctga 780
acgagtctgt gcagatgaac tgcacgaggc ccaacaacaa taccaggaag agtatccata 840
tcggtcccgg cagggcattc tataccaccg gcgagatcat cggcgacatc aggcaggccc 900
actgtaacct tagcaggaca aagtggaacg agactctgaa gaggatcgtg atcaagctga 960
gggagcagta cgagaacaag accatcgtct ttaatcaatc cagcggcggg gaccctgaga 1020
ttgtgatgct gagcttcaac tgcggtgggg agttcttcta ctgtaactca accaagctgt 1080
ttaatagcac ttggaacggc actgagtcta acaacaccgg tgatgacccc atcgtgctgc 1140
catgcaggat caagcaggtg atcaacatgt ggcaggaagt gggcaaggcc atgtatgccc 1200
ctcccatcag gggtcagatt aggtgcagca gcaatattac cggcctgcta ctgacccgcg 1260
acggcggtaa cagcaacgag accaacacca ccgagatctt caggcctggg ggcggcaaca 1320
tgaaggacaa ttggaggagc gagttataca aatataaggt ggtgaggatt gagcctctgg 1380
gtatcgcccc caccagggcc aagaggaggg tggtgcagta a 1421

<210> 157
<211> 1443
<212> DNA
<213> Human HBV

<400> 157
ttgtgggtca cagtctatta tggggtacct gtgtggaaag aagcaaaaac tactctattc 60
tgtgcatcag atgctaaatc atatgagaaa gaagtgcata atgtctgggc tacacatgcc 120
tgtgtaccca cagaccccaa cccacaagaa atagttttgg gaaatgtaac agaaaatttt 180
aacatgtgga aaaatgacat ggtggatcag atgcatgagg atataatcag tttatgggat 240
caaagcctaa agccatgtgt aaagttgacc ccactctgtg tcactttaaa ttgtacagag 300
gttaatgtta ccagaaatgt taataatagc gtggttaata ataccacaaa tgttaataat 360
agcatgaatg gagacatgaa aaattgctct ttcaacataa ccacagaact aaaagataag 420
aaaaagaatg tgtatgcact tttttataaa cttgatatag tatcacttaa tgagactgac 480
gactctgaga ctggcaactc tagtaaatat tatagattaa taaattgtaa tacctcagcc 540
ctaacacaag cctgtccaaa ggtctctttt gacccaattc ctatacatta ttgtgctcca 600
gctggttatg cgattctaaa gtgtaataat aagacattca atgggacagg accatgccat 660
aatgtcagca cagtacaatg tacacatgga attaagccag tggtatcaac tcaactactg 720
ttaaatggta gcctagcaga agaagggata ataattagat ctgaaaatct gacaaacaat 780
gtcaaaacaa taatagtaca tcttaataga tctatagaaa ttgtgtgtgt aagacccaac 840
aataatacaa gacaaagtat aagaatagga ccaggacaaa cattctatgc aacaggagac 900
ataataggag acataagaca agcacattgt aacattagta ggactaactg gactaagact 960
ttacgagagg taaggaacaa attaagagaa cacttcccta ataaaaatat aacatttaaa 1020
ccatcctcag gaggggacct agaaattaca acacatagct ttaattgtag aggagaattt 1080
ttctattgca atacatcggg cctgtttagt ataaattata cagaaaataa tacagatggt 1140
acacccatca cactcccatg cagaataaga caaattataa atatgtggca ggaagtagga 1200
cgagcaatgt acgcccctcc cattgaagga aacatagcat gtaaatcaga tatcacaggg 1260
ctactattgg ttcgggatgg aggaagcaca aatgatagca caaataataa cacagagata 1320
ttcagacctg caggaggaga tatgagggac aattggagga gtgaattgta taagtataaa 1380
gtggtagaaa ttaagccatt gggaatagca cccactgagg caaaaaggag agtggtggag 1440
taa 1443

<210> 158
<211> 1464
<212> DNA
<213> Human HBV

<400> 158
tggggcaacc tgtgggtgac cgtgtactac ggcgtgcccg tgtggaagga ggccaagacc 60
accctgttct gcgccagcga cgccaagagc tacgagaagg aggtgcacaa cgtgtgggcc 120

acccacgcct gcgtgcccac cgaccccaac ccccaggaga tcgtgctggg caacgtgacc 180
gagaacttca acatgtggaa gaacgacatg gtggaccaga tgcacgagga catcatcagc 240
ctgtgggacc agagcctgaa gccctgcgtg aagctgaccc ccctgtgcgt gaccctgaac 300
tgcaccgagg tgaacgtgac ccgcaacgtg aacaacagcg tggtgaacaa caccaccaac 360
gtgaacaaca gcatgaacgg cgacatgaag aactgcagct tcaacatcac caccgagctg 420
aaggacaaga agaagaacgt gtacgccctg ttctacaagc tggacatcgt gagcctgaac 480
gagaccgacg acagcgagac cggcaacagc agcaagtact accgcctgat caactgcaac 540
accagcgccc tgacccaggc ctgccccaag gtgagcttcg accccatccc catccactac 600
tgcgcccccg ccggctacgc catcctgaag tgcaacaaca agaccttcaa cggcaccggc 660
ccctgccaca acgtgagcac cgtgcagtgc acccacggca tcaagcccgt ggtgagcacc 720
cagctgctgc tgaacggcag cctggccgag gagggcatca tcatccgcag cgagaacctg 780
accaacaacg tgaagaccat catcgtgcac ctgaaccgca gcatcgagat cgtgtgcgtg 840
cgccccaaca acaacacccg ccagagcatc cgcatcggcc ccggccagac cttctacgcc 900
accggcgaca tcatcggcga catccgccag gcccactgca acatcagccg caccaactgg 960
accaagaccc tgcgcgaggt gcgcaacaag ctgcgcgagc acttccccaa caagaacatc 1020
accttcaagc ccagcagcgg cggcgacctg gagatcacca cccacagctt caactgccgc 1080
ggcgagttct tctactgcaa caccagcggc ctgttcagca tcaactacac cgagaacaac 1140
accgacggca cccccatcac cctgccctgc cgcatccgcc agatcatcaa catgtggcag 1200
gaggtgggcc gcgccatgta cgcccccccc atcgagggca acatcgcctg caagagcgac 1260
atcaccggcc tgctgctggt gcgcgacggc ggcagcacca acgacagcac caacaacaac 1320
accgagatct tccgccccgc cggcggcgac atgcgcgaca actggcgcag cgagctgtac 1380
aagtacaagg tggtggagat caagcccctg ggcatcgccc ccaccgaggc caagcgccgc 1440
gtggtggagc gcgagaagcg ctga 1464

<210> 159
<211> 1407
<212> DNA
<213> Human HBV

<400> 159
ttgtgggtca cagtctatta tggggtacct gtgtggaaag atgcagatac caccctattt 60
tgtgcatcag atgccaaagc acatgagaca gaagtgcaca atgtctgggc cacacatgcc 120
tgtgtaccca cagaccccaa cccacaagaa atacacctgg aaaatgtaac agaaaatttt 180
aacatgtgga aaaataaaat ggtagagcag atgcaggagg atgtaatcag tttatgggat 240
caaagtctaa agccatgtgt aaagttaact cctctctgcg ttactttgac ttgtaccaat 300
gctactctga attgtaccaa tttgaccaat ggcaataaga caactaatgt ctctaacata 360
ataggaaatc taacagatga agtaagaaac tgttcttttc atatgaccac agaactaaga 420
gataagaagc agaaggtcta tgcacttttt tataagcttg atatagtaca aattaatagt 480
agtgagtata ggttaataaa ttgtaatact tcagtcatta agcaggcttg tccaaagata 540
tcctttgatc caattcctat acattattgt actccagctg gttatgcgat tttaaagtgt 600
aatgataaga atttcaatgg gacagggcca tgtaaaaatg tcagctcagt acaatgcaca 660
catggaatta agccagtggt atcaactcaa ttgctgttaa atggcagtct agcagaagaa 720
gagataataa tcagctctga aaatctcaca aacaatgcca aaaccataat agtgcacctt 780
aataaatctg tagaaatcag ttgtaccaga ccctccacca atacaagaac aagtatacgt 840
ataggaccag gacaagtatt ctatagaaca ggagacataa caggagatat aagaaaagca 900
tattgtgaga ttaatgaaac aaaatggaat gaagctttaa aacaggtagc tgggaaatta 960
aaagaacact ttaataagac aataatcttt caaccaccct caggaggaga tctagaaatt 1020
acaatgcatc attttaattg tagaggggaa tttttctatt gcgatacaac acaactgttt 1080
aatagaactt ggggagaaaa tgaaaccaga gaggggcgta atatcacact tccatgcaag 1140
ataaagcaaa ttgtaaacat gtggcaggga gcagggcaag caatgtatgc tcctcccatc 1200
agtggaataa ttaagtgtgt atcaaatatt acaggaatac tattgacaag agatggtggt 1260
gctaataatt cggctagtga gaccttcaga cctggaggag gaaatataaa ggacaattgg 1320
agaagtgaat tatataaata taaagtagta caaattgaac cactaggaat agcacccacc 1380
agggcaaaga gaagagtggt ggagtaa 1407

<210> 160
<211> 1407
<212> DNA
<213> Human HBV

<400> 160
ctgtgggtga ccgtgtacta cggcgtgccc gtgtggaagg acgccgacac caccctgttc 60
tgcgccagcg aggccaaggc ccacgagacc gaggtgcaca acgtgtgggc cacccacgcc 120

tgcgtgccca ccgaccccaa cccccaggag atccacctgg agaacgtgac cgagaacttc 180
aacatgtgga agaacaagat ggtggagcag atgcaggagg acgtgatcag cctgtgggac 240
cagagcctga agccctgcgt gaagctgacc cccctgtgcg tgaccctgac ctgcaccaac 300
gccaccctga actgcaccaa cctgaccaac ggcaacaaga ccaccaacgt gagcaacatc 360
atcggcaacc tgaccgacga ggtgcgcaac tgcagcttcc acatgaccac cgagctgcgc 420
gacaagaagc agaaggtgta cgccctgttc tacaagctgg acatcgtgca gatcaacagc 480
agcgagtacc gcctgatcaa ctgcaacacc agcgtgatca agcaggcctg ccccaagatc 540
agcttcgacc ccatccccat ccactactgc acccctgctg gctacgccat cctgaagtgc 600
aacgacaaga acttcaacgg caccggaccc tgcaagaacg tgagcagcgt gcagtgcacc 660
cacggcatca agcccgtggt gagcacccag ctgctgctga acggcagcct ggccgaggag 720
gagatcatca tcagcagcga gaacctgacc aacaacgcca agaccatcat cgtgcacctg 780
aacaagagcg tggagatcag ctgcactcgc cccagcacca acacccgcac cagcatccgc 840
atcggacctg gccaggtgtt ctaccgcacc ggcgacatca ccggcgacat ccgcaaggcc 900
tactgcgaga tcaacgagac caagtggaac gaggccctga agcaggtggc cggcaagctg 960
aaggagcact tcaacaagac catcatcttc cagcctccca gcggaggcga cctggagatc 1020
accatgcacc acttcaactg cagaggcgag ttcttctact gcgacaccac ccagctgttc 1080
aaccgcacct ggggcgagaa cgagacccgc gagggcagga acatcaccct gccctgcaag 1140
atcaagcaga tcgtgaacat gtggcaggga gctggccagg ccatgtacgc cccacccatc 1200
agcggcatca tcaagtgcgt gagcaacatc accggcatcc tgctgacccg cgacggcggt 1260
gccaacaaca gcgccagcga gaccttcagg ccaggcggtg gcaacatcaa ggacaactgg 1320
cgcagcgagc tgtacaagta caaggtggtg cagatcgagc ccctgggcat cgcccccact 1380
cgcgccaagc gccgcgtggt ggagtaa 1407

<210> 161
<211> 1410
<212> DNA
<213> Human HBV

<400> 161
ttgtgggtca cagtctatta tggggtacct gtgtggaaag atgcagagac taccttattt 60
tgtgcatcag atgcgaaagc atatgataca gaagtgcata atgtctgggc tacgcatgcc 120
tgtgtaccta cagaccccaa cccacaagaa atatatatgg aaaatgtgac agaagagttt 180
aacatgtgga aaaataacat ggtagagcag atgcatacag atataatcag tctatgggac 240
caaagcctaa aaccatgtgt acagttaacc cctctctgcg ttactttaga ttgtagctat 300
aacatcagca ataatatcac caatagcatc accaatagct cagttaacat gagagaagaa 360
ataaaaaact gctctttcaa tatgaccaca gaattaaggg ataagaatcg gaaggtatat 420
tcactttttt ataaacttga tgtagtacaa attaataatg gtaataacag tagtaatctg 480
tatagattaa taaattgtaa tacctcagcc cttacacagg cttgtccaaa ggtaaccttt 540
gagccaattc ccatacgtta ttgtgcccca gctggttatg cgattctaaa atgtaatgat 600
aaggagttca atggaacagg gctatgcaaa aatgtcagca cagtgcaatg cacacatgga 660
atcaggccag tagtatcaac tcaactgctg ttaaatggca gtttagcaga aggaaaggta 720
atgattagat ctgaaaatat cacaaacaat gtcaaaaaca taatagtaca acttaacgag 780
actgtaacaa ttaattgtac cagacctaac aacaatacaa gaaaaagtgt acgtatagga 840
ccaggacaaa cattctatgc aacaggtgat ataatagggg atataagaca agcacattgt 900
aatgtcagtg ggtcacaatg gaatagagct ttacaccagg tagttggaca attaagagaa 960
tactggaaca caacaataat ctttaaaaac tcctcaggag gggatttaga aattacaaca 1020
catagtttta attgtggagg agaatttttc tattgtaata catcaggcct gtttaatagt 1080
aattggacac ataatgacac tgccagcatg aaaccaaatg acactataac actcccatgc 1140
agaataaagc aaattataaa tatgtggcag agagtaggac aagcaatata tgcccctccc 1200
attcaaggag taataaggtg tgaatcaaac attacaggac taatattaac aagagatggt 1260
gggggtaaca tcaatgaaag tcaaatcttc agacctggag gaggagatat gagggacaat 1320
tggagaagtg aattatataa gtataaggta gtaagaattg aaccactagg agtagcaccc 1380
accaaggcaa agagaagagt ggtggagtaa 1410

<210> 162
<211> 1410
<212> DNA
<213> Human HBV

<400> 162
ctgtgggtga ccgtgtacta cggcgtgccc gtgtggaagg acgccgagac caccctgttc 60
tgcgccagcg acgccaaggc ctacgacacc gaggtgcaca acgtgtgggc cacccacgcc 120
tgcgtgccca ccgaccccaa cccccaggag atctacatgg agaacgtgac cgaggagttc 180

aacatgtgga agaacaacat ggtggagcag atgcacaccg acatcatcag cctgtgggac 240
cagagcctga agccctgcgt gcagctgacc cccctgtgcg tgaccctgga ctgcagctac 300
aacatcacca acaacatcac caacagcatc accaacagca gcgtgaacat gcgcgaggag 360
atcaagaact gcagcttcaa catgaccacc gagctgcgcg acaagaaccg caaggtgtac 420
agcctgttct acaagctgga cgtggtgcag atcaacaacg gcaacaacag cagcaacctg 480
taccgcctga tcaactgcaa caccagcgcc ctgacccagg cctgccccaa ggtgaccttc 540
gagcccatcc ccatccgcta ctgcgccccc gccggctacg ccatcctgaa gtgcaacgac 600
aaggagttca acggcaccgg cctgtgcaag aacgtgagca ccgtgcagtg cacccacggc 660
atccgccccg tggtgagcac ccagctgctg ctgaacggca gcctggccga gggcaaggtg 720
atgatccgca gcgagaacat caccaacaac gtgaagaaca tcatcgtgca gctgaacgag 780
accgtgacca tcaactgcac ccgccccaac aacaacaccc gcaagagcgt gcgcatcggc 840
cccggccaga ccttctacgc caccggcgac atcatcggcg acatccgcca ggcccactgc 900
aacgtgagcg gcagccagtg gaaccgcgcc ctgcaccagg tggtgggcca gctgcgcgag 960
tactggaaca ccaccatcat cttcaagaac agcagcggcg gcgacctgga gatcaccacc 1020
cacagcttca actgcggcgg cgagttcttc tactgcaaca ccagcggcct gttcaacagc 1080
aactggaccc acaacgacac cgccagcatg aagcccaacg acaccatcac cctgccctgc 1140
cgcatcaagc agatcatcaa catgtggcag cgcgtgggcc aggccatcta cgcccctccc 1200
atccagggcg tgatccgctg cgagagcaac atcaccggcc tgatcctgac ccgcgacggc 1260
ggcggcaaca tcaacgagag ccagatcttc cgccccggcg gcggcgacat gcgcgacaac 1320
tggcgcagcg agctgtacaa gtacaaggtg gtgcgcatcg agcccctggg cgtggccccc 1380
accaaggcca agcgccgcgt ggtggagtaa 1410

## Description

### FIELD OF THE INVENTION

Embodiments of the present invention relate to the discovery of several formulations of a transdermal delivery composition that delivers low and high molecular weight compounds, particularly drugs and cosmetic agents to a subject. Aspects of the invention include said transdermal delivery compositions, transdermal delivery devices for providing said compositions to subjects in need thereof and methods of making and using the foregoing.

### BACKGROUND OF THE INVENTION

The skin provides a protective barrier against foreign materials and infection. In mammals this is accomplished by forming a highly insoluble protein and lipid structure on the surface of the corneocytes termed the cornified envelope (CE). (Downing et al., Dermatology in General Medicine, Fitzpatrick, et al., eds., pp. 210-221 (1993), Ponec, M., The Keratinocyte Handbook, Leigh, et al., eds., pp. 351-363 (1994)). The CE is composed of polar lipids, such as ceramides, sterols, and fatty acids, and a complicated network of cross-linked proteins; however, the cytoplasm of stratum corneum cells remains polar and aqueous. The CE is extremely thin (10 microns) but provides a substantial barrier. Because of the accessibility and large area of the skin, it has long been considered a promising route for the administration of drugs, whether dermal, regional, or systemic effects are desired.

A topical route of drug administration is sometimes desirable because the risks and inconvenience of parenteral treatment can be avoided; the variable absorption and metabolism associated with oral treatment can be circumvented; drug administration can be continuous, thereby permitting the use of pharmacologically active agents with short biological half-lives; the gastrointestinal irritation associated with many compounds can be avoided; and cutaneous manifestations of diseases can be treated more effectively than by systemic approaches.

Most transdermal delivery compositions achieve epidermal penetration by using a skin penetration enhancing vehicle. Such compounds or mixtures of compounds are known in the art as "penetration enhancers" or "skin enhancers". While many of the skin enhancers in the literature enhance transdermal absorption, several possess certain drawbacks in that (i) some are regarded as toxic; (ii) some irritate the skin; (iii) some have a thinning effect on the skin after prolonged use; (iv) some change the intactness of the skin structure resulting in a change in the diffusability of the drug; and (v) all are incapable of delivering high molecular weight pharmaceuticals and cosmetic agents. Despite these efforts, there remains a need for transdermal delivery compositions that deliver a wide-range of pharmaceuticals and cosmetic agents.

### BRIEF SUMMARY OF THE INVENTION

Disclosed herein are formulations of transdermal delivery compositions used to deliver pharmaceuticals, therapeutic compounds, diagnostics, and cosmetic agents of various molecular weights. In several embodiments, the transdermal delivery composition comprises a unique formulation of penetration enhancer (an ethoxylated lipid, modified lipid, fatty acid, fatty alcohol, or fatty amine therein having 10-19 ethoxylations per molecule) or transdermal delivery enhancer (an ethoxylated compound with a multi-functional backbone) that delivers a wide range of pharmaceuticals and cosmetic agents having molecular weights of less than 100 daltons to greater than 500,000 daltons. For example, embodiments of the transdermal delivery composition include formulations that deliver a therapeutically effective amount of a pharmaceutical, including NSAIDs, capsaicin or Boswellin-containing pain-relief solutions, other drugs or chemicals, dyes, low and high molecular weight peptides (*e.g.,* collagens or fragments thereof), hormones, nucleic acids, antibiotics, vaccine preparations, and immunogenic preparations. Methods of making the transdermal delivery compositions described herein and systems for their delivery are embodiments. Further embodiments include methods of using said compositions (*e.g*., the treatment and prevention of undesired human conditions or diseases or cosmetic applications).

Aspects of the invention concern transdermal delivery compositions that comprise lipospheres. In some embodiments, the liposphere comprises an ethoxylated composition having a carbon chain length of at least 10, wherein the ethoxylated composition (e.g., a fatty acid, fatty alcohol, or fatty amine), comprises, consists of, or consists essentially of at least 10, 11, 12, 13, 14, 15, 16, 17, 18, or 19 ethoxylations per molecule. Aspects of the invention also include propoxylated compositions or compositions that comprise a combination of propoxylated and ethoxylated compositions. In some formulations, the ethoxylated or propoxylated composition is a fatty moiety, such as a fatty acid (e.g., an unsaturated fatty acid or a polyunsaturated fatty acid). In other formulations, the fatty moiety is a fatty alcohol. In other embodiments, the liposphere comprises an ethoxylated or propoxylated oil or lipid having carbon chain lengths of at least 10, wherein the ethoxylated or propoxylated oil or lipid (e.g., a nut oil, a tri-alcohol, a tri-fatty amine, a glycolipid, a sphingolipid, a glycosphingolipid, or any other modified lipid moiety), comprises, consists of, or consists essentially of at least 10, 11, 12, 13, 14, 15, 16, 17, 18, or 19 ethoxylations per molecule.

In preferred embodiments, the number of ethoxylations or propoxylations per molecule is the same as the number of carbons in the fatty moiety or lipid moiety. Desirably, the fatty moiety has a carbon chain length of at least 10, 12, 14, 16, 18, 20, 22, or 24. The liposphere comprises a homogeneous mixture of an ethoxylated or propoxylated fatty moiety in some embodiments, while in other embodiments, the liposphere comprises a heterogeneous mixture of an ethoxylated or propoxylated fatty moiety.

Other aspects of the invention concern transdermal delivery compositions comprised of an ethoxylated lipid moiety, such as an oil, glycolipid, sphingolipid, or glycosphingolipid. The ethoxylated oil that can be used in the formulations described herein can be a vegetable, nut, animal, or synthetic oil having at least 10, 11, 12, 13, 14, 15, 16, 17, 18, 19, or more ethoxylations per molecule. Preferred oils include macadamia nut oil or meadowfoam (*limnanthes alba*)*.* It should be understood that when an oil is ethoxylated, one or more of the components of the oil are ethoxylated (e.g., fatty acids, fatty alcohols, and/or fatty amines) and it is generally recognized in the field that an average number of ethoxylations for the oil and components is obtained and therefore provided. That is, the measured composition is the algebraic sum of the compositions of the species in the mix.

Still other aspects of the invention relate to transdermal delivery compositions comprising a delivery enhancer. As used herein, the term "transdermal delivery enhancer" refers to a molecule that comprises a multi-functional backbone having at least two reactive (R) groups. The R groups on the multifunctional backbone comprise a reactive hydrogen, such as -OH, COOH, amines, sulfydryl groups, and aldehydes. Thus, multifunctional backbones include trialcholols, triacids, amino acids, dipeptides, tripeptides, sugars, and other compounds such as glucosamine. At least one R group is substituted with a fatty moiety, and least one reactive group is substituted with a polyethoxy or polyethoxy/polypropoxy group, wherein the polyethoxy or the polyethoxy/polypropoxy group comprises between 10 and 19 ethoxy or propoxy/ethoxy substituents, respectively.

In several formulations, the ethoxylated fatty moiety is about 0.1% to greater than 99% by weight of the transdermal delivery composition described herein.

In some embodiments of the invention, the transdermal delivery composition further comprises an alcohol and/or water and/or an aqueous adjuvant. In some embodiments, the aqueous adjuvant is a plant extract from the family of *Liliaceae,* such as *Aloe Vera*. Other embodiments of the invention include the transdermal delivery composition described above, wherein about 0/1% to 15% by weight or volume is alcohol or 0.1% to 15% is water or both, or wherein about 0.1% to 85% by weight or volume is water or *Aloe Vera* or another aqueous adjuvant.

Alcohol, water, and other aqueous adjuvants are not present in some formulations of the transdermal delivery composition described herein. It has been discovered that some delivered agents (*e.g*., steroids) are soluble and stable in ethoxylated oil in the absence of alcohol or water and some delivered agents are soluble and stable in ethoxylated oil/alcohol emulsions, ethoxylated oil/water emulsions, ethoxylated oil/alcohol/water emulsions, and ethoxylated oil/alcohol/water/*Aloe Vera* emulsions. In particular, it was found that a particular *Aloe Vera*, alcohol, or water mixture was not essential to obtain a transdermal delivery composition provided that an appropriately ethoxylated oil was mixed with the delivered agent. That is, the alcohol, water, and *Aloe Vera* can be removed from the formulation by using a light oil *(e.g.,* macadamia nut oil) that has been ethoxylated to approximately 10-19 ethoxylations/molecule, desirably 11-19 ethoxylations/molecule, more desirably 12-18 ethoxylations/molecule, still more desirably 13-17 ethoxylations/molecule, preferably 14 -16 ethoxylations/molecule and most preferably 15 or 16 ethoxylations/molecule. For example, some ethoxylated oils (*e.g.,* macadamia nut oil comprising, consisting of or consisting essentially of 10, 11, 12, 13, 14, 15, 16, 17, 18, or 19 ethoxylations/molecule) can deliver low and high molecular weight peptides (*e.g*., collagen and fragments of collagen) or amino acids in the absence of alcohol and *Aloe Vera*. Some embodiments, however, have a ratio of ethoxylated lipid:alcohol:aqueous adjuvant selected from the group consisting of 1:1:4, 1:1:14, 3:4:3, and 1:10:25.

In still other embodiments, the transdermal delivery compositions described herein can also include fragrances, creams, bases and other ingredients that stabilize the formulation, facilitate delivery, or protect the delivered agent from degradation (*e.g*., agents that inhibit DNAse, RNAse, or proteases).

The transdermal delivery compositions described herein are useful for the delivery of a wide variety of delivered agents. In certain embodiments, the transdermal delivery composition comprises delivered agents that are hormones. In some embodiments, the delivered agent is a peptide hormone. Non-limiting examples of peptide hormones include oxytocin, vasopressin, melanocyte-stimulating hormone, corticortropin, lipotropin, thyrotropin, growth hormone, prolactin, luteinizing hormone, human chorionic gonadotropin, follicle stimulating hormone, corticotropin-releasing factor, gonadotropin-releasing factor, prolactin-releasing factor, prolactin-inhibiting factor, growth-hormone releasing factor, somatostatin, thyrotropin-releasing factor, calcitonin gene-related peptide, parathyroid hormone, glucagon-like peptide 1, glucose-dependent insulinotropic polypeptide, gastrin, secretin, cholecystokinin, motilin, vasoactive intestinal peptide, substance P, pancreatic polypeptide, peptide tyrosine tyrosine, neuropeptide tyrosine, amphiregulin, insulin, glucagon, placental lactogen, relaxin, angiotensin II, calctriol, atrial natriuretic peptide, melatonin, and insulin.

In other embodiments, the delivered agent is a non-peptide hormone. Non-limiting examples of hormones that are not peptide hormones useful in embodiments include thyroxine, triiodothyronine, calcitonin, estradiol, estrone, progesterone, testosterone, cortisol, corticosterone, aldosterone, epinephrine, norepinepherine, androstiene, or calcitriol.

Other peptides such as collagen, or fragments thereof, are delivered agents in certain embodiments.

In additional embodiments, the delivered agent is a pharmacologically active small compound. For example, in certain embodiments, the delivered agent is an anesthetic such as articaine, procaine, tetracaine, chloroprocaine and benzocaine, novocain, mepivicaine, bupivicaine, benzocaine, or lidocaine. Analgesics are delivered agents in other embodiments. Thus, in certain embodiments the delivered agent is tramadol hydrochloride, fentanyl, metamizole, morphine sulphate, ketorolac tromethamine, hydrocodone, oxycodone, morporine, loxoprofen, Capsaicin, or Boswellin.

Other pharmacologically active compounds that are suitable delivered agents include non-steroidal anti-inflammatory drugs ("NSAIDs"). Thus, in embodiments of the invention the delivered agent is ibuprofen (2-(isobutylphenyl)-propionic acid); methotrexate (N-[4-(2, 4 diamino 6 - pteridinyl - methyl] methylamino] benzoyl)-L-glutamic acid); aspirin (acetylsalicylic acid); salicylic acid; diphenhydramine (2-(diphenylmethoxy)-NN-dimethylethylamine hydrochloride); naproxen (2-naphthaleneacetic acid, 6-methoxy-9-methyl-, sodium salt, (-)); phenylbutazone (4-butyl-1,2-diphenyl-3,5-pyrazolidinedione); sulindac-(2)-5-fluoro-2-methyl-1-[[p-(methylsulfinyl)phenyl]methylene-]-1H-indene-3-acetic acid; diflunisal (2',4', -difluoro-4-hydroxy-3-biphenylcarboxylic acid; piroxicam (4-hydroxy-2-methyl-N-2-pyridinyl-2H-1, 2-benzothiazine-2-carboxamide 1, 1-dioxide, an oxicam; indomethacin (1-(4-chlorobenzoyl)-5-methoxy-2-methyl-H-indole-3-acetic acid); meclofenamate sodium (N-(2, 6-dichloro-m-tolyl) anthranilic acid, sodium salt, monohydrate); ketoprofen (2- (3-benzoylphenyl)-propionic acid; tolmetin sodium (sodium 1-methyl-5-(4-methylbenzoyl-1H-pyrrole-2-acetate dihydrate); diclofenac sodium (2-[(2,6-dichlorophenyl)amino] benzeneatic acid, monosodium salt); hydroxychloroquine sulphate (2-{[4-[(7-chloro-4-quinolyl) amino] pentyl] ethylamino}ethanol sulfate (1:1); penicillamine (3-mercapto-D-valine); flurbiprofen ([1,1-biphenyl]-4-acetic acid, 2-fluoro-alphamethyl-, (+-.)); cetodolac (1-8- diethyl-13,4,9, tetra hydropyrano-[3-4-13] indole-1-acetic acid; mefenamic acid (N-(2,3-xylyl)anthranilic acid; and diphenhydramine hydrochloride (2-diphenyl methoxy-N, N-di-methylethamine hydrochloride).

In other embodiments, the delivered agent is a steroidal anti-inflammatory compound, such as hydrocortisone, prednisolone, triamcinolone, or piroxicam.

In yet other embodiments, the delivered agent is an anti-infective agent. By way of example, in some embodiments, the delivered agent is an antimicrobial or antifungal agent such as amoxicillin, clavulanate potassium, itraconazole, flucanazole, erythromycin ehtysuccinate, acetyl sulfisoxazole, penicillin V, erythromycin, azithromycin, tetracycline, ciproflaxin, gentamycin sulfathiazole. In still other embodiments, the delivered agent is an anti-viral compound, such as for example acyclovir, lamivudine, indinavir sulfate, stavudine, saquinavir, ritonavir or hepsysls.

In still other embodiments, the delivered agent is a nucleic acid. In some embodiments, the nucleic acid is an oligonulcoeitde consisting of cysteine and guanidine, e.g., a CpG molecule. In further embodiments, the nucleic acid is a polynucleotide. In some embodiments, the polynucleotide comprises a nucleic acid sequence that is capable of eliciting an immune response from an animal. For example, in some embodiments, the nucleic acid comprises nucleic acid sequences from HIV, influenza A virus, hepatitis C virus, hepatitis A virus, hepatitis B virus, hantavirus, SARS, or sequences encoding members of the Inhibitor of Apoptosis family of proteins.

Embodiments of the transdermal delivery compositions disclosed herein also include transdermal delivery systems that comprise adjuvants and immunogenic compositions. Thus, some formulations of transdermal delivery compositions comprise an immunogenic peptide or nucleic acid encoding said peptide, a vaccine, such as a DNA vaccine, polypeptide vaccine, or other vaccine, and an adjuvant, such as aluminium hydroxide, calcium phosphate, cytokines (such as, e.g., interleukin-12 (IL-12)), co-stimulatory molecules (such as, e.g., B7-1 (CD80) or B7-2 (CD86)), and haptens, such as dinitrophenyl (DNP), and the like.

In yet other embodiments, the delivered agent is an immune response modifier. For example, in some embodiments, the delivered immune response modifier is an imidazoquinoline amine including, but not limited to, substituted imidazoquinoline amines. For example in some embodiments the delivered agent is an amide substituted imidazoquinoline amine, a sulfonamide substituted imidazoquinoline amine, a urea substituted imidazoquinoline amine, an aryl ether substituted imidazoquinoline amine, a heterocyclic ether substituted imidazoquinoline amine, an amido ether substituted imidazoquinoline amine, a sulfonamido ether substituted imidazoquinoline amine, a urea substituted imidazoquinoline ether, a thioether substituted imidazoquinoline amine, or a 6-, 7-, 8-, or 9-aryl or heteroaryl substituted imidazoquinoline amine. In other embodiments, the delivered agent is a tetrahydroimidazoquinoline amine such as an amide substituted tetrahydroimidazoquinoline amine, a sulfonamide substituted tetrahydroimidazoquinoline amine, a urea substituted tetrahydroimidazoquinoline amine, an aryl ether substituted tetrahydroimidazoquinoline amine, a heterocyclic ether substituted tetrahydroimidazoquinoline amine, an amido ether substituted tetrahydroimidazoquinoline amine, a sulfonamido ether substituted tetrahydroimidazoquinoline amine, a urea substituted tetrahydroimidazoquinoline ether, or a thioether substituted tetrahydroimidazoquinoline amine. In other embodiments, the deleivered agent is an imidazopyridine amine such as an amide substituted imidazopyridine amine, a sulfonamide substituted imidazopyridine amine, a urea substituted imidazopyridine amine, an aryl ether substituted imidazopyridine amine, a heterocyclic ether substituted imidazopyridine amine, an amido ether substituted imidazopyridine amine, a sulfonamido ether substituted imidazopyridine amine, a urea substituted imidazopyridine ether, or a thioether substituted imidazopyridine amines. In yet other embodiments, the delivered agent is a 1,2-bridged imidazoquinoline amine; 6,7-fused cycloalkylimidazopyridine amine, a idazonaphthyridine amine, a tetrahydroimidazonaphthyridine amines, an oxazoloquinoline amine, a thiazoloquinoline amine; an oxazolopyridine amine, a thiazolopyridine amine, a oxazolonaphthyridine amine, a thiazolonaphthyridine amine, a 1H-imidazo dimers fused to a pyridine amine, a quinoline amine, a tetrahydroquinoline amine, a naphthyridine amine, or a tetrahydronaphthyridine amine.

In further embodiments, the immune response modifier is a purine derivative, an imidazoquinoline amide derivative, a 1H-imidazopyridine derivative, a benzimidazole derivatives, a derivative of a 4-aminopyrimidine fused to a five membered nitrogen containing heterocyclic ring (including adenine derivatives), a 3-.beta.-D-ribofuranosylthiazolo[4,5-d]pyri- midine derivative, or a 1H-imidazopyridine derivatives.

Examples of particular immune response modifier compounds useful as delivered agents include 2-propyl[1,3]thiazolo[4,5-c]quinolin-4-amine, 4-amino-.alpha.,.alpha.-dimethyl-1H-imidazo[4,5-c]quinoline-1-ethanol, and 4-amino-2-(ethoxymethyl)-.alpha.,.alpha.-dimethyl-6,7- ,8,9-tetrahydro-1H-imidazo[4,5-c]quinoline-1-ethanol. Other examples of Immune response modifier compounds include N-[4-(4-amino-2-butyl-1H-imidazo[4,5-c][1,5]naphthyridin-1-yl)butyl]-N'-c- yclohexylurea, 2-methyl-1-(2-methylpropyl)-1H-imidazo[4,5-c][1,5]naphthyri- din-4-amine, 1-(2-methylpropyl)-1H-imidazo[4,5-c][1,5]naphthyridin-4-amine-, N-{2-[4-amino-2-(ethoxymethyl)-1H-imidazo[4,5-c]quinolin-1-yl]-1,1-dimet- hylethyl}methanesulfonamide, N-[4-(4-amino-2-ethyl-1H-imidazo[4,5-c]quinol- in-1-yl)butyl]methanesulfonamide, 2-methyl-1-[5-(methylsulfonyl)pentyl]-1H- -imidazo[4,5-c]quinolin-4-amine, N-[4-(4-amino-2-propyl-1H-imidazo[4,5-c]q- uinolin-1-yl)butyl]methanesulfonamide, 2-butyl-1-[3-(methylsulfonyl)propyl- ]-1H-imidazo[4,5-c]quinoline-4-amine, 2-butyl-1-{2-[(1-methylethyl)sulfony- 1]ethyl}-1H-imidazo[4,5-c]quinolin-4-amine, N-{2-[4-amino-2-(ethoxymethyl)- -1H-imidazo[4,5-c]quinolin-1-yl]-1,1-dimethylethyl}-N'-cyclohexylurea, N-{2-[4-amino-2-(ethoxymethyl)-1H-imidazo[4,5-c]quinolin-1-yl]-1,1-dimethylethyl}cyclohexanecarboxamide, N-{2-[4-amino-2-(ethoxymethyl)-1H-imidazo[- 4,5-c]quinolin-1-yl]ethyl}-N'-isopropylurea. Resiquimod, and 4-amino-2-ethoxymethyl-.alpha.,.alpha.-dimethyl-1H-imidazo[4,5-c]quinolin- e-1-ethanol.

In certain embodiments, the delivered agent is an analgesic. Non-limiting examples of analgesiscs include tramadol hydrochloride, fentanyl, metamizole, morphine sulphate, ketorolac tromethamine, morphine, and loxoprofen sodium. In other embodiments, the delivered agent is a migraine therapeutic, such as ergotamine, melatonin, sumatriptan, zolmitriptan, or rizatriptan.

In yet other embodiments, the delivered agent is an imaging component, such as iohexol, technetium, TC99M, sestamibi, iomeprol, gadodiamide, oiversol, and iopromide. Diagnostic contrast components such as alsactide, americium, betazole, histamine, mannitol, metyraphone, petagastrin, phentolamine, radioactive B12, gadodiamide, gadopentetic acid, gadoteridol, perflubron are delivered agents in certain embodiments.

Another aspect of the invention concerns methods of making lipopsheres useful for transdermal delivery of a delivered agent. In one embodiment, a liposphere is made by identifying a delivered agent for incorporation into a liposphere and mixing said delivered agent with an ethoxylated or propoxylated fatty moiety, ethoxylated or propoxylated lipid moiety, or ethoxylated or propoxylated multifunctional backbone, wherein said ethoxylated fatty moiety, lipid moiety, or multifunctional backbone has between 10 and 19 ethoxylations per molecule. In preferred embodiments, the fatty moiety, or at least one of the fatty components of the lipid moiety or multifunctional backbone has a carbon chain length of between about 10 and 24 carbon residues.

The formulations described herein are placed into a vessel that is joined to an applicator such that the active ingredients can be easily provided to a subject. Applicators include, but are not limited to, roll-ons, bottles, jars, tubes, sprayer, atomizers, brushes, swabs, gel dispensing devices, and other dispensing devices.

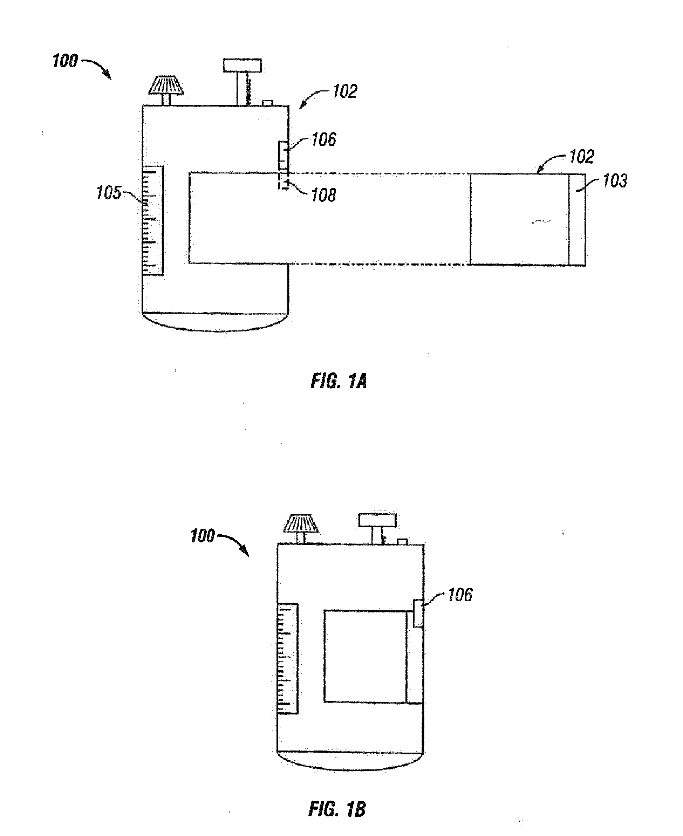

Aspects of the present invention also concern compositions comprising a transdermal delivery and a transdermal delivery device, which provides a measured amount of said transdermal delivery system. Accordingly, desired dosages of delivered agents can be delivered to a subject in need. An exemplary transdermal delivery device is depicted in Figures 1A-4B.

Yet other aspects of the present invention relate to methods of delivering an amount of a transdermal delivery composition comprising providing a transdermal delivery composition within a transdermal delivery device, wherein the device is designed to administer a measured amount of the transdermal delivery composition and providing a transdermal delivery composition to be administered to a subject.

Several methods of using the transdermal delivery compositions are also embodiments. For example, one approach involves a method of reducing pain or inflammation by using a transdermal delivery composition that comprises an anti-inflammatory molecule (e.g., an NSAID or MSM) on a subject in need of a reduction of pain or inflammation. Monitoring the reduction in inflammation may also be desired as part of a rehabilitation program.

NSAIDs and other chemotherapeutic agents have also been shown to improve the health, welfare, or survival of subjects that have cancer or Alzheimer's disease. The tendency of these compounds to cause adverse side effects such as gastrointestinal irritation liver and kidney problems renders them particularly desirable transdermal delivery agents. Accordingly, some embodiments concern methods of using transdermal delivery compositions that comprise delivered agents (e.g., any one or combination of the NSAIDs disclosed above or other chemotherapeutic agents such as fluorouracil) to treat or prevent cancer or hyperproliferative cell disorders (e.g., basal cell carcinoma or actinic keratosis.) For example, a method to improve the health, welfare, or survival of a subject that has cancer or Alzheimer's disease or a method of treating or preventing cancer or Alzheimer's disease in said subject can be conducted by using a transdermal delivery composition that comprises a COX enzyme inhibitor and providing said transdermal delivery composition to said subject.

Some formulations of transdermal delivery compositions can be used to reduce oxidative stress to cells, tissues and the body of a subject. For example, a method to improve the health, welfare, or survival of a subject that is in need of a reduction in oxidative stress to a cell, tissue, or the body as a whole involves providing to said subject a transdermal delivery composition that comprises an antioxidant such as ascorbic acid, tocopherol or tocotrienol or an anti-stress compound such as Bacocalmine (Bacopa Monniera Extract obtained from Sederma Laboratories). Methods of treating or preventing diseases or conditions associated with oxidative stress or vitamin deficiency and methods of reducing an oxidative stress or a vitamin deficiency in a subject in need thereof are also embodiments.

Other formulations of transdermal delivery composition can be used to reduce psoriasis or eczema or a related condition or can be used to promote wound healing in a subject in need thereof. By one approach, a transdermal delivery composition that comprises peptides that promote wound healing (*e.g*., peptides comprising the sequence LKEKK **(SEQ. ID. NO:1**), are provided to a subject in need of a treatment or reduction in psoriasis or eczema or a condition associated with psoriasis or eczema (*e.g*., allergies) or treatment of a wound.

An exemplary formulation for the treatment of Psoriasis, or Eczema is as follows:

| | |
|---|---|
| Hepsyl | 2 Grams |
| Distilled Water with Sodium Bi Carbonate (to alter ph to 8.4 +/- 0.2) | 18 mls |
| Ethoxylated Macadamia Nut Oil | 20 mls |
| Ethyl Alcohol Anhydrous | 20 mls |

Admix in the listed order and pour off into 60 ml roll on bottles and apply directly to affected areas morning and evening.

Other formulations of transdermal delivery composition can be used to relax the muscles of a subject. By one approach, a transdermal delivery composition that comprises a compound that relaxes the muscles (*e.g*., chlorzoxazone or ibuprofen) is provided to a subject in need of a muscle relaxant. Accordingly methods of treating or preventing muscle soreness are embodiments.

Other formulations of transdermal delivery composition can be used to raise the levels of a hormone in a subject in need thereof. By one approach, a transdermal delivery composition that comprises a hormone (*e.g*., any one of or combination of the hormones disclosed above or derivatives or functional analogues thereof) is provided to a subject in need thereof. Accordingly methods of treating or preventing a hormone deficiency or methods of increasing the level of a hormone in a subject using one of the transdermal delivery compositions described herein are embodiments.

Other formulations of transdermal delivery composition can be used to raise the levels of a hormone, for example, growth factor in a subject in need thereof. By one approach, a transdermal delivery composition that comprises a growth factor *(e.g.,* a growth factor contained in Bioserum, which is obtainable through Atrium Biotechnologies of Quebec City, Canada) is provided to a subject in need thereof. In other embodiments, a transdermal delivery composition comprising a peptide that comprises the sequence LKEKK (SEQ ID NO:1) is provided to a subject in need of an increase in a growth factor. Accordingly methods of treating or preventing a growth factor deficiency or methods of increasing the level of a growth factor in a subject using one of the transdermal delivery compositions described herein are embodiments. By another approach, a transdermal delivery composition that comprises oxytocin, vasopressin, insulin, melanocyte-stimulating hormone, corticortropin, lipotropin, thyrotropin, growth hormone, prolactin, luteinizing hormone, human chorionic gonadotropin, follicle stimulating hormone, corticotropin-releasing factor, gonadotropin-releasing factor, prolactin-releasing factor, prolactin-inhibiting factor, growth-hormone releasing factor, somatostatin, thyrotropin-releasing factor, calcitonin, calcitonin gene-related peptide, parathyroid hormone, glucagon-like peptide 1, glucose-dependent insulinotropic polypeptide, gastrin, secretin, cholecystokinin, motilin, vasoactive intestinal peptide, substance P, pancreatic polypeptide, peptide tyrosine tyrosine, neuropeptide tyrosine, amphiregulin, insulin, glucagon, placental lactogen, relaxin, angiotensin II, atrial natriuretic peptide, melatonin, thyroxine, triiodothyronine, estradiol, estrone, progesterone, testosterone, cortisol, corticosterone, aldosterone, epinephrine, norepinepherine, or calctriol, is provided to a subject in need of the same.

Other formulations of the transdermal delivery composition described herein are used to brighten the skin, reduce age spots or skin discolorations, reduce stretch marks, reduce spider veins, or add dyes, inks, (*e.g*., tattoo ink), perfumes, or fragrances to the skin of a subject. In some embodiments, for example, transdermal delivery compositions that comprise a compound that brightens the skin or reduces age spots or skin discolorations (*e.g*., Melaslow, a citrus-based melanin (tyrosinase) inhibitor obtainable from Revivre Laboratories of Singapore or Etioline, a skin brightener made from an extract from the *Mitracarpe* leaf obtainable from Krobell, USA), or a compound that reduces stretch marks (Kayuuputih Eucalyptus Oil, obtainable from Striad Laboratories) or add dyes, inks, (e.g., tattoo ink), perfumes, or fragrances are provided to the skin of a subject.

It has also been discovered that ethoxylated oil by itself, preferably macadamia nut oil having 10-19 ethoxylations/molecule (*i.e*., 10, 11, 12, 13, 14, 15, 16, 17, 18, or 19 ethoxylations/molecule), has therapeutic and cosmetic properties. For example, application of an ethoxylated oil (e.g., macadamia nut oil having 16 ethoxylations/molecule) was found to reduce stretch marks and spider veins on a subject in need thereof. Application of an ethoxylated oil (e.g., macadamia nut oil having 16 ethoxylations/molecule) to a burn *(e.g.,* a sun burn or a skin burn obtained from overheated metal) was found to significantly expedite recovery from the burn, oftentimes without blistering. Accordingly, some embodiments concern a transdermal delivery composition comprising an ethoxylated oil (*e.g*., macadamia nut oil that was ethoxylated 10-19 ethoxylations per molecule, 11-19 per molecule, 12-18 ethoxylations per molecule, 13-17 ethoxylations per molecule, 14-16 ethoxylations per molecule, or 15 ethoxylations per molecule) and these compositions are used to reduce the appearance of stretch marks and spider veins or facilitate the recovery from burns of the skin.

In addition to the delivery of low and medium molecular weight delivered agents, several compositions that have high molecular weight delivered agents (*e.g*., collagens) and methods of use of such compositions are embodiments of the invention. Preferred formulations of the transdermal delivery composition comprise a collagen (natural or synthetic) or fragment thereof at least 2, 4, 6, 8, 10, 12, 14, 16, 18, 20, 24, 30, 40, 50, 100, 250, 500, 1000, 1500, 2000, 2500, 3000, 5000, or more amino acids in length and these compositions are used to reduce wrinkles and fine lines on a subject.

For example, some embodiments concern a transdermal delivery composition comprising an ethoxylated fatty moiety, an ethoxylated lipid (*e.g*., macadamia nut oil that was ethoxylated 10-19 ethoxylations per molecule, 11-19 per molecule, 12-18 ethoxylations per molecule, 13-17 ethoxylations per molecule, 14-16 ethoxylations per molecule, or 15 ethoxylations per molecule), or an ethoxylated transdermal delivery enhancer and a therapeutically effective amount of a collagen or fragment thereof (*e.g.,* marine collagen). In some aspects of the invention, a transdermal delivery composition comprising an ethoxylated oil and collagen also contains water and/or an alcohol and/or an aqueous adjuvant such as *Aloe Vera*.

In different embodiments of this transdermal delivery composition, the collagen has a molecular weight less than, or equal to 6,000 daltons or greater than 6,000 daltons. Thus, in some embodiments, the collagen can have an approximate molecular weight as low as 2,000 daltons or lower. In other embodiments, the molecular weight is from about 300,000 daltons to about 500,000 daltons. Further, these transdermal delivery compositions can have a therapeutically effective amount of collagen or fragment thereof by weight or volume that is 0.1% to 85.0%. The collagen can be any natural or synthetic collagen, for example, Hydrocoll EN-55, bovine collagen, human collagen, a collagen derivative, marine collagen, Solu-Coll, or Plantsol, recombinant or otherwise man made collagens or derivatives or modified versions thereof (*e.g*., protease resistant collagens). As above, an apparatus having a vessel joined to an applicator that houses the transdermal delivery composition containing collagen is also an embodiment and preferred applicators or dispensers include a roll-on or a sprayer.

Accordingly, some of the embodied methods concern the reduction of wrinkles and or the improvement of skin tone by using a transdermal delivery composition comprising an ethoxylated oil and a collagen and/or a fragment thereof. Some formulations to be used to reduce wrinkles and improve skin tone include an ethoxylated fatty moiety, an ethoxylated lipid moiety (*e.g*., macadamia nut oil that was ethoxylated 10-19 ethoxylations per molecule, 11-19 per molecule, 12-18 ethoxylations per molecule, 13-17 ethoxylations per molecule, 14-16 ethoxylations per molecule, or 15 ethoxylations per molecule), or an ethoxylated transdermal delivery enhancer, and a therapeutically effective amount of a collagen or fragment thereof (*e.g*., marine collagen) that is at least 2, 4, 6, 8, 10, 12, 14, 16, 18, 20, 24, 30, or 40 amino acids in length. Some formulations that can be used to practice the method above include a transdermal delivery composition comprising an ethoxylated oil and collagen or fragment thereof, as described above, and, optionally, water and/or an alcohol and/or an aqueous adjuvant such as *Aloe Vera*. For example, by one approach, a method of reducing wrinkles or improving skin tone is practiced by identifying a subject in need thereof and providing said subject a transdermal delivery composition, as described herein and, optionally, monitoring the subject for restoration or improvement of skin tone and the reduction of wrinkles.

### BRIEF DESCRIPTION OF THE DRAWINGS

Embodiments of a transdermal delivery device are depicted in Figures 1A through 4B. In aspects of the invention, a transdermal delivery system comprises a transdermal delivery composition and a transdermal delivery device.
Figure 1A schematically depicts in an exploded state a dispenser for delivery of a transdermal drug delivery system fluid comprising a removable cartridge.
Figure 1B schematically depicts the dispenser of Figure 1A in an assembled state.
Figure 2 schematically depicts a cross section of the dispenser of Figure 1B.
Figure 3A schematically depicts a cross section of the upper portion of a partially filled dosing chamber having an upper wall configured allow air to escape, but prevent fluid from escaping.
Figure 3B schematically depicts the upper portion of the dosing chamber of Figure 3A, where the dosing chamber is full and fluid is prevented from escaping.
Figure 4A schematically depicts a cross-section of the dispenser of Figure 2, taken along the line 4, wherein the slidable member is in a first position permitting filling of the dosing chamber.
Figure 4B schematically depicts the cross-section of Figure 4A, wherein the slidable member is in a second position permitting delivery of the dosed fluid.

### DETAILED DESCRIPTION OF THE INVENTION

Several transdermal delivery compositions and devices for providing said compositions to a subject are described herein. Embodiments of the invention can be used to transdermally deliver low or high (or both low and high) molecular weight pharmaceuticals, prophylactics, diagnostics, and cosmetic agents to a subject. The transdermal delivery compositions disclosed herein are useful for the delivery of various types of compounds including but not limited to nucleic acids, peptides, modified peptides, small molecules, immunogenic preparations, and the like. Some embodiments include transdermal delivery compositions that can administer compounds having molecular weights greater than 6,000 daltons. One embodiment, for example, includes a transdermal delivery composition that can administer a therapeutically effective amount of a non-steroidal anti-inflammatory drug (NSAID). Still more embodiments concern transdermal delivery compositions that can administer hormones, anesthetics, collagen preparations *e.g.,* soluble collagens, hydrolyzed collagens, and fragments of collagen), cardiovascular pharmaceutical compounds, anti-infective compounds (e.g. antibiotics and antiviral compounds), diabetes-related treatments, immunogenic compositions, vaccines, immune response modifiers, enzyme inhibitors, analgesics (*e.g.,* a formulation comprising capsaicin or Boswellin or both), migraine therapies, sedatives, imaging and contrast compounds. These examples are provided to demonstrate that embodiments of the invention can be used to transdermally deliver both low and high molecular weight compounds and it should be understood that many other molecules can be effectively delivered to the body, using the embodiments described herein, in amounts that are therapeutically, prophylactically, or cosmetically beneficial.

Some transdermal delivery compositions described herein comprise a liposphere that is configured to deliver a wide variety of delivered agents. As used herein, the term "liposphere" refers to a spherical or ovoid-shaped structure comprising an ethoxylated or propoxylated fatty moiety, which contains or is associated with (e.g., joined to) a delivered agent. That is, the term "lipospheres" includes, but is not limited to, liposomes that comprise an ethoxylated or propoxylated oil, fatty acid, fatty amine, or fatty alcohol. Accordingly, the term "fatty moiety" can refer to a fatty acid, a fatty alcohol, fatty amine, or other fatty acid derivative. The ethoxylated fatty moiety or lipid moiety has both hydrophobic and hydrophilic properties, in that the hydrocarbon chain of the fatty moiety or lipid moiety is hydrophobic, and the polyethoxy groups confer hydrophilicity on the molecule. The preparation of propoxylated fatty moieties and lipid moieties is well known. (*See, e.g.,* Raths et al., supra). Due to their similarity in structure, propoxylated fatty moieties and lipids will share many of the same characteristics as ethoxylated fatty moieties and lipids. Accordingly, fatty moieties and lipid moieties that are propoxylated or ethoxylated and propoxylated are contemplated penetration enhancers and transdermal delivery enhancers.

In the embodiments disclosed herein, the number of ethoxylations is adjusted to between 10 and 19 ethoxylations per molecule to achieve optimal transdermal delivery of the delivered agent. Ethoxylated fatty acids, fatty alcohols, and fatty amines, are commercially available (*e.g.,* Ethox Chemicals, LLC, Greenville, SC; A&E Connock, Ltd., Hampshire, England; Floratech, Glibert, AZ). Alternatively ethoxylated fatty moieties are synthesized using methods known to those skilled in the art *(See,* U.S. Patent No. 6,300,508 to Raths et al.; U.S. Patent No. 5,936,107 to Raths et al.) by reacting fatty moieties with ethylene oxide.

By way of example, ethoxylated oils can be prepared using a two-step process that starts with trans-esterification with added glycerol followed by ethoxylation of the product of this reaction. Trans-esterification is performed by any method available to those skilled in the art, such as heating an ester, such as the glycerol esters present in natural vegetable oils, in the presence of another alcohol or polyol, such as glycerol, in the presence of a catalyst. Catalysts useful in the transesterification reaction include gaseous catalysts, such hydrochloric acid bubbled through the reaction mixture. Alternatively, solid catalysts such as zinc oxide or the acetates of copper, cobalt or zinc can also be used. The transesterificaiton reaction produces one or two fatty acids attached to a molecule of glycerol. The ratio of mono- and di-esters can be controlled by the amount of glycerol used in the reaction (i.e. higher ratios of glycerol:oil will yield more reactive -OH and fewer fatty acid moieties per molecule, and a lower ratio of glycerol:oil would give more fatty acids, as is apparent to those skilled in the art. The hydroxyl groups are subsequently reacted with ethylene oxide in the presence of an appropriate catalyst, (e.g., aluminum) using methods known to those skilled in the art.

Purified fatty moieties commercially available from a variety of sources (*e.g.,* SIGMA-Aldrich, St. Louis, MO) are suitable for use in the transdermal delivery compositions described above.

Alternative embodiments of transdermal delivery compositions described herein comprise a penetration enhancer that includes an ethoxylated lipid moiety. It was discovered that ethoxylated lipids *(e.g.,* ethoxylated oils) can be used as transdermal penetration enhancers in that they effectively transport low and high molecular weight compounds through the skin. It was also discovered that ethoxylated oils, by themselves, have therapeutic and cosmetic applications *(e.g.,* the reduction of the appearance of spider veins and stretch marks or promoting expedited recovery from burns to the skin). Ethoxylated lipids can be created in many ways, however, a preferred approach involves the reaction of ethylene oxide with a vegetable, nut (*e.g.,* macadamia nut), animal, or synthetic oil. In embodiments where the transdermal delivery composition comprises an ethoxylated oil, it is contemplated that in some embodiments, ethoxylated fatty moieties are used to fortify or supplement ethoxylated oils in some embodiments. By way of example, ethoxylated macadamia nut oil can be fortified with ethoxylated palmitic or oleic acid.

Several transdermal delivery enhancers disclosed herein are compounds having a multifunctional backbone. The multifunctional backbone can be one of many chemical structures that have at least two reactive hydrogen residues, such that the multifunctional backbone is the basis of a transdermal delivery enhancer with least one fatty moiety and at least one polyethoxy group. The reactive hydrogen residues (R) are present in -OH, COOH, SH, and NH₂, groups.

The polyethoxy group has the structure:

-O-(CH₂-CH₂-O-)ₙH

Embodiments wherein n is between 10 and 19 per molecule of transdermal delivery enhancer to possess superior transdermal delivery properties.

In preferred embodiments, the fatty moiety component of the transdermal delivery enhancer has a carbon chain of at least 10 carbon residues. The chain length of the fatty moiety can be for example 10, 12, 14, 16, 18, 20, 22, or 24 residues. Further, the fatty moiety may be saturated, unsaturated, or polyunsaturated.

Desirably, the multifunctional backbone has at least three reactive groups. The reactive groups can be homogeneous. For example, in some embodiments, the multifunctional backbone is a tri-alcohol comprising three -OH groups, such as 1, 2, 3-butanetriol, 1, 2, 4 butantetriol, pyrogallol (1, 2, 3-benezentriol), hydroxyquinol (1, 2, 4-benzenetriol), trimethyololpropane, 1, 2, 6-hexanetriol and the like. Other examples of multifunctional backbones suitable as the foundation of a transdermal delivery enhancer include tri-acids, comprising three carboxylate groups, such as hemi-mellitic acid, trimellitic acid, trimesic acid, nitrilotriacetic acid, and the like. Those skilled in the art will appreciate that other tricarboxylic acids are suitable as multifunctional backbones.

Alternative multifunctional backbones have heterogeneous reactive groups, e.g., a combination of at least two different reactive groups (e.g., a COOH group and an NH₂ group). For example, amino acids such as glutamic acid, aspartic acid, cysteine, glutamine, serine, threonine, tryrosine, and lysine have three reactive groups and are suitable as multifunctional backbones. Similarly, di- and tri-peptides will have three or more reactive groups and are thus suitable as multifunctional backbones.

Triethanolamine, diethanolamine, dimethylolurea, and glucosamine are other exemplary multifunctional backbones with heterogeneous reactive groups.

Simple carbohydrates are small straight-chain aldehydes and ketones with several hydroxyl groups, usually one on each carbon except the functional group. Due to the presence of the multiple -OH groups on carbohydrates such as tetroses, pentoses, hexoses, and so forth, these compounds are another source of multifunctional backbones useful as components of transdermal delivery enhancers. Exemplary carbohydrates that are useful components of transdermal delivery enhancers include glucose, mannose, fructose, ribose, xylose, threose, erythrose, and the like.

Sugar alcohols such as sorbitol, mannitol, xylitol, erythritol, petaerythritol, and inositol are useful components of transdermal delivery enhancers.

Not wanting to be tied to any particular mechanism or mode of action and offered only to expand the knowledge in the field, it is contemplated that the ethoxylated fatty moiety, ethoxylated lipid moiety, or ethoxylated multifunctional backbone encapsulates the delivered agent in a sphere-like composition, forming a "liposphere" that exhibits greatly enhanced transdermal delivery properties.

Each of the disclosed transdermal delivery compositions can contain additional compounds such as alcohols, nonionic solubilizers or emulsifiers. In some compositions, these compounds are added to improve the solubility of the delivered agent or effectiveness or fluidity of the liposphere, penetration enhancer, or transdermal delivery enhancer. Suitable hydrophilic components include, but are not limited to, ethylene glycol, propylene glycol, dimethyl sulfoxide (DMSO), dimethyl polysiloxane (DMPX), oleic acid, caprylic acid, isopropyl alcohol, 1-octanol, ethanol (denatured or anhydrous), and other pharmaceutical grade or absolute alcohols.

Other embodiments of the transdermal delivery compositions comprise an aqueous adjuvant. Aqueous adjuvants include, but are not limited to, water (distilled, deionized, filtered, or otherwise prepared), *Aloe Vera* juice, and other plant extracts such as chlorophyll or *Spirulina*. Thus, several embodiments of the invention have a hydrophobic/hydrophilic component comprising an ethoxylated fatty moiety (*e.g.,* palmitoleic acid, oleic acid, or palmitic acid) or an ethoxylated oil (*e.g.,* macadamia nut oil, coconut oil, eucalyptus oil, synthetic oils, castor oil, glycerol, corn oil, jojoba oil, or emu oil) and may contain a hydrophilic component comprising an alcohol, a nonionic solubilizer, or an emulsifier (*e.g.,* isopropyl alcohol) and/or, optionally, an aqueous adjuvant, such as water and/or *Aloe Vera* extract.

Other materials can also be components of a transdermal delivery composition of the invention including fragrance, creams, ointments, colorings, and other compounds so long as the added component does not deleteriously affect transdermal delivery of the delivered agent. It has been found that the *Aloe Vera*, which allows for transdermal delivery of high molecular weight delivered agents, including collagen having an average molecular weight greater than 6,000 daltons, can be removed from transdermal delivery compositions comprising a light oil (*e.g.,* macadamia nut oil) that has been ethoxylated to the range of 10 - 19 ethoxylations/molecule. Formulations lacking *Aloe Vera* provide the unexpected benefit of efficient transdermal delivery, uniform application and quick penetration making these formulations superior to formulations that contain *Aloe Vera*.

Similarly, formulations of transdermal delivery compositions that lack alcohol provide the unexpected benefit of efficient transdermal delivery, uniform application, and quick penetration without the drying or irritation brought about by the alcohol. Additionally, formulations lacking water or other aqueous adjuvants provide efficient transdermal delivery while maintaining the highest possible concentration of delivered agent and, also, provide for quick penetration without the skin-drying effects seen with some formulations that contain alcohol.

A molecule or a mixture of molecules (*e.g.,* a pharmaceutical, chemical, or cosmetic agent) that are delivered to the body using an embodiment of a transdermal delivery composition are termed "delivered agents". A delivered agent that can be administered to the body using an embodiment of the invention can include, for example, a protein or peptide, a sugar, a nucleic acid, a chemical, a lipid, or derivatives of the same. Desirable delivered agents include, but are not limited to, glycoproteins, enzymes, genes, nucleic acids, peptides, drugs, and ceramides. Preferred delivered agents include NSAIDS, collagens or fragments thereof, capsaicin, and Boswellin. In some embodiments, a transdermal delivery composition comprises a combination of any two of the aforementioned delivered agents. Other delivered agents include, for example, hormones, anti-inflammatory drugs, anesthetics, analgesics, sedatives, migraine therapies, cardiovascular pharmaceuticals, anti-infective agents, diabetes-related therapies, vaccines, imaging agents, contrast agents, glucosamine, chondroitin sulfate, MSM, perfumes, melasin, nicotine, nicotine analogs, peptides, amino acids, nucleic acids, and peptidomimetics.

In addition to the aforementioned compositions, methods of making and using the embodiments of the invention are provided. In one aspect, a transdermal delivery composition is prepared by mixing an ethoxylated fatty moiety with a delivered agent.

In another aspect, a transdermal delivery composition is prepared by mixing a hydrophilic component with a hydrophobic component and an aqueous adjuvant. Depending on the solubility of the delivered agent, the delivered agent can be solubilized in either the ethoxylated oil, a hydrophobic, hydrophilic, or aqueous adjuvant or water prior to mixing.

In addition to physical mixing techniques, (*e.g.,* magnetic stirring or rocker stirring), embodiments of the methods contemplate heat can be applied to help coalesce the mixture. Desirably, the temperature is not raised above 40°C.

Several formulations of transdermal delivery compositions are within the scope of aspects of the invention. In embodiments wherein the transdermal delivery composition includes an aqueous adjuvant, in further embodiments, the formulation comprises a ratio of hydrophilic component:hydrophobic component:aqueous adjuvant of 3:4:3. The amount of delivered agent that is incorporated into the penetration enhancer depends on the compound, desired dosage, and application. The amount of delivered agent in a particular formulation can be expressed in terms of percentage by weight, percentage by volume, or concentration. Several specific formulations of delivery systems are provided in the Examples described herein.

Methods of treatment and prevention of pain, inflammation, and human disease are also provided. In some embodiments, a transdermal delivery composition comprising an NSAID, capsaicin, Boswellin or any combination thereof is provided to a patient in need of treatment, such as for relief of pain and/or inflammation. A patient can be contacted with the transdermal delivery composition and treatment continued for a time sufficient to reduce pain or inflammation or inhibit the progress of disease.

Additionally, a method of reducing wrinkles, removing age spots, and increasing skin tightness and flexibility is provided. By this approach, a transdermal delivery composition comprising a collagen or fragment thereof or melaslow or other skin brightening agent is provided to a patient in need, the patient is contacted with the transdermal delivery composition, and treatment is continued for a time sufficient to restore a desired skin tone (*e.g.,* reduce wrinkles, age spots, or restore skin brightness, tightness and flexibility). In the disclosure below, there is provided a description of several of the delivered agents that can be incorporated into the transdermal delivery compositions described herein.

### Delivered agents

Many different delivered agents can be incorporated into the various transdermal delivery compositions described herein. While the transdermal delivery of molecules having a molecular weight in the vicinity of 6000 daltons has been reported, it has not been possible, until the present invention, to administer molecules of greater size transdermally. *(See* U.S. Pat. No. 5,614,212 to D'Angelo et al.).

The described embodiments can be organized according to their ability to deliver a low or high molecular weight delivered agent. Low molecular weight molecules (*e.g.,* a molecule having a molecular weight less than 6,000 daltons) can be effectively delivered using an embodiment of the invention and high molecular weight molecules (*e.g.,* a molecule having a molecular weight greater than 6,000 daltons) can be effectively delivered using an embodiment of the invention. Desirably, a transdermal delivery composition described herein provides a therapeutically, prophylactically, diagnostically, or cosmetically beneficial amount of a delivered agent having a molecular weight of 50 daltons to less than 6,000 daltons. Preferably, however, a transdermal delivery composition described herein provides a therapeutically, prophylactically, diagnostically, or cosmetically beneficial amount of a delivered agent having a molecular weight of 50 daltons to 2,000,000 daltons or less. That is, a transdermal delivery composition described herein, preferably, provides a delivered agent having a molecular weight of less than or equal to or greater than 50, 100, 200, 500, 1,000, 1,500, 2,000, 2,500, 3,000, 3,500, 4,000, 4,500, 5,000, 5,500, 6,000, 7,000, 8,000, 9,000, 10,000, 11,000, 12,000, 13,000, 14,000, 15,000, 16,000, 17,000, 18,000, 19,000, 20,000, 21,000, 22,000, 23,000, 24,000, 25,000, 26,000, 27,000, 28,000, 29,000, 30,000, 31,000, 32,000, 33,000, 34,000, 35,000, 36,000, 37,000, 38,000, 39,000, 40,000, 41,000, 42,000, 43,000, 44,000, 45,000, 46,000, 47,000, 48,000, 49,000, 50,000, 51,000, 52,000, 53,000, 54,000, 55,000, 56,000, 57,000, 58,000, 59,000, 60,000, 61,000, 62,000, 63,000, 64,000, 65,000, 66,000, 67,000, 68,000, 69,000, 70,000, 75,000, 80,000, 85,000, 90,000, 95,000, 100,000, 125,000, 150,000, 175,000, 200,000, 225,000, 250,000, 275,000, 300,000, 350,000, 400,000, 450,000, 500,000, 600,000, 700,000, 800,000, 900,000, 1,000,000, 1,500,000, 1,750,000, and 2,000,000 daltons.

In one aspect, a low molecular weight compound (*e.g*., a pain relieving substance or mixture of pain relieving substances) is transdermally delivered to cells of the body using an embodiment described herein. The delivered agent can be, for example, any one or more of a number of compounds, including non-steroidal anti-inflammatory drugs (NSAIDs) that are frequently administered systemically. These include ibuprofen (2-(isobutylphenyl)-propionic acid); methotrexate (N-[4-(2, 4 diamino 6 - pteridinyl - methyl] methylamino] benzoyl)-L-glutamic acid); aspirin (acetylsalicylic acid); salicylic acid; diphenhydramine (2-(diphenylmethoxy)-NN-dimethylethylamine hydrochloride); naproxen (2-naphthaleneacetic acid, 6-methoxy-9-methyl-, sodium salt, (-)); phenylbutazone (4-butyl-1,2-diphenyl-3,5-pyrazolidinedione); sulindac-(2)-5-fluoro-2-methyl-1-[[p-(methylsulfinyl)phenyl]methylene-]-1H-indene-3-acetic acid; diflunisal (2',4', -difluoro-4-hydroxy-3-biphenylcarboxylic acid; piroxicam (4-hydroxy-2-methyl-N-2-pyridinyl-2H-1, 2-benzothiazine-2-carboxamide 1, 1-dioxide, an oxicam; indomethacin (1-(4-chlorobenzoyl)-5-methoxy-2-methyl-H-indole-3-acetic acid); meclofenamate sodium (N-(2, 6-dichloro-m-tolyl) anthranilic acid, sodium salt, monohydrate); ketoprofen (2- (3-benzoylphenyl)-propionic acid; tolmetin sodium (sodium 1-methyl-5-(4-methylbenzoyl-1H-pyrrole-2-acetate dihydrate); diclofenac sodium (2-[(2,6-dichlorophenyl)amino] benzeneatic acid, monosodium salt); hydroxychloroquine sulphate (2-{[4-[(7-chloro-4-quinolyl) amino] pentyl]ethylamino}ethanol sulfate (1:1); penicillamine (3-mercapto-D-valine); flurbiprofen ([1,1-biphenyl]-4-acetic acid, 2-fluoro-alphamethyl-, (+-.)); cetodolac (1-8- diethyl-13,4,9, tetra hydropyrano-[3-4-13] indole-1-acetic acid; mefenamic acid (N-(2,3-xylyl)anthranilic acid; and diphenhydramine hydrochloride (2-diphenyl methoxy-N, N-di-methylethamine hydrochloride).

The transdermal delivery compositions described herein, which contain NSAIDs, desirably comprise an amount of the compound that is therapeutically beneficial for the treatment or prevention of disease or inflammation. Several studies have determined an appropriate dose of an NSAID for a given treatment or condition. (*See e.g.,* Woodin, RN, August: 26-33 (1993) and Amadio et al., Postgrduate Medicine, 93(4):73-97 (1993)). The maximum recommended daily dose for several NSAIDs is listed in **TABLE 1.**

A sufficient amount of NSAID can be incorporated into a transdermal delivery composition described herein such that a therapeutically effective amount of NSAID is effectively delivered to a subject. For example, about 0.5ml of the transdermal delivery composition described herein is applied in a single application. A therapeutically effective amount of ibuprofen is about 800mg/dose. Accordingly, a 30 ml bottle containing a transdermal delivery system formulation and ibuprofen can contain 24 grams of ibuprofen such that 800mg of ibuprofen is provided in each 1.0 ml. Because the transdermal delivery compositions described herein can provide a delivered agent in a site-specific manner, a lower total dose of therapeutic agent, as compared to the amounts provided systemically, will provide therapeutic benefit. Additionally, greater therapeutic benefit can be gained by using a transdermal delivery composition described herein because a greater concentration of therapeutic agent (*e.g*., an NSAID) can be provided to the particular site of inflammation. That is, in contrast to systemic administration, which applies the same concentration of therapeutic to all regions of the body, a transdermal delivery composition can site-specifically provide the therapeutic agent and, thereby, provide a much greater regional concentration of the agent than if the same amount of therapeutic were administered systemically.

**TABLE 1**

| Agent | Maximum Recommended Daily Dose |
|---|---|
| Indomethacin | 100 mg |
| Ibuprofen | 3200 mg |
| Naproxen | 1250 mg |
| Fenoprofen | 3200 mg |
| Tolmetin | 2000 mg |
| Sulindac | 400 mg |
| Meclofenamate | 400 mg |
| Ketoprofen | 300 mg |
| Proxicam | 10 mg |
| Flurbiprofen | 300 mg |
| Diclofenac | 200 mg |

Additional embodiments include a transdermal delivery composition that provides a pain relieving mixture comprising capsaicin (*e.g.*, oleoresin capsicum) or Boswellin or both. Capsaicin (8-methyl-N-vanillyl-6-nonenamide), the pungent component of paprika and peppers, is a potent analgesic. (*See* U.S. Patent Nos. 5,318,960 to Toppo, 5,885,597 to Botknecht et al., and 5,665,378 to Davis et al., herein expressly incorporated by reference in their entireties). Capsaicin produces a level of analgesia comparable to morphine, yet it is not antagonized by classical narcotic antagonists such as naloxone. Further, it effectively prevents the development of cutaneous hyperalgesia, but appears to have minimal effects on normal pain responses at moderate doses. At high doses capsaicin also exerts analgesic activity in classical models of deep pain, elevating the pain threshold above the normal value. Capsaicin can be readily obtained by the ethanol extraction of the fruit of *Capsicum frutescens* or *Capsicum annum.* Capsaicin and analogs of capsaicin are available commercially from a variety of suppliers, and can also be prepared synthetically by published methods. Aspects of the invention encompass the use of synthetic and natural capsaicin, capsaicin derivatives, and capsaicin analogs.

A form of capsaicin used in several desirable embodiments is oleoresin capsicum. Oleoresin capsicum contains primarily capsaicin, dihydrocapsaicin, nordihydrocapsaicin, homocapsaicin, and homodihydrocapsaicin. The term "capsaicin" collectively refers to all forms of capsaicin, capsicum, and derivatives or modifications thereof. The pungency of these five compounds, expressed in Scoville units, is provided in **TABLE 2.**

**TABLE 2**

| Compound Pungency | x 100,000 SU |
|---|---|
| Capsaicin | 160 |
| Dihydrocapsaicin | 160 |
| Nordihydrocapsaicin | 91 |
| Homocapsaicin | 86 |
| Homodihydrocapsaicin | 86 |

The transdermal delivery compositions that are formulated to contain capsaicin desirably comprise by weight or volume 0.01% to 1.0% capsaicin or 1.0% to 10% oleoresin capsicum. Preferred amounts of this delivered agent include by weight or volume 0.02% to 0.75% capsaicin or 2.0% to 7.0% oleoresin capsicum. For example, the transdermal delivery compositions that contain capsaicin can comprise by weight or volume less than or equal to 0.01%, 0.015%, 0.02%, 0.025%, 0.03%, 0.035%, 0.04%, 0.045%, 0.05%, 0.055%, 0.06%, 0.065%, 0.07%, 0.075%, 0.08%, 0.085%, 0.09%, 0.095%, 0.1%, 0.15%, 0.175%, 0.2%, 0.225%, 0.25%, 0.275%, 0.3%, 0.325%, 0.35%, 0.375%, 0.4%, 0.425%, 0.45%, 0.475%, 0.5%, 0.55%, 0.6%, 0.65%, 0.7%, 0.75%, 0.8%, 0.85%, 0.9%, 0.95%, and 1.0% capsaicin. The transdermal delivery compositions of that contain capsaicin can also comprise an amount of capsaicin by weight or volume that is greater than 1.0%, such as 1.2%, 1.5%, 1.8%, 2.0%, 2.2%, 2.5%, 2.8%, 3.0%, 3.5%, 4.0%, 4.5%, and 5.0%. Similarly, the transdermal delivery compositions that contain oleoresin capsicum can comprise an amount of oleoresin capsicum less than 1.0%, 1.5%, 2.0%, 2.5%, 3.0%, 3.5%, 4.0%, 4.5%, 5.0%, 5.5%, 6.0%, 6.5%, 7.0%, 7.5%, 8.0%, 8.5%, 9.0%, 9.5%, 10.0%, 11.0%, 12.0%, and 13.0%.

Boswellin, also known as Frankincense, is an herbal extract of a tree of the *Boswellia* family. Boswellin can be obtained, for example, from *Boswellia thurifera*, *Boswellia carteri, Boswellia sacra*, and *Boswellia serrata.* There are many ways to extract Boswellin and Boswellin gum resin and boswellic acids are obtainable from several commercial suppliers (a 65% solution of Boswellic acid is obtainable from Nature's Plus). Some suppliers also provide creams and pills having Boswellin with and without capsaicin and other ingredients. Embodiments of the invention comprise Boswellin and the term "Boswellin" collectively refers to Frankincense, an extract from one or more members of the *Boswellia* family, Boswellic acid, synthetic Boswellin, or modified or derivatized Boswellin.

The transdermal delivery compositions that contain Boswellin desirably comprise 0.1% to 10% Boswellin by weight or volume. Preferred amounts of this delivered agent include 1.0% to 5.0% Boswellin by weight. For example, the transdermal delivery compositions that contain Boswellin can comprise by weight or volume less than or equal to 0.1%, 0.15%, 0.2%, 0.25%, 0.3%, 0.35%, 0.4%, 0.45%, 0.5%, 0.55%, 0.6%, 0.65%, 0.7%, 0.75%, 0.8%, 0.85%, 0.9%, 0.95%, 1.0%, 1.1%, 1.15%, 1.2%, 1.25%, 1.3%, 1.35%, 1.4%, 1.45%, 1.5%, 1.55%, 1.6%, 1.65%, 1.7%, 1.75%, 1.8%, 1.85%, 1.9%, 1.95%, and 2.0%, 2.1%, 2.15%, 2.2%, 2.25%, 2.3%, 2.35%, 2.4%, 2.45%, 2.5%, 2.55%, 2.6%, 2.65%, 2.7%, 2.75%, 2.8%, 2.85%, 2.9%, 2.95%, 3.0%, 3.1%, 3.15%, 3.2%, 3.25%, 3.3%, 3.35%, 3.4%, 3.45%, 3.5%, 3.55%, 3.6%, 3.65%, 3.7%, 3.75%, 3.8%, 3.85%, 3.9%, 3.95%, 4.0%,. 4.1%, 4.15%, 4.2%, 4.25%, 4.3%, 4.35%, 4.4%, 4.45%, 4.4%, 4.45%, 4.5%, 4.55%, 4.6%, 4.65%, 4.7%, 4.75%, 4.8%, 4.85%, 4.9%, 4.95%, and 5.0% Boswellin. The transdermal delivery compositions that contain Boswellin can also comprise amounts of Boswellin by weight that are greater than 5.0%, such as 5.5%, 5.7%, 6.0%, 6.5%%, 6.7%, 7.0%, 7.5%, 7.7%, 8.0%, 8.5%, 8.7%, 9.0%, 9.5%, 9.7%, and 10.0% or greater. Additionally, Boswellin from different sources can be combined to compose the Boswellin component of an embodiment. For example, in one embodiment an extract from *Boswellia thurifera* is combined with an extract from *Boswellia serrata*.

Additional embodiments of the invention comprise a transdermal delivery composition that can administer a pain relieving solution comprising two or more members selected from the group consisting of NSAIDs, capsacin, and Boswellin. The transdermal delivery compositions that include two or more members selected from the group consisting of NSAIDs, capsacin, and Boswellin desirably comprise an amount of delivered agent that can be included in a delivered agent having an NSAID, capsaicin, or Boswellin by itself. For example, if the delivered agent comprises an NSAID, the amount of NSAID that can be used can be an amount recommended in the literature (See *e.g.,* Woodin, RN, August: 26-33 (1993) and Amadio, et al., Postgrduate Medicine, 93(4):73-97 (1993)), or an amount listed in TABLE 1. Similarly, if capsaicin is a component of the delivered agents then the transdermal delivery composition can comprise by weight or volume less than or equal to 0.01%, 0.015%, 0.02%, 0.025%, 0.03%, 0.035%, 0.04%, 0.045%, 0.05%, 0.055%, 0.06%, 0.065%, 0.07%, 0.075%, 0.08%, 0.085%, 0.09%, 0.095%, 0.1%, 0.15%, 0.175%, 0.2%, 0.225%, 0.25%, 0.275%, 0.3%, 0.325%, 0.35%, 0.375%, 0.4%, 0.425%, 0.45%, 0.475%, 0.5%, 0.55%, 0.6%, 0.65%, 0.7%, 0.75%, 0.8%, 0.85%, 0.9%, 0.95%, and 1.0% capsaicin or less than 1.0%, 1.5%, 2.0%, 2.5%, 3.0%, 3.5%, 4.0%, 4.5%, 5.0%, 5.5%, 6.0%, 6.5%, 7.0%, 7.5%, 8.0%, 8.5%, 9.0%, 9.5%, 10.0%, 11.0%, 12.0%, 13.0%, oleoresin capsicum. Further, if Boswellin is a component of the delivered agents, then the delivery system can comprise by weight or volume less than or equal to 0.1%, 0.15%, 0.2%, 0.25%, 0.3%, 0.35%, 0.4%, 0.45%, 0.5%, 0.55%, 0.6%, 0.65%, 0.7%, 0.75%, 0.8%, 0.85%, 0.9%, 0.95%, 1.0%, 1.1%, 1.15%, 1.2%, 1.25%, 1.3%, 1.35%, 1.4%, 1.45%, 1.5%, 1.55%, 1.6%, 1.65%, 1.7%, 1.75%, 1.8%, 1.85%, 1.9%, 1.95%, 2.0%, 2.1%, 2.15%, 2.2%, 2.25%, 2.3%, 2.35%, 2.4%, 2.45%, 2.5%, 2.55%, 2.6%, 2.65%, 2.7%, 2.75%, 2.8%, 2.85%, 2.9%, 2.95%, 3.0%, 3.1%, 3.15%, 3.2%, 3.25%, 3.3%, 3.35%, 3.4%, 3.45%, 3.5%, 3.55%, 3.6%, 3.65%, 3.7%, 3.75%, 3.8%, 3.85%, 3.9%, 3.95%, 4.0%,. 4.1%, 4.15%, 4.2%, 4.25%, 4.3%, 4.35%, 4.4%, 4.45%, 4.4%, 4.45%, 4.5%, 4.55%, 4.6%, 4.65%, 4.7%, 4.75%, 4.8%, 4.85%, 4.9%, 4.95%, 5.0%, 5.5%, 5.7%, 6.0%, 6.5%%, 6.7%, 7.0%, 7.5%, 7.7%, 8.0%, 8.5%, 8.7%, 9.0%, 9.5%, 9.7%, and 10.0% Boswellin.

Other analgesics are useful delivered agents in the transdermal delivery compositions described herein. For example, tramadol hydrochloride, fentanyl, metamizole, morphine sulphate, ketorolac tromethamine, hydrocodone, oxycodone, morphine and loxoprofen sodium are delivered agents in certain embodiments.

Steroidal anti-inflammatory compounds are also useful delivered agents in the transdermal delivery compositions described herein. For example, hydrocortisone, prednisolone, triamcinolone, and priroxicam are delivered agents in certain embodiments.

Local anesthetics are low molecular weight compounds that are useful as delivered agents in the transdermal delivery compositions described herein. The transdermal delivery compositions disclosed herein are particularly useful in the context of local anesthetics, where a local, concentrated dose of a delivered agent is desirable. Embodiments of the transdermal delivery compositions include local anesthetics, such as articaine, procaine, tetracaine, chloroprocaine and benzocaine, novocain, mepivicaine, bupivicaine, benzocaine, and lidocaine, and the like. The maximum single dose for local anesthetic solutions is somewhere between 70 mg to 500 mg, depending upon the age and health of the patient.

Compounds that have anti-infective activity are also useful in the present invention, particularly in the context of dermal bacterial, fungal, or viral infections. Antibiotics are compounds that either kill bacterial or fungal cells, or prevent them from multiplying. Several antibiotics are known to those skilled in the art and are delivered agents in certain embodiments of the transdermal delivery compositions, including but not limited to amoxicillin, clavulanate potassium, itraconazole, acyclovir, fluconazole, terbinafine hydrochloride, erythromycin ethylsuccinate, acetyl sulfisoxazole, penicillin V, cephalexin, erythromycin, azithromycin, tetracycline, ciproflaxin, gentamycin, sulfathiazole, nitrofurantoin, norfloxacin, flumequine, and ibafloxacin, metronidazole, and nystatin. Likewise, several compounds that have antiviral activity useful as delivered agents include but are not limited to acyclovir, lamivudine, indinavir sulfate, and stavudine. Those skilled in the art will appreciate that analogs and derivatives of the anti-infective compounds now known (e.g. valacyclovir) and discovered in the future are contemplated in the present invention.

In addition to low molecular weight delivered agents, many medium molecular weight delivered agents (eg., humates) can be delivered to cells in the body by using an embodiment of the transdermal delivery composition. Synthetic humates ("hepsyls") are medium molecular weight compounds (1,000 to 100,000 daltons), which are known to be strong antiviral and antimicrobial medicaments. *(See* International Application Publication No. WO 9834629 to Laub). Hepsyls are generally characterized as polymeric phenolic materials comprised of conjugated aromatic systems to which are attached hydroxyl, carboxyl, and other covalently bound functional groups. A transdermal delivery composition that can provide hepsyls to cells of the body has several pharmaceutical uses, including but not limited to, treatment of topical bacterial and viral infections.

Accordingly, in another aspect of the invention, a transdermal delivery system that can provide a medium molecular weight compound (*e.g*., a form of hepsyl) to cells of the body is provided. As described above, many different medium molecular weight compounds can be provided using an embodiment of a transdermal delivery composition described herein and the use of a medium molecular weight hepsyl as a delivered agent is intended to demonstrate that embodiments of the invention can deliver many medium molecular weight compounds to cells of the body.

In some embodiments, amino acids, peptides, nucleotides, nucleosides, and nucleic acids are transdermally delivered to cells in the body using an embodiment of the transdermal delivery composition described herein. That is, any amino acid or peptide having at least, less than, more than, or equal to 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 75, 100, 125, 150, 200, 300, 400, 500, 600, 700, 800, 900, 1000, 1500, 2000, 2500, 3000, 3500, 4000, 4500, 5000, 7000, or 10, 000 amino acids can be incorporated into a transdermal delivery composition described herein and said delivered agent can be delivered to cells in the body shortly after application of the composition. These embodiments can be used, for example, to stimulate an immune response, promote wound healing, induce collagen synthesis, or to supplement collagen. These embodiments are also useful for the delivery of peptide hormones. Non-limiting examples of peptide hormones that are delivered agents in certain embodiments include oxytocin (SEQ ID NO:2), vasopressin (SEQ ID NO:3), melanocyte-stimulating hormone (SEQ ID NO:4 (alpha) SEQ ID NO:5 (beta) SEQ ID NO:6 (gamma)), corticortropin (SEQ ID NO:7), lipotropin (SEQ ID NO:8 (beta) SEQ ID NO:9 (gamma)), thyrotropin (SEQ ID NO:10), growth hormone (SEQ ID NO:1), prolactin (SEQ ID NO:11), luteinizing hormone (SEQ ID NO:12), human chorionic gonadotropin (available from SIGMA-Aldrich, St. Louis, MO, Cat. No. C1063), follicle stimulating hormone, corticotropin-releasing factor (SEQ ID NO:13) gonadotropin-releasing factor (SEQ ID NO:43), prolactin-releasing factor (SEQ ID NO:14), prolactin-inhibiting factor (SEQ ID NO:15), growth-hormone releasing factor (SEQ ID NO:16), somatostatin (SEQ ID NO:17), thyrotropin-releasing factor (SEQ ID NO:18), calcitonin (SEQ ID NO:19), calcitonin gene-related peptide (SEQ ID NO:20), parathyroid hormone (SEQ ID NO:21), glucagon-like peptide 1 (SEQ ID NO:22), glucose-dependent insulinotropic polypeptide (SEQ ID NO:23), gastrin (SEQ ID NO:24), secretin (SEQ ID NO:25), cholecystokinin (SEQ ID NO:26), motilin (SEQ ID NO:27), vasoactive intestinal peptide (SEQ ID NO:28), substance P (SEQ ID NO:30), pancreatic polypeptide (SEQ ID NO:31), peptide tyrosine tyrosine (SEQ ID NO:32), neuropeptide tyrosine (SEQ ID NO:33), amphiregulin (SEQ ID NO:34), insulin (available from SIGMA Aldrich, St. Louis, MO, Cat. No. I643), glucagon (SEQ ID NO:35), placental lactogen (SEQ ID NO:37), relaxin (SEQ ID NO:38), inhibin A (SEQ ID NO:39), Inhibin B (SEQ ID NO:40), Endorphins (e.g., SEQ ID NO:41), angiotensin II (SEQ ID NO:42), atrial natriuretic peptide (SEQ ID NO:),

Several other hormones are not peptide hormones, but are nevertheless suitable delivered agents in embodiments of the invention. Accordingly, embodiments of the invention include cortisol (available from SIGMA Aldrich, St. Louis, MO, Cat. No. H3160), corticosterone (available from SIGMA Aldrich, St. Louis, MO, Cat. No. C27840), aldosterone (available from SIGMA Aldrich, St. Louis, MO, Cat. No. 05521), epinephrine (available from SIGMA Aldrich, St. Louis, MO, Cat. No. 02252), noepinephrine (available from SIGMA Aldrich, St. Louis, MO, Cat. No. 74460), calcitriol (available from SIGMA Aldrich, St. Louis, MO, Cat. No. 17936), progesterone (available from SIGMA Aldrich, St. Louis, MO, Cat. No. P8783), testosterone (available from SIGMA Aldrich, St. Louis, MO, Cat. No. T1500), androstene (available from SIGMA Aldrich, St. Louis, MO) and melatonin (available from SIGMA Aldrich, St. Louis, MO, Cat. No. 63610).

Any nucleotide or nucleoside, modified nucleotide or nucleoside, or nucleic acid having at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 75, 100, 125, 150, 200, 300, 400, 500, 600, 700, 800, 900, 1000, 1500, 2000, 2500, 3000, 3500, 4000, 4500, 5000, 7000, or 10, 000 or more nucleotides can be incorporated into a transdermal delivery composition described herein and said delivered agent can be delivered to cells in the body shortly after application of the composition. These embodiments can also be used, for example, to stimulate an immune response, promote wound healing, or induce collagen synthesis.

Several nucleic acid immunogens and/or vaccines and therapies are known in the art and are useful as delivered agents in embodiments of the transdermal delivery compositions disclosed herein. Several nucleic acid immunogens that induce an immune response (both humoral and cellular) upon administration to a host have been described. DNA vaccines for several viruses, as well as for tumors, are known. Those skilled in the art will appreciate that nucleic acid immunogens contain essential regulatory elements such that upon administration to a host, the immunogen is able to direct host cellular machinery to produce translation products encoded by the respective delivered nucleic acids. Furthermore, those skilled in the art will appreciate that the specific sequences disclosed herein are non-limiting, and that while Applicants reference specific nucleic acids, allelic variants, fragments of nucleic acids, as well as orthologs and paralogs, now known or later discovered such as those made publicly available on databases such as Genbank™ are contemplated in the present invention.

Several immunogens for Human Immunodeficiency Virus (HIV), have been described. International Publication No. WO 01/46393 teaches that compositions comprising the nucleic acid encoding the HIV Nef gene, fragments thereof, or variants that are optimized for efficacy as vaccines in humans, are capable of inducing a cellular immune response in a host. The HIV Nef protein has been shown to promote viral replication. DNA sequences comprising the Nef sequence, including the sequences of SEQ ID NOs:52, 53, and 54 are known to be capable of inducing a cellular immune response in individuals. International Publication No. WO 04/050856 discloses that DNA vaccines comprising the nucleic acid sequences and variants of HIV gp120 (SEQ ID NOs:153, 154, 155, 156) and a codon-optimized nucleic acid encoding HIV-1 Gag (SEQ ID NO:152) are capable of inducing antibody and humoral immune responses. Nucleic acids encoding HIV-1 Gag and variants thereof have also been shown to induce an immune response when administered to a host (Qui et al., 2000, J. Virology. 74(13):5997-6005). Any of the above sequences from HIV are useful delivered agents for the transdermal delivery compositions disclosed herein.

Influenza A is the causative agent of the flu in humans. Flu epidemics cause morbidity and mortality worldwide, and each year in the USA alone more than 200,000 patients are admitted to hospitals because of influenza and there are approximately 36,000 influenza-related deaths. Immunogens directed against Influenza A generally comprise attenuated strains of the virus. WO 04/060720 teaches that a DNA vaccine comprising nucleic acids of sequence SEQ ID NO:51 are capable of inducing a cellular immune response against Influenza virus A.

Much work has also been done on nucleic acid-based immunogens and vaccines for the hepatitis viruses, such as hepatitis C, hepatitis B and hepatitis A. ("HCV", "HBV", and "HAV") The amino acid sequence encoded by the complete coding sequence of the prototype HCV-1 genome (HCVgp1) is provided (SEQ ID NO:128). Houghton et al. (U.S.S.N. 2002/0002272) disclose nucleic acids that encode several portions of HCVgp1 that are capable of inducing a humoral immune response. For example, nucleic acids encoding the HCV E2 envelope protein or portions thereof (SEQ ID NOs:129, 130, 131, 132), or nucleic acids encoding both HCVE1/E2 envelope proteins (SEQ ID NOs:133, 134) were capable of eliciting an immune response. Schiver et al. (International Pub. No. WO 01/43693) disclose other nucleic acid sequences from HCV that elicit protective immune responses, including the sequences of SEQ ID NO's:52, 53, 54.

Embodiments of the present invention also contemplate sequences from HBV, such as nucleic acids that encode HBV core antigen (SEQ ID NO:135); HBVsAg (Genbank™ Accession No. AR141190), and the like. Additionally, nucleic acid sequences from the HAV genome (Genbank™ Accession No. NC_001489) are contemplated as delivered agents.

Various other nucleic acid-based immunogens and vaccines against viral pathogens have been described in the art, such as vaccines comprising nucleic acids from Hantavirus. Hantavirus is the causative agent of Hantavirus Pulmonary Syndrome (HPS), a form of adult respiratory disease syndrome that is potentially fatal in humans. WO 04/058808 discloses sequences (SEQ ID NOs:126, 127) that are useful delivered agents. Chen (International Pub. No. WO 04/110483) discloses several amino acid sequences, (SEQ ID NOs:147, 148, 149 150), the encoding nucleic acid sequences of which are useful as delivered agents for vaccines SARS.

Vaccines and immunogens comprising nucleic acids that encode a member of the Inhibitor of Apoptosis (IAP) family of proteins are also useful in the context of cancer treatment. For Example, Xiang et al. (International Publication No. WO 04/099389) teach DNA vaccines comprising sequences encoding members of the Inhibitor of Apoptosis (IAP) family of proteins, such as nucleic acids encoding the sequences of SEQ ID NO's:136, 137, 138, and 139. These sequences are also useful as delivered agents in one or more of the transdermal delivery systems described herein for the purposes of anti-tumor therapy.

Globulins constitute a diverse group of proteins that share the common characteristic of being soluble in water or dilute salt solutions. Due to their ability to specifically bind to target antigens, antibodies are an extremely valuable example of globulins. Offered as non-limiting examples of therapeutically valuable antibody therapies that are contemplated as delivered agents are: e.g. Rituxin™ for lymphoma; human rabies immune globulin (HRIG) for rabies; bacterial polysaccharide immune globulin (BPIG) for passive immunization of infants against bacterial infections; pertussis immune globulin, and Herceptin™ for breast cancer;. Those skilled in the art will appreciate that a wide variety of antibody therapies - polyclonal, monoclonal, including chemically or physically modified antibodies - can be used as delivered agents in the transdermal delivery compositions described herein.

Other globulins are involved in the transport of a variety of substances, including lipids, hormones, and inorganic ions. For example, sex-hormone binding globulin binds to and transports testosterone, and to a lesser degree estrogens, and thyronine-binding globulin binds to thyoxine. Globulins other than immunoglobulins are also contemplated as delivered agents in the transdermal delivery system described herein.

Immune response modifiers ("IRMs") are compounds that act on the immune system by inducing and/or suppressing cytokine biosynthesis. IRMs possess potent immunostimulating activity including, but not limited to, antiviral and antitumor activity, and can also down-regulate other aspects of the immune response, for example shifting the immune response away from a TH₂ immune response, which is useful for treating a wide range of TH₂ mediated diseases. IRMs can also be used to modulate humoral immunity by stimulating antibody production by B cells. Some IRMs are small organic compounds having a molecular weight under about 1000 daltons, preferably under about 500 daltons.

Examples of classes of small molecule IRM compounds include, but are not limited to, compounds having a 2-aminopyridine fused to a five-membered nitrogen-containing heterocyclic ring. Such compounds include, for example, imidazoquinoline amines including, but not limited to, substituted imidazoquinoline amines such as, for example, amide substituted imidazoquinoline amines, sulfonamide substituted imidazoquinoline amines, urea substituted imidazoquinoline amines, aryl ether substituted imidazoquinoline amines, heterocyclic ether substituted imidazoquinoline amines, amido ether substituted imidazoquinoline amines, sulfonamido ether substituted imidazoquinoline amines, urea substituted imidazoquinoline ethers, thioether substituted imidazoquinoline amines, and 6-, 7-, 8-, or 9-aryl or heteroaryl substituted imidazoquinoline amines; tetrahydroimidazoquinoline amines including, but not limited to, amide substituted tetrahydroimidazoquinoline amines, sulfonamide substituted tetrahydroimidazoquinoline amines, urea substituted tetrahydroimidazoquinoline amines, aryl ether substituted tetrahydroimidazoquinoline amines, heterocyclic ether substituted tetrahydroimidazoquinoline amines, amido ether substituted tetrahydroimidazoquinoline amines, sulfonamido ether substituted tetrahydroimidazoquinoline amines, urea substituted tetrahydroimidazoquinoline ethers, and thioether substituted tetrahydroimidazoquinoline amines; imidazopyridine amines including, but not limited to, amide substituted imidazopyridine amines, sulfonamide substituted imidazopyridine amines, urea substituted imidazopyridine amines, aryl ether substituted imidazopyridine amines, heterocyclic ether substituted imidazopyridine amines, amido ether substituted imidazopyridine amines, sulfonamido ether substituted imidazopyridine amines, urea substituted imidazopyridine ethers, and thioether substituted imidazopyridine amines; 1,2-bridged imidazoquinoline amines; 6,7-fused cycloalkylimidazopyridine amines; imidazonaphthyridine amines; tetrahydroimidazonaphthyridine amines; oxazoloquinoline amines; thiazoloquinoline amines; oxazolopyridine amines; thiazolopyridine amines; oxazolonaphthyridine amines; thiazolonaphthyridine amines; and 1H-imidazo dimers fused to pyridine amines, quinoline amines, tetrahydroquinoline amines, naphthyridine amines, or tetrahydronaphthyridine amines.

Additional examples of small molecule IRMs said to induce interferon (among other things), include purine derivatives (such as those described in U.S. Pat. Nos. 6,376,501, and 6,028,076), imidazoquinoline amide derivatives (such as those described in U.S. Pat. No. 6,069,149), 1H-imidazopyridine derivatives (such as those described in Japanese Patent Application No. 9-255926), benzimidazole derivatives (such as those described in U.S. Pat. No. 6,387,938), derivatives of a 4-aminopyrimidine fused to a five membered nitrogen containing heterocyclic ring (such as adenine derivatives described in U.S. Pat. Nos. 6,376,501; 6,028,076 and 6,329,381; and in International Publication No. WO 02/08595), and certain 3-.beta.-D-ribofuranosylthiazolo[4,5-d]pyri- midine derivatives (such as those described in U.S. Patent Publication No. 2003/0199461). 1H-imidazopyridine derivatives (such as those described in U.S. Pat. No. 6,518,265 and European Patent Application EP No. 1 256 582)) are said to inhibit TNF and IL-1 cytokines.

Examples of small molecule IRMs that comprise a 4-aminopyrimidine fused to a five-membered nitrogen-containing heterocyclic ring include adenine derivatives (such as those described in U.S. Pat. Nos. 6,376,501; 6,028,076 and 6,329,381; and in International Publication No. WO 02/08595).

Examples of particular IRM compounds include 2-propyl[1,3]thiazolo[4,5-c]quinolin-4-amine, which is considered predominantly a TLR 8 agonist (and not a substantial TLR 7 agonist), 4-amino-.alpha.,.alpha.-dimethyl-1H-imidazo[4,5-c]quinoline-1-ethanol, which is considered predominantly a TLR 7 agonist (and not a substantial TLR 8 agonist), and 4-amino-2-(ethoxymethyl)-alpha,alpha.-dimethyl-6,7,8,9-tetrahydro-1H-imidazo[4,5-c]quinolines-1-ethanol, which is a TLR 7 and TLR 8 agonist. In addition to its TLR 7 activity (and TLR 6 activity, but low TLR 8 activity), 4-amino-alpha,alpha-dimethyl-1H-imidazo[4,5-c]quinoline-1-ethanol has beneficial characteristics, including that it has a much lower CNS effect when delivered systemically compared to imiquimod. Other examples of specific IRM compounds include, e.g., N-[4-(4-amino-2-butyl-1H-imidazo[4,5-c][1,5]naphthyridin-1-yl)butyl]-N'-cyclohexylurea, 2-methyl-1-(2-methylpropyl)-1H-imidazo[4,5-c][1,5]naphthyridin-4-amine, 1-(2-methylpropyl)-1H-imidazo[4,5-c][1,5]naphthyridin-4-amine, N-{2-[4-amino-2-(ethoxymethyl)-1H-imidazo[4,5-c]quinolin-1-yl]-1,1-dimethylethyl}methanesulfonamide, N-[4-(4-amino-2-ethyl-1H-imidazo[4,5-c]quinolin-1-yl)butyl]methanesulfonamide, 2-methyl-1-[5-(methylsulfonyl)pentyl]-1H-imidazo[4,5-c]quinolin-4-amine, N-[4-(4-amino-2-propyl-1H-imidazo[4,5-c]quinolin-1-yl)butyl]methanesulfonamide, 2-butyl-1-[3-(methylsulfonyl)propyl-]-1H-imidazo[4,5-c]quinoline-4-amine, 2-butyl-1-{2-[(1-methylethyl)sulfonyl]ethyl}-1H-imidazo-[4,5-c]quinolin-4-amine, N-{2-[4-amino-2-(ethoxymethyl)- -1H-imidazo[4,5-c]quinolin-1-yl]-1,1-dimethylethyl}-N'-cyclohexylurea, N-{2-[4-amino-2-(ethoxymethyl)-1H-imidazo[4,5-c]quinolin-1-yl]-1,1-dimethylethyl}cyclohexanecarboxamide, N-{2-[4-amino-2-(ethoxymethyl)-1H-imidazo[-4,5-c]quinolin-1-yl]ethyl}-N'-isopropylurea. Resiquimod, 4-amino-2-ethoxymethyl-.alpha.,.alpha.-dimethyl-1H-imidazo[4,5-c]quinoline-1-ethanol, may also be used in certain situations where a combination TLR 7 and TLR 8 agonist is desired.

Other IRMs include large biological molecules such as oligonucleotide sequences. Some IRM oligonucleotide sequences contain cytosine-guanine dinucleotides (CpG) and are described, for example, in U.S. Pat. Nos. 6,194,388; 6,207,646; 6,239,116; 6,339,068; and 6,406,705. Some CpG-containing oligonucleotides can include synthetic immunomodulatory structural motifs such as those described, for example, in U.S. Pat. Nos. 6,426,334 and 6,476,000. Other IRM nucleotide sequences lack CpG and are described, for example, in International Patent Publication No. WO 00/75304. IRMs are delivered agents in embodiments of the transdermal delivery compositions of the present invention.

Embodiments of the invention are also useful for delivery of compounds used to facilitate imaging of tissues and organs within the body. Several imaging methods commonly used include Xray, CT scans, ultrasound, and magnetic resonance imaging. Various compounds are administered to individuals that facilitate the imaging process. Thus, other embodiments are useful for the delivery of diagnostic or contrast components useful in imaging methods now known or later discovered include iohexol, technetium, Tc99M, sestamibi, iomeprol, gadodiamide, oiversol, iopromide, alsactide, americium, betazole, histamine, mannitol, metyraphone, petagastrin, phentolamine, radioactive B12, gadodiamide, gadopentetic acid, gadoteridol, or perflubron as delivered agents.

In addition to low molecular weight delivered agents and medium molecular weight delivered agents, several high molecular weight delivered agents (e.g., glycoproteins) can be delivered to cells in the body by using an embodiment of the transdermal delivery composition. Glycoproteins are high molecular weight compounds, which are generally characterized as conjugated proteins containing one or more heterosaccharides as prosthetic groups. The heterosaccharides are usually branched but have a relatively low number of sugar residues, lack a serially repeating unit, and are covalently bound to a polypeptide chain. Several forms of glycoproteins are found in the body. For example, many membrane bound proteins are glycoproteins, the substances that fill the intercellular spaces (e.g., extracellular matrix proteins) are glycoproteins, and the compounds that compose collagens, proteoglycans, mucopolysaccharides, glycosaminoglycans, and ground substance are glycoproteins. A delivery system that can administer glycoproteins to cells of the body has several pharmaceutical and cosmetic uses, including but not limited to, the restoration of skin elasticity and firmness (e.g., the reduction in the appearance of fine lines and wrinkles by transdermal delivery of collagen) and the restoration of flexible and strong joints (e.g., water retention in joints can be increased by transdermal delivery of proteoglycans).

Accordingly, in another aspect of the invention, a transdermal delivery composition that can administer a high molecular weight compound (e.g., a form of collagen or fragment thereof) to cells of the body is provided. As described above, many different high molecular weight compounds can be administered by using an embodiment of a transdermal delivery composition of the invention and the use of a high molecular weight collagen as a delivered agent is intended to demonstrate that embodiments of the invention can deliver many high molecular weight compounds to cells of the body.

Collagens exist in many forms and can be isolated from a number of sources. Additionally, several forms of collagen can be obtained commercially (e.g., Brooks Industries Inc., New Jersey). Many low molecular weight collagens can be made, for example, by hydrolysis. Several transdermal delivery compositions of the invention can deliver collagens having molecular weights below 6,000 daltons. Additionally, several high molecular weight collagens exist. Some are isolated from animal or plant sources and some are synthesized or produced through techniques common in molecular biology. Several transdermal delivery compositions of the invention can deliver collagens having molecular weights of 1,000 daltons to greater than 2,000,000 daltons. That is, embodiments of the transdermal delivery compositions can deliver collagens having molecular weights of less than or equal to or greater than 1,000, 1,500, 2,000, 2,500, 3,000, 3,500, 4,000, 4,500, 5,000, 5,500, 6,000, 7,000, 8,000, 9,000, 10,000, 11,000, 12,000, 13,000, 14,000, 15,000, 16,000, 17,000, 18,000, 19,000, 20,000, 21,000, 22,000, 23,000, 24,000, 25,000, 26,000, 27,000, 28,000, 29,000, 30,000, 31,000, 32,000, 33,000, 34,000, 35,000, 36,000, 37,000, 38,000, 39,000, 40,000, 41,000, 42,000, 43,000, 44,000, 45,000, 46,000, 47,000, 48,000, 49,000, 50,000, 51,000, 52,000, 53,000, 54,000, 55,000, 56,000, 57,000, 58,000, 59,000, 60,000, 61,000, 62,000, 63,000, 64,000, 65,000, 66,000, 67,000, 68,000, 69,000, 70,000, 75,000, 80,000, 85,000, 90,000, 95,000, 100,000, 125,000, 150,000, 175,000, 200,000, 225,000, 250,000, 275,000, 300,000, 350,000, 400,000, 450,000, 500,000, 600,000, 700,000, 800,000, 900,000, 1,000,000, 1,500,000, 1,750,000, and 2,000,000 daltons.

In some embodiments, the commercially available collagen "Hydrocoll EN-55" was provided as the delivered agent and was delivered to cells of a test subject. This form of collagen is hydrolyzed collagen and has a molecular weight of 2,000 daltons. In another embodiment, the commercially available "Ichtyocollagene" or marine collagen (Sederma or Croda of Parsippany, New Jersey) was provided as the delivered agent and was delivered to a test subject. This form of soluble collagen has a molecular weight of greater than 100,000 daltons. In another embodiment, the commercially available collagen "Solu-Coll" was provided as the delivered agent and was delivered to cells of a test subject. This form of collagen is a soluble collagen having a molecular weight of 300,000 daltons. An additional embodiment includes the commercially available collagen "Plantsol", which is obtained from yeast and has a molecular weight of 500,000 daltons. This collagen was also provided as a delivered agent and was delivered to cells of a test subject.

The transdermal delivery compositions that contain a form of collagen or fragment thereof desirably comprise by weight or volume between 0.1% to 85.0% of the delivered agent depending on the type and form of the collagen, its solubility, and the intended application. That is, some transdermal delivery compositions comprise by weight or volume less than or equal to or greater than 0.1%, 0.15%, 0.2%, 0.25%, 0.3%, 0.35%, 0.4%, 0.45%, 0.5%, 0.55%, 0.6%, 0.65%, 0.7%, 0.75%, 0.8%, 0.85%, 0.9%, 0.95%, 1.0%, 1.25%, 1.5%, 1.75%, 2.0%, 2.25%, 2.5%, 2.75%, 3.0%, 3.25%, 3.5%, 3.75%, 4.0%,. 4.25%, 4.5%, 4.75%, 5.0%, 5.25%, 5.5%, 5.75%, 6.0%, 6.25%, 6.5%, 6.75%, 7.0%, 7.25%, 7.5%, 7.75%, 8.0% 8.25%, 8.5%, 8.75%, 9.0%, 9.25%, 9.5%, 9.75%, 10.0%, 10.25%, 10.5%, 10.75%, 11.0%,. 11.25%, 11.5%, 11.75%, 12.0%, 12.25%, 12.5%, 12.75%, 13.0%, 13.25%, 13.5%, 13.75%, 14.0%, 14.25%, 14.5%, 14.75%, 15.0%, 15.5%, 16.0%, 16.5%, 17.0%, 17.5%, 18.0%, 18.5%, 19.0%, 19.5%, 20.0%, 20.5%, 21.0%, 21.5%, 22.0%, 22.5%, 23.0%, 23.5%, 24.0%, 24.5%, 25.0%, 25.5%, 26.0%, 26.5%, 27.0%, 27.5%, 28.0%, 28.5%, 29.0%, 29.5%, 30.0%, 30.5%, 31.0%, 31.5%, 32.0%, 32.5%, 33.0%, 33.5%, 34.0%, 34.5%, 35.0%, 35.5%, 36.0%, 36.5%, 37.0%, 37.5%, 38.0%, 38.5%, 39.0%, 39.5%, 40.0%, 41.0%, 42.0%, 43.0%, 44.0%, 45.0%, 46.0%, 47.0%, 48.0%, 49.0%, 50.0%, 51%, 52%, 53%, 54%, 55%, 56%, 57%, 58%, 59%, 69%, 70%, 71%, 72%, 73%, 74%, 75%, 76%, 77%, 78%, 79%, 80%, 81%, 82%, 83%, 84%, or 85% collagen or fragment thereof.

For example, embodiments having Hydrocoll-EN55 can comprise by weight or volume less than or equal to or greater than 1.0%, 1.25%, 1.5%, 1.75%, 2.0%, 2.25%, 2.5%, 2.75%, 3.0%, 3.25%, 3.5%, 3.75%, 4.0%,. 4.25%, 4.5%, 4.75%, 5.0%, 5.25%, 5.5%, 5.75%, 6.0%, 6.25%, 6.5%, 6.75%, 7.0%, 7.25%, 7.5%, 7.75%, 8.0% 8.25%, 8.5%, 8.75%, 9.0%, 9.25%, 9.5%, 9.75%, 10.0%, 10.25%, 10.5%, 10.75%, 11.0%,. 11.25%, 11.5%, 11.75%, 12.0%, 12.25%, 12.5%, 12.75%, 13.0%, 13.25%, 13.5%, 13.75%, 14.0%, 14.25%, 14.5%, 14.75%, 15.0%, 15.5%, 16.0%, 16.5%, 17.0%, 17.5%, 18.0%, 18.5%, 19.0%, 19.5%, 20.0%, 20.5%, 21.0%, 21.5%, 22.0%, 22.5%, 23.0%, 23.5%, 24.0%, 24.5%, 25.0%, 25.5%, 26.0%, 26.5%, 27.0%, 27.5%, 28.0%, 28.5%, 29.0%, 29.5%, 30.0%, 30.5%, 31.0%, 31.5%, 32.0%, 32.5%, 33.0%, 33.5%, 34.0%, 34.5%, 35.0%, 35.5%, 36.0%, 36.5%, 37.0%, 37.5%, 38.0%, 38.5%, 39.0%, 39.5%, 40.0%, 41.0%, 42.0%, 43.0%, 44.0%, 45.0%, 46.0%, 47.0%, 48.0%, 49.0%, 50.0%, 51%, 52%, 53%, 54%, 55%, 56%, 57%, 58%, 59%, 69%, 70%, 71%, 72%, 73%, 74%, 75%, 76%, 77%, 78%, 79%, 80%, 81%, 82%, 83%, 84%, or 85% Hydrocoll-EN-55.

Embodiments having marine collagen can comprise by weight or volume less than or equal to or greater than 1.0%, 1.25%, 1.5%, 1.75%, 2.0%, 2.25%, 2.5%, 2.75%, 3.0%, 3.25%, 3.5%, 3.75%, 4.0%,. 4.25%, 4.5%, 4.75%, 5.0%, 5.25%, 5.5%, 5.75%, 6.0%, 6.25%, 6.5%, 6.75%, 7.0%, 7.25%, 7.5%, 7.75%, 8.0% 8.25%, 8.5%, 8.75%, 9.0%, 9.25%, 9.5%, 9.75%, 10.0%, 10.25%, 10.5%, 10.75%, 11.0%,. 11.25%, 11.5%, 11.75%, 12.0%, 12.25%, 12.5%, 12.75%, 13.0%, 13.25%, 13.5%, 13.75%, 14.0%, 14.25%, 14.5%, 14.75%, 15.0%, 15.5%, 16.0%, 16.5%, 17.0%, 17.5%, 18.0%, 18.5%, 19.0%, 19.5%, 20.0%, 20.5%, 21.0%, 21.5%, 22.0%, 22.5%, 23.0%, 23.5%, 24.0%, 24.5%, 25.0%, 25.5%, 26.0%, 26.5%, 27.0%, 27.5%, 28.0%, 28.5%, 29.0%, 29.5%, 30.0%, 30.5%, 31.0%, 31.5%, 32.0%, 32.5%, 33.0%, 33.5%, 34.0%, 34.5%, 35.0%, 35.5%, 36.0%, 36.5%, 37.0%, 37.5%, 38.0%, 38.5%, 39.0%, 39.5%, 40.0%, 41.0%, 42.0%, 43.0%, 44.0%, 45.0%, 46.0%, 47.0%, 48.0%, 49.0%, 50.0%, 51%, 52%, 53%, 54%, 55%, 56%, 57%, 58%, 59%, 69%, 70%, 71%, 72%, 73%, 74%, 75%, 76%, 77%, 78%, 79%, 80%, 81%, 82%, 83%, 84%, or 85% marine collagen.

Further, transdermal delivery compositions that contain Solu-Coll can comprise by weight or volume less than or equal to or greater than 0.1%, 0.15%, 0.2%, 0.25%, 0.3%, 0.35%, 0.4%, 0.45%, 0.5%, 0.55%, 0.6%, 0.65%, 0.7%, 0.75%, 0.8%, 0.85%, 0.9%, 0.95%, 1.0%, 1.1%, 1.15%, 1.2%, 1.25%, 1.3%, 1.35%, 1.4%, 1.45%, 1.5%, 1.55%, 1.6%, 1.65%, 1.7%, 1.75%, 1.8%, 1.85%, 1.9%, 1.95%, or 2.0% Solu-Coll.

Additionally, transdermal delivery compositions that contain Plantsol can comprise by weight or volume less than or equal to or greater than 0.1%, 0.15%, 0.2%, 0.25%, 0.3%, 0.35%, 0.4%, 0.45%, 0.5%, 0.55%, 0.6%, 0.65%, 0.7%, 0.75%, 0.8%, 0.85%, 0.9%, 0.95%, 1.0%, 1.1%, 1.15%, 1.2%, 1.25%, 1.3%, 1.35%, 1.4%, 1.45%, 1.5%, 1.55%, 1.6%, 1.65%, 1.7%, 1.75%, 1.8%, 1.85%, 1.9%, 1.95%, 2.0%, 2.1%, 2.15%, 2.2%, 2.25%, 2.3%, 2.35%, 2.4%, 2.45%, 2.5%, 2.55%, 2.6%, 2.65%, 2.7%, 2.75%, 2.8%, 2.85%, 2.9%, 2.95%, 3.0%, 3.1%, 3.15%, 3.2%, 3.25%, 3.3%, 3.35%, 3.4%, 3.45%, 3.5%, 3.55%, 3.6%, 3.65%, 3.7%, 3.75%, 3.8%, 3.85%, 3.9%, 3.95%, or 4.0% Plantsol.

In other embodiments of the invention, a transdermal delivery composition that can provide a collagen solution comprising two or more forms of collagen *(e.g.,* Hydro-Coll EN-55, marine collagen, Solu-coll, or Plantsol) is provided. The transdermal delivery compositions that include two or more forms of collagen desirably comprise an amount of delivered agent that can be included in a delivered agent having the specific type of collagen by itself. For example, if the mixture of delivered agents comprises Hydro-Coll EN55, the amount of Hydro-Coll EN55 in the transdermal delivery composition can comprise by weight or volume less than or equal to or greater than 1.0%, 1.25%, 1.5%, 1.75%, 2.0%, 2.25%, 2.5%, 2.75%, 3.0%, 3.25%, 3.5%, 3.75%, 4.0%,. 4.25%, 4.5%, 4.75%, 5.0%, 5.25%, 5.5%, 5.75%, 6.0%, 6.25%, 6.5%, 6.75%, 7.0%, 7.25%, 7.5%, 7.75%, 8.0% 8.25%, 8.5%, 8.75%, 9.0%, 9.25%, 9.5%, 9.75%, 10.0%, 10.25%, 10.5%, 10.75%, 11.0%,. 11.25%, 11.5%, 11.75%, 12.0%, 12.25%, 12.5%, 12.75%, 13.0%, 13.25%, 13.5%, 13.75%, 14.0%, 14.25%, 14.5%, 14.75%, 15.0%, 15.5%, 16.0%, 16.5%, 17.0%, 17.5%, 18.0%, 18.5%, 19.0%, 19.5%, 20.0%, 20.5%, 21.0%, 21.5%, 22.0%, 22.5%, 23.0%, 23.5%, 24.0%, 24.5%, 25.0%, 25.5%, 26.0%, 26.5%, 27.0%, 27.5%, 28.0%, 28.5%, 29.0%, 29.5%, 30.0%, 30.5%, 31.0%, 31.5%, 32.0%, 32.5%, 33.0%, 33.5%, 34.0%, 34.5%, 35.0%, 35.5%, 36.0%, 36.5%, 37.0%, 37.5%, 38.0%, 38.5%, 39.0%, 39.5%, 40.0%, 41.0%, 42.0%, 43.0%, 44.0%, 45.0%, 46.0%, 47.0%, 48.0%, 49.0%, 50.0%, 51%, 52%, 53%, 54%, 55%, 56%, 57%, 58%, 59%, 69%, 70%, 71%, 72%, 73%, 74%, 75%, 76%, 77%, 78%, 79%, 80%, 81%, 82%, 83%, 84%, or 85% Hydrocoll-EN-55.

If the mixture of delivered agents has marine collagen, then the amount of marine collagen in the delivery system can comprise by weight or volume less than or equal to or greater than 1.0%, 1.25%, 1.5%, 1.75%, 2.0%, 2.25%, 2.5%, 2.75%, 3.0%, 3.25%, 3.5%, 3.75%, 4.0%,. 4.25%, 4.5%, 4.75%, 5.0%, 5.25%, 5.5%, 5.75%, 6.0%, 6.25%, 6.5%, 6.75%, 7.0%, 7.25%, 7.5%, 7.75%, 8.0% 8.25%, 8.5%, 8.75%, 9.0%, 9.25%, 9.5%, 9.75%, 10.0%, 10.25%, 10.5%, 10.75%, 11.0%,. 11.25%, 11.5%, 11.75%, 12.0%, 12.25%, 12.5%, 12.75%, 13.0%, 13.25%, 13.5%, 13.75%, 14.0%, 14.25%, 14.5%, 14.75%, 15.0%, 15.5%, 16.0%, 16.5%, 17.0%, 17.5%, 18.0%, 18.5%, 19.0%, 19.5%, 20.0%, 20.5%, 21.0%, 21.5%, 22.0%, 22.5%, 23.0%, 23.5%, 24.0%, 24.5%, 25.0%, 25.5%, 26.0%, 26.5%, 27.0%, 27.5%, 28.0%, 28.5%, 29.0%, 29.5%, 30.0%, 30.5%, 31.0%, 31.5%, 32.0%, 32.5%, 33.0%, 33.5%, 34.0%, 34.5%, 35.0%, 35.5%, 36.0%, 36.5%, 37.0%, 37.5%, 38.0%, 38.5%, 39.0%, 39.5%, 40.0%, 41.0%, 42.0%, 43.0%, 44.0%, 45.0%, 46.0%, 47.0%, 48.0%, 49.0%, 50.0%, 51%, 52%, 53%, 54%, 55%, 56%, 57%, 58%, 59%, 69%, 70%, 71%, 72%, 73%, 74%, 75%, 76%, 77%, 78%, 79%, 80%, 81%, 82%, 83%, 84%, or 85% marine collagen.

Similarly if the mixture of delivered agents has Solu-coll, then the amount of Solu-coll in the delivery system can comprise by weight or volume less than or equal to or greater than 0.1%, 0.15%, 0.2%, 0.25%, 0.3%, 0.35%, 0.4%, 0.45%, 0.5%, 0.55%, 0.6%, 0.65%, 0.7%, 0.75%, 0.8%, 0.85%, 0.9%, 0.95%, 1.0%, 1.1%, 1.15%, 1.2%, 1.25%, 1.3%, 1.35%, 1.4%, 1.45%, 1.5%, 1.55%, 1.6%, 1.65%, 1.7%, 1.75%, 1.8%, 1.85%, 1.9%, 1.95%, or 2.0% or Solu-Coll. Further, if the mixture of delivered agents has Plantsol, then the amount of Plantsol in the delivery system can comprise by weight or volume less than or equal to or greater than 0.1%, 0.15%, 0.2%, 0.25%, 0.3%, 0.35%, 0.4%, 0.45%, 0.5%, 0.55%, 0.6%, 0.65%, 0.7%, 0.75%, 0.8%, 0.85%, 0.9%, 0.95%, 1.0%, 1.1%, 1.15%, 1.2%, 1.25%, 1.3%, 1.35%, 1.4%, 1.45%, 1.5%, 1.55%, 1.6%, 1.65%, 1.7%, 1.75%, 1.8%, 1.85%, 1.9%, 1.95%, 2.0%, 2.1%, 2.15%, 2.2%, 2.25%, 2.3%, 2.35%, 2.4%, 2.45%, 2.5%, 2.55%, 2.6%, 2.65%, 2.7%, 2.75%, 2.8%, 2.85%, 2.9%, 2.95%, 3.0%, 3.1%, 3.15%, 3.2%, 3.25%, 3.3%, 3.35%, 3.4%, 3.45%, 3.5%, 3.55%, 3.6%, 3.65%, 3.7%, 3.75%, 3.8%, 3.85%, 3.9%, 3.95%, or 4.0% Plantsol.

Additionally, modified or stabilized collagens or collagen derivatives are contemplated for use in some of the embodiments described herein. Particularly preferred are collagens that are resistant to proteases. Recombinant engineering can be used to generate collagens or fragments thereof that lack protease cleavage sites for example. Resistant collagens or fragments thereof can also be prepared by incorporating D-amino acids in synthetically prepared collagens or fragments thereof. Cross-linked collagens can also be used. (*See e.g.,* Charulatha, Biomaterials Feb;24(5):759-67 (2003)). Still further, amidated collagen or collagen fragments can be prepared using synthetic chemistry and these collagen derivatives can be mixed with an ethoxylated oil with or without water or alcohol so as to form a transdermal delivery composition containing collagen. Several techniques to create synthetic, recombinant, or cross-linked collagens are known to those of skill in the art and many are commercially available.

Still further, protease resistant fragments of collagen can be prepared and isolated using conventional techniques. By one approach, marine collagen, procollagen, or collagen obtained from human placenta is incubated with bovine serum, pepsin, or bacterial collagenase for one hour and the preparation is then separated by gel electrophoresis, size exclusion, reverse phase, or ionic exchange chromatography (e.g., FPLC or HPLC). Protease resistant fragments of collagen *(e.g.,* 15 kDa or 30kDa; *see e.g.,* Tasab et al., JBC 277(38):35007 (2002) or 38kDa *see e.g.,* Odermatt et al., Biochem J. May 1;211(2):295-302 (1983) both of which are herein expressly incorporated by reference in their entireties) are separated from the hydrolytic products and these fragments are isolated from the column and concentrated (e.g., centricon filters) or lyophilized using conventional techniques. The protease resistant fragments of collagen are then incorporated into a transdermal delivery composition, as described herein. Alternatively, the protease resistant domain of collagen can be prepared synthetically or obtained commercially (e.g., pepsinized collagens can also be obtained from Chemicon of Temecula, CA).

An additional delivered agent that can be included in a transdermal delivery composition is Etioline (Sederma or Croda of Parsippany, New Jersey). Etioline is a tyrosinase inhibitor made from the extract *Mitracarpe* and bearberry that effectively whitens the skin. Formulations of a transdermal delivery composition described herein containing Etioline *(e.g.,* at 1%, 2%, 3%, 4%, 5%, 6%, 7%, 8%, 9%, 10%, 11%, 12%, 13%, 14%, 15%, 16%, 17%, 18%, 19%, or 20%) are also embodiments of the invention. Another skin brightening or whitening formulation of a transdermal delivery composition comprises Melaslow (Sederma of Parsippany, New Jersey). Melaslow is an extract made from *Citrus reticulate Blanco* var. Unshiu. Melaslow is also an inhibitor of melanogenesis and formulations of a transdermal delivery composition described herein containing Melaslow (*e.g*., at 1%, 2%, 3%, 4%, 5%, 6%, 7%, 8%, 9%, 10%, 11%, 12%, 13%, 14%, 15%, 16%, 17%, 18%, 19%, or 20%) are also embodiments of the invention. An additional delivered agent that can be included in a transdermal delivery composition is Matrixyl (Sederma or Croda of Parsippany, New Jersey). Matrixyl is a compound comprising the peptide KTTKS (SEQ. ID. NO:2), which has been shown to stimulate collagen synthesis. *See* Katayama et al., J. Biol. Chem. 268, 9941 (1993). Formulations of a transdermal delivery composition described herein containing Matrixyl or the peptide KTTKS (SEQ. ID. NO:2) (*e.g.,* at 1%, 2%, 3%, 4%, 5%, 6%, 7%, 8%, 9%, 10%, 11%, 12%, 13%, 14%, 15%, 16%, 17%, 18%, 19%, or 20%) are also embodiments of the invention. The section below describes the manufacture and use of several penetration enhancers that deliver both low and high molecular weight molecules to cells of the body.

### Penetration Enhancers

A penetration enhancer included in many embodiments of the invention is comprised of two components -- a hydrophobic component and a hydrophilic component. Desirably, the hydrophobic component comprises a polyether compound, such as an ethoxylated fatty moiety, preferably, an ethoxylated oil, such as vegetable, nut, synthetic, or animal oil, which has the ability to reduce the surface tension of materials that are dissolved into it. Not wanting to be tied to any particular mechanism or mode of action and offered only to expand the knowledge in the field, it is contemplated that the attachment of poly (ethylene oxide) to the components of a particular oil occurs not on a particular functional group but rather the polyethylene oxide chains begin to grow from unsaturated C=C bonds and from the occasional glycerol unit. Because an ethoxylated oil, such as ethoxylated macadamia nut oil, is a mixture of various fatty acids, fatty alcohols, and fatty amines, the components of the oil may have varying amounts of ethoxylation. Accordingly, measurements of ethoxylation/molecule *(e.g.,* 16 ethoxylations/molecule) are an average of the amount of ethoxylation present on the components of the oil rather than on any specific component itself.

Preferred ethoxylated oils can be obtained or created from, for example, macadamia nut oil, meadowfoam, castor oil, jojoba oil, corn oil, sunflower oil, sesame oil, and emu oil. Many of these oils are commercially available from Floratech of Gilbert, Arizona or other suppliers. Alternatively, ethoxylated oils can be prepared by reacting the oil with ethylene oxide. Pure carrier oils that are suitable for ethoxylation so as to create a penetration enhancer for use with the transdermal delivery compositions described herein are included in **TABLES 3-17** and can be obtained from Esoteric oils Pty. Ltd., Pretoria South Africa. **TABLES 3-17** also list the component fatty acids of these oils, all of which are individually suitable for ethoxylation and incorporation into an embodiment of a transdermal delivery composition. That is, it is contemplated that ethoxylated fatty acids, ethoxylated fatty alcohols, and ethoxylated fatty amines, in particular ethoxylated fatty acids, ethoxylated fatty alcohols, and ethoxylated fatty amines that contain 12, 13, 14, 15, 16, 17, 18, or 19 ethoxylations are suitable penetration enhancers for use in the transdermal delivery compositions described herein. These ethoxylated oil components can be used individually as penetration enhancers or as supplements to other penetration enhancers (e.g., ethoxylated macadamia nut oil).

**TABLE 3**

| MACADAMIA NUT OIL | | |
|---|---|---|
| Fatty acids | | Range |
| Myristic | C14 | 0.6-1.6% |
| Palmitic | C16 | 7.0 - 11.0% |
| Palmitoleic | C16:1 | 18.0 -25.0% |
| Stearic | C18 | 2.0 - 4.0 % |
| Oleic | C18:1 | 55.0 - 62.0 % |
| Linoleic | C18:2 | 1.0 -4.0% |
| Arachidic | C20 | 2.0 - 4.0 % |
| Eicosenoic | C20:1 | 2.0 - 4.0 % |

**TABLE 4**

| APRICOT KERNEL OIL | | | |
|---|---|---|---|
| Fatty acids | | Range | T ypical |
| Palmitic | C1 6:0 | 3.0 - 6.0 % | 4. 28 % |
| Palmitoleic | C1 6:1 | trace - 1.4 % | 0. 70% |
| Stearic | C1 8:0 | trace - 2.0 % | 1. 12% |
| Oleic | C1 8:1 | 55.0 - 70.0% | 6 9.62% |
| Linoleic | C1 8:2 | 20.0 - 35.0 % | 2 3.34 % |
| Linolenic | C1 8:3 | trace - 1.0 % | 0. 22% |
| Eicosenoic | C2 0:1 | trace - 1.0 % | 0. 18 % |

**TABLE 5**

| AVOCADO OIL | | | |
|---|---|---|---|
| Fatty acids | | Range | Typical |
| Palmitic | C16:0 | 12.0 - 20.0 % | 14.25 % |
| Palmitoleic | C16:1 | 2.0 - 10.0 % | 5.84 % |
| Stearic | C18:0 | 0.1-2.0% | 0.1 % |
| Oleic | C18:1 | 55.0 - 75.0 % | 65.4 % |
| Linoleic | C18:2 | 9.0 - 17.0 % | 14.74 % |
| Linolenic | C18:3 | 0.1-2.0% | 0.8 % |

**TABLE 6**

| EVENING PRIMROSE OIL | | | |
|---|---|---|---|
| Fatty acids | | Range | Typical |
| Palmitic | C16:0 | 5.5 - 7.0 % | 5.9 % |
| Stearic | C18:0 | 1.5 - 2.5 % | 1.7 % |
| Oleic | C18:1 | 5.0-11.0 % | 5.8 % |
| Linoleic | C18:2 | 70.0 - 77.0 % | 75.1 % |
| Gamma Linolenic | C18:3 | 9.0 - 10.9 % | 10.6 % |
| Alpha Linolenic | C18:3 | 1.0 % max | 0.4 % |
| Icosanoic | C20:0 | 1.0 % max | 0.2 % |
| Icosenoic | C20:1 | 1.0 % max | .01% |

**TABLE 7**

| GRAPE SEED OIL | | | |
|---|---|---|---|
| Fatty acids | | Range | Typical |
| Palmitic | C16:0 | 6.0 -9.0 % | 6.5 % |
| Palmitoleic | C16:1 | less 1 % | 0.2 % |
| Stearic | C18:0 | 3.0 -6.0 % | 3.7 % |
| Oleic | C18:1 | 12.0-25.0 % | 23.4 % |
| Linoleic | C18:2 | 60.0 - 75.0 % | 65.3 % |
| Alpha Linolenic | C18:3 | less than 1.5 % | 0.2 % |
| Icosanoic | C20:0 | less than 0.5 % | 0.2 % |
| Icosenoic | C20:1 | less than 0.5 % | 0.2 % |
| Docosanoic | C22:0 | less than 0.3 % | 0.2 % |

**TABLE 8**

| HAZELNUT OIL | | |
|---|---|---|
| Fatty acids | | Range |
| Palmitic | C16:0 | 4.0- 8.0 % |
| Palmitoleic | C16:1 | 0.1 - 0.6% |
| Stearic | C18:0 | 1.5 - 3.5 % |
| Oleic | C18:1 | 68.0 -85.0 % |
| Linoleic | C18:2 | 7.0 - 15.0 % |
| Linolenic | C18:3 | 0.1 - 0.5 % |
| Arachidic | C20:0 | 0.1 - 0.5 % |
| Gadoleic | C20:1 | 0.1 - 0.3 % |
| Behenic | C22:0 | 3.0 % MAX |

**TABLE 9**

| JOJOBA OIL | | |
|---|---|---|
| Fatty acids | | Range |
| Palmitic | C16:0 | 3.0 % max |
| Palmitoleic | C16:1 | 1.0 %max |
| Stearic | C18:0 | 1.0 %max |
| Oleic | C18:1 | 5.0 - 15.0 % |
| Linoleic | C18:2 | 5.0 % max |
| Linolenic | C18:3 | 1.0 % max |
| Arachidic | C20:0 | 0.5 % max |
| Eicosenoic | C20:1 | 65.0 - 80.0 % max |
| Behenic | C22:0 | 0.5 % max |
| Erucic | C22:1 | 10.0 - 20.0 % max |
| Lignoceric | C24:0 | 5.0 % max |

**TABLE 10**

| OLIVE OIL | | |
|---|---|---|
| Fatty acids | | Range |
| Palmitic | C16:0 | 5.0 - 12.0 % |
| Palmitoleic | C16:1 | 1.0 % max |
| Stearic | C18:0 | 3.5 % max |
| Oleic | C18:1 | 65.0 - 80.0 % |
| Linoleic | C18:2 | 6.0 - 25.0 % |
| Linolenic | C18:3 | 1.0 % max |
| Arachidic | C20:0 | 0.6 % max |
| Gadoleic | C20:1 | 0.5 % max |
| Behenic | C22:0 | 0.3 % max |
| Erucic | C22:1 | 0.2 % max |

**TABLE 11**

| PUMPKIN SEED OIL | | |
|---|---|---|
| Fatty acids | | Range |
| Palmitic | C16:0 | 6.0 - 21.0 % |
| Stearic | C18:0 | 3.0 - 8.0 % |
| Oleic | C18:1 | 24.0 - 41.0 % |
| Linoleic | C18:2 | 42.0 - 60.0 % |
| Linolenic | C18:3 | 2.0 % max |
| Others | | 2.0 % max |

**TABLE 12**

| ROSE HIP OIL | | |
|---|---|---|
| Fatty acids | | Range |
| Mirystic | C14:0 | 0.0 -0.3 % |
| Palmitic | C16:0 | 3.4 - 4.4 % |
| Palmitoleic | C16:1 | 0.1 - 0.18 % |
| Stearic | C18:0 | 1.5 -2.5 % |
| Oleic | C18:1 | 14.0 - 16.0 % |
| Linoleic | C18:2 | 43.0 - 46.0 % |
| Linolenic | C18:3 | 31.0-34.0% |
| Arachidic | C20:0 | 0.1 - 0.9 % |
| Gadoleic | C20:1 | 0.0 - 0.5 % |
| Eicosenoic | C20:2 | 0.0 - 0.5 % |
| Behenic | C22:0 | 0.1 -0.4 % |

**TABLE 13**

| SAFFLOWER OIL | | |
|---|---|---|
| Fatty acids | | Range |
| Palmitic | C16:0 | 4.0 - 9.0 % |
| Palmitoleic | C16:1 | Trace |
| Stearic | C18:0 | trace - 2.5 % |
| Oleic | C18:1 | 72.0 - 80.0 % |
| Linoleic | C18:2 | 12.0 - 16.0 % |
| Linolenic | C18:3 | trace - 0.5 % |

**TABLE 14**

| SESAME OIL | | |
|---|---|---|
| Fatty acids | | Range |
| Palmitic | C16:0 | 7.0 - 12.0 % |
| Palmitoleic | C16:1 | trace - 0.5 % |
| Stearic | C18:0 | 3.5 - 6.0 % |
| Oleic | C18:1 | 35.0 - 50.0 % |
| Linoleic | C18:2 | 35.0 - 50.0 % |
| Linolenic | C18:3 | trace - 1.0 % |
| Eicosenoic | C20:1 | trace - 1.0 % |

**TABLE 15**

| SUNFLOWER OIL | | |
|---|---|---|
| Fatty acids | | Range |
| Palmitic | C16:0 | 5.8 % |
| Palmitoleic | C16:1 | 0.1 % |
| Stearic | C18:0 | 3.9 % |
| Oleic | C18:1 | 15.9 % |
| Linoleic | C18:2 | 71.7 % |
| Alpha Linolenic | C18:3 | 0.6 % |
| Gamma Linolenic | C18:3 | 0.1 % |
| Arachidic | C20:0 | 0.3 % |
| Gadoleic | C20:1 | 0.2 % |
| Tetracosanoic | C24:0 | 0.5 % |
| Behenic | C22:0 | 0.7 % |

**TABLE 16**

| WALNUT OIL | | | |
|---|---|---|---|
| Fatty acids | | Range | Typical |
| Palmitic | C16:0 | 5.0 - 8.0 % | 6.0 % |
| Palmitoleic | C16:1 | less than 1.0 % | 0.1 % |
| Stearic | C18:0 | 3.0 - 7.0 % | 4.0 % |
| Oleic | C18:1 | 25.0 - 35.0 % | 29.8 % |
| Linoleic | C18:2 | 45.0 - 60.0 % | 58.5 % |
| Alpha Lino lenic | C18:3 | less than 0.8 % | 0.4 % |
| Arachidic | C20:0 | less than 0.5 % | 0.3 % |
| Eicosenoic | C20:1 | less than 0.5 % | 0.2 % |

**TABLE 17**

| WHEAT GERM OIL | | | |
|---|---|---|---|
| Fatty acids | | Range | Typical |
| Palmitic | C16:0 | 11.0-16.0% | 12.5 % |
| Palmitoleic | C16:1 | 1.0 % max | 0.2 % |
| Stearic | C18:0 | 2.0 - 6.0 % | 2.5 % |
| Oleic | C18:1 | 12.0 - 39.0 % | 27.3 % |
| Linoleic | C18:2 | 30.0 -57.0 % | 53.7 % |
| Linolenic | C18:3 | 2.0 - 10.0 % | 3.0% |
| Arachidic | C20:0 | 1.0 % max | 0.4 % |
| Gadoleic | C20:1 | 0.5 % max | 0.2 % |
| Behenic | C22:0 | 1.0 % max | 0.1 % |

In some embodiments, an ethoxylated oil comprises a molar ratio of ethylene oxide:oil of 35:1. A 99% pure ethylene oxide/castor oil having such characteristics can be obtained commercially (BASF) or such an ethoxylated compound can be synthesized using conventional techniques. In other embodiments, the ethoxylated oil is itself the penetration enhancer. That is, it has been discovered that oils that have been ethoxylated 10, 11, 12, 13, 14, 15, 16, 17, 18, or 19 ethoxylations/molecule are sufficiently hydrophobic and sufficiently hydrophilic to allow for transdermal delivery of a variety of delivered agents without water, alcohol, or an aqueous adjuvant. Although the ethoxylated oil can comprise at least 20-25 ethoxylations per molecule or more, preferably, the ethoxylated lipid comprises less than 20 ethoxylations per molecule, *e.g.,* 19, 18, 17, 16, 15, 14, 13, 12, 11, or 10 ethoxylations per molecule.

By using a light, ethoxylated oil (*e.g*., macadamia nut oil containing approximately 16 ethoxylations/molecule) efficient transdermal delivery of high molecular weight collagen was observed in the absence of *Aloe Vera* and alcohol. Formulations of a transdermal delivery composition that contain *Aloe Vera* and an oil with 20-30 ethoxylations/molecule are not as effective as formulations of a transdermal delivery composition that contain an oil with 10-19 ethoxylations/molecule *(e.g.,* 16 ethoxylations/molecule) but lacking *Aloe Vera* and alcohol. A greater reduction of fine lines and wrinkles was observed with a transdermal delivery composition composed of macadamia nut oil (16 ethoxylations/molecule) and water as compared with a transdermal delivery composition composed of castor oil (25 ethoxylations/molecule), water, alcohol, and *Aloe Vera*, for example.

Unexpectedly, it was discovered that a reduction in the number of ethoxylations on a light oil produced a superior transdermal delivery product. This was unexpected because as the amount of ethoxylations on a molecule of oil decreases its miscibility with the aqueous components of the delivery system decreases. Surprisingly, formulations containing 10 - 19 ethoxylations/ molecule were not only miscible but provided very efficient transdermal delivery in the absence of *Aloe Vera*.

Desirable compounds often found in ethoxylated oils that are beneficial for some embodiments and methods described herein are glycerol-polyethylene glycol ricinoleate, the fatty esters of polyethylene glycol, polyethylene glycol, and ethoxylated glycerol. Some of these desirable compounds exhibit hydrophilic properties and the hydrophilic-lipophilic balance (HLB) is preferably maintained between 10 and 18. Any number of methods have been devised to characterize HLB, but perhaps the most widely used is the octanol/water coefficient. (*See* Calculating log Poct from Structures", by Albert J. Leo, Chemical Reviews, vol 93, pp 1281).

Accordingly, some of the components of the oils in the table above and related fatty acids, fatty alcohols, and fatty amines can be ethoxylated and used as a penetration enhancer or to enhance another penetration enhancer (e.g., ethoxylated macadamia nut oil). For example, some embodiments comprise a penetration enhancer that consists of, consists essentially of, or comprises ethoxylated palmitoleic acid, ethoxylated oleic acid, ethoxylated gondoic acid, or ethoxylated erucic acid. These compounds can be prepared synthetically or isolated or purified from oils that contain large quantities of these fatty acids and the synthesized, isolated, or purified fatty acids can then be reacted with ethylene oxide.

That is, a transdermal delivery composition of the invention can comprise a penetration enhancer that contains, for example, ethoxylated palmitoleic acid, ethoxylated oleic acid, ethoxylated gondoic acid, or ethoxylated erucic acid, wherein the amount of one or more of the fatty acids is at least, less than, more than, or an amount equal to 0.1%, 0.15%, 0.2%, 0.25%, 0.3%, 0.35%, 0.4%, 0.45%, 0.5%, 0.55%, 0.6%, 0.65%, 0.7%, 0.75%, 0.8%, 0.85%, 0.9%, 0.95%, 1.0%, 1.25%, 1.5%, 1.75%, 2.0%, 2.25%, 2.5%, 2.75%, 3.0%, 3.25%, 3.5%, 3.75%, 4.0%,. 4.25%, 4.5%, 4.75%, 5.0%, 5.25%, 5.5%, 5.75%, 6.0%, 6.25%, 6.5%, 6.75%, 7.0%, 7.25%, 7.5%, 7.75%, 8.0% 8.25%, 8.5%, 8.75%, 9.0%, 9.25%, 9.5%, 9.75%, 10.0%, 10.25%, 10.5%, 10.75%, 11.0%,. 11.25%, 11.5%, 11.75%, 12.0%, 12.25%, 12.5%, 12.75%, 13.0%, 13.25%, 13.5%, 13.75%, 14.0%, 14.25%, 14.5%, 14.75%, 15.0%, 15.5%, 16.0%, 16.5%, 17.0%, 17.5%, 18.0%, 18.5%, 19.0%, 19.5%, 20.0%, 20.5%, 21.0%, 21.5%, 22.0%, 22.5%, 23.0%, 23.5%, 24.0%, 24.5%, 25.0%, 25.5%, 26.0%, 26.5%, 27.0%, 27.5%, 28.0%, 28.5%, 29.0%, 29.5%, 30.0%, 30.5%, 31.0%, 31.5%, 32.0%, 32.5%, 33.0%, 33.5%, 34.0%, 34.5%, 35.0%, 35.5%, 36.0%, 36.5%, 37.0%, 37.5%, 38.0%, 38.5%, 39.0%, 39.5%, 40.0%, 40.25%, 40.5%, 40.75%, 41.0%, 41.25%, 41.5%, 41.75%, 42.0%, 42.25%, 42.5%, 42.75%, 43.0%,. 43.25%, 43.5%, 43.75%, 44.0%, 44.25%, 44.5%, 44.75%, 45.0%, 45.25%, 45.5%, 45.75%, 46.0%, 46.25%, 46.5%, 46.75%, 47.0% 47.25%, 47.5%, 47.75%, 48.0%, 48.25%, 48.5%, 48.75%, 49.0%, 49.25%, 49.5%, 49.75%, 50.0%,. 50.25%, 50.5%, 50.75%, 51.0%, 51.25%, 51.5%, 51.75%, 52.0%, 52.25%, 52.5%, 52.75%, 53.0%, 53.25%, 53.5%, 53.75%, 54.0%, 54.5%, 54.0%, 54.5%, 55.0%, 55.5%, 56.0%, 56.5%, 57.0%, 57.5%, 58.0%, 58.5%, 59.0%, 59.5%, 60.0%, 60.5%, 61.0%, 61.5%, 62.0%, 62.5%, 63.0%, 63.5%, 64.0%, 64.5%, 65.0%, 65.5%, 66.0%, 66.5%, 67.0%, 67.5%, 68.0%, 68.5%, 69.0%, 69.5%, 70.0%, 70.5%, 71.0%, 71.5%, 72.0%, 72.5%, 73.0%, 73.5%, 74.0%, 74.5%, 75.0%, 75.5%, 76.0%, 76.5%, 77.0%, 77.5%, 78.0%, 78.5%, 79.0%, 79.5%, 80.0%, 80.5%, 81%, 81.5%, 82%, 82.5%, 83%, 83.5%, 84%, 84.5%, 85%. 85.5%, 86%, 86.5%, 87%, 87.5%, 88%, 88.5%, 89%, 89.5%, 90%, 90.5%, 91%, 91.5%, 92%, 92.5%, 93%, 93.5%, 94%, 94.5%, 95%, 95.5%, 96%, 96.5%, 97%, 97.5%, 98%, 98.5%, 99%, or 100% of the total fatty acid content in the composition. In some embodiments, more than one ethoxylated compound is added or another hydrophobic compound is added (e.g., Y-Ling-Y-Lang oil; Young Living Essential Oils, Lehl, Utah)) to balance or enhance the penetration enhancer. Preferred embodiments include ethoxylated macadamia nut oil that has been supplemented with ethoxylated palmitoleic acid, ethoxylated oleic acid, ethoxylated gondoic acid, or ethoxylated erucic acid.

Depending on the type of delivered agent and the intended application, the amount of ethoxylated lipid(s) in the delivery system can vary. For example, delivery systems of the invention can comprise between 0.1% and 99% by weight or volume ethoxylated compound(s). That is, embodiments of the invention can comprise by weight or volume at least, less than, or equal to or greater than 0.1%, 0.15%, 0.2%, 0.25%, 0.3%, 0.35%, 0.4%, 0.45%, 0.5%, 0.55%, 0.6%, 0.65%, 0.7%, 0.75%, 0.8%, 0.85%, 0.9%, 0.95%, 1.0%, 1.25%, 1.5%, 1.75%, 2.0%, 2.25%, 2.5%, 2.75%, 3.0%, 3.25%, 3.5%, 3.75%, 4.0%,. 4.25%, 4.5%, 4.75%, 5.0%, 5.25%, 5.5%, 5.75%, 6.0%, 6.25%, 6.5%, 6.75%, 7.0%, 7.25%, 7.5%, 7.75%, 8.0% 8.25%, 8.5%, 8.75%, 9.0%, 9.25%, 9.5%, 9.75%, 10.0%, 10.25%, 10.5%, 10.75%, 11.0%,. 11.25%, 11.5%, 11.75%, 12.0%, 12.25%, 12.5%, 12.75%, 13.0%, 13.25%, 13.5%, 13.75%, 14.0%, 14.25%, 14.5%, 14.75%, 15.0%, 15.5%, 16.0%, 16.5%, 17.0%, 17.5%, 18.0%, 18.5%, 19.0%, 19.5%, 20.0%, 20.5%, 21.0%, 21.5%, 22.0%, 22.5%, 23.0%, 23.5%, 24.0%, 24.5%, 25.0%, 25.5%, 26.0%, 26.5%, 27.0%, 27.5%, 28.0%, 28.5%, 29.0%, 29.5%, 30.0%, 30.5%, 31.0%, 31.5%, 32.0%, 32.5%, 33.0%, 33.5%, 34.0%, 34.5%, 35.0%, 35.5%, 36.0%, 36.5%, 37.0%, 37.5%, 38.0%, 38.5%, 39.0%, 39.5%, 40.0%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% ethoxylated lipid(s), preferably an ethoxylated oil or fatty acid or combination of fatty acids.

The hydrophilic component of the penetration enhancer can comprise an alcohol, a non-ionic solubilizer, or an emulsifier. Compounds such as ethylene glycol, propylene glycol, dimethyl sulfoxide (DMSO), dimethyl polysiloxane (DMPX), oleic acid, caprylic acid, isopropyl alcohol, 1-octanol, ethanol (denatured or anhydrous), and other pharmaceutical grade or absolute alcohols with the exception of methanol can be used. Preferred embodiments comprise an alcohol (e.g., absolute isopropyl alcohol), which is commercially available. As above, the amount of hydrophilic component in the penetration enhancer depends on the type of the delivered agent and the intended application. The hydrophilic component of a penetration enhancer of the invention can comprise between 0.1% and 50% by weight or volume. That is, a delivery system of the invention can comprise by weight or volume at least, less than or equal to or greater than 0.1%, 0.15%, 0.2%, 0.25%, 0.3%, 0.35%, 0.4%, 0.45%, 0.5%, 0.55%, 0.6%, 0.65%, 0.7%, 0.75%, 0.8%, 0.85%, 0.9%, 0.95%, 1.0%, 1.25%, 1.5%, 1.75%, 2.0%, 2.25%, 2.5%, 2.75%, 3.0%, 3.25%, 3.5%, 3.75%, 4.0%,. 4.25%, 4.5%, 4.75%, 5.0%, 5.25%, 5.5%, 5.75%, 6.0%, 6.25%, 6.5%, 6.75%, 7.0%, 7.25%, 7.5%, 7.75%, 8.0% 8.25%, 8.5%, 8.75%, 9.0%, 9.25%, 9.5%, 9.75%, 10.0%, 10.25%, 10.5%, 10.75%, 11.0%,. 11.25%, 11.5%, 11.75%, 12.0%, 12.25%, 12.5%, 12.75%, 13.0%, 13.25%, 13.5%, 13.75%, 14.0%, 14.25%, 14.5%, 14.75%, 15.0%, 15.5%, 16.0%, 16.5%, 17.0%, 17.5%, 18.0%, 18.5%, 19.0%, 19.5%, 20.0%, 20.5%, 21.0%, 21.5%, 22.0%, 22.5%, 23.0%, 23.5%, 24.0%, 24.5%, 25.0%, 25.5%, 26.0%, 26.5%, 27.0%, 27.5%, 28.0%, 28.5%, 29.0%, 29.5%, 30.0%, 30.5%, 31.0%, 31.5%, 32.0%, 32.5%, 33.0%, 33.5%, 34.0%, 34.5%, 35.0%, 35.5%, 36.0%, 36.5%, 37.0%, 37.5%, 38.0%, 38.5%, 39.0%, 39.5%, 40.0%, 41.0%, 42.0%, 43.0%, 44.0%, 45.0%, 46.0%, 47.0%, 48.0%, 49.0%, or 50.0% hydrophilic component.

In addition to a delivered agent and penetration enhancer, the transdermal delivery compositions described herein can comprise an aqueous adjuvant. The section below describes the incorporation of aqueous adjuvants in formulations of transdermal delivery compositions, in particular, *Aloe Vera,* which can enhance the delivery of both low and high molecular weight molecules to the skin cells of the body.

### Aqueous Adjuvants

Several embodiments of the transdermal delivery composition described herein comprise an aqueous adjuvant such as *Aloe Vera* juice or water or both. The term *"Aloe"* refers to the genus of South African plants of the *Liliaceae* family, of which the *Aloe barbadensis* plant is a species. *Aloe* is an intricate plant, which contains many biologically active substances. (Cohen, et al. in Wound Healing/Biochemical and Clinical Aspects, 1st ed. WB Saunders, Philadelphia (1992)). Over 300 species of *Aloe* are known, most of which are indigenous to Africa. Studies have shown that the biologically active substances are located in three separate sections of the *Aloe* leaf--a clear gel fillet located in the center of the leaf, in the leaf rind or cortex of the leaf and in a yellow fluid contained in the pericyclic cells of the vascular bundles, located between the leaf rind and the internal gel fillet, referred to as the latex. Historically, *Aloe* products have been used in dermatological applications for the treatment of burns, sores and other wounds. These uses have stimulated a great deal of research in identifying compounds from *Aloe* plants that have clinical activity, especially anti-inflammatory activity. (*See e.g.,* Grindlay and Reynolds (1986) J. of Ethnopharmacology 16:117-151; Hart, et al. (1988) J. of Ethnopharmacology 23:61-71). As a result of these studies there have been numerous reports of *Aloe* compounds having diverse biological activities, including anti-tumor activity, anti-gastric ulcer, anti-diabetic, anti-tyrosinase activity, (*See e.g.,* Yagi, et al. (1977) Z. Naturforsch. 32c:731-734), and antioxidant activity (International Application Serial No. PCT/US95/07404).

Recent research has also shown that *Aloe Vera,* a term used to describe the extract obtained from processing the entire leaf, isolated from the *Aloe Vera* species of *Aloe,* can be used as a vehicle for delivering hydrocortisone, estradiol, and testosterone propionate. (*See* Davis, et al, JAPMA 81:1 (1991) and U.S. Pat. No. 5,708,038 to Davis)). As set forth in Davis (U.S. Pat. No. 5,708,308), one embodiment of *"Aloe Vera"* can be prepared by "whole-leaf processing" of the whole leaf of the *Aloe barbadensis* plant. Briefly, whole leaves obtained from the *Aloe barbadensis* plant are ground, filtered, treated with cellulase (optional) and activated carbon and lyophilized. The lyophilized powder is then reconstituted with water prior to use.

*Aloe Vera* can be obtained commercially through Aloe Laboratories, for example. In other embodiments, the *Aloe Vera* is made as follows. First, the leaves are manually harvested. Next, the leaves are washed with water and the thorns on both ends are cut. The leaves are then hand-filleted so as to extract the inner part of the leaf. The inner gel is passed through a grinder and separator to remove fiber from the gel. Then the gel is put into a pasteurizing tank where L-Ascorbic Acid (Vitamin C) and preservatives are added. The gel is pasteurized at 85°C for 30 minutes. After pasteurization, the gel is put into a holding tank for about one or two days, after which the gel is sent through a ½ micron filter. Finally, the gel is cooled down through a heat exchanger and stored in a steamed, sanitized and clean 55 gallon drum. The above described sources and manufacturing methods of *Aloe Vera* are given as examples and not intended to limit the scope of the invention. One of ordinary skill in the art will recognize that *Aloe Vera* is a well known term of art, and that *Aloe Vera* is available from various sources and manufactured according to various methods.

Absolute *Aloe Vera* (100% pure) can also be obtained from commercial suppliers (Lily of the Desert, Irving, Texas). *Aloe Vera* juice, prepared from gel fillet, has an approximate molecular weight of 200,000 to 1,400,000 daltons. Whole leaf *Aloe Vera* gel has a molecular weight of 200,000 to 3,000,000 depending on the purity of the preparation. Although, preferably, the embodiments of the invention having *Aloe Vera* comprise *Aloe Vera* juice, other extracts from a member of the *Liliaceae* family can be used *(e.g.,* an extract from another *Aloe* species).

Transdermal delivery compositions having *Aloe Vera* can comprise between 0.1% to 85.0% by weight or volume *Aloe Vera*. That is, embodiments of the invention can comprise by weight or volume at least, less than or equal to or greater than 0.1%, 0.15%, 0.2%, 0.25%, 0.3%, 0.35%, 0.4%, 0.45%, 0.5%, 0.55%, 0.6%, 0.65%, 0.7%, 0.75%, 0.8%, 0.85%, 0.9%, 0.95%, 1.0%, 1.25%, 1.5%, 1.75%, 2.0%, 2.25%, 2.5%, 2.75%, 3.0%, 3.25%, 3.5%, 3.75%, 4.0%,. 4.25%, 4.5%, 4.75%, 5.0%, 5.25%, 5.5%, 5.75%, 6.0%, 6.25%, 6.5%, 6.75%, 7.0%, 7.25%, 7.5%, 7.75%, 8.0% 8.25%, 8.5%, 8.75%, 9.0%, 9.25%, 9.5%, 9.75%, 10.0%, 10.25%, 10.5%, 10.75%, 11.0%,. 11.25%, 11.5%, 11.75%, 12.0%, 12.25%, 12.5%, 12.75%, 13.0%, 13.25%, 13.5%, 13.75%, 14.0%, 14.25%, 14.5%, 14.75%, 15.0%, 15.5%, 16.0%, 16.5%, 17.0%, 17.5%, 18.0%, 18.5%, 19.0%, 19.5%, 20.0%, 20.5%, 21.0%, 21.5%, 22.0%, 22.5%, 23.0%, 23.5%, 24.0%, 24.5%, 25.0%, 25.5%, 26.0%, 26.5%, 27.0%, 27.5%, 28.0%, 28.5%, 29.0%, 29.5%, 30.0%, 30.5%, 31.0%, 31.5%, 32.0%, 32.5%, 33.0%, 33.5%, 34.0%, 34.5%, 35.0%, 35.5%, 36.0%, 36.5%, 37.0%, 37.5%, 38.0%, 38.5%, 39.0%, 39.5%, 40.0%, 40.25%, 40.5%, 40.75%, 41.0%, 41.25%, 41.5%, 41.75%, 42.0%, 42.25%, 42.5%, 42.75%, 43.0%,. 43.25%, 43.5%, 43.75%, 44.0%, 44.25%, 44.5%, 44.75%, 45.0%, 45.25%, 45.5%, 45.75%, 46.0%, 46.25%, 46.5%, 46.75%, 47.0% 47.25%, 47.5%, 47.75%, 48.0%, 48.25%, 48.5%, 48.75%, 49.0%, 49.25%, 49.5%, 49.75%, 50.0%,. 50.25%, 50.5%, 50.75%, 51.0%, 51.25%, 51.5%, 51.75%, 52.0%, 52.25%, 52.5%, 52.75%, 53.0%, 53.25%, 53.5%, 53.75%, 54.0%, 54.5%, 54.0%, 54.5%, 55.0%, 55.5%, 56.0%, 56.5%, 57.0%, 57.5%, 58.0%, 58.5%, 59.0%, 59.5%, 60.0%, 60.5%, 61.0%, 61.5%, 62.0%, 62.5%, 63.0%, 63.5%, 64.0%, 64.5%, 65.0%, 65.5%, 66.0%, 66.5%, 67.0%, 67.5%, 68.0%, 68.5%, 69.0%, 69.5%, 70.0%, 70.5%, 71.0%, 71.5%, 72.0%, 72.5%, 73.0%, 73.5%, 74.0%, 74.5%, 75.0%, 75.5%, 76.0%, 76.5%, 77.0%, 77.5%, 78.0%, 78.5%, 79.0%, 79.5%, 80.0%, 80.5%, 81%, 81.5%, 82%, 82.5%, 83%, 83.5%, 84%, 84.5%, and 85% *Aloe Vera.*

The amount of water in the delivery system generally depends on the amount of other reagents (e.g., delivered agent, penetration enhancer, and other aqueous adjuvants or fillers). Although water is used as the sole aqueous adjuvant in some embodiments, preferred embodiments use enough water to make the total volume of a particular preparation of a delivery system such that the desired concentrations of reagents in the penetration enhancer, aqueous adjuvant, and delivered agent are achieved. Suitable forms of water are deionized, distilled, filtered or otherwise purified. Clearly, however, any form of water can be used as an aqueous adjuvant.

Transdermal delivery compositions having water can comprise between 0.1% to 85.0% by weight or volume water. That is, embodiments of the invention can comprise by weight or volume at least, less than or equal to or greater than 0.1%, 0.15%, 0.2%, 0.25%, 0.3%, 0.35%, 0.4%, 0.45%, 0.5%, 0.55%, 0.6%, 0.65%, 0.7%, 0.75%, 0.8%, 0.85%, 0.9%, 0.95%, 1.0%, 1.25%, 1.5%, 1.75%, 2.0%, 2.25%, 2.5%, 2.75%, 3.0%, 3.25%, 3.5%, 3.75%, 4.0%,. 4.25%, 4.5%, 4.75%, 5.0%, 5.25%, 5.5%, 5.75%, 6.0%, 6.25%, 6.5%, 6.75%, 7.0%, 7.25%, 7.5%, 7.75%, 8.0% 8.25%, 8.5%, 8.75%, 9.0%, 9.25%, 9.5%, 9.75%, 10.0%, 10.25%, 10.5%, 10.75%, 11.0%,. 11.25%, 11.5%, 11.75%, 12.0%, 12.25%, 12.5%, 12.75%, 13.0%, 13.25%, 13.5%, 13.75%, 14.0%, 14.25%, 14.5%, 14.75%, 15.0%, 15.5%, 16.0%, 16.5%, 17.0%, 17.5%, 18.0%, 18.5%, 19.0%, 19.5%, 20.0%, 20.5%, 21.0%, 21.5%, 22.0%, 22.5%, 23.0%, 23.5%, 24.0%, 24.5%, 25.0%, 25.5%, 26.0%, 26.5%, 27.0%, 27.5%, 28.0%, 28.5%, 29.0%, 29.5%, 30.0%, 30.5%, 31.0%, 31.5%, 32.0%, 32.5%, 33.0%, 33.5%, 34.0%, 34.5%, 35.0%, 35.5%, 36.0%, 36.5%, 37.0%, 37.5%, 38.0%, 38.5%, 39.0%, 39.5%, 40.0%, 40.25%, 40.5%, 40.75%, 41.0%, 41.25%, 41.5%, 41.75%, 42.0%, 42.25%, 42.5%, 42.75%, 43.0%,. 43.25%, 43.5%, 43.75%, 44.0%, 44.25%, 44.5%, 44.75%, 45.0%, 45.25%, 45.5%, 45.75%, 46.0%, 46.25%, 46.5%, 46.75%, 47.0% 47.25%, 47.5%, 47.75%, 48.0%, 48.25%, 48.5%, 48.75%, 49.0%, 49.25%, 49.5%, 49.75%, 50.0%,. 50.25%, 50.5%, 50.75%, 51.0%, 51.25%, 51.5%, 51.75%, 52.0%, 52.25%, 52.5%, 52.75%, 53.0%, 53.25%, 53.5%, 53.75%, 54.0%, 54.5%, 54.0%, 54.5%, 55.0%, 55.5%, 56.0%, 56.5%, 57.0%, 57.5%, 58.0%, 58.5%, 59.0%, 59.5%, 60.0%, 60.5%, 61.0%, 61.5%, 62.0%, 62.5%, 63.0%, 63.5%, 64.0%, 64.5%, 65.0%, 65.5%, 66.0%, 66.5%, 67.0%, 67.5%, 68.0%, 68.5%, 69.0%, 69.5%, 70.0%, 70.5%, 71.0%, 71.5%, 72.0%, 72.5%, 73.0%, 73.5%, 74.0%, 74.5%, 75.0%, 75.5%, 76.0%, 76.5%, 77.0%, 77.5%, 78.0%, 78.5%, 79.0%, 79.5%, 80.0%, 80.5%, 81%, 81.5%, 82%, 82.5%, 83%, 83.5%, 84%, 84.5%, and 85% water. In addition to the aforementioned compositions, methods of making and using the transdermal delivery compositions are described in the following section.

### Preparing Transdermal delivery compositions

In general, transdermal delivery compositions are prepared by combining an ethoxylated fatty moiety or a penetration enhancer with a delivered agent and, optionally, an aqueous adjuvant. Depending on the solubility of the delivered agent, the delivered agent can be solubilized in either the hydrophobic or hydrophilic components of the penetration enhancer. In some formulations, (e.g., formulations containing oil soluble delivered agents such as steroid hormones), the delivered agent readily dissolves in the ethoxylated oil without water, alcohol, or an aqueous adjuvant. In other formulations, the delivered agent (*e.g.,* an NSAID or collagen or fragments thereof) readily dissolves in water, which is then mixed with the ethoxylated oil. Additionally, some delivered agents can be solubilized in the aqueous adjuvant prior to mixing with the penetration enhancer. Desirably, the pH of the mixture is maintained between 3 and 11 and preferably between 5 and 9. That is, during preparation and after preparation the pH of the solution is desirably maintained at less than, more than, at least, or equal to 3.0, 3.25, 3.5, 3.75, 4.0, 4.25, 4.5, 4.75, 5.0, 5.25, 5.5, 5.75, 6, 6.25, 6.5, 6.75, 7.0, 7.25, 7.5, 7.75, 8.0, 8.25, 8.5, 8.75, 9.0, 9.25, 9.5, 9.75, 10.0, 10.25, 10.5, 10.75, or 11.0.

Several physical mixing techniques can be employed to help the delivery system coalesce. For example, a magnetic stir plate and bar can be used, however, the speed of stirring is preferably minimized so as not to drive air into the mixture and/or destroy the delivered agent (e.g., when the delivered agent is a peptide or a protein). Additionally, a rocker can be used to bring components of the delivery system together. Heat can also be applied to help coalesce the mixture but desirably, the temperature is not raised above 40°C so that labile aqueous adjuvants or labile delivered agents are not degraded. Preferably, once the delivery system has coalesced, other components such as fragrances and colors are added or the delivery system is incorporated into a cream or ointment or a device for applying the delivery system.

Several formulations of delivery system are within the scope of aspects of the invention. In embodiments that include an aqueous adjuvant, the ratio of hydrophilic component:hydrophobic component:aqueous adjuvant is desirably 3:4:3, but preferred formulations comprise 1:1:4, 1:1:14, and 1:10:25. As described above, a sufficient amount of delivered agent to suit the intended purpose is incorporated into the delivery system. The amount of delivered agent that is incorporated into the penetration enhancer depends on the compound, desired dosage, and application.

In some embodiments, the transdermal delivery composition is made by providing an ethoxylated oil, mixing the ethoxylated oil with an alcohol, non-ionic solubilizer, or emulsifier so as to form a penetration enhancer, mixing the penetration enhancer with an aqueous adjuvant (*e.g.,* an extract from a plant of the *Liliaeacae* family), and mixing the penetration enhancer and aqueous adjuvant with a delivered agent and thereby making the transdermal delivery composition. For example, an embodiment of a transdermal delivery composition comprising a pain relief solution is manufactured as follows. A solution of 2.0% to 7.0% oleoresin capsicum, 2.5 grams of Boswellin is mixed with 400ml of absolute carpilic alcohol or isopropyl alcohol, 300ml of ethoxylated castor oil, and 300ml of a 100% solution of *Aloe Vera*. This transdermal delivery composition has been observed to alleviate pain when rubbed on a targeted area.

The transdermal delivery compositions having a form of Hepsyl as a delivered agent desirably are comprised by weight or volume of between 0.005% to 12.0% Hepsyl, depending on the type of Hepsyl, its solubility, and the intended application. For example, embodiments having Hepsyl CA 1501C. Hepsyl CGA 1501K., and Hepsyl RA 150K can be comprised by weight or volume of 0.01-2 grams of Hepsyl delivered agent, 0-50 mL of hydrophobic penetration enhancers (*e.g*., ethoxylated castor oil, jojoba oil, etc.), 0-50 mL of hydrophilic penetration enhancers, nonionic solubilizers, or emulsifiers (*e.g*., isopropyl. alcohol, DMSO, etc.), and 0-50 mL of aqueous adjuvant (*e.g.,* water, *Aloe Vera* extract, etc.). A particularly desirable embodiment of the invention is comprised of 0.1-0.5 gram of Hepsyl, 5-10 mL of ethoxylated castor oil, 5-10 mL of isopropyl alcohol, and 5-10 mL of *Aloe Vera* extract. By using these formulations, other delivered agents can be incorporated into a transdermal delivery composition. Formulations of transdermal delivery compositions having collagens are described in the examples. The following section describes several therapeutic, prophylactic and cosmetic applications.

### Therapeutic, Prophylactic, and Cosmetic Applications

Many embodiments are suitable for treatment of subjects either as a preventive measure (e.g., to avoid pain or skin disorders) or as a therapeutic to treat subjects already afflicted with skin disorders or who are suffering pain. In general, most drugs, chemicals, and cosmetic agents that can be incorporated into a pharmaceutical or cosmetic can be formulated into a transdermal delivery composition of the invention. Because the various formulations of transdermal delivery composition described herein have a considerable range in hydrophobic and hydrophilic character, most drugs, chemicals, and cosmetic preparations can be incorporated therein. That is, by adjusting the amount of ethoxylation, alcohol, and water in a particular formulation most pharmaceutical and cosmetic agents are solubilized in a transdermal delivery composition with little effort. Furthermore, because the transdermal delivery compositions described herein can deliver a wide range of materials of both high and low molecular weight to skin cells, the utility of the transdermal delivery compositions described herein is incredibly broad. The aspects of the invention that follow are for exemplary purposes only, and one of skill in the art can readily appreciate the wide spread applicability of a transdermal delivery composition described herein and the incorporation of other delivered agents into a formulation of transdermal delivery composition is straight forward.

In one embodiment, for example, a method of treatment or prevention of inflammation, pain, or human diseases, such as cancer, arthritis, and Alzheimer's disease, comprises using a transdermal delivery composition described herein that has been formulated with an NSAID. Because delivered agents such as NSAIDs, capsaicin, and Boswellin interfere and/or inhibit cyclooxygenase enzymes (COX-1 and COX-2), they provide a therapeutically beneficial treatment for cancer and Alzheimer's disease when administered by a transdermal delivery composition described herein. (*See* U.S. Pat. No. 5,840,746 to Ducharme et al., and U.S. Pat. No. 5,861,268 to Tang et al.).

By one approach, a transdermal delivery composition comprising a delivered agent that is effective at reducing pain or inflammation (e.g., NSAIDS, capsaicin, Boswellin, or any combination thereof) is administered to a subject in need and the reduction in pain or inflammation is monitored. An additional approach involves identifying a subject in need of a COX enzyme inhibitor (*e.g*., a subject suffering from cancer or Alzheimer's disease) and administering a transdermal delivery composition comprising a delivered agent that inhibits a COX enzyme (*e.g*., NSAIDS, capsaicin, Boswellin, or any combination thereof). Although many individuals can be at risk for contracting cancer or Alzheimer's disease, those with a family history or a genetic marker associated with these maladies are preferably identified. Several diagnostic approaches to identify persons at risk of developing these diseases have been reported. (See *e.g.,* U.S. Pat. Nos., 5,891,857; 5,744,368; 5,891,651; 5,837,853; and 5,571,671). The transdermal delivery composition is preferably applied to the skin at a region of inflammation or an area associated with pain or the particular condition and treatment is continued for a sufficient time to reduce inflammation, pain, or inhibit the progress of the disease. Typically, pain and inflammation will be reduced in 5-20 minutes after application. Cancer and Alzheimer's disease can be inhibited or prevented with prolonged use.

In another method, an approach to reduce wrinkles and increase skin tightness and flexibility (collectively referred to as "restoring skin tone") is provided. Accordingly, a transdermal delivery composition comprising a form of collagen or fragment thereof as a delivered agent is provided and contacted with the skin of a subject in need of treatment. By one approach, a subject in need of skin tone restoration is identified, a transdermal delivery composition comprising collagen or a fragment thereof is administered to the subject, and the restoration of the skin tone is monitored. Identification of a person in need of skin restoration can be based solely on visible inspection and the desire to have tight, smooth, and flexible skin. Treatment with the delivery system is continued until a desired skin tone is achieved. Typically a change in skin tone will be visibly apparent in 15 days but prolonged use may be required to retain skin tightness and flexibility. The form of collagen in the delivered agent can be from various sources and can have many different molecular weights, as detailed above. Preferably, high molecular weight natural collagens are used, however, recombinant collagens, modified collagens, protease resistant collagens, and fragments thereof may be used with some of the transdermal delivery compositions described herein.

The transdermal delivery compositions described herein can be processed in accordance with conventional pharmacological, veterinary and cosmetological methods to produce medicinal, veterinary, and cosmetic agents for administration to animals and humans in need thereof (*e.g*., mammals including humans, dogs, cats, horses, cattle, and other companion or farm animals). The transdermal delivery compositions described herein can be incorporated into a pharmaceutical or cosmetic product with or without modification. The compositions of the invention can be employed in admixture with conventional excipients, e.g., pharmaceutically acceptable organic or inorganic carrier substances suitable for topical application that do not deleteriously react with the molecules that assemble the delivery system. The preparations can be sterilized and if desired mixed with auxiliary agents, e.g., lubricants, preservatives, stabilizers, coloring, aromatic substances and the like that do not deleteriously react with the active compounds. They can also be combined where desired with other active agents. Embodiments described herein can be made according to good manufacturing processes (e.g., certified GMP), can be approved by a governmental body, such as the Food and Drug Administration, and may have indicia that indicates that said compositions were manufactured GMP or were approved by a governmental body, with or without structure-function indicia (e.g., indicia that indicates the product's usefulness for improvement of one's appearance or the general health and welfare of individuals that use the product).

The effective dose and method of administration of a transdermal delivery system formulation can vary based on the individual patient and the stage of the disease, as well as other factors known to those of skill in the art. Although several doses of delivered agents have been indicated above, the therapeutic efficacy and toxicity of such compounds in a delivery system of the invention can be determined by standard pharmaceutical or cosmetological procedures with experimental animals, e.g., ED50 (the dose therapeutically effective in 50% of the population) and LD50 (the dose lethal to 50% of the population). The dose ratio of toxic to therapeutic effects is the therapeutic index, and it can be expressed as the ratio, LD50/ED50. Pharmaceutical and cosmetological compositions that exhibit large therapeutic indices are preferred. The data obtained from animal studies is used in formulating a range of dosages for human use. The dosage of such compounds lies preferably within a range of circulating concentrations that include the ED50 with little or no toxicity. The dosage varies within this range depending upon the dosage form employed, sensitivity of the patient, and the route of administration.

The exact dosage is chosen by the individual physician in view of the patient to be treated. Dosage and administration are adjusted to provide sufficient levels of the active moiety or to maintain the desired effect. Additional factors that may be taken into account include the severity of the disease state, age, weight and gender of the patient; diet, time and frequency of administration, drug combination(s), reaction sensitivities, and tolerance/response to therapy. Short acting compositions are administered daily whereas long acting pharmaceutical compositions are administered every 2, 3 to 4 days, every week, or once every two weeks. Depending on half-life and clearance rate of the particular formulation, the pharmaceutical compositions of the invention are administered once, twice, three, four, five, six, seven, eight, nine, ten or more times per day.

Routes of administration of the delivery systems of the invention are primarily topical, although it is desired to administer some embodiments to cells that reside in deep skin layers. Topical administration is accomplished via a topically applied cream, gel, rinse, etc. containing a delivery system of the invention. Compositions of delivery system-containing compounds suitable for topical application include, but are not limited to, physiologically acceptable ointments, creams, rinses, and gels.

In some embodiments, the transdermal delivery composition is incorporated into a device that facilitates application. The embodied compositions generally have a vessel joined to an applicator, wherein a transdermal delivery composition of the invention is incorporated in the vessel. Some devices, for example, facilitate delivery by encouraging vaporization of the mixture. These apparatus have a transdermal delivery composition of the invention incorporated in a vessel that is joined to an applicator such as a sprayer *(e.g.,* a pump-driven sprayer). These embodiments can also comprise a propellant for driving the incorporated transdermal delivery composition out of the vessel. Other apparatus can be designed to allow for a more focused application. A device that facilitates a focused application of a transdermal delivery composition of the invention can have a roll-on or swab-like applicator joined to the vessel that houses the transdermal delivery composition. Several devices that facilitate the administration of a delivery system of the invention have a wide range of cosmetic or therapeutic applications. An exemplary transdermal delivery device is described in the section that follows.

### Transdermal Delivery Dispenser

In some embodiments, the transdermal delivery composition is provided in a single dose application containing a pre-measured amount of a delivered agent. For example, septum sealed vials with or without an applicator (e.g., a swab) containing a pre-measured amount of transdermal delivery composition (e.g., 0.5ml) containing a pre-measured amount of a delivered agent (*e.g*., 400mg of ibuprofen, 0.6mg marine collagen, or 1g of aspirin) are embodiments of the invention. These embodiments have significant utility because pre-determined doses of certain delivered agents facilitate appropriate treatment regimens and the individually sealed doses of the transdermal delivery composition with delivered agent maintain sterility of the composition between applications.

Figures 1A and 1B show an exemplary embodiment of a dispenser 100. As can be seen in Figure 1A, in which the dispenser 100 is shown in an exploded state, the dispenser 100 comprises a removable cartridge 102 and a body portion 104. A latch member 106 on the body portion 104, is shown in an unsecured state, permitting the insertion and removal of the removable cartridge 102. The latch member 106 is slidable between the unsecured position as shown and a secured position 108, shown in shadow, in which the insertion and/or removal of a removable cartridge 102 is inhibited. Although a slidable latch member 106 is shown in this embodiment, it will be understood that any method of securing the removable cartridge 102 to the body portion 104 can be used. For example, a pin attached to the body portion 104 could engage an aperture on the removable cartridge 102. Alternately, if the body portion is formed from a sufficiently resilient material, the body portion can be designed such that a snug fit is formed without any need for additional securing methods. Transparent portion 103 permits the user to view the amount of fluid remaining in removable cartridge 102. Similarly, transparent portion 105 allows the user to see the amount of fluid to be dosed.

Figure 1B shows the dispenser 100 in an assembled state, where the removable cartridge 102 has been inserted into the body portion 104. The latch member 106 has been moved to the secured position 108 shown in shadow in Figure 1A.

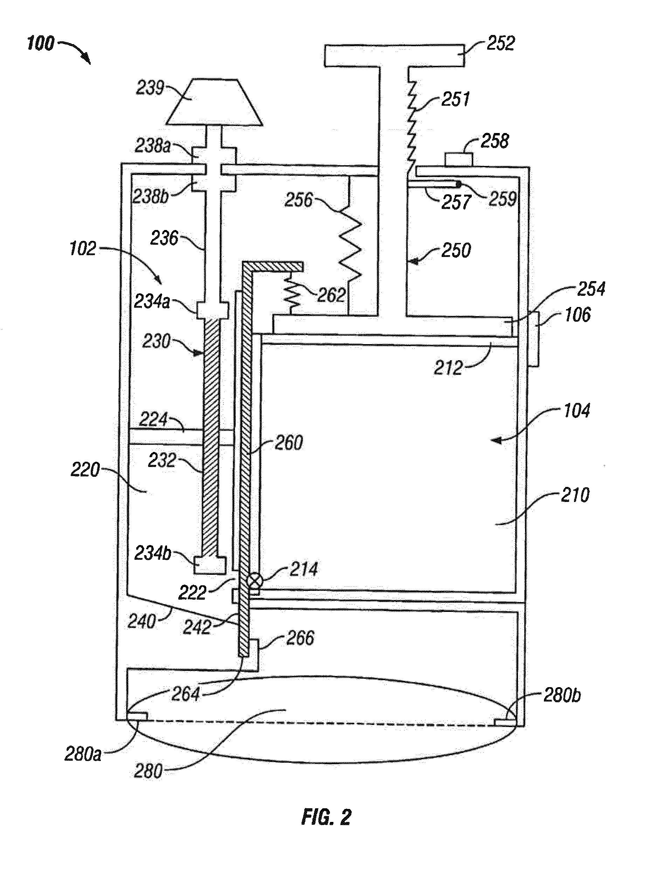

Figure 2 schematically depicts a cross-section of the dispenser 100 of Figure 1, in an assembled state. It can be seen that the removable cartridge 104 comprises a fluid reservoir 210, which is configured to hold the therapeutic drug delivery fluid. The removable cartridge 104 further comprises a movable upper wall 212, which forms the upper wall of the fluid reservoir 210. The movable upper wall is displaceable in at least the downward direction. The removable cartridge 102 also includes a one-way valve 214, such as a check valve, located at the bottom of the removable cartridge, which is in fluid communication with the fluid reservoir 210. As will be described in greater detail below, displacement of the movable upper wall 212 in the downward direction will cause fluid to flow from the fluid reservoir 210 through the valve 214.

Still with respect to Figure 2, the body portion 102 of the dispenser 100 includes a dosing chamber 220. When the dispenser 100 is in an assembled space, the dosing chamber 220 is in fluid communication with the valve 214 of the removable cartridge via an aperture 222 in the dosing chamber aligned with the valve 214. The upper wall of the dosing chamber 220 is formed by the lower surface of a movable member 224, alternately referred to as a dosing member. In this embodiment, movable member 224 comprises a threaded aperture through which a threaded portion 232 portion of shaft 230 extends. Stop members 234a and 234b are located at the upper and lower ends, respectively, of the shaft 230. A non-threaded portion 236 of shaft 230 extends through an aperture in the top of the body portion 104, and wide sections 238a and 238b of shaft 230 constrain vertical translation of the shaft 230 with respect to the body portion 102. A knob 239 at the top of the shaft 220 facilitates rotation by a user.

The size of the dosing chamber 220 can be adjusted by rotating the knob 239, causing rotation of the shaft 230. As the dosing chamber 220 and the movable member 224 have a non-circular shape, the movable member 224 cannot rotate along with the shaft 230. The rotational movement of the shaft therefore results in vertical translation of the movable member 224, changing the volume of the dosing chamber 220. When the movable member reaches one of stop members 234a,b, the rotational movement of the shaft 230 will be inhibited. The movable member 224 may comprise a ring of partially deformable material (not shown), such as a rubberized material, around the edges of the movable member which come in contact with the walls of the dosing chamber, in order to facilitate a tight seal between the edges of the movable member and the walls of the dosing chamber, so as to prevent undesired leakage along the sides of the movable member.

The lower end of the dosing chamber 220 comprises a sloped surface 240, and an aperture 242 in the wall of the dosing chamber. This aperture 242 preferably extends to the bottom of the dosing chamber at least one point along the bottom surface of the dosing chamber, such that all fluid in the dosing chamber 220 can flow out of the aperture 242.

The body portion 104 further comprises a plunger 250 having an upper end 252 and a lower end 254 configured to engage the movable upper wall of the removable cartridge 102. The plunger 250 extends through an aperture in the top of the body portion 104. The plunger 250 is preferably biased to return to a position in which the upper surface of the removable cartridge is not engaged. This may be done, for example, via a spring 256 connecting the body portion 104 and the plunger 250. As will be discussed in greater detail below, it may be desirable to permit the user to control the timing of the return to the initial position. This may be accomplished via the inclusion of teeth 251 along the shaft of the plunger, and a locking member 257 which is attached to the interior of the body portion 102 and biased to remain in a position against the plunger 250, as shown. The teeth 251 therefore permit the downward movement of the plunger, but inhibit the return of the plunger upward to its original position. Locking member 257 is operably connected to a release button 258 on the exterior of the dispenser 100. Engaging the release button rotates locking member 257 about pivot point 259, and permits the return of the plunger to its original position. When the release button is disengaged, the bias of locking member 257 returns it to the position shown in Figure 2.

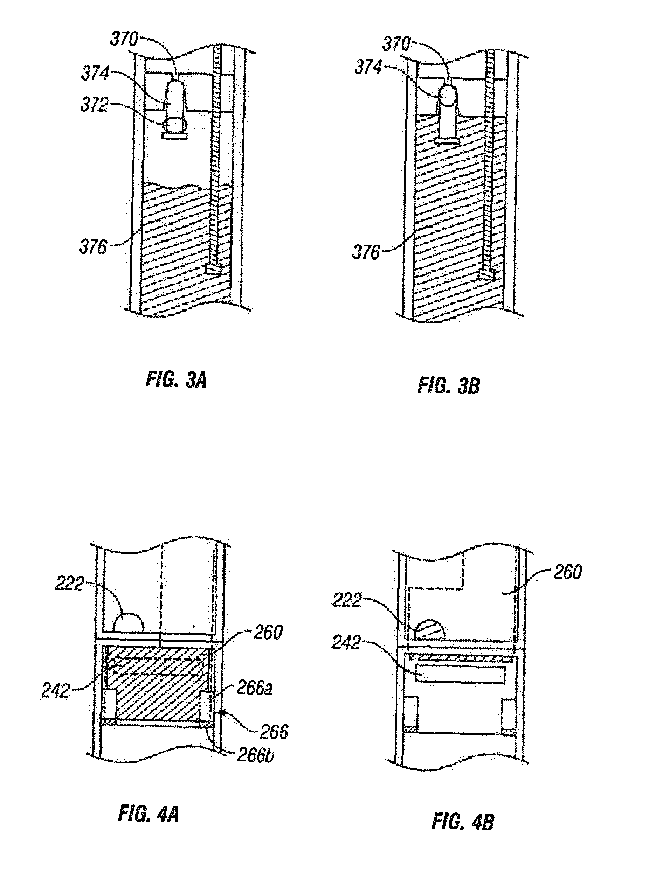

The body portion 104 further comprises a slidable member 260 which is movable between a first position in which the slidable member 260 inhibits fluid flow out of the dosing chamber 220 through the aperture 242, and a second position in which the slidable member 250 inhibits fluid flow from the removable cartridge 102 to the dosing chamber 220 via aperture 222. The slidable member 260 is in the first position when the plunger is in a depressed position, and the second position when the plunger retracts to an undepresed position. This may be accomplished, for example, via spring 262, which connects the plunger 250 to the slidable member 262. When the plunger is depressed, the spring 262 holds the slidable member 260 within a slot 264, located below the sloped surface 240 which forms the bottom of the chamber. As the pressure increases, the sliding member is prevented from flexing away from the dosing chamber by tabs 266. Thus, when the sliding member 260 is in the first position, shown in Figure 2, the dosing chamber can be filled and fluid will not leak out. Fluid is permitted to flow into the dosing chamber due to the shape of sliding member 260, discussed in greater detail with respect to Figure 4. When the plunger is moved to an undepressed position, the slidable member will be pushed upward to the second position, where the flow of fluid through the aperture 242 is permitted.

At the bottom of the dispenser 100 is an applicator. In the present embodiment, the applicator consists of an ellipsoidal applicator 280 mounted on pins 280a and 280b which extend at least partially into the applicator 280 along the axis of the applicator. Applicator 280 thus provides a roll-on applicator, such that once the therapeutic fluid is released from the dosing chamber after the release button 258 is pressed, the fluid will flow downwards onto applicator 280. The applicator can then be placed in contact with the skin of the user, and the dispenser moved to cause the applicator to roll across the skin of the patient, applying the desired dose of the therapeutic fluid to the patient. It will be understood that alternate non-invasive applicators can be used in place of the roll-on applicator. These alternate non-invasive applicators may include, but are not limited to, an absorbent applicator tip, such as a sponge, or an applicator surface having perforations through which the therapeutic fluid can flow.

In the above embodiment, the dosing chamber will be partially filled with fluid when the plunger is depressed, but the air in the dosing chamber will not be permitted to escape, and will therefore be compressed in the dosing chamber. In order to protect the dispenser 100 from damage due to excessive pressure created in the dosing chamber, the valve 214 may be designed to close when a certain pressure has been reached. Taking into account this pressure, and the volume of the trapped air at that pressure, accurate dosing can be obtained by accounting for the volume of the trapped air in the dosing chamber.

In an alternative embodiment, the movable member 224 may comprise a mechanism for allowing air to exit the dosing chamber without permitting fluid. An exemplary system for doing so is shown in Figures 3A and 3B. In Figure 3A, it can be seen that the movable member 324 includes an aperture 370. A sphere 372, which is buoyant relative to the fluid 376which will be used, is suspended within a track 374, which permits movement of the sphere 372 upward to engage the aperture 370, forming a seal, but inhibits movement of the sphere 372 downward below a level necessary to allows air to flow over the sphere 372 and out through the aperture 370.

Figure 3B shows the dosing chamber full of fluid 376. The buoyant sphere 372 is lifted as the fluid level rises within the dosing chamber. Because the sphere 372 is kept level with the fluid, almost all of the air is allowed to escape, but the fluid cannot escape through the aperture once the sphere engages the aperture. The sphere 372 may advantageously be formed of a partially deformable material, to facilitate the forming of a seal between the sphere and the movable member 324. Bouancy of the sphere 372 may be achieved through selection of an appropriate material, or through the use of a hollow sphere, in order to increase buoyancy.

It will be understood that alternate methods of permitting air to escape while preventing fluid flow through the movable member 220 may be utilized, including the use of specialized valves which permit the flow of air while inhibiting the flow of fluid through the valve.

The operation of the sliding member 370 is now described with respect to Figures 4A and 4B. Figure 4A depicts a portion of the cross section of the dispenser 100 of Figure 2, taken along line 4 of Figure 2. In particular, it can be seen that Figure 4A depicts an embodiment in which the slidable member 260, shown partially in shadow where it is locate behind other features, is in a first position in which the slidable member does not inhibit the flow of fluid from the fluid reservoir 210 through the aperture 222 into the dosing chamber 220 (for simplicity, the valve 214 is not depicted, but would be in line with aperture 222). This is due to the design of the slidable member 260 such that it is substantially L-shaped. It can be seen that when the slidable member 260 is in this first position, flow of the fluid out of the dosing chamber 230 through the aperture 242 is prevented by the lower portion of slidable member 260. Slidable member 260 is prevented from flexing away from aperture 242 by the upper portions 266a of tab members 266. In addition, the lower portions 266b of tab members 266 prevent additional downward movement of slidable member 260. Thus, tab members 266 form a slot 264 (see Figure 2) which constrains the slidable member such that it forms a sufficiently rigid barrier to constrain fluid flow out of the dosing chamber.

In Figure 4B, the slidable member 260 has been moved to a second position in which the slidable member 260 obstructs the flow of fluid through aperture 222 into dosing chamber 220, but permits the flow of fluid from dosing chamber 220 through aperture 242, and downward to applicator 280 (not shown). Because the slidable member 260 is operably connected via spring 262 to plunger 250, the slidable member 260 is in the first position when the plunger is depressed and fluid is being dispensed into the dosing chamber, and in the second position when the plunger returns to its original position after the release button is depressed. As can be seen, the lower, thicker portion of the slidable member 260 desirably has sufficient height that at intermediate positions of the slidable member, both of the apertures 222, 242 are completely occluded. Thus, no additional fluid beyond what is already in the dosing chamber will be dispensed.

Although the above detailed description has shown, described, and pointed out novel features of the invention as applied to various embodiments, it will be understood that various omissions, substitutions, and changes in the form and details of the device illustrated may be made by those skilled in the art without departing from the spirit of the invention.

For example, in another embodiment, the dispenser may not include a fluid reservoir contained within a removable cartridge, but may instead be a disposable dispenser without a replaceable cartridge. In another embodiment, the volume of the dosing chamber need not be adjustable by the user. Such an embodiment may be advantageous in situations where precise dosing is required, or where regular fixed doses are required.

In another embodiments, the user actuatable knob which controls the size of the dosing chamber need not be fixed directly to the rotatable shaft, but may instead be operably connected to the rotatable shaft via a gear or a series of gears, so as to facilitate either rapid adjustment of the dosing volume or very precise adjustment of the dosing volume, depending on the relative properties of the gears. In alternate embodiments, one or more of the operably connected features need not be mechanically connected, as described and depicted above. For instance, electrical connections between features and electrical actuators, such as servo motors, stepper motors, or hydraulics, can be used to replace the mechanical interconnections described above. For example, the knob 239 could be replaced by two buttons, electrically connected to a motor, one of which causes the motor to drive the rotatable shaft in one direction, and the other of which causes the motor to drive the rotatable shaft in the other direction. Similarly, the plunger could be replaced by a plunger which is electronically actuatable at the push of a button. A pressure sensor within the dosing chamber could be used to release the plunger once a sufficient pressure has been reached.

Thus, these and other modifications to the above described devices can be made by persons having ordinary skill in the art without departing from the spirit of the invention. As will be recognized, the present invention may be embodied within a form that does not provide all of the features and benefits set forth herein, as some features may be used or practiced separately from others.

Example 1 below describes a clinical study that was performed to evaluate the efficacy of a transdermal delivery composition that comprised capsaicin.

### EXAMPLE 1

In this example, evidence is provided that a transdermal delivery composition of the invention can administer a therapeutically effective amount of a low molecular weight delivered agent *(e.g.,* 0.225% oleoresin capsicum). A clinical study was performed to evaluate the effectiveness of a transdermal delivery composition of the invention comprising 0.225% capsaicin ("EPRS") as compared to a commercially available cream comprising Boswellin, 10% methyl salicylate, and 0.25% capsaicin. (Nature's Herbs). The two pain relief preparations were tested on six subjects who suffer from degenerative arthritis, debilitating back pain, and/ or bursitis. For the first five days of the study, the subjects applied the commercially available cream three times a day. On day six, application of the commercially available cream was stopped and subjects applied the EPRS transdermal delivery composition. The EPRS pain relief solution was also applied for five days, three times a day. Daily analysis of the efficacy of the particular pain relief formulations was taken by the subjects and observations such as the time of administration, odor, and therapeutic benefit were recorded after each administration.

The five day use of the commercially available cream was found to provide only minimal therapeutic benefit. The cream was reported to irritate the skin, have a noxious smell, and provide little decrease in pain or increase in flexibility or range of motion. In contrast, the five day use of EPRS was reported to provide significant pain relief, relative to the relief obtained from the oral consumption of NSAIDs. Further, EPRS was reported to increase flexibility and range of motion within five to twenty minutes after application. Additionally, EPRS did not present a significant odor nor did it cause skin irritation. The results of this study demonstrate that a delivery system comprising a low molecular weight compound, capsaicin, can effectively administer the delivered agent to cells of the body where it provides therapeutic benefit. The next example describes a clinical study that was performed to evaluate the efficacy of several different formulations of transdermal delivery composition that comprised low and high molecular weight collagens.

### EXAMPLE 2

In this example, evidence is provided that a transdermal delivery composition of the invention can administer a therapeutically effective amount of a low and high molecular weight delivered agent *(e.g.,* a low and high molecular weight collagens). A clinical study was performed to evaluate the effectiveness of several transdermal delivery compositions comprising various penetration enhancers, aqueous adjuvants, and collagen delivered agents. The various transdermal delivery compositions that were evaluated are provided in **TABLE 18.** Of the formulations that were originally screened, three were extensively evaluated by ten subjects (three men and seven women) in a single blind study. The formulations analyzed in the single blind study are indicated in **TABLE 18** by a dagger. That is, the three different formulations ("P1", "P2", and "F4") were evaluated.

The P1 formulation comprised approximately 0.73% to 1.46% Solu-Coll, a soluble collagen having a molecular weight of 300,000 daltons. The P2 formulation comprised approximately 1.43% to 2.86% Plantsol, a plant collagen obtained from yeast having a molecular weight of 500,000 daltons. The F4 formulation comprised approximately 11.0% of HydroColl EN-55, a hydrolyzed collagen having a molecular weight of 2,000 daltons. The evaluation of the P1, P2, and F4 formulations was as follows. Left, right, and center mug-shot photographs were taken with a Pentax camera having a zoom 60X lens and Kodak-Gold 100 film before beginning the study. Shortly after, each subject was given a bottle having a formulation of transdermal delivery composition and was instructed to apply the solution to the right side of the face and neck, leaving the left side untreated, twice daily for 15 days. The F4 formulation was tested first and the application was carried out after showering or washing and before application of any other product to the treated area of the face. After the 15 day period, three mug-shot photographs were again taken, the subjects recorded their observations on the effectiveness of the formulation in a questionnaire, and a 7 day period without application of a collagen product provided. The questionnaire requested the subject to assign a score *(e.g.,* a numerical value that represents effectiveness) on characteristics of the transdermal delivery composition formulation. Characteristics that were evaluated included tackiness, odor, marketability, and overall effectiveness of the formulation, as well as, whether the formulation tightened the skin, decreased lines, conditioned or softened the skin, and had any negative side-effects. The scale for the scoring was 1-10, with 1 being the worst rating and 10 being the best rating.

Following the test of F4, the evaluation detailed above was conducted on the P1 formulation. Again, photographs were taken before and after the second 15 day protocol, a questionnaire evaluating the efficacy of the particular formulation was completed, and a 7 day period without application of a collagen product was provided. Further, after the test of P1, the same evaluation was conducted on the P2 formulation, photographs were taken before and after the trial, and a questionnaire evaluating the efficacy of the particular formulation was completed.

The data from the three evaluation questionnaires were pooled, analyzed using a "t-table" and standard deviation calculations were made. See **TABLE 19.** An overall rating for each particular formulation was assigned. A perfect score by this system was a 7.875 overall rating. P1 was found to have a 4.25 overall rating (approximately 54% effective), P2 was found to have a 4.625 overall rating (approximately 59% effective), and F4 was found to have a 5.625 overall rating (approximately 71% effective).

The before and after treatment photographs also revealed that the three tested transdermal delivery compositions provided therapeutic benefit. A decrease in wrinkles was observed and an increase in skin tightness and firmness can be seen. That is, P1, P2, and F4 all provided therapeutic and/or cosmetic benefit in that they restored skin tone in the subjects tested. The results presented above also demonstrate that transdermal delivery compositions of the invention can be used to administer high molecular weight delivered agents.

**TABLE 18**

| ECO | *Aloe* | IPA | Plantsol | EN-55 | Solu-coll | DMPX | YYO | Score | ID |
|---|---|---|---|---|---|---|---|---|---|
| | | | | | | | | | |
| 29.7% * | 50.0%* | 5.0%* | 0* | 8.3%* | 0* | 0* | 0* | 2 | F-1 |
| 10.4% | 79.0% | 5.3% | 0 | 8.7% | 0 | 0 | 0 | 3 | F-2 |
| 5.2% | 63.0% | 5.3% | 0 | 17.4% | 0 | 0 | 0 | 3 | F-3 |
| 5.0% | 70.0% | 5.0% | 0 | 11.0% | 0 | 0 | 0 | 3+ | F-4 t |
| 4.5% | 18.2% | 4.6% | 0 | 0 | 0.7% to 1.5% | 0 | 0 | 3+ | P-1 † |
| 8.3% | 8.3% | 8.3% | 0.7% to 1.4% | 4.6% | 0.3% to 0.7% | 0 | 0 | 2 | Y-500 |
| 0.7% | 22.2% | 11.1% | 1.3% to 2.7% | 0 | 0 | 0 | 0 | 3+ | P-501 |
| 0.4% | 35.7% | 3.6% | 1.1% to 2.1% | 0 | 0 | 0 | 0 | 2 | P-502 |
| 0.9% | 8.7% | 0 | 0 | 0 | 2.3% to 4.6% | 0 | 0 | 1 | SC-1 |
| 1.8% | 18.5% | 0 | 0 | 44.8% | 0 | 0 | 0 | 3+ | SC-2 |
| 1.8% | 17.9% | 7.1% | 0 | 43.2% | 0 | 0 | 0 | 3 | SC-3 |
| 0.9% | 9.4% | 4.7% | 0 | 34.3% | 0.3% to 0.6% | 0 | 0 | 1 | PSC EN |
| 1.8% | 31.3% | 6.3% | 1.3% to 2.5% | 0 | 0 | 0 | 0 | 3+ | P-1A |
| 0.8% | 19.2% | 3.8% | 1.5% to 3.1% | 0 | 0 | 7.7% | 0.3% | 5 | P-1C |
| 0.7% | 17.9% | 7.1% | 1.4% to 2.9% | 0 | 0 | 1.1% | 0.3% | 5 | P-2 t |
| 0.7% | 22.2% | 11.1% | 1.3% to 2.7% | 0 | 0 | 0 | 0 | 3+ | P-501 |

| | | | | | | | | | |
|---|---|---|---|---|---|---|---|---|---|
| *Abbreviations:* ECO - ethoxylated castor oil (BASF) *Aloe - Aloe Vera (Aloe* Labs; (800)-258-5380) IPA - Absolute isopropyl alcohol (Orange County Chemical, Santa Ana, California) Plantsol - Yeast extract collagen (Brooks Industries Inc., Code No. 06485) EN-55 - hydrolyzed bovine collagen (Brooks Industries Inc., Code No. 01000) SoluColl - soluble collagen (Brooks Industries Inc., Code No. 01029) DMPX - dimethyl polysiloxane (5 centistokes) (Sigma) YYO - Y-ling-Y-lang oil (Young Living Essential Oils, Lehl, Utah) ID - Identification number * The percentages reflect volume to volume. † Sample used in the 45 day clinical trial. | | | | | | | | | |

**TABLE 19**

| COLLAGEN T-TABLE | | | | |
|---|---|---|---|---|
| Formulations | P1 | P2 | F4 | standard deviation |
| Tackiness | 5 | 3 | 10 | 2.94 |
| Skin tightness | 7 | 5 | 8 | 1.25 |
| Odor | 2 | 8 | 8 | 2.83 |
| Decrease lines | 2 | 2 | 1 | 0.47 |
| Soften skin | 8 | 7 | 4 | 1.7 |
| Total skin restoration | 5 | 5 | 6 | 0.47 |
| Market Buying Power | 5 | 7 | 8 | 1.25 |
| Side effects | 0 | 0 | 0 | 0 |
| Total Score (Average) | 4.25 | 4.63 | 5.63 | 1.36 |

Several *in vitro* techniques are now widely used to assess the percutaneous absorption of delivered agents. (*See e.g.,* Bronaugh and Collier in In vitro Percutaneous absorption studies:Principle, Fundamentals, and Applications, eds. Bronaugh and Maibach, Boca Raton, Fl, CRC Press, pp237-241 (1991) and Nelson et al., J. Invest. Dermatol. 874-879 (1991)). Absorption rates, and skin metabolism can be studied in viable skin without the interference from systemic metabolic processes. The next example describes several approaches that can be used to evaluate the ability of a particular formulation of transdermal delivery composition to deliver a particular delivered agent.

### EXAMPLE 3

Skin barrier function can be analyzed by examining the diffusion of fluorescent and colored proteins and dextrans of various molecular weights ("markers") across the skin of nude mice or swine. Swine skin is preferred for many studies because it is inexpensive, can be maintained at -20°C, and responds similarly to human skin. Prior to use, frozen swine skin is thawed, hair is removed, and subcutaneous adipose tissue is dissected away. Preferably, a thickness of skin that resembles the thickness of human skin is obtained so as to prepare a membrane that accurately reflects the thickness of the barrier layer. A dermatome can be pushed across the surface of the skin so as to remove any residual dermis and prepare a skin preparation that accurately reflects human skin. Elevation of temperature can also be used to loosen the bond between the dermis and the epidermis of hairless skin. Accordingly, the excised skin is placed on a hot plate or in heated water for 2 minutes at a temperature of approximately 50°C - 60°C and the dermis is removed by blunt dissection. Chemical approaches (e.g., 2M salt solutions) have also been used to separate the dermis from the epidermis of young rodents.

Many different buffers or receptor fluids can be used to study the transdermal delivery of delivered agents across excised skin prepared as described above. Preferably, the buffer is isotonic, for example a normal saline solution or an isotonic buffered solution. More physiological buffers, which contain reagents that can be metabolized by the skin, can also be used. (See *e.g.,* Collier et al., Toxicol. Appl. Pharmacol. 99:522-533 (1989)).

Several different markers with molecular weight from 1,000 daltons to 2,000,000 daltons are commercially available and can be used to analyze the transdermal delivery compositions of the invention. For example, different colored protein markers having a wide range of molecular weights (6,500 to 205,000 daltons) and FITC conjugated protein markers (*e.g.*, FITC conjugated markers from 6,500 to 205,000 daltons) are available from Sigma (C3437, M0163, G7279, A2065, A2190, C1311, T9416, L8151, and A2315). Further, high molecular weight FITC conjugated dextrans (*e.g.*, 250,000, 500,000, and 2,000,000 daltons) are obtainable from Sigma. (FD250S, FD500S, and FD2000S).

Accordingly, in one approach, swine skin preparations, obtained as described above, are treated with a delivery system lacking a delivered agent and control swine skin preparations are treated with water. Subsequently, the skin is contacted with a 1mM solution of a marker with a known molecular weight suspended in Ringer's solution (pH 7.4) at 37°C. After one hour, the skin is frozen and sliced at a thickness of 5µm. The sections are counter stained with 5µg/ml propidium and, if the marker is FITC conjugated, the sections are analyzed by fluoresence microscopy. If the marker is a colored marker, diffusion of the marker can be determined by light microscope. The marker will be retained in the upper layers of the stratum corneum in the skin(delete "untreated mice") but the skin treated with the delivery system will be found to have the dye distributed throughout the stratum corneum and any dermal layer that remains.

Additionally, modifications of the experiments described above can be performed by using a delivery system comprising various molecular weight markers. Accordingly, skin preparations are treated with the delivery system comprising one or more markers and control skin preparations are treated with water. After one hour, the skin is frozen and sliced at a thickness of 5µm. The sections can be counter stained with 5µg/ml propidium iodide and can be analyzed by fluoresence microscopy (*e.g.*, when a fluorescent marker is used) or alternatively, the sections are analyzed under a light microscope. The marker will be retained in the upper layers of the stratum corneum in the skin(delete "untreated mice") but the skin treated with the delivery system will be found to have the dye distributed throughout the stratum corneum and any dermal layer that remains.

In another method, the transdermal water loss (TEWL) of penetration enhancer-treated skin preparations can be compared to that of untreated skin preparations. Accordingly, skin preparations are obtained, as described above, and are treated with a delivery system of the invention lacking a delivered agent *(e.g.,* a penetration enhancer). Control skin preparations are untreated. To assess TEWL, an evaporimeter is used to analyze the skin preparation. The Courage and Khazaka Tewameter TM210, an open chamber system with two humidity and temperature sensors, can be used to measure the water evaporation gradient at the surface of the skin. The parameters for calibrating the instrument and use of the instrument is described in Barel and Clarys Skin Pharmacol. 8: 186-195 (1995) and the manufacturer's instructions. In the controls, TEWL will be low. In contrast, TEWL in penetration enhancer-treated skin preparations will be significantly greater.

Further, skin barrier function can be analyzed by examining the percutaneous absorption of labeled markers (*e.g.*, radiolabeled, fluorescently labeled, or colored) across skin preparations in a diffusion chamber. Delivery systems of the invention having various molecular weight markers, for example, the proteins and dextrans described above, are administered to swine skin preparations. Swine skin preparations are mounted in side-by-side diffusion chambers and are allowed to stabilize at 37°C with various formulations of penetration enhancer. Donor and receiver fluid volumes are 1.5ml. After 1 hour of incubation, a labeled marker is added to the epidermal donor fluid to yield a final concentration that reflects an amount that would be applied to the skin in an embodiment of the invention. Five hundred microliters of receiver fluid is removed at various time points, an equal volume of penetration enhancer is added to the system. The aliquot of receiver fluid removed is then analyzed for the presence of the labeled marker (*e.g*., fluorescent detection, spectroscopy, or scintillation counting). Control swine skin preparations are equilibrated in Ringer's solution (pH 7.4) at 37°C; the same concentration of labeled marker as used in the experimental group is applied to the donor fluid after one hour of equilibration; and 500µl of receiver fluid is analyzed for the presence of the marker. In the experimental group, the steady-state flux of labeled marker in the skin will be significantly greater than that of the control group. By using these approaches, several transdermal delivery compositions can be evaluated for their ability to transport low and high molecular weight delivered agents across the skin. The next example describes several different formulations of transdermal delivery composition that were made to comprise various delivered agents, demonstrating the wide-range of utility of aspects of the invention.

### EXAMPLE 4

In this example, several different formulations of transdermal delivery composition containing various delivered agents are provided. The formulations described include: compositions for removing age spots and restoring skin brightness, compositions for advanced pain relief, muscle relaxers, hormone replacement products, wound healing formulations, products for reducing fine lines and wrinkles, stretch mark reducing products, growth factor products, and anti-psoriasis products.

**SKIN BRIGHTENING OR AGE SPOT REDUCING PRODUCT:**

| | |
|---|---|
| Melaslow (10%) | 30 ml |
| Ethoxylated Macadamia nut oil (16 ethoxylations/molecule) | 160 ml |
| Ethanol | 80 ml |
| Water | 40 ml |
| Marine collagen (1%) | 40 ml |
| Etioline (5%) | 30 ml |

This formulation was found to rapidly reduce the appearance of age spots in a subject that applied daily amounts of the product for thirty days.

**STRETCH MARK REDUCING PRODUCTS: FORMULATION #1**

| | |
|---|---|
| Eucalyptus oil | 400 ml |
| Ethanol | 180 ml |
| Ethoxylated macadamia nut oil (16 ethoxylations/molecule) | 180 ml |
| Distilled water | 40 ml |
| various perfumes were added including | |
| lemon oil or | 30 drops |
| lavender or | 30 drops |
| sweet orange or | 1 ml |
| tangerine | 30 drops |

**FORMULATION #2**

| | |
|---|---|
| Eucalyptus oil | 500 ml |
| Ethanol | 225 ml |
| Ethoxylated macadamia nut oil (16 ethoxylations/molecule) | 225 ml |
| Distilled water | 50 ml |

**FORMULATION #3**

| | |
|---|---|
| Eucalyptus oil (*Kayuuputih* oil) | 400 ml |
| Ethanol | 220 ml |
| Ethoxylated macadamia nut oil (16 ethoxylations/molecule) | 180 ml |
| Distilled water | 40 ml |
| Y-Ling-Y-Lang | 22 drops |
| Coconut oil | 3 ml |

These formulations were found to rapidly reduce the appearance of stretch marks in a subject that applied daily amounts of the products for thirty days.

**TESTOSTERONE SUPPLEMENTATION PRODUCTS: FORMULATION #1**

| | |
|---|---|
| Ethanol | 30 ml |
| Ethoxylated macadamia nut oil (16 ethoxylations/molecule) | 30 ml |
| Water | 20 ml |
| Testosterone | 10 ml (200 mg/ml) |
| Coconut oil | 10 drops |

**FORMULATION #2**

| | |
|---|---|
| Ethanol | 40 ml |
| Ethoxylated macadamia nut oil (16 ethoxylations/molecule) | 40 ml |
| Water | 5 ml |
| Testosterone | 5 ml (200 mg/ml) |
| Coconut oil | 10 drops |
| Y-Ling-Y-Lang oil | 10 drops |

**FORMULATION #3**

| | |
|---|---|
| Testosterone | 10 ml (200 mg/ml) |
| Ethanol | 40 ml |
| Ethoxylated macadamia nut oil (16 ethoxylations/molecule) | 40 ml |
| Coconut oil | 10 drops |
| Y-Ling-Y-Lang oil | 10 drops |
| Water | 3 ml |

**FORMULATION #4**

| | |
|---|---|
| Testosterone | 1,000 mg in 5 ml |
| Ethanol | 50 ml |
| Ethoxylated macadamia nut oil (16 ethoxylations/molecule) | 40 ml |
| Water | 5 ml |
| Y-Ling-Y-Lang oil | 15 drops |
| Rain water | 15 drops |

These formulations were found to rapidly increase the amount of testosterone in the blood of a subject that applied approximately 0.5ml of the product daily.

**PAIN RELIEF PRODUCTS: FORMULATION #1**

| | |
|---|---|
| Ethyl alcohol | 10.4 g |
| White willow bark extract | 10.4 g |
| Glucosamine HCL | 10 g |
| MSM | 10 g |
| Chrondroitan sulfate sodium | 10g |
| Marine collagen (1%) | 100 ml |
| *Aloe Vera* (whole leaf) concentrate | 100 ml |
| Ethoxylated macadamia nut oil (16 ethoxylations/molecule) | 300 ml |
| Y-Ling-Y-Lang oil | 28 drops |
| Coconut oil | 3 ml |
| Ibuprofen | 16 g |

**FORMULATION #2**

| | |
|---|---|
| Ibuprofen | 3 g |
| Methocarbanol | 3 g |
| Chlorzoxazone | 5 g |
| Ethanol | 75 ml |
| Macadamia nut oil (16 ethoxylations/molecule) | 75 ml |
| *Aloe Vera* (whole leaf) concentrate | 5 ml |
| Y-Ling-Y-Lang oil | 10 drops |

Compounds brought into solution with slight heat.

**FORMULATION #3**

| | |
|---|---|
| Acetyl salicylic acid | 22 g |
| Ibuprofen | 8.5 g |
| Ethanol (undenatured) | 500 ml |
| Ethoxylated macadamia nut oil (16 ethoxylations/molecule) | 400 ml |
| Distilled water | 100 ml |
| Peppermint oil | 20 drops |

**FORMULATION #4**

| | |
|---|---|
| Acetyl salicylic acid | 44 g |
| Undenatured ethanol | 800 ml |
| Ethoxylated macadamia nut oil (16 ethoxylations/molecule) | 200 ml |
| Distilled water | 40 drops |
| Y-ling Y-lang oil | 40 drops |
| Peppermint oil | 40 drops |

**FORMULATION #5**

| | |
|---|---|
| Acetyl salicylic acid | 44 g |
| Undenatured ethanol | 900 ml |
| Ethoxylated macadamia nut oil (16 ethoxylations/molecule) | 1000 ml |
| Distilled water | 100 ml |
| Y-ling y-lang oil | 40 drops |
| Peppermint oil | 40 drops |

**FORMULATION #6**

| | |
|---|---|
| Liquid aspirin | 44 g |
| Undenatured ethanol | 800 ml |
| Ethoxylated macadamia nut oil (16 ethoxylations/molecule) | 200 ml |
| Distilled water | 40 drops |
| Y-ling y-lang oil | 20 drops |
| Peppermint oil | 40 drops |

These formulations were found to reduce pain in several subjects within 5-20 minutes after application. Depending on the formulation, the period of pain reduction lasted from 45 minutes (*e.g.*, acetyl salicylic acid preparations) to several hours (e.g., ibuprofen containing preparations).

**SKIN CARE/ANTI-PSORIASIS/ANTI-ECZEMA/ WOUND HEALING PRODUCTS: FORMULATION #1**

| | |
|---|---|
| Dmae bitartrate | 22.5 g |
| Alpha lipoic acid | 5 g |
| Ethyl alcohol | 25 ml |
| Marine collagen (1%) | 25 ml |
| *Aloe Vera* | 25 ml |
| Macadamia nut oil (16 ethoxylations/molecule) | |

The Dmae bitartrate and alpha lipoic acid was brought into solution and filtered prior to mixture with the ethoxylated macadamia nut oil.

**FORMULATION #2**

| | |
|---|---|
| Ichtyocollagene (1%) | 500 ml |
| Distilled water | 248 ml |
| LKEKK **(SEQ. ID. No. 1)** | 1 vial (about 1 ml ~10µg) |
| Ethoxylated macadamia nut oil (16 ethoxylations/molecule) | 150 ml |
| Ethanol | 25 ml |
| Phenochem (*i.e.,* a mixture of Methyl Paraben, Ethyl Paraben, Propyl Paraben, Butyl Paraben, and Isobutyl Paraben) | 39 ml |

**FORMULATION #3**

| | |
|---|---|
| Distilled water | 100 ml |
| LKEKK **(SEQ. ID. No. 1)** | 5 bottles (∼50µg) |
| Ethoxylated macadamia nut oil (16 ethoxylations/molecule) | 40 ml |
| Ethanol | 5 ml |

These formulations were found to improve the healing of a wound (a laceration) and were found to reduce psoriasis and eczema in an afflicted subject.

**FORMULATION #4**

| | |
|---|---|
| Distilled Water with Sodium Bi Carbonate (pH 8.2-8.6) | 18 ml |
| Hepsyl | 5 g |
| Ethoxylated macadamia nut oil (16 ethoxylations/molecule) | 20 ml |
| Ethyl Alcohol Anhydrous | 20 ml |

This formulation reduces psoriasis and eczema in an afflicted subject.

**PRODUCTS THAT REDUCE THE APPEARANCE OF FINE LINES AND WRINKLES FORMULATION #1**

| | |
|---|---|
| Ichtyocollagene (1%) | 2,990 ml |
| Distilled water | 1,483 ml |
| Ethoxylated Macadamia nut oil (16 ethoxylations/molecule) | 922 ml |
| Ethanol | 150 ml |
| Matrixyl (8%) | 236 ml |
| Phenochem | 236 ml |
| Ethoxydiglycol | 33 ml |

**FORMULATION #2**

| | |
|---|---|
| Ichtyocollagene (6%) | 250 ml |
| Distilled water | 124 ml |
| Ethoxylated macadamia nut oil (16 ethoxylations/molecule) | 78 ml |
| Phenochem | 20 ml |
| Bio-ten (Atrium Biotechnologies, Inc., Quebec, Canada) | 1 ml |
| Ethanol | 10 ml |

**FORMULATION #3**

| | |
|---|---|
| Ichtyocollagene (1%) | 500 ml |
| Distilled water | 250 ml |
| Ethoxylated macadamia nut oil (16 ethoxylations/molecule) | 125 ml |
| Ethanol | 2 ml |
| Bio-ten | 3 ml |
| Phenochem | 40 ml |

**FORMULATION #4**

| | |
|---|---|
| Ichtyocollagene (1%) | 2,990 ml |
| Distilled water | 1,483 ml |
| Ethoxylated macadamia nut oil (16 ethoxylations/molecule) | 922 ml |
| Ethyl alcohol | 150 ml |
| Matrixyl | 236 ml |
| Phenochem | 236 ml |

**FORMULATION #5**

| | |
|---|---|
| Ichtyocollagene (1%) | 1,994 ml |
| Distilled water | 999 ml |
| Ethoxylated macadamia nut oil (16 ethoxylations/molecule) | 675 ml |
| Ethanol | 100 ml |
| Bioserum (Atrium Biotechnologies, Inc., Quebec, Canada) | 24 ml |
| Phenochem | 157 ml |

**FORMULATION #6**

| | |
|---|---|
| Ichtyocollagene (1%) | 500 ml |
| Distilled water | 250 ml |
| Ethoxylated macadamia nut oil (16 ethoxylations/molecule) | 168.75 ml |
| Ethanol | 25 ml |
| Bioserum | 10 ml |
| Phenochem | 43.75 ml |

**FORMULATION #7**

| | |
|---|---|
| Ichtyocollagene (1%) | 1,000 ml |
| Ethoxylated macadamia nut oil (16 ethoxylations/molecule) | 338 ml |
| Distilled water | 500 ml |
| Ethanol | 50 ml |
| Matrixyl | 76 ml |
| Phenochem | 76 ml |

**FORMULATION #8**

| | |
|---|---|
| Ichtyocollagene (1%) | 22.55 ml |
| Distilled Water | 11.7 ml |
| Ethoxylated macadamia nut oil (16 ethoxylations/molecule) | 7 ml |
| .Phenochem | 0.5 ml |
| Ethanol | 1.5 ml |
| Bio Serum | 1 ml |
| | |
| TOTAL | 44.25 ml |

**FORMULATION #9**

| | |
|---|---|
| Ichtyocollagene (1%) | 15.03 ml |
| Distilled Water | 7.8 ml |
| Ethoxylated macadamia nut oil (16 ethoxylations/molecule) | 4.67 ml |
| Phenochem | 0.333 ml |
| Ethanol | 1 ml |
| Bio Serum | 0.67 ml |
| | |
| TOTAL | 29.5 ml |

**FORMULATION #10**

| | |
|---|---|
| Ichtyocollagene (1%)(Marine Collagen, Sderma) | 150 ml |
| Distilled Water | 400 ml |
| Ethoxylated macadamia nut oil (16 ethoxylations/molecule) | 120 ml |
| Ethyl Alcohol (anhydrous) | 10 ml |
| Yling Ylang | 16 drops |
| Crodaderm B (Croda, Inc.) | 5.0 ml |
| Phenochem | 2.0 ml |
| Sepigel | 15 g |

Formulation #10 is mixed in the order listed, and heated to 80°F.

These formulations were found to reduce the appearance of fine lines and wrinkles in subjects that applied the formulations daily for thirty days. It should be noted that Bioserum, which is obtainable from Atrium Biosciences, Ontario Canada, may contain one or more of the following: placental protein, amniotic fluid, calf skin extract, and serum protein. Also, phenochem may contain one or more of the following: Methyl Paraben, Ethyl Paraben, Propyl Paraben, Butyl Paraben, and Isobutyl Paraben, and sodium methylparaban imidizolidinyl urea. Additional components that may be included in some formulations of products that reduce the appearance of fine lines and wrinkles include: igepal cephene distilled, synasol, ethoxylated glycerides, trisodium EDTA, potassium sorbate, citric acid, ascorbic acid, and distilled water. For example, one formulation contains: Collagen (Marine), Distilled Water, Igepal Cephene Distilled, Methyl Paraben, Ethyl Paraben, Propyl Paraben, Butyl Paraben, Isobutyl Paraben, Synasol, Serum Protein, Purified Water, Amniotic Fluid. Placental Protein. Calfskin Extract, Hydrolyzed Collagen Sodium Methylparaben Imidazolidinyl Urea. Ethoxylated Glycerides, Trisodium EDTA, Potassium Sorbate, Citric Acid, and Ascorbic Acid.

**SPOT FAT REDUCERS FORMULATION #1**

| | |
|---|---|
| Epigallocatechin Gallate (ECGC) | 40 g |
| Ethyl Alcohol | 100 ml |
| Distilled Water | 100 ml |
| Ethoxylated macadamia nut oil (16 ethoxylations/molecule) | 60 ml |
| Lipase | 1 ml |

**FORMULATION #2**

| | |
|---|---|
| Epigallocatechin Gallate (ECGC)(DSM, Netherlands) | 40 g |
| Ethyl Alcohol | 100 ml |
| Distilled Water | 100 ml |
| Ethoxylated macadamia nut oil (16 ethoxylations/molecule) | 60 ml |
| Lipase | 1 ml |
| Caffeine | 2.0 g |

These formulations were found to reduce fat when applied to the body in individual. Polyphenols other than ECGC, such as analogs of green tea extract, are suitable in the above formulations and can be substituted for or used in combination with ECGC in the above forumations. The following example describes experiments that employed two different skin cell model systems to evaluate the ability of a transdermal delivery composition containing collagen to transport collagen to skin cells.

### EXAMPLE 5

In this example, it is shown that a transdermal delivery composition of the invention comprising marine type 1 collagen or native collagen efficiently transported the delivered agent to skin cells. Two different *in vitro* skin cell model systems were used, human cadaver skin and a cellulose acetate skin cell model system. Based on the physiology of the skin, three possible pathways exist for passive transport of molecules through the skin to the vascular network: (1) intercellular diffusion through the lipid lamellae; (2) transcellular diffusion through both the keratinocytes and lipid lamellae; and (3) diffusion through appendages (hair follicles and sweat ducts). The cellulose acetate skin model evaluates the ability of the delivered agent to transport using the first two pathways and the human cadaver skin evaluates the ability to use all three pathways.

In brief, the transdermal delivery composition comprising collagen was applied to the cellulose acetate and the human cadaver skin in a diffusion chamber and the results were recorded after 10 minutes, 30 minutes and one hour. The diffused material was analyzed by a spectrophotometer (Hitachi U2000 multiscan spectrophotometer). A portion of the diffused material was also separated on a gel by electrophoresis and the collagen was stained using a collagen-specific dye. A portion of the diffused material was also immunoprecipitated using polyclonal antibodies specific for collagens types 1-7 and the immunoprecipitates were analyzed by immunodiffusion.

The table below provides the collagen concentration in the various samples of transdermal delivery compositions tested. The protein concentration was determined using a micro-protein assay (Bio-Rad).

**TABLE 20**

| Sample number | Protein Concentrations | |
|---|---|---|
| | Native type 1 Collagen | Marine type 1 collagen |
| Sample 1 | 0.40 mg/ml | 1.14 mg /ml |
| Sample 2 | 0.44 mg/ml | 1.09 mg /ml |
| Sample 3 | 0.42 mg/ml | 1.14 mg /ml |
| Average | 0.42 | 1.12 |
| Standard error | 0.011 | 0.017 |
| Variance | 0.0004 | 0.0008 |
| Standard deviation | 0.02 | 0.03 |

### Penetration analysis

The transdermal delivery composition containing either marine collagen or native collagen was applied to the human cadaver skin and the cellulose acetate skin model systems. The penetration studies were performed in a diffusion chamber and the results were recorded at 10 minutes, 30 minutes and an hour later. Sections of skin or cellulose acetate were stained with a collagen specific dye and a light microscope was used to visualize the transported collagen. **TABLE 21** provides the results of these experiments. Note, that the native collagen appeared to penetrate the skin in less time than the marine collagen. This may be due to the differing concentrations of collagen used in the transdermal delivery compositions (*i.e.,* the concentration of the native collagen was 0.40 mg/ml and the concentration of the marine collagen was 1.14 mg/ml). Nevertheless, by one hour, almost all of both types of collagen had penetrated the skin in the model systems employed.

**TABLE 21**

| Product Hydroderm | Percent Penetration as per time interval | | | |
|---|---|---|---|---|
| Marine Collagen Vial A | 10 minutes | 20 minutes | 30 minutes | 60 minutes |
| Sample A1 | 40% | 60% | 75% | 95% |
| Sample A2 | 40% | 60% | 75% | 95% |
| Sample A3 | 40% | 60% | 75% | 95% |

| Marine Collagen Vial B | | | | |
|---|---|---|---|---|
| Sample B1 | 40% | 60% | 75% | 95% |
| Sample B1 | 40% | 60% | 75% | 95% |
| Sample B1 | 40% | 60% | 75% | 95% |

| Marine collagen Vial C | | | | |
|---|---|---|---|---|
| Sample C1 | 40% | 60% | 75% | 95% |
| Sample C1 | 40% | 60% | 75% | 95% |
| Sample C1 | 40% | 60% | 75% | 95% |

| Native Collagen | | | | |
|---|---|---|---|---|
| Sample 1 | 80% | 95% | | |
| Sample 2 | 80% | 95% | | |
| Sample 3 | 80% | 95% | | |

When similar concentrations of native collagen and marine collagen were used in a transdermal delivery composition, the native collagen and the marine collagen penetrated the upper three layers of the epidermis in approximately one hour. The marine collagen and the native collagen were localized in the upper three layers of the human cadaver epidermis using a collagen specific dye. A similar distribution of the collagen was confirmed by the cellulose acetate skin model. *See* **TABLES 22** and **23.**

**TABLE 22**

| PENETRATION IN THE LAYERS OF THE HUMAN SKIN EPIDERMIS | | | | | |
|---|---|---|---|---|---|
| | Penetration of Epidermis layers of the Skin (Human Skin diffusion chamber study) | | | | |
| Marine collagen Vial A | *Stratum Corneum* | *Stratum lucidum* | *Stratum Granulosum* | *Stratum Spinosum* | *Stratum Basale* |
| Sample A1 | √ | √ | √ | - | - |
| Sample A2 | √ | √ | √ | - | - |
| Sample A3 | √ | √ | √ | - | - |

| Marine collagen Vial B | | | | | |
|---|---|---|---|---|---|
| Sample B1 | √ | √ | √ | - | - |
| Sample B1 | √ | √ | √ | - | - |
| Sample B1 | √ | √ | √ | - | - |

| Marine collagen Vial C | | | | | |
|---|---|---|---|---|---|
| Sample C1 | √ | √ | √ | - | - |
| Sample C1 | √ | √ | √ | - | - |
| Sample C1 | √ | √ | √ | - | - |

| Native collagen | | | | | |
|---|---|---|---|---|---|
| Sample 1 | √ | √ | √ | - | - |
| Sample 2 | √ | √ | √ | - | - |
| Sample 3 | √ | √ | √ | - | - |

Note: (√) indicates the presence of the product in the above layers of the epidermis as determined by collagen specific staining observed by light microscopy after one hour of product application. (-) indicates absence of products in these layers of the epidermis.

**TABLE 23**

| | Penetration Hydroderm in Epidermis layers of the Skin (Cellulose Acetate model skin diffusion chamber study) | | | | |
|---|---|---|---|---|---|
| Marine collagen Vial A | *Stratum Corneum* | *Stratum lucidum* | *Stratum Granulosum* | *Stratum Spinosum* | *Stratum Basale* |
| Sample A1 | √ | √ | √ | - | - |
| Sample A2 | √ | √ | √ | - | - |
| Sample A3 | √ | √ | √ | - | - |

| Marine collagen Vial B | | | | | |
|---|---|---|---|---|---|
| Sample B1 | √ | √ | √ | - | - |
| Sample B1 | √ | √ | √ | - | - |
| Sample B1 | √ | √ | √ | - | - |

| Marine collagen Vial C | | | | | |
|---|---|---|---|---|---|
| Sample C1 | √ | √ | √ | - | - |
| Sample C1 | √ | √ | √ | - | - |
| Sample C1 | √ | √ | √ | - | - |

| Native Collagen | | | | | |
|---|---|---|---|---|---|
| Sample 1 | √ | √ | √ | - | - |
| Sample 2 | √ | √ | √ | - | - |
| Sample 3 | √ | √ | √ | - | - |

Note: z) indicates the presence of the product in the above layers of the epidermis as determined by collagen specific staining observed by light microscopy after one hour of product application. (-) indicates absence of products in these layers of the epidermis.

### Spectrophotometric Analysis

Spectrophotometric analysis of the diffused material revealed that the transdermal delivery composition enabled significant transport of both types of collagens. *See* **TABLE 24.**

**TABLE 24**

| Sample number | Spectral Absorbance at wavelength 280nm | |
|---|---|---|
| | Native type 1 collagen | Marine type 1 collagen |
| Sample 1 | 2.35 | 2.832 |
| Sample 2 | 2.766 | 2.772 |
| Sample 3 | 2.751 | 2.683 |
| Average | 2.622 | 2.762 |
| Standard error | 0.136 | 0.043 |
| Variance | 0.0557 | 0.0056 |
| Standard deviation | 0.24 | 0.07 |

### Electrophoresis Analysis

A portion of the diffused material was then separated by electrophoresis and visualized by staining with a collagen-specific dye. The penetrated marine collagen remained intact during and after the analysis because the labeled marine collagen detected in the diffused material was observed to have the same molecular weight as marine collagen that had not undergone the analysis (control sample). The results showed that the marine collagen prior to the penetration study and after the penetration study maintained its molecular structure around 500 kilodaltons (KD). The native collagen also maintained a molecular weight around 500KD before and after penetration of the epidermis, demonstrating that the native collagen that was delivered by the transdermal delivery composition, like the marine collagen, remained intact into the epidermis.

### Immunoprecipitation Analysis

When the transdermal delivery composition containing marine collagen was immunoprecipitated using polyclonal antibodies specific for collagens types 1-7 before and after the penetration study, more evidence that the marine collagen remained in tact after the transdermal delivery was obtained. Immuno-diffusion studies verified that the marine collagen prior to penetration of the skin and post penetration of skin consisted mainly of type I collagen. This further confirmed that the collagen remained intact post penetration.

The penetration study described above provided strong evidence that the transdermal delivery compositions described herein are effective at transporting high molecular weight molecules to skin cells. It was found, for example, that marine collagen type 1 (-500 kD) effectively penetrated the upper 3 layers of the epidermis and remained intact within an hour. These findings were supported by histology, spectrophotometric analysis, electrophoretic separation analysis, immunoprecipitation analysis, and immuno-diffusion analysis. The following example describes a clinical study that was performed, which verified that the transdermal delivery compositions described herein effectively reduce wrinkles and improve skin tone in humans in need thereof.

### EXAMPLE 6

A clinical study was performed to evaluate the ability of a transdermal delivery composition containing collagen, prepared as described herein, to reduce wrinkles and fine lines and otherwise restore skin tone to subjects in need thereof. The medial half of the facial region including the neck and the upper chest areas were assigned as the regions under investigation. During a subject's routine application of the product, three times a day, digital pictures were taken at days 0, 3, 7, 14 and 21 of the regions under investigation of the face including the symmetrical region of the face where the product was not applied. Micrometer measurements of the wrinkles were then made from the digital pictures and also from the facial areas under investigation.

Subjects invited to participate in the study had facial wrinkles and were 25 years or older. Non-facial wrinkle individuals were also invited and served as the control group. The source of subjects for the study was randomly selected from the ethnically diverse population group ages ranging from 25 years to 88 years old.

**Table 25**

| *Description of the subjects participating in the study* | | | | |
|---|---|---|---|---|
| Identification Number | Gender | Ethnicity | Age | General Description |
| F101601 | Female | Hispanic American | 88 | Distinct facial wrinkles |
| F101602 | Female | Hispanic American | 67 | Distinct facial wrinkles |
| F101603 | Female | Hispanic American | 25 | Distinct facial wrinkles around the eyes |
| F101604 | Female | Caucasian | 28 | Distinct facial wrinkles around the eye region |
| M101605 | Male | Asian | 40 | Distinct facial wrinkles around the eye region |

Subjects that signed the study consent form received 30 mls of a transdermal delivery composition comprising marine collagen. Micrometer measurements of the wrinkles were performed using a 10X magnification objective eye piece. The measurements were recorded and tabulated together with the digital photographs before and after application of the product. The wrinkle measurements were determined within the 3-week duration of the study. The tabulated results provided in **TABLE 26,** which indicates the general observations by subjects utilizing the product, and **TABLE 27,** which shows the wrinkle measurements. **TABLE 28** shows the average percent of wrinkle reduction data generated after 21 days of application of the transdermal delivery composition comprising collagen.

**TABLE 26**

| **Identification Number** | **Days of product application on one half of the face including the upper chest and neck regions** | | | |
|---|---|---|---|---|
| | **Day 3** | **Day 7** | **Day 14** | **Day 21** |
| F101601 | Skin felt soft, and clear, when compared to the other half without product application, slight burning sensation for 3-5 minutes upon product application. | The right half of the face cleared up and felt smooth, the slight burning sensation was still present for 3-5 minutes. | The right half of the face cleared up and felt smooth, the slight burning sensation no longer present. | The right half of the face cleared up and felt smooth, the slight burning sensation no longer present. |
| F101602 | Skin felt soft, and clear, when compared to the other half without product application, slight burning sensation for 3-5 minutes upon product application. | The right half of the face cleared up and felt smooth, the slight burning sensation was still present for 3-5 minutes. | The right half of the face cleared up and felt smooth, the slight burning sensation no longer present. | The right half of the face cleared up and felt smooth, the slight burning sensation no longer present. |
| F101603 | Skin felt soft, and clear, when compared to the other half without product application, slight burning sensation for 3-5 minutes upon product application. | The right half of the face cleared up and felt smooth, the slight burning sensation was still present for 3-5 minutes. | The right half of the face cleared up and felt smooth, the slight burning sensation no longer present. | The right half of the face cleared up and felt smooth, the slight burning sensation no longer present.. |
| F101604 | Skin felt soft, and clear, when compared to the other half without product application, slight burning sensation for 3-5 minutes upon product application. | The skin felt smooth and very soft in the facial region where product was applied. | Developed rashes in the neck region, stopped using product. | The rashes cleared up, and the skin had normal appearance as the other half in which the product was not applied. |
| M101605 | Skin felt soft, and clear, when compared to the other half without product application, slight burning sensation for 3-5 minutes upon product application. | The right half of the face cleared up and felt smooth, the slight burning sensation was still present for 3-5 minutes. | The right half of the face cleared up and felt smooth, the slight burning sensation still present for 3-5 minutes. | The right half of the face cleared up and felt smooth, the slight burning sensation still present for 3-5 minutes. |

**TABLE 27**

| **Subject's Identification Number** | **Average wrinkle measurements with product application on one half of the face including the upper chest and neck areas in µm** | | | | | | |
|---|---|---|---|---|---|---|---|
| | **Regions of the face** | **Day 0** | **Day 3** | **Day 5** | **Day 7** | **Day 14** | **Day 21** |
| F101601 | Around eyes | 6 µm | 6 µm | 6 µm | 5 µm | 4.5 µm | 4.5 µm |
| | Temporal cheek | 7 µm | 7 µm | 7 µm | 7 µm | 6 µm | 5.5 µm |
| | Chin | 7.5 µm | 7.5 µm | 7.5 µm | 7.5 µm | 7.0 µm | 6.5 µm |
| | Around mouth | 6.5 µm | 6.5 µm | 6.5 µm | 6.5 µm | 6.0 µm | 5.5 µm |
| F101602 | Around eyes | 3.5 µm | 3.5 µm | 3.5 µm | 3.5 µm | 3.5 µm | 3.2 µm |
| | Temporal cheek | 4.1 µm | 4.1 µm | 4.1 µm | 4.1 µm | 3.9 µm | 3.5 µm |
| | Chin | 2.5 µm | 2.5 µm | 2.5 µm | 2.5 µm | 2.0 µm | 2.0 µm |
| | Around mouth | 2.0 µm | 2.0 µm | 2.0 µm | 2.0 µm | 2.0 µm | 2.0 µm |
| F101603 | Around eyes | 1.5 µm | 1.5 µm | 1.5 µm | 1.5 µm | 1.5 µm | 1.2 µm |
| | Temporal cheek | 1.0 µm | 1.0 µm | 1.0 µm | 1.0 µm | 1.0 µm | 1.0 µm |
| | Chin | 0.9 µm | 0.9 µm | 0.9 µm | 0.9 µm | 0.9 µm | 0.85 µm |
| | Around mouth | 0.5 µm | 0.5 µm | 0.5 µm | 0.5 µm | 0.5 µm | 0.45 µm |
| F101604 | Around eyes | 0.2 µm | 0.2 µm | 0.2 µm | 0.2 µm | 0.2 µm | ** |
| | Temporal cheek | 1.5 µm | 1.5 µm | 1.5 µm | 1.5 µm | 1.5 µm | ** |
| | Chin | 1.0 µm | 1.0 µm | 1.0 µm | 1.0 µm | 1.0 µm | ** |
| | Around mouth | 0.5 µm | 0.5 µm | 0.5 µm | 0.5 µm | 0.5 µm | ** |
| M101605 | Around eyes | 1.5 µm | 1.5 µm | 1.5 µm | 1.5 µm | 1.5 µm | 1.0 µm |
| | Temporal cheek | 0.5 µm | 0.5 µm | 0.5 µm | 0.5 µm | 0.5 µm | 0.3 µm |
| | Chin | 1.0 µm | 1.0 µm | 1.0 µm | 1.0 µm | 1.0 µm | 0.9 µm |
| | Around mouth | 1.5 µm | 1.5 µm | 1.5 µm | 1.5 µm | 1.5 µm | 1.2 µm |

| | | | | | | | |
|---|---|---|---|---|---|---|---|
| Note ** Indicates the subject stopped using the product. | | | | | | | |

**TABLE 28**

| **Subject's Identification Number** | **The percent reduction of wrinkle measurement on the regions of the face at day 21 of Hydroderm product application** | | | |
|---|---|---|---|---|
| | **Around eyes** | **Temporal cheek** | **Chin** | **Around mouth** |
| F101601 | 25% | 21.4% | 13.3% | 15.4% |
| F101602 | 8.6% | 14.6% | 20.0% | 0.0% |
| F101603 | 20.0% | 0.0% | 5.6% | 10.0% |
| F101604 | 0.0% | 0.0% | 0.0% | 0.0% |
| M101605 | 33.3% | 40.0% | 10% | 20.0% |
| | | | | |
| Average % | 17.42% | 15.20% | 9.78% | 9.08% |
| Overall effectiveness | On the entire facial region where the product was applied. | | | 10.29% |

The data generated from this study indicates that the overall effectiveness of transdermal delivery composition comprising marine collagen as a wrinkle reducer is 10.29% when applied twice daily for 21 days. As indicated by **Table 28,** the percent reduction of the wrinkles varies with the various areas of the face where it is applied, with 17.4% reduction around the eye regions and 15.20% at the temporal cheeks at the high end and around 9% at the chin and mouth regions. The next example sets forth experiments that demonstrate that transdermal delivery compositions containing ethoxylated oils of less than 20 ethoxylations/molecule transfer a delivered agent to the skin more effectively than transdermal delivery compositions containing ethoxylated oils of 20 or more ethoxylations/molecule.

### EXAMPLE 7

Several transdermal delivery composition formulations containing collagen (1.2mg/ml) and an ethoxylated oil having different amounts of ethoxylations/molecule are prepared. Formulations containing ethoxylated oil of either 12, 16, 18, 20, 24, and 30 ethoxylations/molecule, water, and marine collagen (1.2mg/ml) are made. Approximately 0.5ml of each of these formulations are applied to human cadaver skin in a diffusion chamber and the penetration of collagen is monitored over time *(e.g.,* 10 minutes, 30 minutes, 45 minutes and one hour). Sections of the skin are taken, stained with a collagen specific dye, and the stained sections are analyzed under a light microscope.

A greater amount of collagen-specific staining will be seen in stained skin sections collected at the various time points with formulations containing less than 20 ethoxylations/molecule than with formulations containing 20 or more ethoxylations/molecule. Formulations containing less than 20 ethoxylations/molecule will also penetrate the skin faster than formulations containing 20 or more ethoxylations/molecule.

In a second set of experiments, the collagen that has penetrated the skin at the various time points above is collected from the diffusion chamber and analyzed in a spectrophotometer. As above, a greater amount of collagen will be detected in samples collected at the various time points with formulations containing less than 20 ethoxylations/molecule than formulations containing 20 or more ethoxylations/molecule. Formulations containing less than 20 ethoxylations/molecule will also be observed to penetrate the skin faster than formulations containing 20 or more ethoxylations/molecule. The next example sets forth experiments that demonstrate that transdermal delivery compositions containing ethoxylated fatty acids having 10-19 ethoxylations/molecule transfer a delivered agent to the skin as effectively as transdermal delivery compositions containing ethoxylated oils having 10-19 ethoxylations/molecule.

### EXAMPLE 8

A transdermal delivery composition containing collagen (1.2mg/ml) and an ethoxylated fatty moiety having 16 ethoxylations/molecule, water and marine collagen is made. Several transdermal delivery compositions containing ethoxylated fatty moieties and having 16 ethoxylations/molecule, water, and marine collagen are made. Approximately 0.5ml of each of these formulations are applied to human cadaver skin in a diffusion chamber and the penetration of collagen is monitored over time *(e.g.,* 10 minutes, 30 minutes, 45 minutes and one hour). Sections of the skin are taken, stained with a collagen specific dye, and the stained sections are analyzed under a light microscope.

The same amount of collagen-specific staining will be seen in stained skin sections collected at the various time points with formulations containing ethoxylated fatty moieties as compared to formulations containing ethoxylated oils. Formulations containing ethoxylated fatty moieties will also penetrate the skin at approximately the same rate as compared to formulations containing ethoxylated oils.

In a second set of experiments, the collagen that has penetrated the skin at the various time points above is collected from the diffusion chamber and analyzed in a spectrophotometer. As above, approximately the same amount of collagen will be detected in samples collected at the various time points with formulations containing ethoxylated fatty moieties as compared to formulations containing ethoxylated oils. Formulations containing ethoxylated fatty moieties will also be observed to penetrate the skin at approximately the same rate as formulations containing ethoxylated oils.

### EXAMPLE 9

A transdermal delivery composition containing collagen (1.2mg/ml) and an ethoxylated oil having 16 ethoxylations/molecule, water and marine collagen is made. A portion of the composition is transferred to a cartridge adapted for the exemplary transdermal delivery device described herein. The transdermal delivery device is preset to load approximately 0.5ml of the formulation. Approximately 0.5ml of the formulation is applied to human cadaver skin, either manually, or using the transdermal delivery device, in a diffusion chamber and the penetration of collagen is monitored over time *(e.g.,* 10 minutes, 30 minutes, 45 minutes and one hour). Sections of the skin are taken, stained with a collagen specific dye, and the stained sections are analyzed under a light microscope. Several samples are prepared and treated at the same time.

The amount of collagen-specific staining seen in stained skin sections collected is substantially more consistent in the samples in which the formulation is administered via the transdermal delivery device than in the samples in which the formulation is delivered manually.

In a second set of experiments, the collagen that has penetrated the skin at the various time points above is collected from the diffusion chamber and analyzed in a spectrophotometer. As above, the amount of collagen detected in samples collected from the various samples in which the formulation is delivered via the transdermal delivery device shows considerably less variation than the amounts of collagen calculated from samples in which the formulation was applied manually.

Although the invention has been described with reference to embodiments and examples, it should be understood that various modifications can be made without departing from the spirit of the invention. Accordingly, the invention is limited only by the following items.

The invention is further described by way of the following items:
1. A transdermal delivery composition comprising a liposphere, wherein said liposphere comprises an ethoxylated fatty moiety or lipid moiety and a delivered agent, wherein the amount of ethoxylation of said fatty moiety or lipid moiety is 10, 11, 12, 13, 14, 15, 16, 17, 18, or 19 ethoxylations per molecule, and wherein said fatty moiety or lipid moiety comprises 10 carbon residues.
2. The transdermal delivery composition of Item 1 further comprising water.
3. The transdermal delivery composition of Item 1 further comprising alcohol.
4. The transdermal delivery composition of Item 1, wherein said fatty moiety or lipid moiety comprises at least 10 carbon residues but less than or equal to 30 carbon residues.
5. The transdermal delivery composition of Item 1, wherein the amount of ethoxylation is the same as the number of carbons residues in the fatty moiety or lipid moeity.
6. The transdermal delivery composition of Item 1, wherein the fatty moiety or lipid moiety is 10 carbon residues in length.
7. The transdermal delivery composition of Item 1, wherein the fatty moiety chain length is 12 carbon residues in length.
8. The transdermal delivery composition of Item 1, wherein the fatty moiety chain length is 14 carbon residues in length.
9. The transdermal delivery composition of Item 1, wherein the fatty moiety chain length is 16 carbon residues in length.
10. The transdermal delivery composition of Item 1, wherein the fatty moiety chain length is 18 carbon residues in length.
11. The transdermal delivery composition of Item 1, wherein the fatty moiety chain length is 20 carbon residues in length.
12. The transdermal delivery composition of Item 1, wherein the fatty moiety chain length is 22 carbon residues in length.
13. The transdermal delivery composition of Item 1, wherein the fatty moiety chain length is 24 carbon residues in length.
14. The transdermal delivery composition of Item 1, wherein the fatty moiety is a fatty acid.
15. The transdermal delivery composition of Item 14, wherein the fatty acid is unsaturated.
16. The transdermal delivery composition of Item 14, wherein the fatty acid is polyunsaturated.
17. The transdermal delivery composition of Item 14, wherein the fatty acid is palmitoleic acid (C16:1, delta 9).
18. The transdermal delivery composition of Item 14, wherein the fatty acid is selected from the group consisting of C16:1, delta 1; C16:1, delta 2; C16:1, delta 3; C16:1, delta 4; C16:1, delta 5; C16:1, delta 6; C16:1, delta 7; C16:1, delta 8; C16:1, delta 9; C16:1, delta 10; C16:1, delta 11; C16:1, delta 12; C16:1, delta 13; and C16:1, delta 14.
19. The transdermal delivery composition of Item 1, wherein said fatty moiety is a fatty amine.
20. The transdermal delivery composition of Item 1, wherein said lipid moiety is a sphingolipid.
21. The transdermal delivery composition of Item 1, wherein said lipid moiety is a glycolipid.
22. The transdermal delivery composition of Item 1, wherein said lipid moiety is a glycosphingolipid.
23. The transdermal delivery composition of Item 1, wherein said liposphere comprises a homogeneous mixture of an ethoxylated fatty moiety.
24. The transdermal delivery composition of Item 1, wherein said liposphere comprises a heterogeneous mixture of ethoxylated fatty acids.
25. The transdermal delivery composition of Item 1, wherein the delivered agent is a hormone.
26. The transdermal delivery composition of Item 25, wherein said hormone is a polypeptide.
27. The transdermal delivery composition of Item 26, wherein the polypeptide is selected from the group consisting of oxytocin, vasopressin, melanocyte-stimulating hormone, corticortropin, lipotropin, thyrotropin, growth hormone, prolactin, luteinizing hormone, human chorionic gonadotropin, follicle stimulating hormone, corticotropin-releasing factor, gonadotropin-releasing factor, prolactin-releasing factor, prolactin-inhibiting factor, growth-hormone releasing factor, somatostatin, thyrotropin-releasing factor, calcitonin, calcitonin gene-related peptide, parathyroid hormone, glucagon-like peptide 1, glucose-dependent insulinotropic polypeptide, gastrin, secretin, cholecystokinin, motilin, vasoactive intestinal peptide, substance P, pancreatic polypeptide, peptide tyrosine tyrosine, neuropeptide tyrosine, amphiregulin, insulin, glucagon, placental lactogen, relaxin, , angiotensin II, atrial natriuretic peptide, and melatonin.
28. The transdermal delivery composition of Item 25, wherein the hormone is a non-peptide hormone.
29. The transdermal delivery composition of Item 25, wherein the hormone is selected from the group consisting of thyroxine, triiodothyronine, estradiol, estrone, progesterone, testosterone, cortisol, corticosterone, aldosterone, epinephrine, norepinepherine, and calctriol.
30. The transdermal delivery composition of Item 1, wherein said delivered agent is collagen.
31. The transdermal delivery composition of Item 1, wherein said delivered agent is an anesthetic.
32. The transdermal delivery composition of Item 31, wherein said anesthetic is selected from the group consisting of articaine, procaine, tetracaine, chloroprocaine and benzocaine, novocain, mepivicaine, bupivicaine, benzocaine, and lidocaine.
33. The transdermal delivery composition of Item 1, wherein said delivered agent is an analgesic.
34. The transdermal delivery composition of Item 33, wherein said delivered agent is selected from the group consisting of tramadol hydrochloride, fentanyl, metamizole, morphine sulphate, ketorolac tromethamine, hydrocodone, oxycodone, morprhine, loxoprofen, Capsaicin, and Boswellin.
35. The transdermal delivery composition of Item 1, wherein said delivered agent is an NSAID.
36. The transdermal delivery composition of Item 35, wherein said non-steroidal anti inflammatory drug (NSAID) is selected from the group consisting of ibuprofen (2-(isobutylphenyl)-propionic acid); methotrexate (N-[4-(2, 4 diamino 6 - pteridinyl - methyl] methylamino] benzoyl)-L-glutamic acid); aspirin (acetylsalicylic acid); salicylic acid; diphenhydramine (2-(diphenylmethoxy)-NN-dimethylethylamine hydrochloride); naproxen (2-naphthaleneacetic acid, 6-methoxy-9-methyl-, sodium salt, (-)); phenylbutazone (4-butyl-1,2-diphenyl-3,5-pyrazolidinedione); sulindac-(2)-5-fuoro-2-methyl-1-[[p-(methylsulfinyl)phenyl]methylene-]-1H-indene-3-acetic acid; diflunisal (2',4', -difluoro-4-hydroxy-3-biphenylcarboxylic acid; piroxicam (4-hydroxy-2-methyl-N-2-pyridinyl-2H-1, 2-benzothiazine-2-carboxamide 1,1-dioxide, an oxicam; indomethacin (1-(4-chlorobenzoyl)-5-methoxy-2-methyl-H-indole-3-acetic acid); meclofenamate sodium (N-(2, 6-dichloro-m-tolyl) anthranilic acid, sodium salt, monohydrate); ketoprofen (2- (3-benzoylphenyl)-propionic acid; tolmetin sodium (sodium 1-methyl-5-(4-methylbenzoyl-1H-pyrrole-2-acetate dihydrate); diclofenac sodium (2-[(2,6-dichlorophenyl)amino] benzeneatic acid, monosodium salt); hydroxychloroquine sulphate (2-{[4-[(7-chloro-4-quinolyl) amino]pentyl]ethylamino}ethanol sulfate (1:1); penicillamine (3-mercapto-D-valine); flurbiprofen ([1,1-biphenyl]-4-acetic acid, 2-fluoro-alphamethyl-, (+-.)); cetodolac (1-8- diethyl-13,4,9, tetra hydropyrano-[3-4-13] indole-1-acetic acid; mefenamic acid (N-(2,3-xylyl)anthranilic acid; and diphenhydramine hydrochloride (2-diphenyl methoxy-N, N-di-methylethamine hydrochloride).
37. The transdermal delivery composition of Item 1, wherein the delivered agent is an anti-inflammatory compound selected from the group consisting of hydrocortisone, prednisolone, triamcinolone, cortisone and piroxicam.
38. The transdermal delivery composition of Item 1, wherein the delivered agent is an anti-infective compound selected from the group consisting of amoxicillin, clavulanate potassium, itraconazole, acyclovir, fluconazole, terbinafine hydrochloride, erythromycin ethylsuccinate, acetyl sulfisoxazole, penicillin V, cephalexin, erythromycin, azithromycin, tetracycline, ciproflaxin, gentamycin, sulfathiazole, nitrofurantoin, norfloxacin, flumequine, and ibafloxacin, metronidazole, nystatin, lamivudine, indinavir sulfate, and stavudine.
39. The transdermal delivery composition of Item 1, wherein the delivered agent is metformin hydrochloride or acarbose.
40. The transdermal delivery composition of Item 1, wherein the delivered agent is an immunogen.
41. The transdermal delivery composition of Item 40, wherein the immunogen comprises a polynucleotide.
42. The transdermal delivery composition of Item 1 wherein the delivered agent is an immune response modifier.
43. The transdermal delivery composition of Item 42, wherein the immune response modifier is selected from the group consisting of purine derivatives, adenine derivatives, cyclosporine, and CpGs.
44. The transdermal delivery composition of Item 1, wherein the delivered agent is an enzyme inhibitor selected from the group consisting of zileuton, captopril, and lisinopril.
45. The transdermal delivery composition of Item 1, wherein the delivered agent is selected from the group consisting of ergotamine, melatonin, sumatriptan, zolmitriptan, and rizatriptan.
46. The transdermal delivery composition of Item 1, wherein the delivered agent is selected from the group consisting of zolpidem, zolpidem tartrate, triazolam, and hycosine butylbromide.
47. The transdermal delivery composition of Item 1, wherein the delivered agent is selected from the group consisting of iohexol, technetium, TC99M, sestamibi, iomeprol, gadodiamide, ioversol, and iopromide.
48. The transdermal delivery composition of Item 1, wherein the delivered agent is selected from the group consisting of alsactide, americium, betazole, histamine, mannitol, metyrapone, petagastrin, phentolamine, radioactive B.sub.12, gadodiamide, gadopentetic acid, gadoteridol, perflubron, cyclosporine, sildenafil citrate, paclitaxel, ritonavir, and saquinavir.
49. A transdermal delivery composition comprising
   a transdermal delivery enhancer comprising a multifunctional backbone, wherein said multifunctional backbone comprises at least two reactive groups (R), wherein at least one reactive group is substituted with a fatty moiety, wherein at least one reactive group is substituted with a polyethoxy moiety, wherein R is selected from the group consisting of -OH, COOH, SH, and NH₂, wherein said fatty moiety is selected from the group consisting of a fatty acid, a fatty alcohol, a fatty amine, and a modified fatty acid; and
   a delivered agent.
50. The transdermal delivery composition of Item 49, wherein said (R) groups on said multifunctional backbone are homogeneous.
51. The transdermal delivery composition of Item 49, wherein said (R) groups on said multifunctional backbone are heterogeneous.
52. The transdermal delivery composition of Item 49, wherein said penetration enhancer comprises at least 10 ethoxylations per molecule but less than or equal to 19 ethoxylations per molecule.
53. The transdermal delivery composition of Item 49, wherein said multifunctional backbone is a tri-alcohol.
54. The transdermal delivery composition of Item 53, wherein said fatty moiety is a fatty acid, and wherein said fatty acid comprises at least 10 carbon residues.
55. The transdermal delivery composition of Item 49, wherein said multifunctional backbone is a triacid.
56. The transdermal delivery composition of Item 55, wherein at least one of said (R) groups on said triacid is substituted with a fatty alcohol.
57. The transdermal delivery composition of Item 55, wherein said triacid has a benzene ring.
58. The transdermal delivery composition of Item 57, wherein said triacid is selected from the group consisting of hemi-mellitic acid, trimellitic acid, trimesic acid, and ntrilotriacetic acid.
59. The transdermal delivery composition of Item 57, wherein said triacid comprises a toluene group.
60. The transdermal delivery composition of Item 57, wherein said triacid comprises a naphthalene group.
61. The transdermal delivery composition of Item 49, wherein said multifunctional backbone is an amino acid.
62. The transdermal delivery composition of Item 61, wherein said amino acid is selected from the group consisting of glutamic acid, aspartic acid, cysteine, glutamine, serine, threonine, tyrosine, lysine and aminomalonic acid.
63. The transdermal delivery composition of Item 49, wherein said multifunctional backbone is triethanolamine.
64. The transdermal delivery composition of Item 49, wherein said multifunctional backbone is diethanolamine.
65. The transdermal delivery composition of Item 49, wherein said multifunctional backbone is dimethylolurea.
66. The transdermal delivery composition of Item 49, wherein said multifunctional backbone is glucosamine.
67. The transdermal delivery composition of Item 49, wherein said multifunctional backbone is a sugar.
68. The transdermal delivery composition of Item 67, wherein said sugar comprises between 4 and 6 R groups, wherein said R groups are -OH.
69. The transdermal delivery composition of Item 49, wherein the delivered agent is a hormone.
70. The transdermal delivery composition of Item 69, wherein said hormone is a polypeptide.
71. The transdermal delivery composition of Item 69, wherein the polypeptide is selected from the group consisting of oxytocin, vasopressin, melanocyte-stimulating hormone, corticortropin, lipotropin, thyrotropin, growth hormone, prolactin, luteinizing hormone, human chorionic gonadotropin, follicle stimulating hormone, corticotropin-releasing factor, gonadotropin-releasing factor, prolactin-releasing factor, prolactin-inhibiting factor, growth-hormone releasing factor, somatostatin, thyrotropin-releasing factor, calcitonin, calcitonin gene-related peptide, parathyroid hormone, glucagon-like peptide 1, glucose-dependent insulinotropic polypeptide, gastrin, secretin, cholecystokinin, motilin, vasoactive intestinal peptide, substance P, pancreatic polypeptide, peptide tyrosine tyrosine, neuropeptide tyrosine, amphiregulin, insulin, glucagon, placental lactogen, relaxin, , angiotensin II, atrial natriuretic peptide, and melatonin.
72. The transdermal delivery composition of Item 69, wherein the hormone is a non-peptide hormone.
73. The transdermal delivery composition of Item 72, wherein the hormone is selected from the group consisting of thyroxine, triiodothyronine, estradiol, estrone, progesterone, testosterone, cortisol, corticosterone, aldosterone, epinephrine, norepinepherine, and calctriol.
74. The transdermal delivery composition of Item 49, wherein said delivered agent is collagen.
75. The transdermal delivery composition of Item 49, wherein said delivered agent is an anesthetic.
76. The transdermal delivery composition of Item 75, wherein said anesthetic is selected from the group consisting of articaine, procaine, tetracaine, chloroprocaine and benzocaine, novocain, mepivicaine, bupivicaine, benzocaine, and lidocaine.
77. The transdermal delivery composition of Item 49, wherein said delivered agent is an analgesic.
78. The transdermal delivery composition of Item 77, wherein said delivered agent is selected from the group consisting of tramadol hydrochloride, fentanyl, metamizole, morphine sulphate, ketorolac tromethamine, hydrocodone, oxycodone, morprhine, loxoprofen, Capsaicin, and Boswellin.
79. The transdermal delivery composition of Item 49, wherein said delivered agent is an NSAID.
80. The transdermal delivery composition of Item 79, wherein said non-steroidal anti inflammatory drug (NSAID) is selected from the group consisting of ibuprofen (2-(isobutylphenyl)-propionic acid); methotrexate (N-[4-(2, 4 diamino 6 - pteridinyl - methyl] methylamino] benzoyl)-L-glutamic acid); aspirin (acetylsalicylic acid); salicylic acid; diphenhydramine (2-(diphenylmethoxy)-NN-dimethylethylamine hydrochloride); naproxen (2-naphthaleneacetic acid, 6-methoxy-9-methyl-, sodium salt, (-)); phenylbutazone (4-butyl-1,2-diphenyl-3,5-pyrazolidinedione); sulindac-(2)-5-fuoro-2-methyl-1-[[p-(methylsulfinyl)phenyl]methylene-]-1H-indene-3-acetic acid; diflunisal (2',4', -difluoro-4-hydroxy-3-biphenylcarboxylic acid; piroxicam (4-hydroxy-2-methyl-N-2-pyridinyl-2H-1, 2-benzothiazine-2-carboxamide 1, 1-dioxide, an oxicam; indomethacin (1-(4-chlorobenzoyl)-5-methoxy-2-methyl-H-indole-3-acetic acid); meclofenamate sodium (N-(2, 6-dichloro-m-tolyl) anthranilic acid, sodium salt, monohydrate); ketoprofen (2- (3-benzoylphenyl)-propionic acid; tolmetin sodium (sodium 1-methyl-5-(4-methylbenzoyl-1H-pyrrole-2-acetate dihydrate); diclofenac sodium (2-[(2,6-dichlorophenyl)amino] benzeneatic acid, monosodium salt); hydroxychloroquine sulphate (2-{[4-[(7-chloro-4-quinolyl) amino] pentyl] ethylamino}ethanol sulfate (1:1); penicillamine (3-mercapto-D-valine); flurbiprofen ([1,1-biphenyl]-4-acetic acid, 2-fluoro-alphamethyl-, (+-.)); cetodolac (1-8- diethyl-13,4,9, tetra hydropyrano-[3-4-13] indole-1-acetic acid; mefenamic acid (N-(2,3-xylyl)anthranilic acid; and diphenhydramine hydrochloride (2-diphenyl methoxy-N, N-di-methylethamine hydrochloride).
81. The transdermal delivery composition of Item 49, wherein the delivered agent is an anti-inflammatory compound selected from the group consisting of hydrocortisone, prednisolone, triamcinolone, cortisone and piroxicam.
82. The transdermal delivery composition of Item 49, wherein the delivered agent is an anti-infective compound selected from the group consisting of amoxicillin, clavulanate potassium, itraconazole, acyclovir, fluconazole, terbinafine hydrochloride, erythromycin ethylsuccinate, acetyl sulfisoxazole, penicillin V, cephalexin, erythromycin, azithromycin, tetracycline, ciproflaxin, gentamycin, sulfathiazole, nitrofurantoin, norfloxacin, flumequine, and ibafloxacin, metronidazole, nystatin, lamivudine, indinavir sulfate, and stavudine.
83. The transdermal delivery composition of Item 49, wherein the delivered agent is metformin, metformin hydrochloride or acarbose.
84. The transdermal delivery composition of Item 49, wherein the delivered agent is an immunogen.
85. The transdermal delivery composition of Item 49, wherein the immunogen comprises a polynucleotide.
86. The transdermal delivery composition of Item 49 wherein the delivered agent is an immune response modifier.
87. The transdermal delivery composition of Item 86, wherein the immune response modifier is selected from the group consisting of purine derivatives, adenine derivatives, cyclosporine, and CpGs.
88. The transdermal delivery composition of Item 49, wherein the delivered agent is selected from the group consisting of zileuton, captopril, and lisinopril.
89. The transdermal delivery composition of Item 49, wherein the delivered agent is selected from the group consisting of ergotamine, melatonin, sumatriptan, zolmitriptan, and rizatriptan.
90. The transdermal delivery composition of Item 49, wherein the delivered agent is selected from the group consisting of zolpidem, zolpidem tartrate, triazolam, and hycosine butylbromide.
91. The transdermal delivery composition of Item 49, wherein the delivered agent is selected from the group consisting of iohexol, technetium, TC99M, sestamibi, iomeprol, gadodiamide, ioversol, and iopromide.
92. The transdermal delivery composition of Item 49, wherein the delivered agent is selected from the group consisting of alsactide, americium, betazole, histamine, mannitol, metyrapone, petagastrin, phentolamine, radioactive B₁₂, gadodiamide, gadopentetic acid, gadoteridol, perflubron, cyclosporine, sildenafil citrate, paclitaxel, ritonavir, and saquinavir.
93. A transdermal delivery device comprising the transdermal delivery composition of Item 1 or 49, wherein said device comprises:
   a removable cartridge, wherein said removable cartridge is configured to contain said transdermal delivery composition, and wherein said removable cartridge comprises a movable wall and a one-way valve;
   a body portion, said body portion being adaptable to receive the removable cartridge, wherein the body portion comprises an internal wall having an aperture configured to communicate with the one-way valve;
   a movable member, wherein said movable member partially defines a dosing chamber along with the internal wall, and wherein movement of said movable member alters the volume of said dosing chamber;
   a plunger, said plunger being actuatable between a first position wherein said plunger interacts with the movable wall of said removable cartridge, and a second position, wherein said plunger is biased to be in said second position;
   a non-invasive applicator; and
   a slidable member attached to said plunger, said slidable member being actuatable between a first position and a second position corresponding to the first and second positions of said plunger, wherein when said slidable member is in said first position, fluid communication between the one-way valve and the dosing chamber is permitted, and wherein when said slidable member is in said second position, fluid communication between said dosing chamber and the non-invasive applicator is permitted.
94. The transdermal delivery device of item 93, further comprising a rotatable shaft, wherein said rotatable shaft has a threaded portion extending through a threaded aperture in the moveable member, whereby rotation of the rotatable shaft results translation of the movable member in the direction of the axis of the shaft.
95. The transdermal delivery device of item 94, wherein the dosing chamber has a non-circular cross-section, and wherein the movable member comprises a layer of deformable material along the edges in contact with the walls of the dosing chamber.
96. The transdermal delivery device of item 94, wherein the rotatable shaft extends through a portion of the moveable member away from the center of the upper surface of the movable member.
97. The transdermal delivery device of item 93, wherein the non-invasive applicator comprises a roll-on applicator.
98. The transdermal delivery device of item 93, wherein the slidable member is operably connected to the plunger via a spring.
99. The transdermal delivery device of item 93, wherein the plunger is biased to be in said second position via a spring connected to said body portion.
100. The transdermal delivery device of item 93, wherein the plunger comprises a toothed surface, said toothed surface configured to interact with a locking member to prevent return of said plunger to the second position.
101. The transdermal delivery device of item 100, wherein the locking member is operably connected to a release button, whereby pressing the release button moves the locking member so as to permit return of the plunger to the second position.
102. A transdermal delivery device for dispensing a measured quantity of the transdermal delivery composition of Item 1 or 49, the device comprising:
   a reservoir, said reservoir comprising a movable wall and a one-way valve, wherein said movable wall can be actuated to result in fluid flow through the one-way valve;
   a plunger, said plunger being actuatable between a first position, wherein said plunger is not in contact with the movable wall of said reservoir, and a second position, wherein said plunger interacts with the movable wall of the reservoir and further translation of the plunger in the direction of the movable wall results in fluid flow through the one-way valve of the reservoir; and
   a dosing member, said dosing member being movable between a plurality of positions, wherein each of said plurality of positions corresponds to a given quantity of transdermal delivery composition to be dispensed.
103. A method of making a liposphere for transdermal delivery of a delivered agent comprising:
   identifying a delivered agent for incorporation into a liposphere; and
   mixing said delivered agent with an ethoxylated fatty moiety, wherein the amount of ethoxylation of said fatty moiety or lipid moiety is 10, 11, 12, 13, 14, 15, 16, 17, 18, or 19 ethoxylations per molecule.
104. The method of Item 103, further comprising mixing said delivered agent and said ethoxylated fatty moiety with an aqueous adjuvant.
105. A method of making a liposphere for transdermal delivery of a delivered agent comprising:
   identifying a delivered agent for incorporation into a liposphere, and
   mixing said delivered agent with an ethoxylated multifunctional backbone, wherein said ethoxylated multifunctional backbone wherein said multifunctional backbone comprises at least two reactive groups (R), wherein at least one reactive group is substituted with a fatty moiety, wherein at least one reactive group is substituted with a polyethoxy moiety, wherein R is selected from the group consisting of -OH, COOH, SH, and NH₂, wherein said fatty moiety is selected from the group consisting of a fatty acid, a fatty alcohol, a fatty amine, and a modified fatty acid; and
   wherein said ethoxylated multifunctional backbone comprises 10, 11, 12, 13, 14, 15, 16, 17, 18, or 19 ethoxylations per molecule.
106. The method of Item 105, further comprising mixing said delivered agent and said ethoxylated fatty moiety with an aqueous adjuvant.
107. A method of making the transdermal delivery composition of Item 1 comprising:
   identifying a fatty moiety for exthoxylation;
   ethoxylating said fatty moiety so as to yield 10, 11, 12, 13, 14, 15, 16, 17, 18, or 19 ethoxylations per molecule; and
   mixing said ethoxylated fatty moiety with a delivered agent.
108. The method of Item 107, further comprising mixing said delivered agent and said ethoxylated fatty moiety with an aqueous adjuvant.
109. A method of making the transdermal delivery composition of Item 49 comprising:
   identifying a multifunctional backbone for ethoxylation, wherein said multifunctional backbone comprises at least two reactive groups (R), wherein at least one reactive group is substituted with a fatty moiety, wherein at least one reactive group is substituted with a polyethoxy moiety, wherein R is selected from the group consisting of -OH, COOH, SH, and NH₂, wherein said fatty moiety is selected from the group consisting of a fatty acid, a fatty alcohol, a fatty amine, and a modified fatty acid;
   ethoxylating said multifunctional backbone so as to yield 10, 11, 12, 13, 14, 15, 16, 17, 18, or 19 ethoxylations per molecule; and
   mixing said ethoxylated multifunctional backbone with a delivered agent.
110. The method of Item 109, further comprising mixing said delivered agent said ethoxylated multifunctional backbone with an aqueous adjuvant.
111. A method of providing a delivered agent to a mammal in need thereof, comprising:
   providing the transdermal delivery device of Item 93;
   obtaining an amount of transdermal delivery system comprising said delivered agent from said transdermal delivery device; and
   contacting said mammal with said transdermal delivery system.

## Claims

1. A transdermal delivery composition comprising a liposphere, wherein said liposphere comprises,
- an ethoxylated fatty moiety or lipid moiety and a delivered agent, wherein the amount of ethoxylation of said fatty moiety or lipid moiety is 10, 11, 12, 13, 14, 15, 16, 17, 18, or 19 ethoxylations per molecule,
- wherein said fatty moiety or lipid moiety comprises 10 carbon residues, and
- wherein said delivered agent is an anti-infective compound.

2. The transdermal delivery composition of claim 1, wherein the delivered agent is an anti-infective selected from the group consisting of an antibiotic and an antiviral compound.

3. The transdermal delivery composition of claims 1-2, wherein said delivered agent has antiviral activity.

4. The transdermal delivery composition of claim 1, further comprising water or alcohol.

5. The transdermal delivery composition of claim 1, wherein said fatty moiety or lipid moiety comprises at least 10 carbon residues but less than or equal to 15 carbon residues.

6. The transdermal delivery composition of claim 1, wherein the amount of ethoxylation is the same as the number of carbons residues in the fatty moiety or lipid moiety.

7. The transdermal delivery composition of claim 1, wherein the fatty moiety or lipid moiety chain length is 10, 12, 14, 16, 18, 20, 22 or 24 carbon residues in length.

8. The transdermal delivery composition of claim 1, wherein the fatty moiety is a fatty acid.

9. The transdermal delivery composition of claim 7, wherein the fatty acid is selected from the group consisting of C16:1, delta 1; C16:1, delta 2; C16:1, delta 3; C16:1, delta 4; C16:1, delta 5; C16:1, delta 6; C16:1, delta 7; C16:1, delta 8; C16:1, delta 9 (palmitoleic acid); C16:1, delta 10; C16:1, delta 11; C16:1, delta 12; C16:1, delta 13; and C16:1, delta 14.

10. The transdermal delivery composition of claim 1, wherein said fatty moiety is a fatty amine.

11. The transdermal delivery composition of claim 1, wherein said lipid moiety is a sphingolipid, a glycolipid or a glycosphingolipid.

12. The transdermal delivery composition of claim 1, wherein said liposphere comprises a homogeneous mixture of an ethoxylated fatty moiety or a heterogeneous mixture of ethoxylated fatty acids.

13. The transdermal delivery composition of claim 1, wherein the fatty moiety is unsaturated or polyunsaturated.

14. The transdermal delivery composition of any of claims 1-13 for use as a medicament.

15. The transdermal delivery composition of any of claims 1-13 for use for the delivery of a delivered agent.
